# EUROPÄISCHE PATENTSCHRIFT

(11) **EP 0 678 503 B1**
(45) Veröffentlichungstag und Bekanntmachung des Hinweises auf die Patenterteilung: **01.09.1999**
(21) Anmeldenummer: 95810236.0
(22) Anmeldetag: 07.04.1995
(51) Int. Cl.: C07C 237/20, A61K 31/16, C07D 213/30, C07D 213/89, C07C 323/12, C07C 317/18, C07D 295/13, C07C 255/13, C07C 271/22, C07D 307/32

(54) **Delta-Amino-gamma-hydroxy-omega-aryl-alkansäureamide mit Enzym-, insbesondere Renin-hemmenden Eigenschaften**
Delta-amino-gamma-hydroxy-omega-aryl alkanoic acid amides with enzyme especially renin inhibiting activities
Amides des acides delta-amino-gamma-hydroxy-oméga-aryl alkanoiques possédant une activité d'inhibiteurs d'enzyme notamment une activité d'inhibiteurs de rénin

(30) Priorität: 18.04.1994 CH 116994
(43) Veröffentlichungstag der Anmeldung: 25.10.1995
(73) Patentinhaber: Novartis AG, 4058 Basel (CH); Novartis-Erfindungen Verwaltungsgesellschaft m.b.H., 1235 Wien (AT)
(72) Erfinder: Göschke, Richard, Dr., CH-4103 Bottmingen (CH); Maibaum, Jürgen Klaus, Dr., D-79576 Weil-Haltingen (DE); Schilling, Walter, Dr., CH-4204 Himmelried (CH); Stutz, Stefan, CH-4053 Basel (CH); Rigollier, Pascal. Dr., F-68510 Sierentz (FR); Yamaguchi, Yasuchika, Dr., CH-4053 Basel (CH); Cohen, Nissim Claude, Dr., F-68300 Village-Neuf (FR); Herold, Peter, Dr., CH-4144 Arlesheim (CH)

(56) Entgegenhaltungen:
- BIOORGANIC & MEDICINAL CHEMISTRY LETTERS, Bd.3, Nr.10, 1993 Seiten 2119 - 2124 M. PLUMMER ET AL. 'Peptidomimetic inhibitors of renin incorporating topographically modified isosteres spanning the P1(-P3)-P1' sites'
- BIOORGANIC & MEDICINAL CHEMISTRY LETTERS, Bd.4, Nr.14, 1994 Seiten 1697 - 1702 S. HANESSIAN, S. RAGHAVAN 'Design and synthesis of a prototypical non-peptidic inhibitor model for the enzyme renin'

## Beschreibung

Die Erfindung betrifft δ-Amino-γ-hydroxy-ω-aryl-alkansäureamide der Formel I 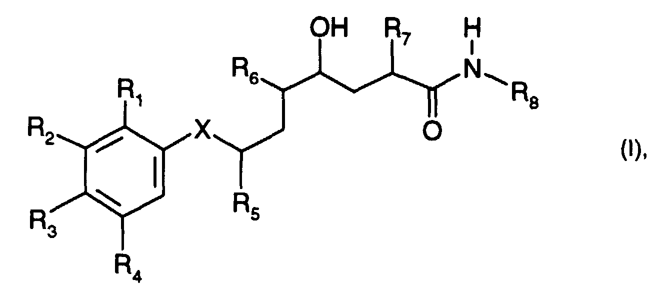 worin
R₁ Wasserstoff, Hydroxy, C₁-C₇-Alkoxy, 3- bis 8-gliedriges Cycloalkoxy, C₁-C₄-Alkoxy-C₂-C₄-alkoxy oder Carboxy-C₁-C₄-alkoxy, C₁-C₄-Alkoxycarbonyl-C₁-C₄-alkoxy, Carbamoyl-C₁-C₄-alkoxy, N-C₁-C₇-Alkylcarbamoyl-C₁-C₄-alkoxy oder N,N-Di-C₁-C₄-alkylcarbamoyl-C₁-C₄-alkoxy bedeutet,
R₂ Wasserstoff, C₁-C₇-Alkyl, 3- bis 8-gliedriges Cycloalkyl, C₁-C₄-Alkoxy-C₁-C₄-alkyl, C₁-C₄-Alkoxy-C₁-C₄-alkoxy-C₁-C₄-alkyl, 3- bis 8-gliedriges Cycloalkoxy-C₁-C₄-alkyl, Hydroxy, C₁-C₇-Alkanoyloxy-C₁-C₄-alkyl, Hydroxy-C₂-C₇-alkoxy, Halogen-C₂-C₇-(hydroxy)alkoxy, C₁-C₇-Alkansulfonyl-C₁-C₄-(hydroxy)alkoxy, Amino-C₁-C₄-alkyl, C₁-C₄-Alkylamino-C₁-C₄-alkyl, N,N-Di-C₁-C₄-alkylamino-C₁-C₄-alkyl, N-C₁-C₄-Alkanoylamino-C₁-C₄-alkyl, C₁-C₇-Alkoxycarbonylamino-C₂-C₇-alkyl, gegebenenfalls partiell hydriertes Pyridyl- oder N-Oxidopyridyl-C₁-C₄-alkyl, Amino-C₁-C₄-alkoxy, C₁-C₄-Alkylamino-C₁-C₄-alkoxy, N,N-Di-C₁-C₄-alkylamino-C₁-C₄-alkoxy, C₁-C₄-Alkanoylamino-C₁-C₄-alkoxy, C₁-C₇-Alkoxycarbonylamino-C₂-C₇-alkoxy, die Alkanoylgruppe in höherer als der α-Stellung tragendes C₁-C₇-Alkanoyl-C₂-C₄-alkoxy, C₁-C₇-Alkoxy, 3- bis 8-gliedriges Cycloalkoxy, C₂-C₇-Alkenyloxy, 3- bis 8-gliedriges Cycloalkoxy-C₁-C₄-alkoxy, C₁-C₄-Alkoxy-C₂-C₄-alkoxy, C₁-C₄-Alkoxy-C₂-C₄-alkenyl, C₂-C₇-Alkenyloxy-C₁-C₄-alkoxy, C₁-C₄-Alkoxy-C₂-C₄-alkenyloxy, C₂-C₇-Alkenyloxy-C₁-C₄-alkyl, C₁-C₄-Alkylthio-C₁-C₄-alkoxy, C₁-C₇-Alkansulfonyl-C₁-C₄-alkoxy, C₁-C₄-Alkylthio-C₁-C₄-(hydroxy)alkoxy, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl- oder Naphthyl-C₁-C₄-alkoxy, gegebenenfalls partiell hydriertes Pyridyl- oder N-Oxidopyridyl-C₁-C₄-alkoxy, Thiazolyl-C₁-C₄-alkoxy, gegebenenfalls N-oxidiertes Morpholino-C₁-C₄-alkoxy, Cyano-C₁-C₄-alkoxy, Carboxy-C₁-C₄-alkoxy, C₁-C₄-Alkoxycarbonyl-C₁-C₄-alkoxy, Carbamoyl-C₁-C₄-alkoxy, N-C₁-C₇-Alkylcarbamoyl-C₁-C₄-alkoxy oder N-Mono- oder N,N-Di-C₁-C₄-alkylcarbamoyl-C₁-C₄-alkoxy, Carboxy-C₁-C₄-alkyl, C₁-C₄-Alkoxycarbonyl-C₁-C₄-alkyl, Carbamoyl-C₁-C₇-alkyl oder N-Mono- oder N,N-Di-C₁-C₄-alkylcarbamoyl-C₁-C₄-alkyl bedeutet,
R₃ Wasserstoff, C₁-C₇-Alkyl, Polyhalogen-C₁-C₄-alkyl, C₁-C₄-Alkoxy-C₁-C₄-alkyl, 3- bis 8-gliedriges Cycloalkoxy-C₁-C₄-alkyl, Hydroxy-C₂-C₇-alkyl, C₁-C₄-Alkylthio-C₁-C₄-alkyl, C₁-C₇-Alkansulfonyl-C₁-C₄-alkyl, Thiazolylthio-C₁-C₄-alkyl, Thiazolinylthio-C₁-C₄-alkyl, Imidazolylthio-C₁-C₄-alkyl, gegebenenfalls N-oxidiertes Pyridylthio-C₁-C₄-alkyl, Pyrimidinylthio-C₁-C₄-alkyl, gegebenenfalls partiell hydriertes Pyridyl- oder N-Oxidopyridyl-C₁-C₄-alkyl, Amino-C₁-C₄-alkyl, C₁-C₄-Alkylamino-C₁-C₄-alkyl, N,N-Di-C₁-C₄-alkylamino-C₁-C₄-alkyl, N-C₁-C₄-Alkanoylamino-C₁-C₄-alkyl, C₁-C₄-Alkansulfonylamino-C₁-C₄-alkyl, Trifluor-C₁-C₇-alkansulfonylamino-C₁-C₄-alkyl, Pyrrolidino-C₁-C₄-alkyl, Piperidino-C₁-C₄-alkyl, Piperazino-C₁-C₄-alkyl, N'-C₁-C₄-Alkylpiperazino-C₁-C₄-alkyl, N'-C₂-C₇-Alkanoylpiperazino-C₁-C₄-alkyl, Morpholino-C₁-C₄-alkyl, Thiomorpholino-C₁-C₄-alkyl, S-Oxothiomorpholino-C₁-C₄-alkyl, S,S-Dioxothiomorpholino-C₁-C₄-alkyl, Cyano-C₁-C₄-alkyl, Carboxy-C₁-C₄-alkyl, C₁-C₄-Alkoxycarbonyl-C₁-C₄-alkyl, Carbamoyl-C₁-C₇-alkyl, N-Mono- oder N,N-Di-C₁-C₄-alkylcarbamoyl-C₁-C₄-alkyl, 3- bis 8-gliedriges Cycloalkyl, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl oder Naphthyl, Hydroxy, C₁-C₄-Alkoxy, 3- bis 8-gliedriges Cycloalkoxy, C₁-C₄-Alkoxy-C₂-C₄-alkoxy, 3- bis 8-gliedriges Cycloalkoxy-C₁-C₄-alkoxy, Hydroxy-C₂-C₇-alkoxy, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl-C₁-C₄-alkoxy oder Naphthyl-C₁-C₄-alkoxy, Polyhalogen-C₁-C₄-alkoxy, C₁-C₄-Alkylthio-C₁-C₄-alkoxy, C₁-C₇-Alkansulfonyl-C₁-C₄-alkoxy, gegebenenfalls partiell hydriertes Pyridyl- oder N-Oxidopyridyl-C₁-C₄-alkoxy, Thiazolyl-C₁-C₄-alkoxy, Morpholino-C₁-C₄-alkoxy, Thiazolylthio-C₁-C₄-alkoxy, Thiazolinylthio-C₁-C₄-alkoxy, Imidazolylthio-C₁-C₄-alkoxy, gegebenenfalls N-oxidiertes Pyridylthio-C₁-C₄-alkoxy, Pyrimidinylthio-C₁-C₄-alkoxy, Amino-C₁-C₄-alkoxy, C₁-C₄-Alkylamino-C₁-C₄-alkoxy, N,N-Di-C₁-C₄-alkylamino-C₁-C₄-alkoxy, C₁-C₇-Alkanoylamino-C₁-C₄-alkoxy, C₁-C₇-Alkansulfonylamino-C₁-C₄-alkoxy, Trifluor-C₁-C₇-alkansulfonyl-C₁-C₄-alkoxy, Pyrrolidino-C₂-C₄-alkoxy, Piperidino-C₁-C₄-alkoxy, Piperazino-C₁-C₄-alkyl, N'-C₁-C₄-Alkylpiperazino-C₁-C₄-alkyl, N'-C₂-C₇-Alkanoylpiperazino-C₁-C₄-alkyl, Morpholino-C₁-C₄-alkyl, Thiomorpholino-C₁-C₄-alkyl, S-Oxothiomorpholino-C₁-C₄-alkyl, S,S-Dioxothiomorpholino-C₁-C₄-alkyl, Cyano-C₁-C₄-alkoxy, Carboxy-C₁-C₄-alkoxy, C₁-C₄-Alkoxycarbonyl-C₁-C₄-alkoxy, Carbamoyl-C₁-C₇-alkoxy oder N-Mono- oder N,N-Di-C₁-C₄-alkylcarbamoyl-C₁-C₄-alkoxy bedeutet oder gemeinsam mit R₄ C₁-C₄-Alkylendioxy oder einen ankondensierten Benzo- oder Cyclohexenoring darstellt,
R₄ gemeinsam mit R₃ C₁-C₄-Alkylendioxy oder einen ankondensierten Benzo- oder Cyclohexenoring darstellt oder für Wasserstoff, C₁-C₇-Alkyl, Hydroxy, C₁-C₄-Alkoxy oder 3- bis 8-gliedriges Cycloalkoxy steht,
X Methylen oder Hydroxymethylen bedeutet,
R₅ für C₁-C₇-Alkyl oder 3- bis 8-gliedriges Cycloalkyl steht,
R₆ Amino, C₁-C₄-Alkylamino, Di-C₁-C₄-alkylamino oder N-C₁-C₇-Alkanoylamino bedeutet,
R₇ C₁-C₇-Alkyl, C₂-C₇-Alkenyl, 3- bis 8-gliedriges Cycloalkyl oder unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl- oder Naphthyl-C₁-C₄-alkyl bedeutet und
R₈ C₁-C₇-Alkyl, Cycloalkyl, Hydroxy-C₂-C₇-alkyl, C₁-C₇-Alkanoyloxy-C₁-C₄-alkyl, C₁-C₄-Alkoxy-C₁-C₄-alkyl, C₂-C₇-Alkenyloxy-C₁-C₄-alkyl, Amino-C₁-C₄-alkyl, N-C₁-C₄-alkylamino-C₁-C₄-alkyl, N,N-Di-C₁-C₄-alkylamino-C₁-C₄-alkyl, N-C₁-C₄-Alkanoylamino-C₁-C₄-alkyl, C₁-C₇-Alkansulfonylamino-C₁-C₄-alkyl, Trifluor-C₁-C₇-alkansulfonylamino-C₁-C₄-alkyl, Pyrrolidino-C₁-C₄-alkyl, Piperidino-C₁-C₄-alkyl, Hydroxypiperidino-C₁-C₄-alkyl, C₁-C₄-Alkoxypiperidino-C₁-C₄-alkyl, Piperazino-C₁-C₄-alkyl, N'-C₁-C₄-Alkylpiperazino-C₁-C₄-alkyl, N'-C₂-C₇-Alkanoylpiperazino-C₁-C₄-alkyl, Morpholino-C₁-C₄-alkyl, Dimethylmorpholino-C₁-C₄-alkyl, Morpholinocarbonyl-C₁-C₄-alkyl, Thiomorpholino-C₁-C₄-alkyl, S,S-Dioxothiomorpholino-C₁-C₄-alkyl, Carboxy-C₁-C₄-alkyl, C₁-C₄-Alkoxycarbonyl-C₁-C₄-alkyl, Carbamoyl-C₁-C₇-alkyl, N-Mono- oder N,N-Di-C₁-C₄-alkylcarbamoyl-C₁-C₄-alkyl, Dicarboxy-C₁-C₄-alkyl, Di-C₁-C₄-alkoxycarbonyl-C₁-C₄-alkyl, Dicarbamoyl-C₁-C₄-alkyl, Di-(N-Mono- oder N,N-Di-C₁-C₄-alkylcarbamoyl-C₁-C₄-alkyl, Carboxy-C₁-C₇-(hydroxy)alkyl, C₁-C₄-Alkoxycarbonyl-C₁-C₇-(hydroxy)alkyl oder Carbamoyl-C₁-C₇-(hydroxy)alkyl, jeweils 5- oder 6-gliedriges Carboxycycloalkyl-C₁-C₄-alkyl, C₁-C₄-Alkoxycarbonylcycloalkyl-C₁-C₄-alkyl, Carbamoylcycloalkyl-C₁-C₄-alkyl oder N-Mono- oder N,N-Di-C₁-C₄-alkylcarbamoylcycloalkyl-C₁-C₄-alkyl, Cyano-C₁-C₄-alkyl, C₁-C₇-Alkansulfonyl-C₁-C₄-alkyl, Thiocarbamoyl-C₁-C₄-alkyl, C₁-C₄-Alkylthiocarbamoyl-C₁-C₄-alkyl, Di-C₁-C₄-alkylthiocarbamamoyl-C₁-C₄-alkyl, Sulfamoyl-C₁-C₄-alkyl, N-C₁-C₇-Alkylsulfamoyl-C₁-C₄-alkyl, N,N-Di-C₁-C₄-alkylsulfamoyl-C₁-C₄-alkyl, einen über ein C-Atom gebundenen, gegebenenfalls partiell hydrierten, oxosubstituierten und/oder benzokondensierten 5-gliedrigen Aza-, Diaza-, Triaza-, Oxadiaza- oder Tetra-aza-arylrest oder 6-gliedrigen Aza- oder Diaza-arylrest oder einen über ein C-Atom gebundenen, gegebenenfalls partiell hydrierten, oxosubstituierten und/oder benzokondensierten 5-gliedrigen Aza-, Diaza-, Triaza-Oxadiaza- oder Tetra-aza-aryl-C₁-C₄-alkylrest oder 6-gliedrigen Aza- oder Diaza-aryl-C₁-C₄-alkylrest darstellt,
und ihre Salze, Verfahren zur Herstellung der erfindungsgemässen Verbindungen, diese enthaltende pharmazeutische Präparate und ihre Verwendung als Arzneimittelwirkstoffe.

3- bis 8-gliedriges Cycloalkoxy sowie Cycloalkoxy in Cycloalkoxy-C₁-C₄-alkoxy ist vorzugsweise 3-, 5- oder 6-gliedriges Cycloalkoxy, wie Cyclopropyloxy, Cyclopentyloxy, Cyclohexyloxy.

3- bis 8-gliedriges Cycloalkyl ist vorzugsweise 3-, 5- oder 6-gliedriges Cycloalkyl, wie Cyclopropyl, Cyclopentyl, Cyclohexyl.

5- oder 6-gliedriges Carboxycycloalkyl-C₁-C₄-alkyl, C₁-C₄-Alkoxycarbonylcycloalkyl-C₁-C₄-alkyl, Carbamoylcycloalkyl-C₁-C₄-alkyl, N-Mono- oder N,N-Di-C₁-C₄-alkylcarbamoylcycloalkyl-C₁-C₄-alkyl ist beispielsweise ω-(1-Carboxycycloalkyl)-C₁-C₄-alkyl, ω-(1-C₁-C₄-Alkoxycarbonylcycloalkyl)-C₁-C₄-alkyl, ω-(1-Carbamoylcycloalkyl)-C₁-C₄-alkyl, ω-(1-C₁-C₄-Alkylcarbamoylcycloalkyl)-C₁-C₄-alkyl oder ω-(1-Di-C₁-C₄-alkylcarbamoylcycloalkyl)-C₁-C₄-alkyl, worin Cycloalkyl beispielsweise Cyclopentyl oder Cyclohexyl, C₁-C₄-Alkoxycarbonyl beispielsweise C₁-C₄-Alkoxycarbonyl, wie Methoxy- oder Ethoxycarbonyl, C₁-C₄-Alkylcarbamoyl beispielsweise C₁-C₄-Alkylcarbamoyl, wie Methylcarbamoyl, Di-C₁-C₄-alkylcarbamoyl beispielsweise Di-C₁-C₄-alkylcarbamoyl, wie Dimethylcarbamoyl, und C₁-C₄-Alkyl beispielsweise C₁-C₄-Alkyl, wie Methyl, Ethyl, Propyl oder Butyl, bedeutet, insbesondere (1-Carboxycyclopentyl)methyl.

5- oder 6-gliedriges Cycloalkoxy-C₁-C₄-alkoxy ist beispielsweise Cyclopentyloxy- oder Cyclohexyloxy-C₁-C₄-alkoxy, wie Cyclopentyloxy- oder Cyclohexyloxymethoxy, 2-Cyclopentyloxy- oder 2-Cyclohexyloxyethoxy, 2- oder 3-Cyclopentyloxy- oder 2- oder 3-Cyclohexyloxypropyloxy oder 4-Cyclopentyloxy- oder 4-Cyclohexyloxybutyloxy, insbesondere Cyclopentyloxy- oder Cyclohexyloxymethoxy.

5- oder 6-gliedriges Cycloalkoxy-C₁-C₄-alkyl ist beispielsweise Cyclopentyloxy- oder Cyclohexyloxy-C₁-C₄-alkyl, wie Cyclopentyloxy- oder Cyclohexyloxymethyl, 2-Cyclopentyloxy- oder 2-Cyclohexyloxyethyl, 2- oder 3-Cyclopentyloxy- oder 2- oder 3-Cyclohexyloxypropyl, 2-Cyclopentyloxy- oder 2-Cyclohexyloxy-2-methyl-propyl, 2-Cyclopentyloxy- oder 2-Cyclohexyloxy-2-ethyl-butyl oder 4-Cyclopentyloxy- oder Cyclohexyloxybutyl, insbesondere Cyclopentyloxy- oder Cyclohexyloxymethyl.

Amino-C₁-C₄-alkoxy ist beispielsweise 2-Aminoethoxy oder 5-Aminopentyloxy, ferner 3-Aminopropyloxy oder 4-Aminobutyloxy.

Amino-C₁-C₄-alkyl ist beispielsweise 2-Aminoethyl, 3-Aminopropyl oder 4-Aminobutyl.

Carbamoyl-C₁-C₇-(hydroxy)alkyl ist beispielsweise wie 1-Carbamoyl-2-hydroxyethyl.

Carbamoyl-C₁-C₄-alkoxy ist beispielsweise Carbamoyl-C₁-C₄-alkoxy, wie Carbamoylmethoxy, 2-Carbamoylethoxy, 3-Carbamoylpropyloxy oder 4-Carbamoylbutyloxy, insbesondere Carbamoylmethoxy.

Carbamoyl-C₁-C₇-alkyl ist beispielsweise Carbamoylmethyl, 2-Carbamoylethyl, 3-Carbamoylpropyl, 2-(3-Carbamoyl)propyl, 2-Carbamoylpropyl 3-(1-Carbamoyl)propyl, 2-(2-Carbamoyl)propyl, 2-Carbamoyl-2-methyl)propyl, 4-Carbamoylbutyl, 1-Carbamoylbutyl, 1-(1-Carbamoyl-2-methyl)butyl, 3-(4-Carbamoyl-2-methyl)butyl.

Carboxy-C₁-C₇-(hydroxy)alkyl ist beispielsweise 1-Carboxy-2-hydroxy-ethyl.

Carboxy-C₁-C₄-alkoxy ist beispielsweise Carboxymethoxy, 2-Carboxyethoxy, 2- oder 3-Carboxypropyloxy oder 4-Carboxybutyloxy, insbesondere Carboxymethoxy.

Carboxy-C₁-C₄-alkyl ist beispielsweise Carboxymethyl, 2-Carboxyethyl, 2- oder 3-Carboxypropyl, 2-Carboxy-2-methyl-propyl, 2-Carboxy-2-ethyl-butyl oder 4-Carboxybutyl, insbesondere Carboxymethyl.

Cyano-C₁-C₄-alkoxy ist beispielsweise Cyanomethoxy, 2-Cyanoethoxy, 2- oder 3-Cyanopropyloxy oder 4-Cyanobutyloxy, insbesondere Cyanomethoxy.

Cyano-C₁-C₄-alkyl ist beispielsweise Cyanomethyl, 2-Cyanoethyl, 2- oder 3-Cyanopropyl, 2-Cyano-2-methyl-propyl, 2-Cyano-2-ethyl-butyl oder 4-Cyanobutyl, insbesondere Cyanomethyl.

Di-(N-Mono- oder N,N-(Di-C₁-C₄-alkylcarbamoyl)-C₁-C₄-alkyl ist beispielsweise 1,2-Di-(N-Mono- oder N,N-Di-C₁-C₄-alkylcarbamoylethyl oder 1,3-Di-(N-Mono- oder N,N-Di-C₁-C₄-alkylcarbamoylpropyl.

Dicarbamoyl-C₁-C₄-alkyl ist beispielsweise 1,2-Dicarbamoylethyl oder 1,3-Dicarbamoylpropyl.

Dicarboxy-C₁-C₄-alkyl ist beispielsweise 1,2-Dicarboxyethyl oder 1,3-Dicarboxypropyl.

Dimethylmorpholino-C₁-C₄-alkoxy kann N-oxidiert sein und ist beispielsweise 2,6-Dimethylmorpholino- oder 3,5-Dimethylmorpholino-C₁-C₄-alkoxy, wie 2,6-Dimethylmorpholino- oder 3,5-Dimethylmorpholinomethoxy, 2-(2,6-Dimethylmorpholino- oder 3,5-Dimethylmorpholino)ethoxy, 3-(2,6-Dimethylmorpholino- oder 3,5-Dimethylmorpholino)propyloxy, 2-(2,6-Dimethylmorpholino- oder 3,5-Dimethylmorpholino-3-methyl)propyloxy, oder 1- oder 2-[4-(2,6-Dimethylmorpholino- oder 3,5-Dimethylmorpholino)]butyloxy.

Dimethylmorpholino-C₁-C₄-alkyl kann N-oxidiert sein und ist beispielsweise 2,6-Dimethylmorpholino- oder 3,5-Dimethylmorpholino-C₁-C₄-alkyl, wie 2,6-Dimethylmorpholino- oder 3,5-Dimethylmorpholinomethyl, 2-(2,6-Dimethylmorpholino- oder 3,5-Dimethylmorpholino)ethyl, 3-(2,6-Dimethylmorpholino- oder 3,5-Dimethylmorpholino)propyl, 2-(2,6-Dimethylmorpholino- oder 3,5-Dimethylmorpholino-3-methyl)propyl, oder 1- oder 2-[4-(2,6-Dimethylmorpholino- oder 3,5-Dimethylmorpholino)]butyl.

Di-C₁-C₄-alkoxycarbonyl-C₁-C₄-alkyl ist beispielsweise 1,2-Dimethoxycarbonylethyl, 1,3-Dimethoxycarbonylpropyl, 1,2-Dimethoxycarbonylethyl oder 1,3-Diethoxycarbonylpropyl.

Di-C₁-C₄-alkylamino ist beispielsweise Dimethylamino, N-Methyl-N-ethylamino, Diethylamino, N-Methyl-N-propylamino oder N-Butyl-N-methyl-amino.

Di-C₁-C₄-alkylamino-C₁-C₄-alkoxy ist 2-Dimethylaminoethoxy, 3-Dimethylaminopropyloxy, 4-Dimethylaminobutyloxy, 2-Diethylaminoethoxy, 2-(N-Methyl-N-ethyl-amino)ethoxy oder 2-(N-Butyl-N-methyl-amino)ethoxy.

Di-C₁-C₄-alkylamino-C₁-C₄-alkyl ist beispielsweise 2-Dimethylaminoethyl, 3-Dimethylaminopropyl, 4-Dimethylaminobutyl, 2-Diethylaminoethyl, 2-(N-Methyl-N-ethyl-amino)ethyl oder 2-(N-Butyl-N-methyl-amino)ethyl.

Di-C₁-C₄-alkylcarbamoyl-C₁-C₄-alkoxy ist beispielsweise Methyl- oder Dimethylcarbamoyl-C₁-C₄-alkoxy, wie N-Methyl-, N-Butyl- oder N,N-Dimethylcarbamoylmethoxy, 2-(N-Methylcarbamoyl)ethoxy, 2-(N-Butylcarbamoyl)ethoxy, 2-(N,N-Dimethylcarbamoyl)ethoxy, 3-(N-Methylcarbamoyl)propyloxy, 3-(N-Butylcarbamoyl)propyloxy, 3-(N,N-Dimethylcarbamoyl)propyloxy oder 4-(N-Methylcarbamoyl)butyloxy, 4-(N-Butylcarbamoyl)butyloxy oder 4-(N,N-Dimethylcarbamoyl)butyloxy, insbesondere N-Methyl-, N-Butyl oder N,N-Dimethylcarbamoylmethoxy.

Di-C₁-C₄-alkylcarbamoyl-C₁-C₄-alkyl ist beispielsweise 2-Dimethylcarbamoylethyl, 3-Dimethylcarbamoylpropyl, 2-Dimethylcarbamoylpropyl, 2-(Dimethylcarbamoyl)-2-methylpropyl oder 2-(1-Dimethylcarbamoyl)-3-methyl-butyl.

Di-C₁-C₄-alkylsulfamoyl-C₁-C₄-alkyl ist beispielsweise N,N-Dimethylsulfamoyl-C₁-C₄-alkyl, wie N,N-Dimethylsulfamoylmethyl, 2-(N,N-Dimethylsulfamoyl)ethyl 3-(N,N-Dimethylcarbamoyl)propyl oder 4-(N,N-Dimethylsulfamoyl)butyl, insbesondere N,N-Dimethylsulfamoylmethyl.

Gegebenenfalls N--C₁-C₄-alkanoyliertes Piperidyl-C₁-C₄-alkyl ist beispielsweise 1-C₁-C₇-C₁-C₄-Alkanoylpiperidin-4-yl-C₁-C₄-alkyl, wie 1-Acetylpiperidin-ylmethyl oder 2-(1-Acetylpiperidin-yl)ethyl.

Gegebenenfalls partiell hydriertes Pyridyl- oder N-Oxidopyridyl-C₁-C₄-alkoxy ist beispielsweise Pyridyl- oder N-Oxidopyridylmethoxy, 2-Pyridylethoxy, 2- oder 3-Pyridylpropyloxy oder 4-Pyridylbutyloxy, insbesondere 3- oder 4-Pyridylmethoxy.

Gegebenenfalls partiell hydriertes Pyridyl- oder N-Oxidopyridyl-C₁-C₄-alkyl ist beispielsweise Pyridyl- oder N-Oxidopyridylmethyl, 2-Pyridylethyl, 2- oder 3-Pyridylpropyl oder 4-Pyridylbutyl, insbesondere 3- oder 4-Pyridylmethyl.

Halogen-(hydroxy)-C₁-C₇-alkoxy ist beispielsweise Halogen-C₂-C₇-(hydroxy)alkoxy, insbesondere Halogen-C₂-C₄-(hydroxy)alkoxy, wie 3-Halogen-, wie 3-Chlor-2-hydroxypropyloxy.

Hydroxy-C₁-C₇-alkoxy ist beispielsweise Hydroxy-C₂-C₇-alkoxy, insbesondere Hydroxy-C₂-C₄-alkoxy, wie 2-Hydroxybutyloxy, 3-Hydroxypropyloxy oder 4-Hydroxybutyloxy.

Hydroxy-C₁-C₇-alkyl ist beispielsweise Hydroxy-C₂-C₇-alkyl, insbesondere Hydroxy-C₂-C₄-alkyl, wie 2-Hydroxyethyl, 3-Hydroxypropyl oder 4-Hydroxybutyl.

Hydroxypiperidino-C₁-C₄-alkyl ist beispielsweise 3- oder 4-Hydroxypiperidino-C₁-C₄-alkoxy, wie 3- oder 4-Hydroxypiperidinomethyl, 2-(3- oder 4-Hydroxypiperidino)ethyl, 3-(3- oder 4-Hydroxypiperidino)propyl oder 4-(3- oder 4-Hydroxypiperidino)butyl.

Imidazolyl-C₁-C₄-alkoxy ist beispielsweise Imidazol-4-ylmethoxy, 2-(Imidazol-4-yl)ethoxy, 3-(Imidazol-4-yl)propyloxy oder 4-(Imidazol-4-yl)butyloxy.

Imidazolyl-C₁-C₄-alkyl ist beispielsweise Imidazol-4-ylmethyl, 2-(Imidazol-4-yl)ethyl, 3-(Imidazol-4-yl)propyl oder 4-(Imidazol-4-yl)butyl.

Morpholinocarbonyl-C₁-C₄-alkyl ist beispielsweise 1-Morpholinocarbonylethyl, 3-Morpholinocarbonylpropyl, oder 1-(Morpholinocarbonyl-2-methyl)propyl.

Morpholino-C₁-C₄-alkoxy kann N-oxidiert sein und ist beispielsweise 1-Morpholinoethoxy, 3-Morpholinopropyloxy, oder 1-(Morpholino-2-methyl)propyloxy.

Morpholino-C₁-C₄-alkyl kann N-oxidiert sein und ist beispielsweise Morpholinomethyl, 2-Morpholinoethyl, 3-Morpholinopropyl oder 1-oder 2-(4-Morpholino)butyl.

C₁-C₇-Alkanoyl ist insbesondere C₂-C₆-Alkanoyl, wie Acetyl, Propionyl, Butyryl, Isobutyryl oder Pivaloyl.

C₁-C₇-Alkanoylamino ist beispielsweise Acetylamino oder Pivaloylamino.

C₁-C₄-Alkanoylamino-C₁-C₄-alkyl ist beispielsweise N-C₁-C₄-Alkanoylamino-C₁-C₄-alkyl, wie 2-Acetaminoethyl.

C₁-C₇-Alkanoyl-C₂-C₄-alkoxy (Oxo-C₂-C₇-alkoxy) trägt die C₁-C₇-Alkanoylgruppe in höherer als der α-Stellung und ist beispielsweise 4-Acetylbutoxy.

C₁-C₇-Alkanoyloxy-C₂-C₄-alkyl trägt die C₁-C₇-Alkanoyloxygruppe in höherer als der α-Stellung und ist beispielsweise 4-Acetoxybutyl.

C₁-C₇-Alkansulfonyl-C₁-C₄-(hydroxy)alkoxy ist beispielsweise 3-Methansulfonyl-2-hydroxypropyloxy.

C₁-C₇-Alkansulfonyl-C₁-C₄-alkoxy ist beispielsweise Methansulfonylmethoxy oder 3-Methansulfonyl-2-hydroxy-propyloxy.

C₁-C₇-Alkansulfonylamino-C₁-C₄-alkoxy ist beispielsweise Ethansulfonylaminomethoxy, 2-Ethansulfonylaminoethoxy, 3-Ethansulfonylaminopropyloxy oder 3-(1,1-Dimethylethansulfonylamino)propyloxy.

C₁-C₇-Alkansulfonylamino-C₁-C₄-alkyl ist beispielsweise Ethansulfonylaminomethyl, 2-Ethansulfonylaminoethyl, 3-Ethansulfonylaminopropyl oder 3-(1,1-Dimethylethansulfonylamino)propyl.

C₁-C₇-Alkansulfonyl-C₁-C₄-alkyl ist beispielsweise Ethansulfonylmethyl, 2-Ethansulfonylethyl, 3-Ethansulfonylpropyl oder 3-(1,1-Dimethylethansulfonyl)propyl.

C₂-C₇-Alkenyl ist beispielsweise Vinyl oder Allyl.

C₂-C₇-Alkenyloxy ist beispielsweise Allyloxy.

C₂-C₇-Alkenyloxy-C₁-C₄-alkoxy ist beispielsweise Allyloxymethoxy.

C₂-C₇-Alkenyloxy-C₁-C₄-alkyl ist beispielsweise Allyloxymethyl.

C₁-C₇-Alkoxy ist beispielsweise C₁-C₅-Alkoxy, wie Methoxy, Ethoxy, Propyloxy, Isopropyloxy, Butyloxy, Isobutyloxy, Sekundärbutyloxy, Tertiärbutyloxy oder Pentyloxy, kann aber auch eine Hexyloxy- oder Heptyloxygruppe sein.

C₁-C₇-Alkoxycarbonyl ist vorzugsweise C₁-C₅-Alkoxycarbonyl, wie Methoxycarbonyl, Ethoxycarbonyl, Propyloxycarbonyl, Isopropyloxycarbonyl, Butyloxycarbonyl, Isobutyloxycarbonyl, Sekundärbutyloxycarbonyl, Tertiärbutyloxy oder Pentyloxycarbonyl, kann aber auch eine Hexyloxycarbonyl- oder Heptyloxycarbonylgruppe sein.

C₁-C₄-Alkoxycarbonyl-C₁-C₇-(hydroxy)alkyl ist beispielsweise 1-Methoxycarbonyl- oder 1-Ethoxycarbonyl-2-hydroxy-ethyl.

C₁-C₇-Alkoxycarbonylamino-C₂-C₇-alkoxy ist vorzugsweise C₂-C₅-Alkoxycarbonylamino-C₂-C₇-alkoxy, wie Methoxycarbonylamino-C₂-C₇-alkoxy, Ethoxycarbonylamino-C₂-C₇-alkoxy, Propyloxycarbonylamino-C₂-C₇-alkoxy, Isopropyloxycarbonylamino-C₂-C₇-alkoxy, Butyloxycarbonylamino-C₂-C₇-alkoxy, Isobutyloxycarbonylamino-C₂-C₇-alkoxy, Sekundärbutyloxycarbonylamino-C₂-C₇-alkoxy oder Tertiärbutyloxyamino-C₂-C₇-alkoxy, worin C₂-C₇-Alkoxy beispielsweise Ethoxy, Propyloxy, Butyloxy, Pentyloxy oder Hexyloxy bedeutet.

C₁-C₇-Alkoxycarbonylamino-C₂-C₇-alkyl ist vorzugsweise C₂-C₅-Alkoxycarbonylamino-C₂-C₇-alkyl, wie Methoxycarbonylamino-C₂-C₇-alkyl, Ethoxycarbonylamino-C₂-C₇-alkyl, Propyloxycarbonylamino-C₂-C₇-alkyl, Isopropyloxycarbonylamino-C₂-C₇-alkyl, Butyloxycarbonylamino-C₂-C₇-alkyl, Isobutyloxycarbonylamino-C₂-C₇-alkyl, Sekundärbutyloxycarbonylamino-C₂-C₇-alkyl oder Tertiärbutyloxyamino-C₂-C₇-alkyl, worin C₂-C₇-Alkyl beispielsweise Ethyl, Propyl, Butyl, Pentyl oder Hexyl bedeutet.

C₁-C₄-Alkoxycarbonyl-C₁-C₄-alkoxy ist beispielsweise Methoxycarbonyl- oder Ethoxycarbonylmethoxy, 2-Methoxycarbonyl- oder 2-Ethoxycarbonylethoxy, 2- oder 3-Methoxycarbonyl- oder 2- oder 3-Ethoxycarbonylpropyloxy oder 4-Methoxycarbonyl- oder 4-Ethoxycarbonylbutyloxy, insbesondere Methoxycarbonyl- oder Ethoxycarbonylmethoxy oder 3-Methoxycarbonyl- oder 3-Ethoxycarbonylpropyloxy.

C₁-C₄-Alkoxycarbonyl-C₁-C₄-alkyl ist beispielsweise Methoxycarbonyl- oder Ethoxycarbonylmethoxy, 2-Methoxycarbonyl- oder 2-Ethoxycarbonylethoxy, 3-Methoxycarbonyl- oder 3-Ethoxycarbonylpropyloxy oder 4-Ethoxycarbonylbutyloxy.

C₁-C₄-Alkoxy-C₂-C₄-alkenyl ist beispielsweise 4-Methoxybut-2-enyl.

C₁-C₄-Alkoxy-C₂-C₄-alkoxy ist beispielsweise 2-Methoxy-, 2-Ethoxy- oder 2-Propyloxyethoxy, 3-Methoxy- oder 3-Ethoxypropyloxy oder 4-Methoxybutyloxy, insbesondere 3-Methoxypropyloxy oder 4-Methoxybutyloxy.

C₁-C₄-Alkoxy-C₁-C₄-alkoxy-C₁-C₄-alkyl ist beispielsweise C₁-C₄-Alkoxy-C₁-C₄-alkoxy-C₁-C₄-alkyl, wie 2-Methoxy-, 2-Ethoxy- oder 2-Propyloxyethoxymethyl, 2-(2-Methoxy-, 2-Ethoxy- oder 2-Propyloxyethoxy)ethyl, 3-(3-Methoxy- oder 3-Ethoxypropyloxy)propyl oder 4-(2-Methoxybutyloxy)butyl, insbesondere 2-(3-Methoxypropyloxy)ethyl oder 2-(4-Methoxybutyloxy)ethyl.

C₁-C₄-Alkoxy-C₁-C₄-alkyl ist beispielsweise Ethoxymethyl, Propyloxymethyl, Butyloxymethyl, 2-Methoxy-, 2-Ethoxy- oder 2-Propyloxyethyl, 3-Methoxy- oder 3-Ethoxypropyl oder 4-Methoxybutyl, insbesondere 3-Methoxypropyl oder 4-Methoxybutyl.

Piperidino-, Hydroxypiperidino- oder C₁-C₄-Alkoxypiperidino-C₁-C₄-alkyl ist beispielsweise 4-C₁-C₄-Alkoxy-, z.B. 4-Methoxypiperidinomethyl.

C₁-C₇-Alkyl kann geradkettig oder verzweigt und/oder überbrückt sein und ist beispielsweise Methyl, Ethyl, Propyl, Isopropyl, Butyl, Isobutyl, Sekundärbutyl, Tertiärbutyl, oder eine Pentyl-, Hexyl- oder Heptylgruppe. C₁-C₇-Alkyl R₂ bzw. R₃ ist insbesondere C₂-C₇-Alkyl, C₁-C₇-Alkyl R₅ bzw. R₇ ist insbesondere verzweigtes C₃-C₇-Alkyl und C₁-C₇-Alkyl R₈ bzw. R₃ ist beispielsweise geradkettiges, verzweigtes oder überbrücktes C₃-C₇-Alkyl.

C₁-C₄-Alkylamino ist beispielsweise Methylamino, Ethylamino, Propylamino, Butylamino, Isobutylamino, Sekundärbutylamino oder Tertiärbutylamino.

C₁-C₄-Alkylamino-C₁-C₄-alkoxy ist beispielsweise Propylaminomethoxy, 2-Methylamino-, 2-Ethylamino-, 2-Propylamino- oder 2-Butylaminoethoxy, 3-Ethylamino- oder 3-Propylaminopropyloxy oder 4-Methylaminobutoxy.

C₁-C₄-Alkylamino-C₁-C₄-alkyl ist beispielsweise Propylaminomethyl, 2-Methylamino-, 2-Ethylamino-, 2-Propylamino- oder 2-Butylaminoethyl, 3-Ethylamino- oder 3-Propylaminopropyl oder 4-Methylaminobutyl.

N-C₁-C₇-Alkylcarbamoyl-C₁-C₄-alkoxy ist beispielsweise Methyl- oder Dimethylcarbamoyl-C₁-C₄-alkoxy, z.B. Methylcarbamoylmethoxy, 2-Methylcarbamoylethoxy oder 3-Methylcarbamoylpropyloxy.

C₁-C₄-Alkylendioxy ist beispielsweise Methylendioxy oder Ethylendioxy, kann aber auch 1,3- oder 1,2-Propylendioxy sein.

N-C₁-C₇-Alkylsulfamoyl-C₁-C₄-alkyl ist beispielsweise N-Methyl-, N-Ethyl- N-Propyl oder N-Butylsulfamoyl-C₁-C₄-alkyl, wie N-Methyl-, N-Ethyl- N-Propyl- oder N-Butylsulfamoylmethyl, 2-(N-Methylsulfamoyl)ethyl, 2-(N-Butylsulfamoyl)ethyl, 3-(N-Methylsulfamoyl)propyl, 3-(N-Butylsulfamoyl)propyl, oder 4-(N-Methylsulfamoyl)butyl, 4-(N-Butylsulfamoyl)butyl oder 4-(N,N-Dimethylsulfamoyl)butyl, insbesondere N-Methyl-, N-Butyl oder N,N-Dimethylsulfamoylmethyl.

C₁-C₄-Alkylthio-C₁-C₄-(hydroxy)alkoxy ist beispielsweise 2-Hydroxy-3-methylthiopropyloxy.

Qxazolyl-C₁-C₄-alkyl ist beispielsweise 2-(1,2,4-Oxadiazol-5-yl)ethyl, 3-(1,2,4-Oxadiazol-5-yl)propyl oder 4-(1,2,4-Oxadiazol-5-yl)butyl.

C₁-C₄-Alkylthio-C₁-C₄-alkoxy ist beispielsweise Methylthio-C₁-C₄-alkoxy, z.B. Methylthiomethoxy, 2-Methylthioethoxy oder 3-Methylthiopropyloxy.

C₁-C₄-Alkylthio-C₁-C₄-alkyl ist beispielsweise Methylthio-C₁-C₄-alkyl, z.B. Methylthiomethyl, 2-Methylthioethyl oder 3-Methylthiopropyl.

N'-C₁-C₄-Alkanoylpiperazino-C₁-C₄-alkoxy 4-Acetylpiperazinomethoxy.

N'-C₁-C₄-Alkanoylpiperazino-C₁-C₄-alkyl ist beispielsweise 4-Acetylpiperazinomethyl.

N'-C₁-C₄-Alkylpiperazino-C₁-C₄-alkyl ist 4-Methylpiperazinomethyl.

Oxo-C₁-C₄-alkoxy ist beispielsweise 3,3-Dimethyl-2-oxo-butyloxy.

Piperazino-C₁-C₄-alkyl ist beispielsweise Piperazinomethyl, 2-Piperazinoethyl oder 3-Piperazinopropyl.

Piperidino-C₁-C₄-alkoxy ist beispielsweise Piperidinomethoxy, 2-Piperidinoethoxy oder 3-Piperidinopropyloxy.

Piperidino-C₁-C₄-alkyl ist beispielsweise Piperidinomethyl, 2-Piperidinoethyl oder 3-Piperidinopropyl.

Polyhalogen-C₁-C₇-alkansulfonylamino-C₁-C₄-alkoxy ist beispielsweise Trifluor-C₁-C₇-alkansulfonylamino-C₁-C₄-alkoxy, wie Trifluormethansulfonylaminobutyloxy.

Polyhalogen-C₁-C₄-alkansulfonylamino-C₁-C₄-alkyl ist beispielsweise Trifluor-C₁-C₇-alkansulfonylamino-C₁-C₄-alkyl, wie Trifluormethansulfonylaminobutyl.

Pyrimidinyl-C₁-C₄-alkoxy ist beispielsweise Pyrimidinylmethoxy, 2-Pyrimidinylethoxy oder 3-Pyrimidinylpropyloxy.

Pyrimidinyl-C₁-C₄-alkyl ist beispielsweise Pyrimidinylmethyl, 2-Pyrimidinylethyl oder 3-Pyrimidinylpropyl.

Pyrrolidino-C₁-C₄-alkoxy ist beispielsweise Pyrrolidino-C₂-C₄-alkoxy, wie 2-Pyrrolidinoethoxy oder 3-Pyrrolidinopropyloxy.

Pyrrolidino-C₁-C₄-alkyl ist beispielsweise Pyrrolidino-C₁-C₄-alkyl, wie Pyrrolidinomethyl, 2-Pyrrolidinoethyl oder 3-Pyrrolidinopropyl.

S,S-Dioxothiomorpholino-C₁-C₄-alkyl ist beispielsweise S,S-Dioxothiomorpholinomethyl oder 2-(S,S-Dioxo)thiomorpholinoethyl.

S-Oxothiomorpholino-C₁-C₄-alkyl ist beispielsweise S-Oxothiomorpholinomethyl oder 2-(S-Oxo)thiomorpholinoethyl.

Sulfamoyl-C₁-C₄-alkyl ist beispielsweise Sulfamoylmethyl, 2-Sulfamoylethyl, 3-Sulfamoylpropyl oder 4-Sulfamoylbutyl.

Tetrazolyl-C₁-C₄-alkyl ist beispielsweise Tetrazol-5-ylmethyl, 2-(Tetrazol-5-yl)ethyl, 3-(Tetrazol-5-yl)propyl oder 4-(Tetrazol-4-yl)butyl.

Thiazolinyl-C₁-C₄-alkoxy ist beispielsweise Thiazolinylmethoxy, 2-Thiazolinylethoxy oder 3-Thiazolinylpropyloxy.

Thiazolinyl-C₁-C₄-alkyl ist beispielsweise Thiazolinylmethyl, 2-Thiazolinylethyl oder 3-Thiazolinylpropyl.

Thiazolyl-C₁-C₄-alkoxy ist beispielsweise Thiazolylmethoxy, 2-Thiazolylethoxy oder 3-Thiazolylpropyloxy.

Thiazolyl-C₁-C₄-alkyl ist beispielsweise Thiazolylmethyl, 2-Thiazolylethyl oder 3-Thiazolylpropyl.

Thiomorpholino-C₁-C₄-alkyl oder S,S-Dioxothiomorpholino-C₁-C₄-alkyl ist beispielsweise Thiomorpholino-C₁-C₄-alkyl, wie -methyl oder -ethyl, oder S,S-Dioxothiomorpholino-C₁-C₄-alkyl, wie -methyl oder -ethyl.

Abhängig vom Vorhandensein asymmetrischer Kohlenstoffatome können die erfindungsgemässen Verbindungen in Form von Isomerengemischen, speziell als Racemate, oder in Form reiner Isomere, speziell von optischen Antipoden vorliegen.

Salze von Verbindungen mit salzbildenden Gruppen sind insbesondere Säureadditionssalze, Salze mit Basen oder bei Vorliegen mehrerer salzbildender Gruppen gegebenenfalls auch Mischsalze oder innere Salze.

Salze sind in erster Linie die pharmazeutisch verwendbaren oder nicht-toxischen Salze von Verbindungen der Formel I.

Solche Salze werden beispielsweise von Verbindungen der Formel I mit einer sauren Gruppe, z.B. einer Carboxy- oder Sulfogruppe, gebildet und sind beispielsweise deren Salze mit geeigneten Basen, wie nicht-toxische, von Metallen der Gruppe Ia, Ib, IIa und IIb des Periodensystems der Elemente abgeleitete Metallsalze, z.B. Alkalimetall-, insbesondere Lithium-, Natrium- oder Kaliumsalze, Erdalkalimetallsalze, beispielsweise Magnesium- oder Calciumsalze, ferner Zinksalze oder Ammoniumsalze, auch solche Salze, welche mit organischen Aminen, wie gegebenenfalls durch Hydroxy substituierten Mono-, Di- oder Trialkylaminen, insbesondere Mono-, Di- oder -C₁-C₄- Tri-C₁-C₄-alkylaminen, oder mit quaternären Ammoniumbasen gebildet werden, z.B. Methyl-, Ethyl-, Diethyl- oder Triethylamin, Mono-, Bis- oder Tris-(2-hydroxy-C₁-C₄-alkyl)-aminen, wie Ethanol-, Diethanol- oder Triethanolamin, Tris-(hydroxymethyl)-methylamin oder 2-Hydroxy-tertiär-butylamin, N,N-Di-C₁-C₄-alkyl-N-(hydroxy-C₁-C₄-alkyl)-amin, wie N,N-Di-N-Dimethyl-N-(2-hydroxyethyl)amin, oder N-Methyl-D-glucamin, oder quaternären Ammoniumhydroxiden, wie Tetrabutylammoniumhydroxid. Die Verbindungen der Formel I mit einer basischen Gruppe, z.B. einer Aminogruppe, können Säureadditionssalze bilden, z.B. mit geeigneten anorganischen Säuren, z.B. Halogenwasserstoffsäure, wie Salzsäure, Bromwasserstoffsäure, Schwefelsäure unter Ersatz eines oder beider Protonen, Phosphorsäure unter Ersatz eines oder mehrerer Protonen, z.B. Orthophosphorsäure oder Metaphosphorsäure, oder Pyrophosphorsäure unter Ersatz eines oder mehrerer Protonen, oder mit organischen Carbon-, Sulfon-, Sulfo- oder Phosphonsäuren oder N-substituierter Sulfaminsäuren, z.B. Essigsäure, Propionsäure, Glykolsäure, Bernsteinsäure, Maleinsäure, Hydroxymaleinsäure, Methylmaleinsäure, Fumarsäure, Äpfelsäure, Weinsäure, Gluconsäure, Glucarsäure, Glucuronsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure, Isonicotinsäure, ferner Aminosäuren, wie z.B. den weiter vom genannten α-Aminosäuren, sowie Methansulfonsäure, Ethansulfonsäure, 2-Hydroxyethansulfonsäure, Ethan-1,2-disulfonsäure, Benzolsulfonsäure, 4-Toluolsulfonsäure, Naphthalin-2-sulfonsäure, 2- oder 3-Phosphoglycerat, Glucose-6-Phosphat, N-Cyclohexylsulfaminsäure (unter Bildung der Cyclamate) oder mit anderen sauren organischen Verbindungen, wie Ascorbinsäure. Verbindungen der Formel I mit sauren und basischen Gruppen können auch innere Salze bilden.

Zur Isolierung und Reinigung können auch pharmazeutisch ungeeignete Salze Verwendung finden.

Die Verbindungen der vorliegenden Erfindung weisen Enzym-hemmende Eigenschaften auf. Insbesondere hemmen sie die Wirkung des natürlichen Enzyms Renin. Letzteres gelangt aus den Nieren ins Blut und bewirkt dort die Spaltung von Angiotensinogen unter Freisetzung des Dekapeptides Angiotensin I, das dann in der Lunge, den Nieren und anderen Organen zum Octapeptid Angiotensinogen II gespalten wird. Das Octapeptid erhöht den Blutdruck sowohl direkt durch arterielle Vasokonstriktion als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II zurückzuführen. Hemmer der enzymatischen Aktivität von Renin bewirken eine Verringerung der Bildung von Angiotensin I. Als Folge davon entsteht eine geringere Menge Angiotensin II. Die verminderte Konzentration dieses aktiven Peptidhormons ist die unmittelbare Ursache für die blutdrucksenkende Wirkung von Renin-Hemmern.

Die Wirkung von Reninhemmern wird unter anderem experimentell mittels in vitro-Tests nachgewiesen, wobei die Verminderung der Bildung von Angiotensin I in verschiedenen Systemen (Humanplasma, gereinigtes humanes Renin zusammen mit synthetischem oder natürlichem Reninsubstrat) gemessen wird. Unter anderem wird der folgende in vitro-Test verwendet: Ein Extrakt von menschlichem Renin aus der Niere (0,5 mGU [Milli-Goldblatt-Einheiten]/ml) wird eine Stunde lang bei 37°C und pH 7,2 in 1-molarer wässriger 2-N-(Tris-hydroxymethylmethyl)amino-ethansulfonsäure-Pufferlösung mit 23 µg/ml synthetischem Reninsubstrat, dem Tetradecapeptid H-Asp-Arg-Val-Tyr-Ile-His-ProPhe-His-Leu-Leu-Val-Tyr-Ser-OH, inkubiert. Die Menge an gebildetem Angiotensin I wird in einem Radioimmunoassay ermittelt. Die erfindungsgemäßen Hemmstoffe werden dem Inkubationgemisch jeweils in verschiedenen Konzentrationen zugefügt. Als IC₅₀ wird diejenige Konzentration des jeweiligen Hemmstoffes bezeichnet, die die Bildung von Angiotensin I um 50 % reduziert. Die Verbindungen der vorliegenden Erfindung zeigen in den in vitro-Systemen Hemmwirkungen bei minimalen Konzentrationen von etwa 10⁻⁶ bis etwa 10⁻¹⁰ Mol/l.

An salzverarmten Tieren bewirken Reninhemmer einen Blutdruckabfall. Das menschliche Renin unterscheidet sich von Renin anderer Spezies. Zur Prüfung von Hemmstoffen des humanen Renins werden Primaten (Marmosets, Callithrix jacchus) verwendet, weil humanes Renin und Primaten-Renin im enzymatisch aktiven Bereich weitgehende homolog sind. Unter anderem wird der folgende in vivo-Test eingesetzt: Die Testverbindungen werden an normotensiven Marmosets beider Geschlechter mit einem Körpergewicht von etwa 350 g, die bei Bewußtsein, freibeweglich und in ihren Normalkäfigen sind, geprüft. Blutdruck und Herzfrequenz werden mit einem Katheter in der Aortha descendens gemessen und radiometrisch erfaßt. Die endogene Freisetzung von Renin wird durch die Kombination einer 1-wöchigen salzarmen Diät mit einer einmaligen intramuskulären Injektion von Furosemid (5-(Aminosulfonyl)-4-chloro-2-[(2-furanylmethyl)amino]benzoesäure) (5 mg/kg) angeregt. 16 Stunden nach der Injektion von Furosemid werden die Testsubstanzen entweder direkt in die Oberschenkelarterie mittels einer Injektionskanüle oder als Suspension oder Lösung über eine Schlundsonde in den Magen verabreicht und ihre Wirkung auf Blutdruck und Herzfrequenz ausgewertet. Die Verbindungen der vorliegenden Erfindung sind in dem beschriebenen in vivo-Test bei Dosen von etwa 0.003 bis etwa 0.3 mg/kg i.v. und bei Dosen von etwa 0.31 bis etwa 30 mg/kg p.o. blutdrucksenkend wirksam.

Die Verbindungen der vorliegenden Erfindung besitzen auch die Eigenschaft, den Augeninnendruck regulieren, insbesondere senken zu können.

Die Messung der Verminderung des Augeninnendruckes nach Applikation eines pharmazeutischen Wirkstoffes der Formel (I) gemäß der vorliegenden Erfindung kann beispielsweise am Tier, insbesondere am Kaninchen oder am Affen bestimmt werden. Nachfolgend sind zwei typische Versuchsanordnungen beschrieben, die die vorliegende Erfindung verdeutlichen, aber in keiner Weise einschränken sollen.

Die in vivo-Testierung am Kaninchen des Typs "Fauve de Bourgogne", zur Bestimmung der Augeninnendruck senkenden Aktivität von topisch applizierten Präparaten, kann beispielsweise wie folgt konzipiert sein. Der Augeninnendruck (AID) wird mit Hilfe eines Applanation-Tonometers jeweils vor dem Experiment und in regelmäßigen zeitlichen Abständen gemessen. Die geeignet formulierte Testsubstanz wird nach einer lokalen Anästhesie topisch in einer präzise definierten Konzentration (z.B. 0.000001 - 5 Gew. %) an einem Auge des jeweiligen Tiers appliziert. Das kontralaterale Auge wird beispielsweise mit physiologischer Kochsalzlösung behandelt. Die so ermittelten Meßwerte werden statistisch ausgewertet.

Die in vivo Tests am Affen der Spezies Macaca Fascicularis, zur Bestimmung der Augeninnendruck senkenden Aktivität von topisch applizierten Präparaten, können beispielsweise folgendermassen durchgeführt werden. Die geeignet formulierte Testsubstanz wird in präzise definierter Konzentration (z.B. 0.000001 -5 Gew.%) jeweils an einem Auge der Affen appliziert. Das zweite Auge der Affen wird entsprechend beispielsweise mit physiologischer Kochsalzlösung behandelt. Vor Beginn der Tests werden die Tiere mit intramuskulären Injektionen von z.B. Ketamin anästhesiert. In regelmäßigen zeitlichen Abständen wird der Augeninnendruck (AID) gemessen. Die Durchführung und Auswertung erfolgt nach den Regeln der "good laboratory practice" (GLP).

Die Verbindungen der vorliegenden Erfindungen können zur Behandlung von Bluthochdruck (Hypertension), Herzinsuffizienz (Congestive Heart Failure), Herzerweiterung (Cardiac Hypertrophy), Herzfibrose (Cardiac Fibrosis), infarktbedingter Herzmuskelschwäche (Cardiomyopathy postinfarction), Komplikationen infolge Diabetes, wie Nephropathie, Vaskulopathie und Neuropathie, Erkrankungen der Herzkranzgefäße, Restenosis nach Angioplastie, erhöhten Augeninnendruck, Glaukom, abnormalem Gefäßwachstum, Hyperaldosteroismus, Angstzuständen und kognitiven Störungen Verwendung finden.

Die im folgenden genannten Verbindungsgruppen sind nicht als geschlossen zu betrachten, sondern es können in sinnvoller Weise, z.B. zur Ersetzung allgemeiner durch speziellere Definitionen, Teile dieser Verbindungsgruppen untereinander oder durch die oben gegebenen Definitionen ausgetauscht oder weggelassen werden.

Die Erfindung betrifft vor allem Verbindungen der Formel I, worin
R₁ Wasserstoff bedeutet,
R₂ C₁-C₇-Alkyl, C₁-C₄-Alkoxy-C₁-C₄-alkyl, C₁-C₄-Alkoxy-C₁-C₄-alkoxy, C₁-C₄-Alkoxy-C₁-C₄-alkoxy-C₁-C₄-alkyl, unsubstituiertes oder durch C₁-C₄-Alkyl, C₁-C₄-Alkoxy, Hydroxy, Halogen, Nitro und/oder Amino substituiertes Phenyl-C₁-C₄-alkoxy, Pyridyl- oder N-Oxidopyridyl-C₁-C₄-alkoxy, C₁-C₄-Alkylthio-C₁-C₄-alkoxy, C₁-C₇-Alkansulfonyl-C₁-C₄-alkoxy, C₁-C₇-Alkanoyl-C₂-C₄-alkoxy, Cyano-C₁-C₄-alkoxy, Carboxy-C₁-C₄-alkoxy, C₁-C₄-Alkoxycarbonyl-C₁-C₄-alkoxy, Carbamoyl-C₁-C₇-alkoxy oder N-Mono- oder N,N-Di-C₁-C₄-alkylcarbamoyl-C₁-C₄-alkoxy darstellt,
R₃ Wasserstoff, C₁-C₇-Alkyl, Hydroxy, C₁-C₄-Alkoxy oder Polyhalogen-C₁-C₄-alkoxy bedeutet oder gemeinsam mit R₄ C₁-C₄-Alkylendioxy darstellt,
R₄ Wasserstoff ist oder gemeinsam mit R₃ C₁-C₄-Alkylendioxy darstellt, X Methylen oder Hydroxymethylen bedeutet,
R₅ für C₁-C₇-Alkyl oder 3- bis 8-gliedriges Cycloalkyl steht,
R₆ Amino, C₁-C₄-Alkylamino, Di-C₁-C₄-alkylamino oder N-C₁-C₇-Alkanoylamino bedeutet,
R₇ C₁-C₇-Alkyl bedeutet und
R₈ C₁-C₇-Alkyl, Hydroxy-C₂-C₇-alkyl, C₁-C₇-Alkanoyloxy-C₂-C₄-alkyl, C₁-C₄-Alkoxy-C₁-C₄-alkyl, C₂-C₇-Alkenyloxy-C₁-C₄-alkyl, Amino-C₁-C₄-alkyl, N-C₁-C₄-Alkanoylamino-C₁-C₄-alkyl, N-C₁-C₄-alkylamino-C₁-C₄-alkyl, N,N-Di-C₁-C₄-alkylamino-C₁-C₄-alkyl,, Piperidino-C₁-C₄-alkyl, Hydroxypiperidino-C₁-C₄-alkyl, C₁-C₄-Alkoxypiperidino-C₁-C₄-alkyl, Morpholino-C₁-C₄-alkyl, Dimethylmorpholino-C₁-C₄-alkyl, Thiomorpholino-C₁-C₄-alkyl, S,S-Dioxothiomorpholino-C₁-C₄-alkyl, Carboxy-C₁-C₄-alkyl, C₁-C₄-Alkoxycarbonyl-C₁-C₄-alkyl, Carbamoyl-C₁-C₇-alkyl, N-Mono- oder N,N-Di-C₁-C₄-alkylcarbamoyl-C₁-C₄-alkyl, Carboxy-C₁-C₇-(hydroxy)alkyl, C₁-C₄-Alkoxycarbonyl-C₁-C₇-(hydroxy)alkyl oder Carbamoyl-C₁-C₇-(hydroxy)alkyl, 5- oder 6-gliedriges Carboxycycloalkyl-C₁-C₄-alkyl, 5- oder 6-gliedriges C₁-C₄-Alkoxycarbonylcycloalkyl-C₁-C₄-alkyl, 5- oder 6-gliedriges Carbamoylcycloalkyl-C₁-C₄-alkyl, 5- oder 6-gliedriges N-Mono- oder N,N-Di-C₁-C₄-alkylcarbamoylcycloalkyl-C₁-C₄-alkyl, Cyano-C₁-C₄-alkyl, C₁-C₇-Alkansulfonyl-C₁-C₄-alkyl, Sulfamoyl-C₁-C₄-alkyl, Imidazolyl-C₁-C₄-alkyl, Oxopyrrolidinyl-C₁-C₄-alkyl, Benzimidazolyl-C₁-C₄-alkyl, Oxadiazolyl-C₁-C₄-alkyl, Pyridyl-C₁-C₄-alkyl, Oxopiperidinyl-C₁-C₄-alkyl oder Chinolinyl-C₁-C₄-alkyl, Piperidin-4-yl-C₁-C₄-alkyl oder 1-C₁-C₇-Alkanoylpiperidin-4-yl-C₁-C₄-alkyl bedeutet,
und ihre Salze.

Die Erfindung betrifft in allererster Linie Verbindungen der Formel I, worin
R₁ und R₄ Wasserstoff bedeuten,
R₂ für C₁-C₄-Alkoxy-C₁-C₄-alkoxy, wie 3-Methoxypropyloxy, oder C₁-C₄-Alkoxy-C₁-C₄-alkyl, wie 4-Methoxybutyl, steht,
R₃ C₁-C₄-Alkyl, wie Isopropyl oder Tertiärbutyl, oder C₁-C₄-Alkoxy, wie Methoxy, darstellt,
R₆ Amino ist, X Methylen ist,
R₅ und R₇ verzweigtes C₁-C₄-Alkyl, wie Isopropyl, bedeuten und
R₈ Carbamoyl-C₁-C₄-alkyl, wie 2- oder 3-Carbamoylpropyl, 2-(3-Carbamoyl)propyl oder 1-(2-Carbamoyl-2-methyl)propyl, N-C₁-C₄-Alkylcarbamoyl-C₁-C₄-alkyl, wie 3-(N-Methylcarbamoyl)propyl, 1-(N-Methylcarbamoyl)prop-2-yl, 2-(N-Methylcarbamoyl)prop-1-yl, vor allem 2(R)-(N-Methylcarbamoyl)prop-1-yl, N,N-Di-C₁-C₄-alkylcarbamoyl-C₁-C₄-alkyl, wie N,N-Dimethylcarbamoylmethyl oder 2-(N,N-Dimethylcarbamoyl)ethyl, 3-(N,N-Dimethylcarbamoyl)propyl, Morpholino-C₁-C₄-alkyl, wie 2-Morpholinoethyl, 3-Morpholinopropyl oder 1-(2-Morpholino-2-methyl)propyl, Thiomorpholino-C₁-C₄-alkyl, wie 2-Thiomorpholinoethyl, 4-(1-C₁-C₄-Alkanoylpiperidyl)-C₁-C₄-alkyl, wie 2-[4-(1-Acetyl)piperidinyl]ethyl, 2-Oxopyrrolidinyl-C₁-C₄-alkyl, wie 2-Oxopyrrolidin-5(S)-ylmethyl oder 2-Oxopyrrolidin-5(R)-ylmethyl,
und ihre Salze.

Besonders wirksam sind jeweils diejenigen Verbindungen der Formel I, worin mindestens ein, beispielsweise ein, zwei, oder vorzugsweise alle vier asymmetrischen C-Atome der Hauptkette die in der Formel Ia 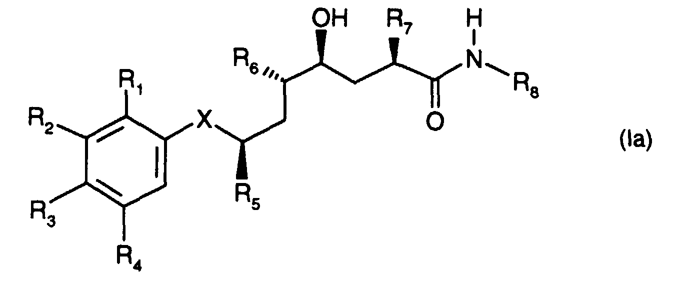 gezeigte Stereotaxie aufweisen, wobei die Variablen jeweils die vorstehend angegebenen Bedeutungen haben, und ihre pharmazeutisch verwendbaren Salze.

Bevorzugter Gegenstand der Erfindung sind dementsprechend Verbindungen der Formel I, worin mindestens ein, beispielsweise ein, zwei, oder vorzugsweise alle vier asymmetrischen C-Atome der Hauptkette die in der Formel Ia gezeigte Stereotaxie aufweisen.

Die Erfindung betrifft ganz besonders diejenigen der vorstehend definierten als bevorzugt bezeichneten Verbindungen der Formeln I und Ia, worin X Methylen bedeutet.

Die Erfindung betrifft namentlich die in den Beispielen genannten Verbindungen der Formel I und ihre Salze, insbesondere ihre pharmazeutisch verwendbaren Salze.

Das erfindungsgemässe Verfahren zur Herstellung von Verbindungen der Formel I ist dadurch gekennzeichnet, dass man
a) eine Verbindung der Formel II 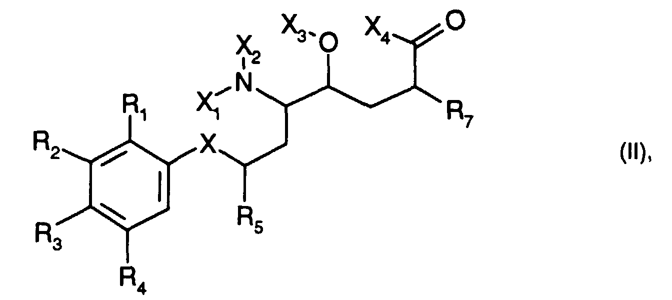 worin X₁ C₁-C₇-Alkyl, C₁-C₇-Alkanoyl oder eine Aminoschutzgruppe bedeutet, X₂ Wasserstoff oder gemeinsam mit X₃ eine bivalente Schutzgruppe darstellt, X₃ Wasserstoff, eine Hydroxyschutzgruppe oder gemeinsam mit X₂ eine bivalente Schutzgruppe darstellt bzw. gemeinsam mit X₄ für eine direkte Bindung steht, X₄ gegebenenfalls reaktionsfähig verethertes oder verestertes Hydroxy, oder gemeinsam mit X₃ eine direkte Bindung darstellt und R₁, R₂, R₃, R₄, X, R₅, R₆ und R₇ die unter der Formel I angegebenen Bedeutungen haben, mit einem Amin der Formel III

   H₂N―R₈ (III),

   worin R₈ eine der unter der Formel I angegebenen Bedeutungen hat, unter Bildung einer Amidbindung umsetzt und vorhandene Schutzgruppen abspaltet, oder
b) in einem Carbonsäureamid der Formel IV 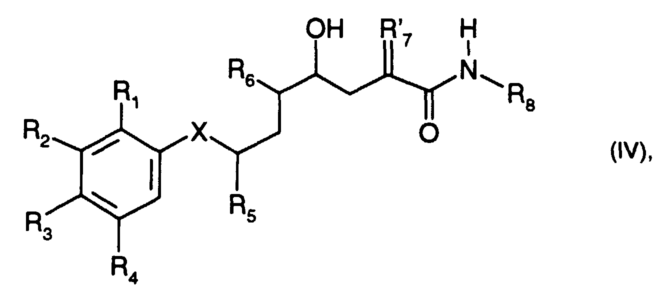 worin R₁, R₂, R₃, R₄, X, R₅, R₆, R₇ und R₈ die unter der Formel I angegebenen Bedeutungen haben und R'₇ eine der C₁-C₄-Alkyl- bzw. Aryl-C₁-C₄-alkylgruppe R₇ entsprechende C₁-C₄-Alkyliden- oder Aryl-C₁-C₄-alkylidengruppe bedeutet, wobei freie funktionelle Gruppen gegebenenfalls in geschützter Form vorliegen, oder ein Salz davon die Gruppe R'₇ durch Behandlung mit einem Hydrierungsmittel zu R₇ reduziert, oder
c) zur Herstellung von Verbindungen der Formel I, worin R₆ Amino bedeutet, in einem 5-Azidocarbonsäurederivat der Formel V 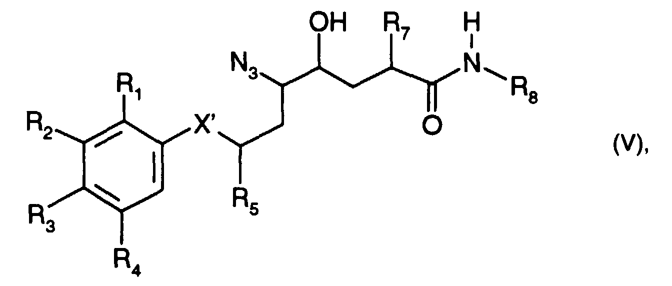 worin R₁, R₂, R₃, R₄, R₅, R₇ und R₈ die unter der Formel I angegebenen Bedeutungen haben, X' Methylen oder gegebenenfalls verestertes oder verethertes Hydroxymethyl bedeutet, und freie funktionelle Gruppen gegebenenfalls in geschützter Form vorliegen, oder in einem Salz davon die Azidogruppe, gewünschtenfalls unter Freisetzung von Hydroxymethyl X oder Reduktion von X' zu Methylen X, zu Amino reduziert und vorhandene Schutzgruppen abspaltet und gewünschtenfalls eine nach einem der vorstehend genannten Verfahren a) bis c) erhältliche Verbindung der Formel I mit mindestens einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

Funktionelle Gruppen in Ausgangsmaterialien, deren Umsetzung vermieden werden soll, insbesondere Carboxy-, Amino-, Hydroxy- und Mercaptogruppen, können durch geeignete Schutzgruppen (conventional protecting groups) geschützt sein, die üblicherweise bei der Synthese von Peptid-Verbindungen, aber auch von Cephalosporinen und Penicillinen sowie Nucleinsäurederivaten und Zuckern verwendet werden. Diese Schutzgruppen können bereits in den Vorstufen vorhanden sein und sollen die betreffenden funktionellen Gruppen gegen unerwünschte Nebenreaktionen wie Acylierungen, Veretherungen, Veresterungen, Oxidationen, Solvolyse etc. schützen. In bestimmten Fällen können die Schutzgruppen darüber hinaus einen selektiven, beispielsweise stereoselektiven Verlauf von Umsetzungen bewirken. Charakteristisch für Schutzgruppen ist, dass sie leicht, d.h. ohne unerwünschte Nebenreaktionen abspaltbar sind, beispielsweise solvolytisch, reduktiv, photolytisch oder auch enzymatisch, z.B. unter physiologischen Bedingungen. Schutzgruppen können aber auch in den Endstoffen vorhanden sein. Verbindungen der Formel I mit geschützten funktionellen Gruppen können eine höhere metabolische Stabilität oder anderweitig verbesserte pharmakodynamische Eigenschaften aufweisen als die entsprechenden Verbindungen mit freien funktionellen Gruppen.

Der Schutz von funktionellen Gruppen durch solche Schutzgruppen, die Schutzgruppen selbst, sowie ihre Abspaltungzreaktionen sind beispielsweise in Standardwerken wie J. F. W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (E. Gross und J. Meienhofer, Herausg.), Academic Press, London und New York 1981, in "Methoden der organischen Chemie", HoubenHouben-Weyll, 4. Auflage, Band 15/I, Georg Thieme Verlag, Stuttgart 1974, in H.-D. Jakubke und H. Jescheit, "Aminosäuren, Peptide, Proteine", Verlag Chemie, Weinheim, Deerfield Beach und Basel 1982, und in Jochen Lehmann, "Chemie der Kohlenhydrate: Monosaccharide und Derivate", Georg Thieme Verlag, Stuttgart 1974, beschrieben.

### Verfahrensvariante a) (Herstellung der Amidbindung):

Aminoschutzgruppen X₁ sind beispielsweise von C₁-C₇-Alkanoyl verschiedenen Acylgruppen, ferner Arylmethyl, C₁-C₄-Alkylthio, 2-Acyl--C₁-C₄-alk-1-enyl oder Silyl. Die Gruppe X₁-N(X₂)- kann auch als Azidogruppe vorliegen.

Von C₁-C₇-Alkanoyl verschiedenen Acylgruppen sind beispielsweise Halogenalkanoyl, z.B. 2-Halogenacetyl, wie 2-Chlor-, 2-Brom-, 2-Jod-, 2,2,2-Trifluor- oder 2,2,2-Trichloracetyl, gegebenenfalls z.B. durch Halogen, C₁-C₇-Alkoxy oder Nitro substituiertes Benzoyl, z.B. Benzoyl, 4-Chlorbenzoyl, 4-Methoxybenzoyl oder 4-Nitrobenzoyl, oder in 1-Stellung des Alkylrestes verzweigtes oder in 1- oder 2-Stellung geeignet substituiertes Aalkoxycarbonyl, beispielsweise Tertiäralkoxycarbonyl, wie Tertiärbutyloxycarbonyl, Arylmethoxycarbonyl mit einem oder zwei Arylresten, die gegebenenfalls, z.B. durch C₁-C₇-Alkyl, z.B. tertiäres C₁-C₇-Alkyl, wie Tertiärbutyl, C₁-C₇-Alkoxy, wie Methoxy, Hydroxy, Halogen, wie Chlor, und/oder Nitro mono- oder polysubstituiertes Phenyl darstellen, z.B. Benzyloxycarbonyl, gegebenenfalls substituiertes Benzyloxycarbonyl, wie 4-Nitrobenzyloxycarbonyl, Diphenylmethoxycarbonyl, Fluorenylmethoxycarbonyl oder substituiertes Diphenylmethoxycarbonyl, wie Di-(4-methoxyphenyl)methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogenalkoxycarbonyl, z.B. 2,2,2-Trichlorethoxycarbonyl, 2-Bromethoxycarbonyl oder 2-Jodethoxycarbonyl, 2-(trisubstituiertes Silyl)-alkoxycarbonyl, z.B.2-Trialkylsilylalkoxycarbonyl, z.B. 2-Trimethylsilylethoxycarbonyl oder 2-(Di-n-butyl-methyl-silyl)-ethoxycarbonyl, oder Triarylsilylalkoxycarbonyl, z.B. 2-Triphenylsilylethoxycarbonyl.

In einem als Aminoschutzgruppe verwendbaren 2-Acyl-alk-1-enylrest ist Acyl z.B. der entsprechende Rest einer C₁-C₇-Alkancarbonsäure, einer gegebenenfalls z.B. durch C₁-C₇-Alkyl, wie Methyl oder Tertiärbutyl, C₁-C₇-Alkoxy, wie Methoxy, Halogen, wie Chlor, und/oder Nitro substituierten Benzoesäure, oder insbesondere eines Kohlensäurehalbesters, wie eines Kohlensäure-alkylhalbesters. Entsprechende Schutzgruppen sind in erster Linie 1-C₁-C₇-Alkanoyl-prop-1-en-2-yl, z.B. 1-Acetyl-prop-1-en-2-yl, oder C₁-C₇-Alkoxycarbonyl-prop-1-en-2-yl, z.B. 1-Ethoxycarbonyl-prop-1-en-2-yl.

Eine Silylaminogruppe ist beispielsweise eine Trialkylsilylaminogruppe, z.B. Trimethylsilylamino. Das Siliziumatom der Silylaminogruppe kann auch nur durch zwei C₁-C₇-Alkylgruppen, z.B. Methylgruppen, und die Aminogruppe oder Carboxylgruppe eines zweiten

Moleküls der Formel I substituiert sein. Verbindungen mit solchen Schutzgruppen lassen sich z.B. mit Dimethylchlorsilan als Silylierungsmittel herstellen.

Eine Aminogruppe kann auch durch Überführung in die protonierte Form geschützt werden; als entsprechende Anionen kommen in erster Linie diejenigen von starken anorganischen Säuren, wie von Schwefelsäure, Phosphorsäure oder Halogenwasserstoffsäuren, z.B. das Chlor- oder Bromanion, oder von organischen Sulfonsäuren, wie p-Toluolsulfonsäure, in Frage.

Bevorzugte Aminoschutzgruppen X₁ sind Acylreste von Kohlensäurehalbestern, wie C₁-C₇-Alkoxycarbonyl, insbesondere Tertiärbutyloxycarbonyl oder Fluorenylmethoxycarbonyl, gegebenenfalls durch C₁-C₇-Alkyl, C₁-C₇-Alkoxy, Nitro und/oder Halogen substituiertes α-Phenyl- oder α,α-Diphenylalkoxycarbonyl, wie Benzyloxycarbonyl, p-Nitrobenzyloxycarbonyl oder Diphenylmethoxycarbonyl, oder 2-Halogenalkoxycarbonyl, z.B. 2,2,2-Trichlorethoxycarbonyl, ferner Trityl oder Formyl.

Hydroxyschutzgruppen X₃ sind beispielsweise Acylgruppen, z.B. durch Halogen, wie Chlor, substituiertes C₁-C₇-Alkanoyl, z.B. 2,2-Dichloracetyl, oder insbesondere für geschützte Aminogruppen genannte Acylreste eines Kohlensäurehalbesters geschützt sein. Eine bevorzugte Hydroxyschutzgruppe ist beispielsweise 2,2,2-Trichlorethoxycarbonyl, 4-Nitrobenzyloxycarbonyl, Diphenylmethoxycarbonyl oder Trityl. Als weitere Hydroxyschutzgruppe X₃ kommen Trialkylsilyl, z.B. Trimethylsilyl, Triisopropylsilyl oder Dimethyltertiärbutylsilyl, leicht abspaltbare verethernde Gruppen, z.B. eine Alkylgruppe, wie tertiäres C₁-C₇-Alkyl, z.B. Tertiärbutyl, oxa- oder thiaaliphatische oder -cycloaliphatische, insbesondere 2-oxa- oder 2-thiaaliphatische oder -cycloaliphatische, Kohlenwasserstoffreste, beispielsweise 1-C₁-C₇-Alkoxyalkyl oder 1-C₁-C₇-Alkylthioalkyl, z.B. Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-7 Ringatomen, z.B. 2-Tetrahydrofuryl oder 2-Tetrahydropyranyl, oder entsprechende Thiaanaloge sowie 1-Phenylalkyl, z.B. Benzyl, Diphenylmethyl oder Trityl, wobei die Phenylreste beispielsweise durch Halogen, z.B. Chlor, C₁-C₇-Alkoxy, z.B. Methoxy, und/oder Nitro substituiert sein können, in Betracht.

Durch X₂ und X₃ gemeinsam dargestellte bivalente Schutzgruppen sind beispielsweise durch ein oder zwei Alkylreste substituierte Methylengruppen und bedeuten somit unsubstituiertes oder substituiertes Alkyliden, wie C₁-C₇-Alkyliden, z.B. Isopropyliden, Cycloalkyliden, z.B. Cyclohexyliden, ferner Carbonyl oder Benzyliden.

Bedeutet X₄ reaktionsfähig verethertes oder verestertes Hydroxy, stellt die endständige Gruppe -(=O)-X₄ eine reaktionsfähig funktionell abgewandelte Carbonsäurefunktion dar und liegt beispielsweise in einer aktivierten Ester- oder Anhydridform vor. Die reaktionsfähigen Säurederivate können auch *in situ* gebildet werden.

Derartige aktivierte Ester von Verbindungen der Formel II sind insbesondere am Verknüpfungskohlenstoffatom des veresternden Restes ungesättigte Ester, z.B. vom Vinylester-Typ, wie Vinylester (erhältlich z.B. durch Umesterung eines entsprechenden Esters mit Vinylacetat; Methode des aktivierten Vinylesters), Carbamoylester (erhältlich z.B. durch Behandeln der entsprechenden Säure mit einem Isoxazoliumreagens; 1,2-Oxazolium- oder Woodward-Methode), oder 1-C₁-C₇-Alkoxyvinylester (erhältlich z.B. durch Behandeln der entsprechenden Säure mit einem C₁-C₇-Alkoxyacetylen; Ethoxyacetylen-Methode), oder Ester vom Amidinotyp, wie N,N'-disubstituierte Amidinoester (erhältlich z.B. durch Behandeln der entsprechenden Säure mit einem geeigneten N,N'-disubstituierten Carbodiimid, z.B. N,N'-Dicyclohexylcarbodiimid; Carbodiimid-Methode), oder N,N-disubstituierte Amidinoester (erhältlich z.B. durch Behandeln der entsprechenden Säure mit einem N,N-disubstituierten Cyananmid; Cyanamid-Methode), geeignete Arylester, insbesondere durch elektronenanziehende Substituenten geeignet substituierte Phenylester (erhältlich z.B. durch Behandeln der entsprechenden Säure mit einem geeignet substituierten Phenol, z.B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester), Cyanmethylester (erhältlich z.B. durch Behandeln der entsprechenden Säure mit Chloracetonitril in Gegenwart einer Base; Cyanmethylester-Methode), Thioester, insbesondere gegebenenfalls, z.B. durch Nitro, substituierte Phenylthioester (erhältlich z.B. durch Behandeln der entsprechenden Säure mit gegebenenfalls, z.B. durch Nitro, substituierten Thiophenolen, u. a. mit Hilfe der Anhydrid- oder Carbodiimid-Methode; Methode der aktivierten Thiolester), oder insbesondere Amino- oder Amidoester (erhältlich z.B. durch Behandeln der entsprechenden Säure mit einer N-Hydroxyamino- bzw. N-Hydroxyamidoverbindung, z.B. N-Hydroxysuccinimid, N-Hydroxypiperidin, N-Hydroxyphthalimid, N-Hydroxy-5-norbomen-2,3-dicarbonsäureimid, 1-Hydroxybenztriazol oder 3-Hydroxy-3,4-dihydro-1,2,3-benztriazin-4-on, z.B. nach der Anhydrid- oder Carbodiimid-Methode; Methode der aktivierten N-Hydroxyester). Es sind auch innere Ester, z.B. γ-Laktone, einsetzbar.

Anhydride von Säuren der Formel II können symmetrische oder vorzugsweise gemischte Anhydride dieser Säuren sein, z.B. Anhydride mit anorganischen Säuren, wie Säurehalogenide, insbesondere Säurechloride (erhältlich z.B. durch Behandeln der entsprechenden Säure mit Thionylchlorid, Phosphorpentachlorid oder Oxalylchlorid; Säurechloridmethode), Azide (erhältlich z.B. aus einem entsprechenden Säureester über das entsprechende Hydrazid und dessen Behandlung mit salpetriger Säure; Azidmethode), Anhydride mit Kohlensäurehalbestern, z.B. Kohlensäurealkylhalbestern (erhältlich z.B. durch Behandeln der entsprechenden Säure mit Chlorameisensäurealkylestem oder mit einem 1-C₁-C₇-Alkoxycarbonyl-2-alkoxy-1,2-dihydrochinolin; Methode der gemischten O-Alkylkohlensäureanhydride), oder Anhydride mit dihalogenierter, insbesondere dichlorierter Phosphorsäure (erhältlich z.B. durch Behandeln der entsprechenden Säure mit Phosphoroxychlorid; Phosphoroxychloridmethode), Anhydride mit anderen Phosphorsäurederivaten (z.B. solchen, die man mit Phenyl-N-Phenylphosphoramidochloridat erhalten kann) oder mit Phosphorigsäurederivaten, oder Anhydride mit organischen Säuren, wie gemischte Anhydride mit organischen Carbonsäuren (erhältlich z.B. durch Behandeln der entsprechenden Säure mit einem gegebenenfalls substituierten C₁-C₇-Alkan- oder Phenylalkancarbonsäurehalogenid, z.B. Phenylessigsäure-, Pivalinsäure- oder Trifluoressigsäurechlorid; Methode der gemischten Carbonsäureanhydride) oder mit organischen Sulfonsäuren (erhältlich z.B. durch Behandeln eines Salzes, wie eines Alkalimetallsalzes, der entsprechenden Säure mit einem geeigneten organischen Sulfonsäurehalogenid, wie C₁-C₇-Alkan- oder Aryl-, z.B. Methan- oder p-Toluolsulfonsäurechlorid; Methode der gemischten Sulfonsäureanhydride), sowie symmetrische Anhydride (erhältlich z.B. durch Kondensation der entsprechenden Säure in Gegenwart eines Carbodiimides oder von 1-Diethylaminopropin; Methode der symmetrischen Anhydride).

Bevorzugte Ausgangsstoffe der Formel II sind Verbindungen der Formeln IIa, IIb und IIc 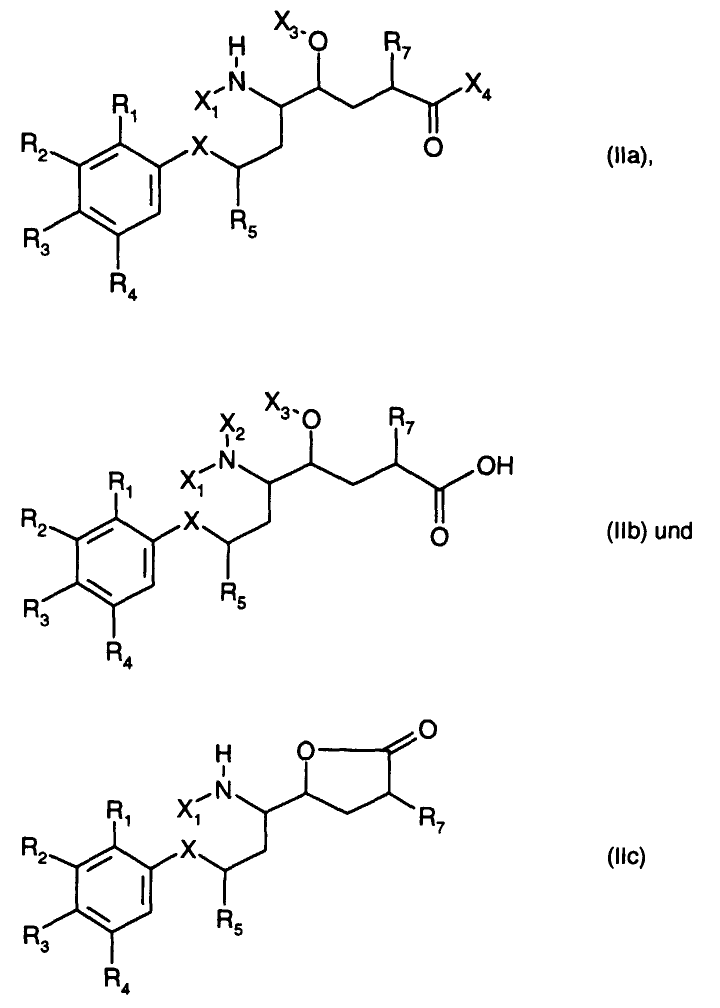 worin X₁ eine Aminoschutzgruppe, insbesondere Tertärbutyloxycarbonyl, X₂ gemeinsam mit X₃ eine bivalente Schutzgruppe, insbesondere C₁-C₇-Alkyliden, wie Isopropyliden, und X₃ in Formel IIa Wasserstoff oder Trialkylsilyl, insbesondere Tertärbutyl(dimethyl)silyl, bzw. in Formel IIb gemeinsam mit X₂ eine bivalente Schutzgruppe, insbesondere C₁-C₇-Alkyliden, wie Isopropyliden, bedeutet und X₄ Hydroxy, C₁-C₇-Alkoxy oder Halogen, wie Chlor, darstellt.

Wie erwähnt, können Derivate von Carbonsäuren, die als Acylierungsmittel verwendet werden, auch *in situ* gebildet werden. So kann man z.B. N,N'-disubstituierte Amidinoester *in situ* bilden, indem man das Gemisch der als Acylierungsmittel verwendeten Säure und des Ausgangsmaterials der Formel III in Gegenwart eines geeigneten N,N'-disubstituierten Carbodiimids, z.B. N,N'-Cyclohexylcarbodiimid, zur Reaktion bringt. Ferner kann man Amino- oder Amidoester der als Acylierungsmittel verwendeten Säuren in Gegenwart des zu acylierenden Ausgangsmaterials der Formel III bilden, indem man das Gemisch der entsprechenden Säure- und Amino-Ausgangsstoffe in Gegenwart eines N,N'-disubstituierten Carbodiimids, z.B. N,N'-Dicyclohexylcarbodiimid, und eines N-Hydroxyamins oder N-Hydroxy-amids, z.B. N-Hydroxysuccinimid, gegebenenfalls in Anwesenheit einer geeigneten Base, z.B. 4-Dimethylamino-pyridin, umsetzt.

Die Kondensation zur Herstellung einer Amidbindung kann in an sich bekannter Weise durchgeführt werden, beispielsweise wie in Standardwerken, wie "Houben-Weyl, Methoden der organischen Chemie", 4. Auflage, Band 15/II (1974), Band IX (1955) Band E 11 (1985), Georg Thieme Verlag, Stuttgart, "The Peptides" (E. Gross und J. Meienhofer, Hg.), Band 1 und 2, Academic Press, London und New York, 1979/1980, oder M. Bodansky, "Principles of Peptide Synthesis", Springer-Verlag, Berlin 1984, beschrieben.

Die Kondensation einer freien Carbonsäure mit dem entsprechenden Amin kann vorzugsweise in Gegenwart eines der üblichen Kondensationsmittel durchgeführt werden. Übliche Kondensationsmittel sind z.B. Carbodiimide, beispielsweise Diethyl-, Dipropyl-, N-Ethyl-N'-(3-dimethylaminopropyl)-carbodiimid oder insbesondere Dicyclohexylcarbodiimid, ferner geeignete Carbonylverbindungen, beispielsweise Carbonyldiimidazol, 1,2-Oxazoliumverbindungen, z.B. 2-Ethyl-5-phenyl-1,2-oxazolium-3'-sulfonat und 2-tert-Butyl-5-methylisoxazoliumperchlorat, oder eine geeignete Acylaminoverbindung, z.B. 2-Ethoxy-1-ethoxycarbonyl-1,2-dihydrochinolin, ferner aktivierte Phosphorsäurederivate, z.B. Diphenylphosphorylazid, Diethylphosphorylcyanid, Phenyl-N-phenylphosphoroamidochloridat, Bis-(2-oxo-3-oxazolidinyl)-phosphinsäurechlorid oder 1-Benztriazolyloxy-tris-(dimethylamino)phosphonium-hexafluorophosphat.

Gewünschtenfalls wird eine organische Base zugegeben, z.B. ein Trialkylamin mit voluminösen Resten, z.B. Ethyldiisopropylamin, und/oder eine heterocyclische Base, z.B. Pyridin, N-Methylmorpholin oder bevorzugt 4-Dimethylaminopyridin.

Die Kondensation aktivierter Ester, reaktionsfähiger Anhydride oder reaktionsfähiger cyclischer Amide mit den entsprechenden Aminen wird üblicherweise in Gegenwart einer organischen Base, z.B. einfachen Trialkylaminen, z.B. Triethylamin oder Tributylamin, oder einer der vorstehend genannten organischen Basen durchgeführt. Gewünschtenfalls wird zusätzlich noch ein Kondensationsmittel verwendet, wie für freie Carbonsäuren beschrieben ist.

Die Kondensation von Säureanhydriden mit Aminen kann z.B. in Gegenwart von anorganischen Carbonaten, z.B. Ammonium- oder Alkalimetallcarbonaten oder - hydrogencarbonaten, wie Natrium- oder Kaliumcarbonat oder -hydrogencarbonat (üblicherweise zusammen mit einem Sulfat), erfolgen.

Carbonsäurechloride, beispielsweise die von der Säure der Formel II abgeleiteten Chlorkohlensäurederivate werden mit den entsprechenden Aminen vorzugsweise in Gegenwart eines organischen Amins, z.B. der vorstehend genannten Trialkylamine oder heterocyclischen Basen, gegebenenfalls in Gegenwart eines Hydrogensulfates, kondensiert.

Die Kondensation wird vorzugsweise in einem inerten, aprotischen, vorzugsweise wasserfreien, Lösungsmittel oder Lösungsmittelgemisch durchgeführt, beispielsweise in einem Carbonsäureamid, z.B. Formamid oder Dimethylformamid, einem halogenierten Kohlenwasserstoff, z.B. Methylenchlorid, Tetrachlorkohlenstoff oder Chlorbenzol, einem Keton, z.B. Aceton, einem cyclischen Ether, z.B. Tetrahydrofuran, einem Ester, z.B. Essigsäureethylester, oder einem Nitril, z.B. Acetonitril, oder in einer Mischung davon, gegebenenfalls bei erniedrigter oder erhöhter Temperatur, z.B. in einem Temperaturbereich von etwa -40° C bis etwa +100° C, bevorzugt von etwa -10° C bis etwa +50° C, im Falle der Verwendung von Arylsulfonylestern auch bei etwa +100° C bis +200° C, und gegebenenfalls unter Inertgas-, z.B. Stickstoff- oder Argonatmosphäre.

Auch wässrige, beispielsweise alkoholische, z.B. Ethanol, oder aromatische Lösungsmittel, z.B. Benzol oder Toluol, sind möglich. Bei Gegenwart von Alkalihydroxiden als Basen kann gegebenenfalls auch Aceton zugesetzt werden.

Die Kondensation kann auch gemäß der als Festphasen-Synthese bekannten Technik erfolgen, die auf R. Merrifield zurückgeht und beispielsweise in Angew. Chem. 97, 801 - 812 (1985), Naturwissenschaften 71, 252 - 258 (1984) oder in R. A. Houghten, Proc. Natl. Acad. Sci. USA 82, 5131 - 5135 (1985) beschrieben ist.

Eine bevorzugte Variante dieses Verfahrens ist dadurch gekennzeichnet, dass man als aktivierten Ester einen von der Carbonsäure der Formel I abgeleiteten inneren Ester (γ-Lakton) der Formel IIc 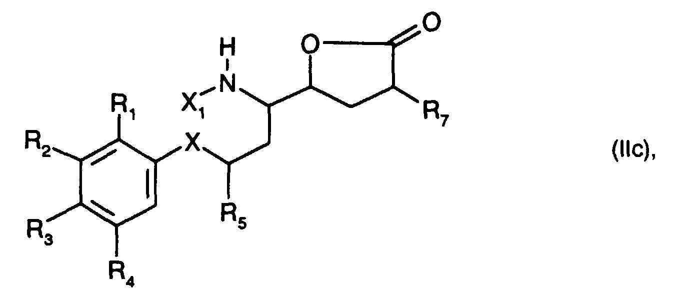 worin X Methylen ist, mit der Verbindung der Formel III umsetzt, wobei in den Reaktionskomponenten vorhandene freie funktionelle Gruppen mit Ausnahme der an der Reaktion teilnehmenden Gruppen gegebenenfalls, wie oben dargelegt, in geschützter Form vorliegen und die vorhandenen Schutzgruppen wie oben beschrieben abgespalten werden. Die Öffnung des Laktonringes unter Bildung der Amidbildung erfolgt unter den vorstehend beschriebenen Bedingungen, gegebenenfalls in Gegenwart eines geeigneten Katalysators. Insbesondere kann ein γ-Lakton IIc mit einem primären Amin III ohne Lösungsmittel oder in Gegenwart eines polaren Lösungsmittels, z.B. einem -C₁-C₄-en Alkohol, wie Methanol oder Ethanol, einem polaren Ether, wie Tetrahydrofuran oder Dioxan, einem Nitril, wie Acetonitril, einem Amid, wie Dimethylformamid, N,N-Dimethylacetamid, N-Methylpyrrolidon oder Hexamethylphosphorsäuretriamid, einem Harnstoff, z.B. N,N'-Dimethyl-N,N'-propylenylharnstoff, einem C₁-C₇-Alkoxy-alkanol, z.B. Diethylenglykolmonomethylether, in Dimethylsulfoxid oder einem Gemisch der genannten Lösungsmittel oder einem Gemisch eines oder mehrerer der genannten Lösungsmittel mit Wasser, bei Temperaturen von Raumtemperatur bis 150° C, bevorzugt von etwa 20° C bis 100°C, und in Gegenwart eines

Katalysators, wie 2-Hydroxypyridin und/oder Triethylamin umgesetzt werden, wobei für die Schutzgruppen das oben Gesagte gilt.

In einer anderen bevorzugten Variante dieses Verfahrens geht man von einer Verbindung der Formeln IIb, worin X Methylen ist, aus und setzt diese in Gegenwart eines Cyanphosphonsäure-diesters, z.B. von Cyanphosphonsäure-diethylester, und eines tertiären organischen Amins, wie eines Tri-C₁-C₄-alkylamins, z.B. von Trimethylamin, und in einem polaren Lösungsmittel, z.B. einem Nitril, wie Acetonitril, einem Amid, wie Dimethylformamid, N,N-Dimethylacetamid, N-Methylpyrrolidon oder Hexamethylphosphorsäuretriamid, einem Harnstoff, z.B. N,N'-Dimethyl-N,N'-propylenylharnstoff, einem C₁-C₇-Alkoxy-alkanol, z.B. Diethylenglykolmonomethylether, in Dimethylsulfoxid oder einem Gemisch der genannten Lösungsmittel oder einem Gemisch eines oder mehrerer der genannten Lösungsmittel mit Wasser, bei Temperaturen von -30° C bis 100°C, bevorzugt von 20° C bis 80°C, mit der Reaktionskomponente der Formel III um, wobei für die Schutzgruppen das oben Gesagte gilt.

Ausgangsstoffe der Formel II können beispielsweise hergestellt werden, indem man eine Verbindung der Formel VI 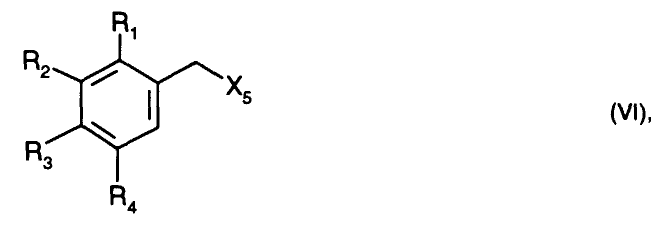 worin X₅ gegebenenfalls reaktionsfähig verestertes Hydroxy, insbesondere Halogen, wie Brom, bedeutet, mit einer Verbindung der Formel VII 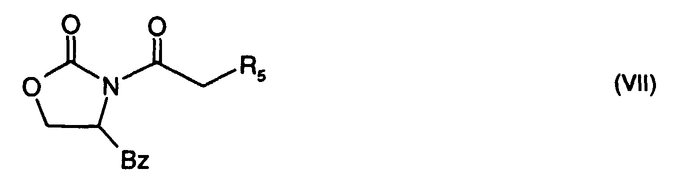 umsetzt, in der erhaltenen Verbindung der Formel VIII 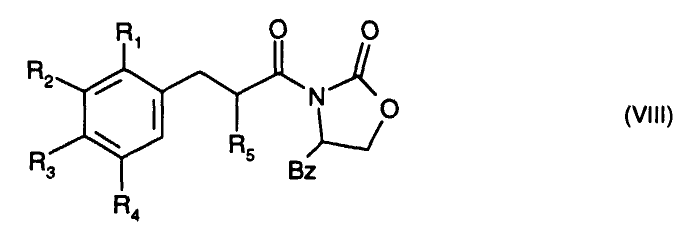 die 4-Benzyl-2-oxo-oxazolidin-1-ylcarbonylgruppe, beispielsweise mittels Lithiumhydroxid/Wasserstoffperoxid, selektiv zu Carboxy hydrolysiert, die Carboxygruppe, beispielsweise mittels Natriumborhydrid/Jod in Tetrahydrofuran, zu Hydroxymethyl reduziert, die Hydroxymethylgruppe, z.B. mit N-Bromsuccinimid/Triphenylphosphin in Dichlormethan, halogeniert und das Reaktionsprodukt der Formel IX 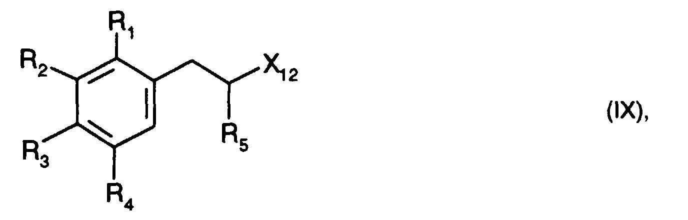 worin X₁₂ Halogenmethyl ist, mit einer Verbindung der Formel X 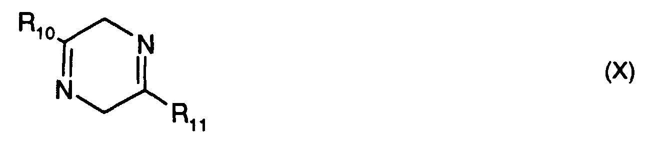 umsetzt, worin R₁₀ und R₁₁ gleiche oder verschiedene C₁-C₇-Alkoxygruppen bedeuten, die erhaltene Verbindung der Formel XI 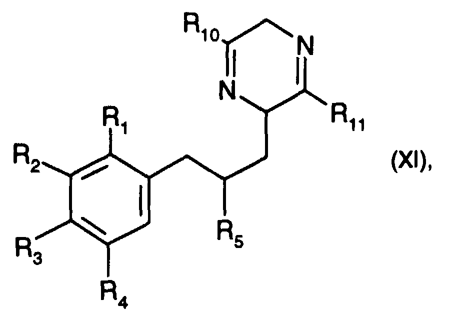 worin R₁, R₂, R₃, R₄ und R₅ die vorstehend angegebenen Bedeutungen haben und R₁₀ und R₁₁ gleiche oder verschiedene C₁-C₇-Alkoxygruppen bedeuten, hydrolysiert, die erhaltene Verbindung der Formel XII 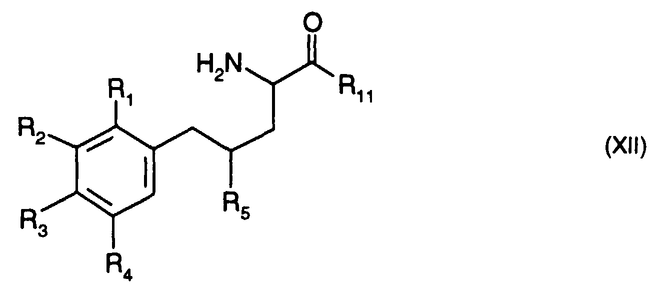 an der Aminogruppe durch eine Aminoschutzgruppe X₁ schützt und gewünschstenfalls die erhaltene Verbindung der Formel XIII 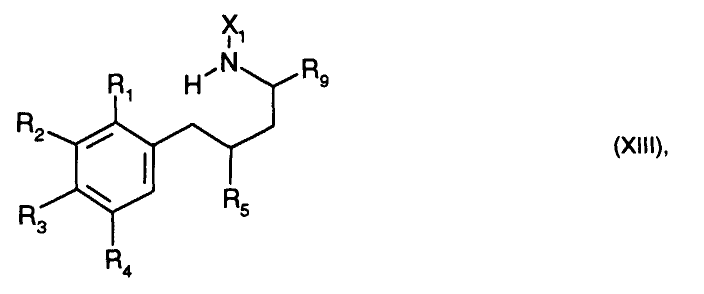 worin R₈ Formyl darstellt, mit einer Verbindung der Formel XVII 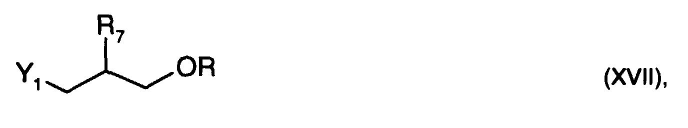 worin Y₁ einen metallischen, insbesondere erdalkalimetallischen Rest, z.B. der Formel - MgHal, worin Hal Brom, Chlor oder Jod bedeutet, und OR verethertes Hydroxy, wie gegebenenfalls substituiertes Benzyloxy, bedeutet, zur entsprechenden Verbindung der Formel XVIII 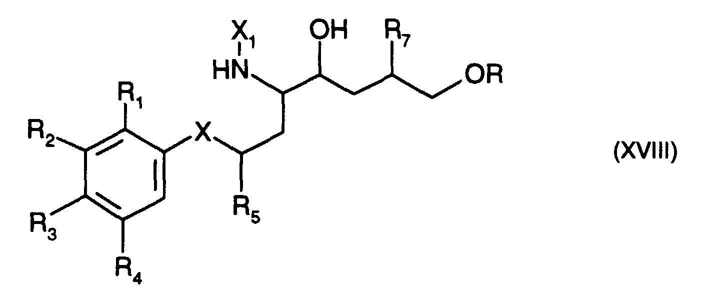 umsetzt, diese an der Amino- und Hydroxygruppe, beispielsweise durch eine bivalente Schutzgruppe -X₂-X₃-, wie C₁-C₇-Alkyliden, insbesondere Isopropyliden, schützt, in der so geschützten Verbindung der Formel XIX 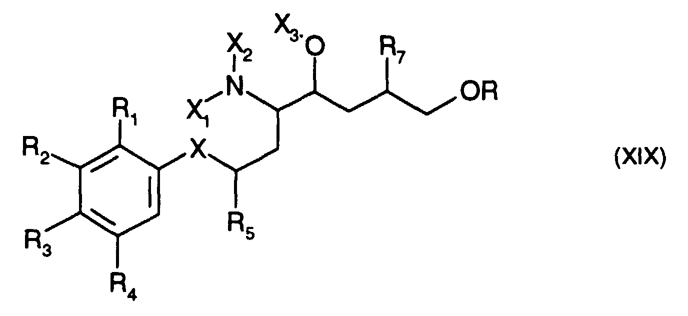 die terminale Hydroxygruppe reduktiv freisetzt und die terminale Hydroxymethylgruppe, z.B. durch Behandeln mit N-Methylmorpholin-N-oxid und Tetrabutylammoniumperruthenat in Chloroform, in Formyl überführt und den erhaltenen Aldehyd in üblicher Weise, beispielsweise durch Behandeln mit Kaliumpermanganat, zur Säure oxidiert bzw. den erhaltenen terminalen Alkohol durch geeignete Massnahmen, beispielsweise Behandeln mit Natriumjodat/Rutheniumchlorid, direkt zur Säure oxidiert und jeweils gewünschtenfalls die Carboxyfunktion reaktiv abwandelt.

Verbindungen der Formel IIc können erhalten werden, indem man eine Verbindung der Formel XV 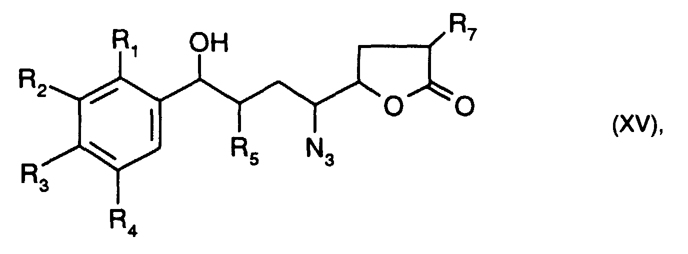 gewünschtenfalls, beispielsweise durch Umsetzung mit einem C₁-C₇-Alkansäureanhydrid, insbesondere Isobuttersäureanhydrid, in Gegenwart von Dimethylaminopyridin in Dichlormethan, an der Hydroxygruppe intermediär schützt, in der erhaltenen Verbindung der Formel XVI 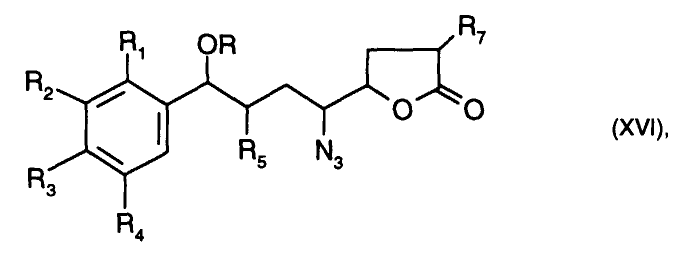 worin R Wasserstoff oder eine Hydroxyschutzgruppe, wie C₁-C₇-Alkanoyl, insbesondere Isobutyryl, bedeutet, die Azidogruppe zu Amino reduziert, beispielsweise durch katalytische Hydrierung unter Verwendung von Palladium auf Kohle, wobei die Gruppe -OR gewünschtenfalls reduktiv durch Wasserstoff ersetzt werden kann, und gegebenenfalls die Schutzgruppe X₁ einführt.

Zur Herstellung von Verbindungen der Formel IIa kann man eine Verbindung der Formel IIc in üblicher Weise unter Öffnung des Laktonringes hydrolysieren, beispielsweise durch Behandeln mit Lithiumhydroxid in einem wasserhaltigen Lösungsmittel, z.B. in DME/Wasser, gegebenenfalls die Hydroxyschutzgruppe X₃ einführen und die terminale Carboxygruppe gewünschtenfalls reaktiv abwandeln.

### Verfahrensvariante b) (Reduktion von C₁-C₇-Alkyliden- oder Arylalkyliden R'₇ zu C₁-C₇-Alkyl- bzw. Arylalkyl R₇)

In einem Ausgangsmaterial der Formel IV sind funktionelle Gruppen, die nicht an der Reaktion teilnehmen sollen, durch geeignete unter a) genannte Schutzgruppen geschützt.

Zur Hydrierung der olefinischen Doppelbindung eignen sich solche Hydrierungsmittel, die unter den Reaktionsbedingungen des Verfahrens die Doppelbindung selektiv oder schneller als die in Verbindungen der Formel IV vorhandenen Amidbindungen reduzieren.

Besonders geeignet sind Hydrierungsmittel wie Wasserstoff in Gegenwart geeigneter Katalysatoren.

Zur Hydrierung geeignete Katalysatoren sind Metalle, wie z.B. Nickel, Eisen, Cobalt oder Ruthenium, oder Edelmetalle bzw. deren Oxide, wie Palladium oder Rhodium bzw. deren Oxide, gegebenenfalls auf geeignetem Trägermaterial, wie Bariumsulfat, Aluminiumoxid oder Aktivkohle aufgezogen, oder als Skelettkatalysatoren, wie z.B. Raney-Nickel, insbesondere aber homogene oder heterogene Metall- oder Edelmetall-Ligand-Komplexe, vor allem solche die die jeweils gewünschte Konfiguration an dem die Gruppe R₄ tragendem C-Atom herbeiführen.

Solche Katalysatoren sind insbesondere Komplexe von Ruthenium oder Rutheniumsalzen, wie Ru-II-halogeniden, wie RuCl₂, Ru₂Cl₂ oder RuHCl, gegebenenfalls halogenierten Ru-II-alkanoylaten, wie Ru(OAc)₂ oder Ru(OOC-CF₃)₂, mit (S)-Bis(2,2'-diphenylphosphino)-1,1'-binaphthyl (S-BINAP) oder Derivaten davon, die anstelle von Phenyl substituierte Phenylreste, wie p-Tolyl oder p-Methoxyphenyl, enthalten, ebenso Rutheniumkomplexe mit (S)-Bis(2,2'-diphenylphosphino)-5,5'-dimethyl-diphenyl und dergl. Bei der Hydrierung mit Komplexen dieser Art arbeitet man vorzugsweise in Alkoholen, wie C₁-C₇-Alkanolen, oder Alkylhaliden, wie Methylenchlorid, im Druckbereich von etwa 1 bis 100, vorzugsweise 20 bis 30 bar und im Temperaturbereich von etwa 10° bis 80° C, vorzugsweise 15 bis 25° C.

Weitere gebräuchliche Lösungsmittel für die katalytische Hydrierung sind polare organische oder anorganische Lösungsmittel, z.B. Wasser, Alkohole, Ester, Dioxan, Eisessig oder Gemische dieser Lösungsmittel. Die Hydrierung erfolgt bei Temperaturen von 0° C bis 250° C, bevorzugt von Raumtemperatur bis etwa 100° C und bei Wasserstoffdrucken von 1 bis 200 bar. Methoden zur Hydrierung finden sich beispielsweise in "Organikum, organischchemisches Grundpraktikum", 17. durchgesehene Auflage, VEB Deutscher Verlag der Wissenschaften, Berlin 1988.

Carbonsäureamide der Formel IV werden beispielsweise erhalten, indem man einen Aldehyd der Formel XIII 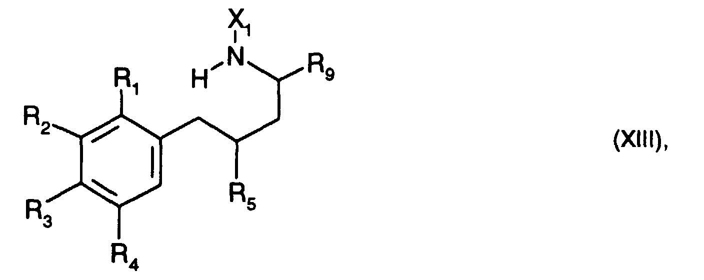 worin R₉ Formyl darstellt, in üblicher Weise mit einer geeigneten metallierten Amidverbindung, beispielsweise erhältlich durch Umsetzung einer Verbindung der Formel XX 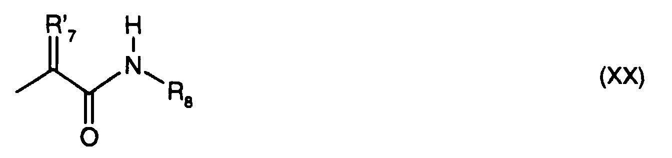 mit Butyllithium und Chlortitantriisopropyloxid, kondensiert.

### Verfahrensvariante c) (Reduktion der Azidogruppe):

In Ausgangsmaterialien der Formel V sind funktionelle Gruppen, die nicht an der Reaktion teilnehmen sollen, durch eine der unter Verfahren a) genannten Schutzgruppen geschützt.

Zur Reduktion der Azidogruppe eignen sich solche Reduktionsmittel, die unter den Reaktionsbedingungen des Verfahrens eine gegebenenfalls funktionalisierte Hydroxygruppe oder Azidogruppe selektiv oder schneller als die in Verbindungen der Formel I vorhandenen Amidgruppen reduzieren.

Die Reduktion wird vorzugsweise mit Wasserstoff in Gegenwart geeigneter Schwermetallkatalysatoren, z.B. Raney-Nickel oder Platin- oder Palladiumkatalysatoren, z.B. Platin- oder Palladium-Aktivkohle, durchgeführt.

Zwischenprodukte der Formel V können beispielsweise hergestellt werden, indem man E-1,4-Dibrombut-2-en zunächst mit einer Verbindung der Formel VII 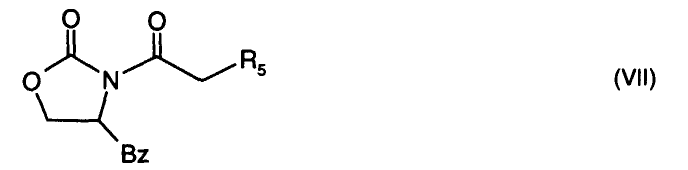 und anschliessend mit einer Verbindung der Formel XXI 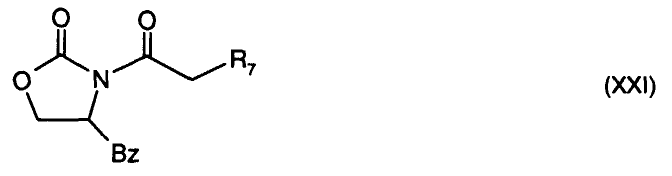 zur entsprechenden Verbindung der Formel XXII 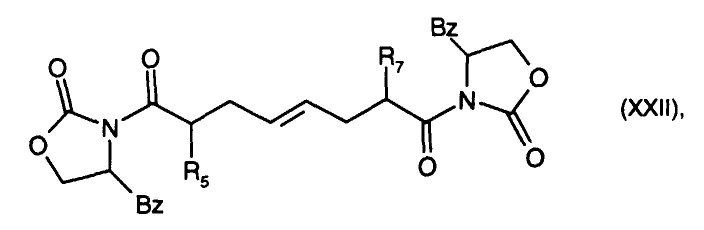 umsetzt, diese, beispielsweise durch Behandeln mit einem üblichen Halogenierungsmittel, wie elementarem Halogen, insbesondere Brom oder Jod, oder vorzugsweise mit einem N-Halogensuccinimid, insbesondere N-Bromsuccinimid, in 1,2-Dimethoxyethan (DME), in die entsprechende Verbindung der Formel XXIII 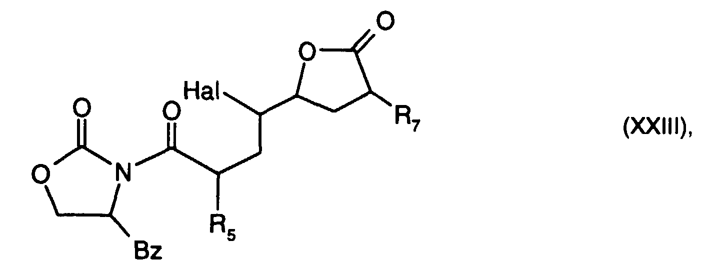 worin Hal Halogen bedeutet, überführt, das gewünschte Isomere in Bezug auf R₅ bzw. R₇ abtrennt und in diesem das Halogenatom, beispielsweise durch Behandeln mit Tetrabenzylammoniumazid in Toluol, durch Azido ersetzt und in der erhaltenen Verbindung der Formel XXIV 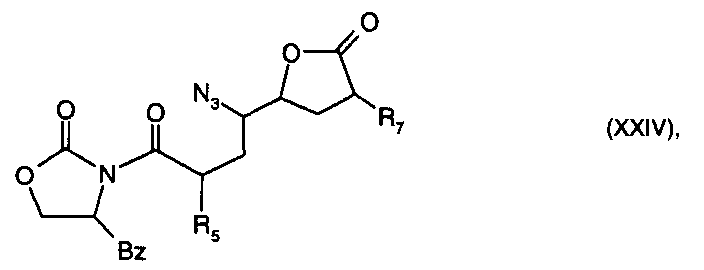 worin R₅ und R₇ die vorstehend angegebenen Bedeutungen haben und Bz Benzyl bedeutet, die 4-Benzyl-2-oxo-oxazolidin-1-ylcarbonylgruppe selektiv zu Carboxy hydrolysiert, beispielsweise durch Behandeln mit einem Alkalimetallhydroxid in Gegenwart eines basischen Hydrolysemittels, insbesondere von Lithiumhydroxid in Gegenwart von Wasserstoffperoxid, einen dabei gegebenenfalls geöffneten Laktonring unter Verwendung eines sauren Katalysators wieder schliesst, in der erhaltenen Verbindung der Formel XXV 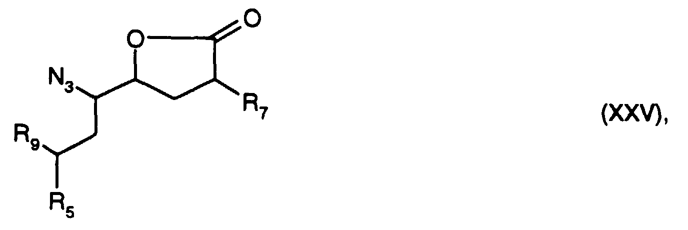 worin R₉ Carboxy ist, die Carboxygruppe, beispielsweise durch Überführung mittels Oxalylchlorid in das Säurechlorid und anschließende Reduktion der Chlorcarbonylgruppe, beispielsweise mit Natriumtritertiärbutyloxyaluminiumhydrid in Tetrahydrofuran, in Formyl umwandelt, die erhaltene Verbindung der Formel XXV, worin R₉ dann Formyl ist, mit einer Verbindung der Formel XIV 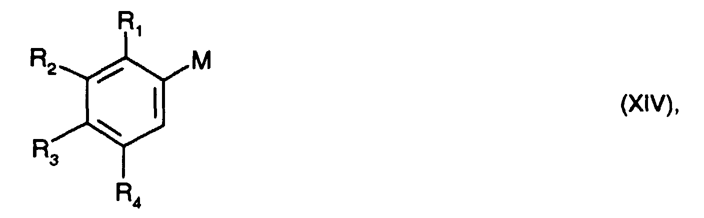 worin M einen metallischen, insbesondere erdalkalimetallischen Rest, z.B. eine Gruppe der Formel Mg-Hal (Hal = Halogen, insbesondere Brom), bedeutet, in üblicher Weise, beispielsweise in einem etherartigen Lösungsmittel, wie Tetrahydrofuran unter Kühlung, z.B. im Temperaturbereich von etwa -80° bis 0°, umsetzt, die erhaltene Verbindung der Formel XV 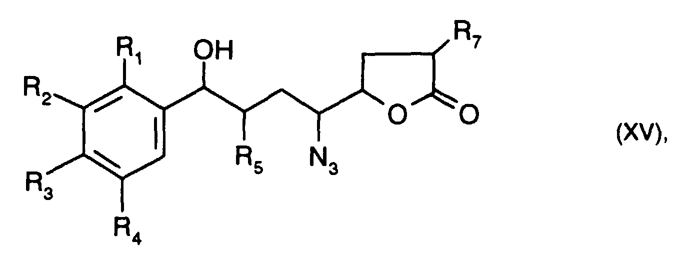 gewünschstenfalls an der Hydroxygruppe verethert oder insbesondere verestert, beispielsweise durch Umsetzung mit einem C₁-C₇-Alkansäureanhydrid, insbesondere Isobuttersäureanhydrid, in Gegenwart von Dimethylaminopyridin in Dichlormethan, an der Hydroxygruppe intermediär schützt, die erhaltene Verbindung der Formel XVI 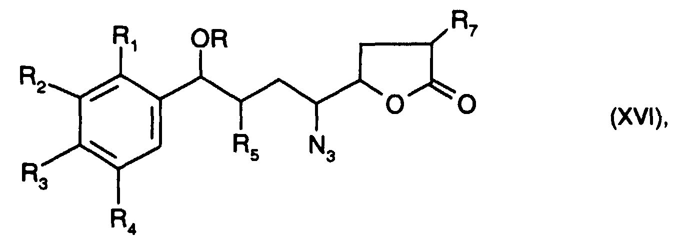 worin die Gruppe -OR eine gegebenenfalls veresterte oder veretherte Hydroxygruppe bedeutet, wobei R vorzugsweise eine Hydroxyschutzgruppe, wie insbesondere Isobutyryl, bedeutet, in üblicher Weise beispielsweise wie unter der Verfahrensvariante a) angegeben, mit einem Amin der Formel III

H₂N―R₈ (III),

worin R₈ eine der unter der Formel I angegebenen Bedeutungen hat, umsetzt und gewünschtenfalls aus der Gruppe -OR Hydroxymethyl freisetzt oder die Gruppe -OR reduktiv durch Wasserstoff ersetzt.

Die sich an die vorstehend beschriebenen Verfahrensvarianten gegebenenfalls anschliessende Abspaltung der Schutzgruppen, die nicht Bestandteil des gewünschten Endproduktes der Formel I sind, z.B. der Carboxy-, Amino-, Hydroxy- und/oder Mercaptoschutzgruppen, erfolgt in an sich bekannter Weise, z.B. mittels Solvolyse, insbesondere Hydrolyse, Alkoholyse oder Acidolyse, oder mittels Reduktion, insbesondere Hydrogenolyse oder chemischer Reduktion, sowie Photolyse, gegebenenfalls stufenweise oder gleichzeitig, wobei auch enzymatische Methoden verwendet werden können. Die Abspaltung der Schutzgruppen ist beispielsweise in den weiter vom im Abschnitt über "Schutzgruppen" genannten Standardwerken beschrieben.

So kann beispielsweise geschütztes Carboxy, z.B. tertiäres Alkoxycarbonyl, in 2-Stellung durch eine trisubstituierte Silylgruppe oder in 1-Stellung durch Alkoxy oder Alkylthio substituiertes Alkoxycarbonyl oder gegebenenfalls substituiertes Diphenylmethoxycarbonyl durch Behandeln mit einer geeigneten Säure, wie Ameisensäure oder Trifluoressigsäure, gegebenenfalls unter Zugabe einer nukleophilen Verbindung, wie Phenol oder Anisol, in freies Carboxy überführt werden. Gegebenenfalls substituiertes Benzyloxycarbonyl kann z.B. mittels Hydrogenolyse, d.h. durch Behandeln mit Wasserstoff in Gegenwart eines metallischen Hydrierungskatalysators, wie eines Palladiumkatalysators, freigesetzt werden. Ferner kann geeignet substituiertes Benzyloxycarbonyl, wie 4-Nitrobenzyloxycarbonyl, auch durch Reduktion, z.B. durch Behandeln mit einem Alkalimetalldithionit, wie Natriumdithionit, oder mit einem reduzierenden Metall, z.B. Zink, oder reduzierenden Metallsalz, wie einem Chrom-II-salz, z.B. Chrom-II-chlorid, üblicherweise in Gegenwart eines Wasserstoffabgebenden Mittels, das zusammen mit dem Metall nascierenden Wasserstoff erzeugen kann, wie einer Säure, in erster Linie einer geeigneten Carbonsäure, wie einer gegebenenfalls, z.B. durch Hydroxy, substituierten Alkancarbonsäure, z.B. Essigsäure, Ameisensäure, Glykolsäure, Diphenylglykolsäure, Milchsäure, Mandelsäure, 4-Chlormandelsäure oder Weinsäure, oder eines Alkohols oder Thiols, wobei man vorzugsweise Wasser zugibt, in freies Carboxy überführt werden. Durch Behandeln mit einem reduzierenden Metall oder Metallsalz, wie oben beschrieben, kann auch 2-Halogenalkoxycarbonyl (gegebenenfalls nach Umwandlung einer 2-Bromalkoxycarbonylgruppe in eine entsprechende 2-Iodalkoxycarbonylgruppe) oder Aroylmethoxycarbonyl in freies Carboxy umgewandelt werden. Aroylmethoxycarbonyl kann ebenfalls durch Behandeln mit einem nukleophilen, vorzugsweise salzbildenden Reagens, wie Natriumthiophenolat oder Natriumjodid, gespalten werden. 2-(trisubstituiertes Silyl)alkoxycarbenyl, wie 2-Trialkylsilylalkoxycarbonyl, kann auch durch Behandeln mit einem das Fluoridanion liefernden Salz der Fluorwasserstoffsäure, wie einem Alkalimetallfluorid, z.B. Natrium- oder Kaliumfluorid, gegebenenfalls in Anwesenheit eines makrocyclischen Polyethers ("Kronenether"), oder mit einem Fluorid einer organischen quaternären Base, wie Tetraalkylammoniumfluorid oder Trialkylarylammoniumfluorid, z.B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy überführt werden. Als organisches Silyloxycarbonyl, wie Trialkylsilyloxycarbonyl, z.B. Trimethylsilyloxycarbonyl, geschütztes Carboxy kann in üblicher Weise solvolytisch, z.B. durch Behandeln mit Wasser, einem Alkohol oder Säure, oder ausserdem Fluorid, wie oben beschrieben, freigesetzt werden. Verestertes Carboxy kann auch enzymatisch freigesetzt werden, beispielsweise durch Esterasen oder geeignete Peptidasen.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenalkoxycarbonylamino (gegebenenfalls nach Umwandlung einer 2-Bromalkoxycarbonylaminogruppe in eine 2-Iodalkoxycarbonylaminogruppe), Aroylmethoxycarbonylamino oder 4-Nitrobenzyloxycarbonylamino kann z.B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetalldithionit, z.B. Natriumdithionit, gespalten werden. Gegebenenfalls substituiertes Diphenylmethoxycarbonylamino, tert-Alkoxycarbonylamino oder 2-(trisubstituiertes Silyl)alkoxycarbenylamino, wie 2-Trialkylsilylalkoxycarbonylamino, kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierungskatalysators, wie eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser gespalten werden und eine als Silylamino geschützten Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs, und anschliessende Solvolyse, wie Alkoholyse oder Hydrolyse, des entstandenen Kondensationsproduktes freigesetzt werden. Eine durch 2-(trisubstituiertes Silyl)-alkoxycarbonyl, wie 2-Trialkylsilylalkoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechenden geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten.

In Form einer Azidogruppe geschütztes Amino wird z.B. durch Reduktion in freies Amino überführt, beispielsweise durch katalytische Hydrierung mit Wasserstoff in Gegenwart eines Hydrierungskatalysators, wie Platinoxid, Palladium oder Raney-Nickel, durch Reduktion mittels Mercaptoverbindungen, wie Dithiothreitol oder Mercaptoethanol, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20° C bis 25° C, oder auch unter Kühlen oder Erwärmen, durchgeführt.

Eine durch eine geeignete Acylgruppe, eine Trialkylsilylgruppe oder durch gegebenenfalls substituiertes 1-Phenylalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z.B. durch basische Hydrolyse, eine durch tert.-Alkyl oder durch einen 2-oxa- oder 2-thia-aliphatischen oder -cycloaliphatischen Kohlenwasserstoffrest geschützte Hydroxy bzw. Mercaptogruppe durch Acidolyse, z.B. durch Behandeln mit einer Mineralsäure oder einer starken Carbonsäure, z.B. Trifluoressigsäure, freigesetzt. Durch Pyridyldiphenylmethyl geschütztes Mercapto kann z.B. durch Quecksilber-II-salze bei pH 2-6, der durch Zink/Essigsäure oder elektrolytische Reduktion, Acetamidomethyl und iso-Butyrylamidomethyl z.B. durch Reaktion mit Quecksilber-II-salzen bei pH 2-6, 2-Chloracetamidomethyl z.B. durch 1-Piperidinothiocarboxamid, S-Ethylthio, S-tert-Butylthio und S-Sulfo z.B. durch Thiolyse mit Thiophenol, Thioglykolsäure, Natriumthiophenolat oder 1,4-Dithiothreitol freigesetzt werden. Zwei Hydroxygruppen oder eine benachbarte Amino- und Hydroxygruppe, die zusammen mittels einer bivalenten Schutzgruppe, vorzugsweise z.B. einer durch Alkyl ein- oder zweifach substituierten Methylengruppe, wie durch Alkyliden, z.B. Isopropyliden, Cycloyalkyliden, z.B. Cyclohexyliden, oder Benzyliden, geschützt sind, können durch saure Solvolyse, besonders in Gegenwart einer Mineralsäure oder einer starken organischen Säure, freigesetzt werden. 2-Halogenalkoxycarbonyl wird auch durch die oben genannten Reduktionsmittel, z.B. reduzierendes Metall, wie Zink, reduzierende Metallsalze, wie Chrom-II-salze, oder durch Schwefelverbindungen, beispielsweise Natriumdithionit oder bevorzugt Natriumsulfid und Schwefelkohlenstoff, entfernt.

Beim Vorhandensein von mehreren geschützten funktionellen Gruppen können, wenn erwünscht, die Schutzgruppen so gewählt werden, dass gleichzeitig mehr als eine solche Gruppe abgespalten werden kann, beispielsweise acidolytisch, wie durch Behandeln mit Trifluoressigsäure, oder mit Wasserstoff und einem Hydrierungskatalysator, wie einem Palladium-Kohle-Katalysator. Umgekehrt können die Gruppen auch so gewählt werden, dass sie nicht alle gleichzeitig, sondern in gewünschter Reihenfolge oder nur teilweise abgespalten werden.

Bei jedem der oben genannten Verfahren können die Ausgangsverbindungen auch als Salze verwendet werden, sofern die Reaktionsbedingungen dies zulassen.

Verfahrensgemäß erhältliche Verbindungen der Formel I können in üblicher Weise in andere Verbindungen der Formel I überführt werden.

So kann man in einer verfahrensgemäß erhältlichen Verbindung der Formel I Hydroxymethyl X reduktiv, beispielsweise durch katalytische Hydrierung in Gegenwart von Palladium auf Kohle, zu Methylen reduzieren.

Ferner kann man in einer verfahrensgemäß erhältlichen Verbindung der Formel I eine in freier oder in reaktionsfähiger Form vorliegende Carboxygruppe verestern oder amidieren bzw. eine veresterte oder amidierte Carboxygruppe in eine freie Carboxygruppe überführen.

Zur Veresterung oder Amidierung einer Carboxygruppe in einer Verbindung der Formel I kann man, wenn erwünscht, die freie Säure verwenden oder die freie Säure in eines der oben genannten reaktionsfähigen Derivate überführen und mit einem Alkohol, Ammoniak, einem primären oder einem sekundären Amin umsetzen, oder man kann zur Veresterung die freie Säure oder ein reaktionsfähiges Salz, z.B. das Cäsiumsalz, mit einem reaktionsfähigen Derivat eines Alkohols umsetzen. Beispielsweise kann man das Cäsiumsalz einer Carbonsäure mit einem dem Alkohol entsprechenden Halogenid oder Sulfonsäureester umsetzen. Die Veresterung der Carboxygruppe kann auch mit anderen üblichen Alkylierungsmitteln erfolgen, z.B. mit Diazomethan, Meerweinsalzen oder 1-substituierten 3-Aryltriazenen.

Zur Überführung einer veresterten oder amidierten Carboxygruppe in die freie Carboxygruppe kann eine der oben bei der Abspaltung der Carboxyschutzgruppen beschriebenen Methoden oder gewünschtenfalls eine alkalische Verseifung nach den im Organikum, 17. Auflage, VEB Deutscher Verlag der Wissenschaften, Berlin 1988, genannten Reaktionsbedingungen angewendet werden.

In einer verfahrensgemäß erhältlichen Verbindung der Formel I kann man eine veresterte Carboxygruppe durch Aminolyse mit Ammoniak oder einem primären oder sekundären Amin, gegebenenfalls in Gegenwart eines geeigneten Kondensationsmittels oder Katalysators, in eine gegebenenfalls substituierte Carboxamidgruppe überführen. Die Aminolyse kann nach den im Organikum, 15. Auflage, VEB Deutscher Verlag der Wissenschaften, Berlin (Ost) 1976, für derartige Umsetzungen genannten Reaktionsbedingungen erfolgen.

In einer verfahrensgemäß erhältlichen Verbindung der Formel I kann man eine vorhandene freie Aminogruppe acylieren oder alkylieren, beispielsweise zur Einführung eines Restes R₆ ausser Wasserstoff. Die Acylierung und die Alkylierung können erfolgen nach einer der für Schutzgruppen genannten Methoden oder nach einem der im Organikum, 17. Auflage, VEB Deutscher Verlag der Wissenschaften, Berlin (Ost) 1988, genannten Verfahren.

Weiterhin kann man in einer verfahrensgemäß erhältlichen Verbindung der Formel I eine vorhandene freie Hydroxygruppe, beispielsweise als Bestandteil des Restes R₈, acylieren.Die Acylierung kann erfolgen mit acylierenden Reagentien nach einer der für Schutzgruppen genannten Methoden oder nach einem der im Organikum, 17. Auflage, VEB Deutscher Verlag der Wissenschaften, Berlin (Ost) 1988, genannten Verfahren.

In einer verfahrensgemäß erhältlichen Verbindung der Formel I kann man weiterhin aus einem Sulfid das entsprechende Sulfoxid oder Sulfon herstellen, d.h. eine Thiogruppe zur Sulfinyl- oder Sulfonylgruppe bzw. ein Sulfinylgruppe zu Sulfonyl, ebenso Thiomorpholino zu S-Oxy- oder S,S-Dioxythiomorpholino oxidieren.

Die Oxidation zum Sulfon kann mit den meisten der üblichen Oxidationsmittel durchgeführt werden. Besonders bevorzugt verwendet man solche Oxidationsmittel, die die Thiogruppe oder den Sulfidschwefel selektiv in Gegenwart anderer funktioneller Gruppen der jeweiligen Verbindung der Formel I, z.B. von Amino oder Hydroxygruppen, oxidieren, beispielsweise aromatische oder aliphatische Peroxycarbonsäuren, z.B. Peroxybenzoesäure, Monoperphthalsäure, m-Chlorperbenzoesäure, Peressigsäure, Perameisensäure oder Trifluorperessigsäure. Die Oxidation mit Peroxycarbonsäuren erfolgt in den üblichen dafür geeigneten Lösungsmitteln, beispielsweise Chlorkohlenwasserstoffen, z.B. Methylenchlorid oder Chloroform, Ethern, wie Diethylether, Estern, wie Essigester oder dergleichen, bei Temperaturen zwischen -78° C und Raumtemperatur, z.B. zwischen -20° C und +10° C, bevorzugt um 0° C. Die Peroxycarbonsäure kann auch in situ gebildet werden, z.B. mit Wasserstoffperoxid in Essigsäure oder Ameisensäure, die gegebenenfalls Essigsäureanhydrid enthält, z.B. mit 30 % oder 90 % Wasserstoffperoxid in Essigsäure/Essigsäureanhydrid. Geeignet sind auch andere Peroxoverbindungen, beispielsweise Kaliumperoxomonosulfat in C₁-C₇-Alkanol/Wasser-Mischungen, z.B. Methanol/Wasser oder Ethanol/Wasser, oder in wässriger Essigsäure bei Temperaturen zwischen -70° C und +30° C, z.B. zwischen -20° C und Raumtemperatur, ferner

Natriummetaperjodat in Methanol oder Methanol/Wasser-Gemischen bei Temperaturen zwischen 0° C und 50° C, z.B. um Raumtemperatur. Bei Einsatz stöchiometrischer Mengen der genannten Oxidationsmittel können auch die entsprechenden Sulfoxide erhalten werden.

Gewünschtenfalls kann durch Reduktion einer Sulfonylgruppe oder eines Sulfonrestes in einer erhältlichen Verbindung der Formel I die entsprechende Thioverbindung oder das entsprechende Sulfid erhalten werden, beispielsweise mit Diisobutylaluminiumhydrid in Ether oder Tetrahydrofuran.

In Verbindungen der Formel I kann man ferner Hydroxy R₁, R₂, R₃ und/oder R₄ durch eine der unter den Formel I genannten veretherten Hydroxygruppen ersetzen, indem man die entsprechende Verbindung der Formel I, worin R₁, R₂, R₃ und/oder R₄ Hydroxy ist, in üblicher Weise, beispielsweise in Gegenwart eines basischen Kondensationsmittels, mit einer Verbindung der Formeln R'₁-Y, R'₂-Y, R'₃-Y und/oder R'₄-Y umsetzt, worin R'₁ C₁-C₇-Alkyl, C₁-C₄-Alkoxy-C₂-C₄-alkyl, Carboxy-C₁-C₄-alkyl, C₁-C₄-Alkoxycarbonyl-C₁-C₄-alkyl, Carbamoyl-C₁-C₄-alkyl, N-C₁-C₇-Alkylcarbamoyl-C₁-C₄-alkyl oder N,N-Di-C₁-C₄-alkylcarbamoyl-C₁-C₄-alkyl bedeutet, R'₂ C₁-C₇-Alkyl, C₁-C₄-Alkoxy-C₁-C₄-alkyl, C₁-C₄-Alkoxy-C₁-C₄-alkoxy-C₁-C₄-alkyl, 3- bis 8-gliedriges Cycloalkoxy-C₁-C₄-alkyl, C₁-C₇-Alkanoyloxy-C₂-C₄-alkyl, Hydroxy-C₂-C₇-alkyl, Halogen-C₂-C₇-(hydroxy)alkyl, C₁-C₇-Alkansulfonyl-C₁-C₄-(hydroxy)alkyl, die Alkanoylgruppe in höherer als der α-Stellung tragendes C₁-C₇-Alkanoyl-C₂-C₄-alkoxy, C₂-C₇-Alkenyl, C₁-C₄-Alkoxy-C₂-C₄-alkenyl, C₂-C₇-Alkenyloxy-C₁-C₄-alkyl, C₁-C₄-Alkylthio-C₁-C₄-alkyl, C₁-C₇-Alkansulfonyl-C₁-C₄-alkyl, C₁-C₄-Alkylthio-C₁-C₄-(hydroxy)alkyl, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl- oder Naphthyl-C₁-C₄-alkyl, gegebenenfalls hydriertes Heteroaryl-C₁-C₄-alkyl, gegebenenfalls partiell hydriertes Pyridyl- oder N-Oxidopyridyl-C₁-C₄-alkyl, Thiazolyl-C₁-C₄-alkyl, gegebenenfalls N-oxidiertes Morpholino-C₁-C₄-alkyl, Cyano-C₁-C₄-alkyl,Carboxy-C₁-C₄-alkyl, C₁-C₄-Alkoxycarbonyl-C₁-C₄-alkyl, Carbamoyl-C₁-C₇-alkyl oder N-Mono- oder N,N-Di-C₁-C₄-alkylcarbamoyl-C₁-C₄-alkyl bedeutet, R'₃ C₁-C₇-Alkyl, C₁-C₄-Alkoxy-C₁-C₄-alkyl, Hydroxy-C₂-C₇-alkyl, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl-C₁-C₄-alkyl oder Naphthyl-C₁-C₄-alkyl, Polyhalogen-C₁-C₄-alkyl, Cyano-C₁-C₄-alkyl,Carboxy-C₁-C₄-alkyl, C₁-C₄-Alkoxycarbonyl-C₁-C₄-alkyl, Carbamoyl-C₁-C₇-alkyl oder N-Mono- oder N,N-Di-C₁-C₄-alkylcarbamoyl-C₁-C₄-alkyl bedeutet, und R'₄ für C₁-C₇-Alkyl steht, und Y reaktionsfähiges verestertes Hydroxy, insbesondere mit einer Mineralsäure, mit Schwefelsäure oder mit einer organischen Sulfonsäure verestertes Hydroxy, wie Halogen, vorzugsweise Chlor, Brom oder Jod, Gruppen der Formel O-SO₂-O-R'_{A}, oder Alkan- oder gegebenenfalls substituiertes Benzolsulfonyloxy, insbesondere Methan-, Ethan-, Benzol-, p-Toluol- oder p-Brombenzolsulfonyloxy, bedeutet. Die Umsetzung erfolgt, wie erwähnt, vorzugsweise in Gegenwart eines basischen Kondensationsmittels, wie einem Alkalimetallcarbonat, beispielsweise von Kaliumcarbonat, in einem inerten Lösungsmittel, wie einem C₁-C₇-Alkanol, wie Methanol, Ethanol, Butanol, Tertiärbutanol oder insbesondere Amylalkohol, vorteilhaft bei erhöhter Temperatur, beispielsweise im Temperaturbereich von etwa 40° bis 140°C, erforderlichenfalls unter, beispielsweise azeotroper, Abdestillation des gebildeten Reaktionswassers.

Ferner kann man verfahrensgemäß erhältliche Salze von Verbindungen der Formel I in an sich bekannter Weise in die freien Verbindungen umwandeln, z.B. durch Behandeln mit einer Base, wie einem Alkalimetallhydroxid, einem Metallcarbonat oder Metallhydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäß gegebenenfalls auch die entsprechenden Salze bzw. freien Verbindungen zu verstehen.

Stereoisomerengemische, also Gemische von Diastereomeren und/oder Enantiomeren, wie beispielsweise racemische Gemische, können in an sich bekannter Weise durch geeignete Trennverfahren in die entsprechenden Isomeren aufgetrennt werden. So können Diastereomerengemische durch fraktionierte Kristallisation, Chromatographie, Lösungsmittelverteilung etc. in die einzelnen Diastereomeren aufgetrennt werden. Racemate können nach Überführung der optischen Antipoden in Diastereomere, beispielsweise durch Umsetzung mit optisch aktiven Verbindungen, z.B. optisch aktiven Säuren oder Basen, durch Chromatographie an mit optisch aktiven Verbindungen belegten Säulenmaterialien oder durch enzymatische Methoden, z.B. durch selektive Umsetzung nur eines der beiden Enantiomeren, voneinander getrennt werden. Diese Trennung kann sowohl auf der Stufe eines der Ausgangsprodukte als auch bei den Verbindungen der Formel I selbst erfolgen.

An einzelnen Chiralitätszentren in einer Verbindung der Formel I kann die Konfiguration gezielt umgekehrt werden. Beispielsweise kann man die Konfiguration asymmetrischer Kohlenstoffatome, welche nukleophile Substituenten, wie Amino oder Hydroxy, tragen, durch nukleophile Substitution zweiter Ordnung, gegebenenfalls nach Überführung des gebundenen nukleophilen Substituenten in eine geeignete nucleofuge Abgangsgruppe und Reaktion mit einem den ursprünglichen Substituenten einführenden Reagenz, umkehren, oder man kann die Konfiguration an Kohlenstoffatomen mit Hydroxygruppen durch Oxidation und Reduktion, analog dem Verfahren in der Europäischen Patentanmeldung EP-A-0 236 734, umkehren.

Vorteilhaft ist auch die reaktionsfähig funktionelle Abwandlung der Hydroxygruppe und anschliessender Ersatz derselben durch Hydroxy unter Konfigurationsumkehr. Dazu werden die in Formel I eingezeichnete Amino- und Hydroxygruppe durch eine bivalente Gruppe, insbesondere Carbonyl überbrückt, wobei man eine Verbindung der Formel XXVI 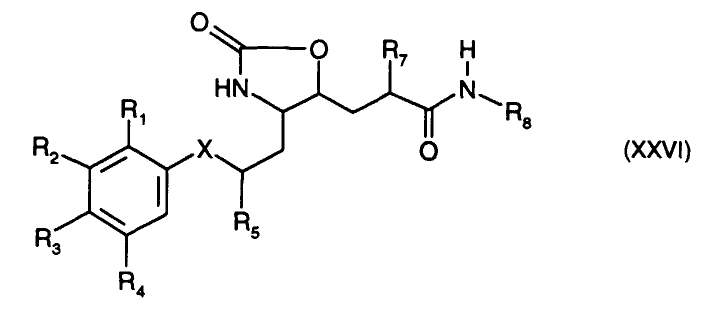 erhält, die bei Behandlung mit Thionylchlorid unter Konfigurationsumkehr wieder gespalten werden kann.

Die Erfindung betrifft auch diejenigen Ausführungsformen des Verfahrens, bei denen man von einer auf irgend einer Stufe als Zwischenprodukt erhältlichen Verbindung ausgeht und die fehlenden Schritte durchführt oder das Verfahren auf irgendeiner Stufe abbricht oder einen Ausgangsstoff unter den Reaktionsbedingungen bildet oder in Form eines reaktionsfähigen Derivates oder Salzes verwendet oder eine nach dem erfindungsgemäßen Verfahren erhältliche Verbindung unter den Verfahrensbedingungen erzeugt und in situ weiterverarbeitet. Dabei geht man vorzugsweise von solchen Ausgangsstoffen aus, die zu den oben als sehr bevorzugt oder ganz besonders bevorzugt beschriebenen Verbindungen führen.

Die neuen Ausgangsstoffe, die speziell für die Herstellung der erfindungsgemäßen Verbindungen entwickelt wurden, insbesondere die zu den eingangs als bevorzugt gekennzeichneten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Dies betrifft Verbindungen der Formel II, die sich, wie erwähnt, als Zwischenprodukte zur Herstellung von Verbindungen der Formel I eignen.

Die Erfindung betrifft dementsprechend auch Verbindungen der Formel II 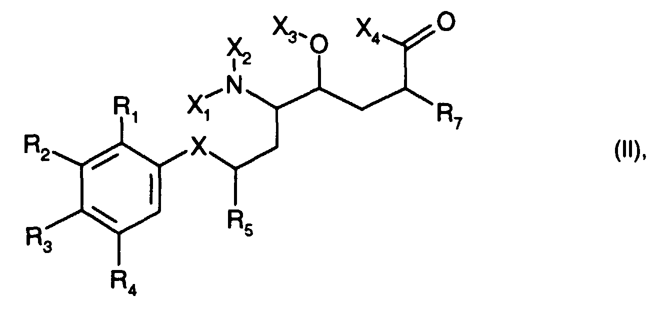 worin
R₁ Wasserstoff, Hydroxy, C₁-C₇-Alkoxy, 3- bis 8-gliedriges Cycloalkoxy, C₁-C₄-Alkoxy-C₂-C₄-alkoxy oder Carboxy-C₁-C₄-alkoxy, C₁-C₄-Alkoxycarbonyl-C₁-C₄-alkoxy, Carbamoyl-C₁-C₄-alkoxy, Carbamoyl-C₁-C₄-alkoxy, N-C₁-C₇-Alkylcarbamoyl-C₁-C₄-alkoxy oder N-Mono- oder N,N-Di-C₁-C₄-alkylcarbamoyl-C₁-C₄-alkoxy bedeutet,
R₂ Wasserstoff, C₁-C₇-Alkyl, 3- bis 8-gliedriges Cycloalkyl, C₁-C₄-Alkoxy-C₁-C₄-alkyl, C₁-C₄-Alkoxy-C₁-C₄-alkoxy-C₁-C₄-alkyl, 3- bis 8-gliedriges Cycloalkoxy-C₁-C₄-alkyl, Hydroxy, C₁-C₇-Alkanoyloxy-C₂-C₄-alkyl, Hydroxy-C₂-C₇-alkoxy, Halogen-C₂-C₇-(hydroxy)alkoxy, C₁-C₇-Alkansulfonyl-C₁-C₄-(hydroxy)alkoxy, Amino-C₁-C₄-alkyl, C₁-C₄-Alkylamino-C₁-C₄-alkyl, N,N-Di-C₁-C₄-alkylamino-C₁-C₄-alkyl, N-C₁-C₄-Alkanoylamino-C₁-C₄-alkyl, C₁-C₇-Alkoxycarbonylamino-C₂-C₇-alkyl, gegebenenfalls partiell hydriertes Pyridyl- oder N-Oxidopyridyl-C₁-C₄-alkyl, Amino-C₁-C₄-alkoxy, C₁-C₄-Alkylamino-C₁-C₄-alkoxy, N,N-Di-C₁-C₄-alkylamino-C₁-C₄-alkoxy, C₁-C₄-Alkanoylamino-C₁-C₄-alkoxy, C₁-C₇-Alkoxycarbonylamino-C₂-C₇-alkoxy, die Alkanoylgruppe in höherer als der α-Stellung tragendes C₁-C₇-Alkanoyl-C₂-C₄-alkoxy, C₁-C₇-Alkoxy, 3- bis 8-gliedriges Cycloalkoxy, C₂-C₇-Alkenyloxy, 3- bis 8-gliedriges Cycloalkoxy-C₁-C₄-alkoxy, C₁-C₄-Alkoxy-C₂-C₄-alkoxy, C₁-C₄-Alkoxy-C₂-C₄-alkenyl, C₂-C₇-Alkenyloxy-C₁-C₄-alkoxy, C₁-C₄-Alkoxy-C₂-C₄-alkenyloxy, C₂-C₇-Alkenyloxy-C₁-C₄-alkyl, C₁-C₄-Alkylthio-C₁-C₄-alkoxy, C₁-C₇-Alkansulfonyl-C₁-C₄-alkoxy, C₁-C₄-Alkylthio-C₁-C₄-(hydroxy)alkoxy, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl- oder Naphthyl-C₁-C₄-alkoxy, gegebenenfalls partiell hydriertes Pyridyl- oder N-Oxidopyridyl-C₁-C₄-alkoxy, Thiazolyl-C₁-C₄-alkoxy, gegebenenfalls N-oxidiertes Morpholino-C₁-C₄-alkoxy, Cyano-C₁-C₄-alkoxy, Carboxy-C₁-C₄-alkoxy, C₁-C₄-Alkoxycarbonyl-C₁-C₄-alkoxy, Carbamoyl-C₁-C₄-alkoxy, N-C₁-C₇-Alkylcarbamoyl-C₁-C₄-alkoxy oder N-Mono- oder N,N-Di-C₁-C₄-alkylcarbamoyl-C₁-C₄-alkoxy, Carboxy-C₁-C₄-alkyl, C₁-C₄-Alkoxycarbonyl-C₁-C₄-alkyl, Carbamoyl-C₁-C₇-alkyl oder N-Mono- oder N,N-Di-C₁-C₄-alkylcarbamoyl-C₁-C₄-alkyl bedeutet,
R₃ Wasserstoff, C₁-C₇-Alkyl, Polyhalogen-C₁-C₄-alkyl, C₁-C₄-Alkoxy-C₁-C₄-alkyl, 3- bis 8-gliedriges Cycloalkoxy-C₁-C₄-alkyl, Hydroxy-C₂-C₇-alkyl, C₁-C₄-Alkylthio-C₁-C₄-alkyl, C₁-C₇-Alkansulfonyl-C₁-C₄-alkyl, Thiazolylthio-C₁-C₄-alkyl bzw. Thiazolinylthio-C₁-C₄-alkyl, Imidazolylthio-C₁-C₄-alkyl, gegebenenfalls N-oxidiertes Pyridylthio-C₁-C₄-alkyl, Pyrimidinylthio-C₁-C₄-alkyl, gegebenenfalls partiell hydriertes Pyridyl- oder N-Oxidopyridyl-C₁-C₄-alkyl, Amino-C₁-C₄-alkyl, C₁-C₄-Alkylamino-C₁-C₄-alkyl, N,N-Di-C₁-C₄-alkylamino-C₁-C₄-alkyl, N-C₁-C₄-Alkanoylamino-C₁-C₄-alkyl, C₁-C₄-Alkansulfonylamino-C₁-C₄-alkyl, Trifluor-C₁-C₇-alkansulfonylamino-C₁-C₄-alkyl, Pyrrolidino-C₁-C₄-alkyl, Piperidino-C₁-C₄-alkyl, Piperazino-C₁-C₄-alkyl, N'-C₁-C₄-Alkylpiperazino-C₁-C₄-alkyl, N'-C₂-C₇-Alkanoylpiperazino-C₁-C₄-alkyl, Morpholino-C₁-C₄-alkyl, Thiomorpholino-C₁-C₄-alkyl, S-Oxothiomorpholino-C₁-C₄-alkyl, S,S-Dioxothiomorpholino-C₁-C₄-alkyl, Cyano-C₁-C₄-alkyl, Carboxy-C₁-C₄-alkyl, C₁-C₄-Alkoxycarbonyl-C₁-C₄-alkyl, Carbamoyl-C₁-C₇-alkyl, N-Mono- oder N,N-Di-C₁-C₄-alkylcarbamoyl-C₁-C₄-alkyl, 3- bis 8-gliedriges Cycloalkyl, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl oder Naphthyl, Hydroxy, C₁-C₄-Alkoxy, 3- bis 8-gliedriges Cycloalkoxy, C₁-C₄-Alkoxy-C₂-C₄-alkoxy, 3- bis 8-gliedriges Cycloalkoxy-C₁-C₄-alkoxy, Hydroxy-C₂-C₇-alkoxy, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl-C₁-C₄-alkoxy oder Naphthyl-C₁-C₄-alkoxy, C₁-C₄-Alkoxy, Polyhalogen-C₁-C₄-alkoxy, C₁-C₄-Alkylthio-C₁-C₄-alkoxy, C₁-C₇-Alkansulfonyl-C₁-C₄-alkoxy, gegebenenfalls partiell hydriertes Pyridyl- oder N-Oxidopyridyl-C₁-C₄-alkoxy, Thiazolyl-C₁-C₄-alkoxy, Morpholino-C₁-C₄-alkoxy, Thiazolylthio-C₁-C₄-alkoxy, Thiazolinylthio-C₁-C₄-alkoxy, Imidazolylthio-C₁-C₄-alkoxy, gegebenenfalls N-oxidiertes Pyridylthio-C₁-C₄-alkoxy, Pyrimidinylthio-C₁-C₄-alkoxy, Amino-C₁-C₄-alkoxy, C₁-C₄-Alkylamino-C₁-C₄-alkoxy, N,N-Di-C₁-C₄-alkylamino-C₁-C₄-alkoxy, C₁-C₇-Alkanoylamino-C₁-C₄-alkoxy, C₁-C₇-Alkansulfonylamino-C₁-C₄-alkoxy, Trifluor-C₁-C₇-alkansulfonyl-C₁-C₄-alkoxy, Pyrrolidino-C₂-C₄-alkoxy, Piperidino-C₁-C₄-alkoxy, Piperazino-C₁-C₄-alkyl, N'-C₁-C₄-Alkylpiperazino-C₁-C₄-alkyl, N'-C₂-C₇-Alkanoylpiperazino-C₁-C₄-alkyl, Morpholino-C₁-C₄-alkyl, Thiomorpholino-C₁-C₄-alkyl, S-Oxothiomorpholino-C₁-C₄-alkyl, S,S-Dioxothiomorpholino-C₁-C₄-alkyl, Cyano-C₁-C₄-alkoxy, Carboxy-C₁-C₄-alkoxy, C₁-C₄-Alkoxycarbonyl-C₁-C₄-alkoxy, Carbamoyl-C₁-C₇-alkoxy, N-Mono- oder N,N-Di-C₁-C₄-alkylcarbamoyl-C₁-C₄-alkoxy bedeutet oder gemeinsam mit R₄ C₁-C₄-Alkylendioxy oder einen ankondensierten Benzo- oder Cyclohexenoring darstellt,
R₄ gemeinsam mit R₃ C₁-C₄-Alkylendioxy oder einen ankondensierten Benzo- oder Cyclohexenoring darstellt oder für Wasserstoff, C₁-C₇-Alkyl, Hydroxy, C₁-C₄-Alkoxy oder 3- bis 8-gliedriges Cycloalkoxy steht, X Methylen oder Hydroxymethylen ist,
R₅ für C₁-C₇-Alkyl oder 3- bis 8-gliedriges Cycloalkyl steht,
R₇ C₁-C₇-Alkyl, C₂-C₇-Alkenyl, 3- bis 8-gliedriges Cycloalkyl oder unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl- oder Naphthyl-C₁-C₄-alkyl bedeutet,
X₁ eine Aminoschutzgruppe bedeutet, X₂ Wasserstoff oder gemeinsam mit X₃ eine bivalente Schutzgruppe darstellt,
X₃ Wasserstoff, eine Hydroxyschutzgruppe oder gemeinsam mit X₂ eine bivalente Schutzgruppe darstellt bzw. gemeinsam mit X₄ für eine direkte Bindung steht und
X₄ gegebenenfalls raktionsfähig verethertes oder verestertes Hydroxy, oder gemeinsam mit X₃ eine direkte Bindung darstellt,und ihre Salze, Verfahren zur Herstellung derselben und ihre Verwendung als Zwischenprodukte für die Herstellung von Arzneimittelwirkstoffen, insbesondere der Formel I.

In den erfindungsgemäß bereitgestellten Verbindungen der Formel II haben die Variablen R₁, R₂, R₃, R₄, X, R₅ und R₇ vorzugsweise die unter der Formel I, die Variablen X₁, X₂, X₃ und X₄ vorzugsweise die unter der Formel II angegebene Bedeutungen.

Die Erfindung betrifft in erster Linie Verbindungen der Formel II, worin
R₁ Wasserstoff, Hydroxy, C₁-C₇-Alkoxy, 3- bis 8-gliedriges Cycloalkoxy, C₁-C₄-Alkoxy-C₂-C₄-alkoxy, Carboxy-C₁-C₄-alkoxy, C₁-C₄-Alkoxycarbonyl-C₁-C₄-alkoxy, Carbamoyl-C₁-C₄-alkoxy, N-C₁-C₇-Alkylcarbamoyl-C₁-C₄-alkoxy oder N-Mono- oder N,N-Di-C₁-C₄-alkylcarbamoyl-C₁-C₄-alkoxy bedeutet,
R₂ Wasserstoff, C₁-C₇-Alkyl, 3- bis 8-gliedriges Cycloalkyl, C₁-C₄-Alkoxy-C₁-C₄-alkyl, C₁-C₄-Alkoxy-C₁-C₄-alkoxy-C₁-C₄-alkyl, 3- bis 8-gliedriges Cycloalkoxy-C₁-C₄-alkyl, Hydroxy, C₁-C₇-Alkanoyloxy-C₂-C₄-alkyl, Hydroxy-C₂-C₇-alkoxy, Halogen-C₂-C₇-(hydroxy)alkoxy, C₁-C₇-Alkansulfonyl-C₁-C₄-(hydroxy)alkoxy, Amino-C₁-C₄-alkyl, C₁-C₄-Alkylamino-C₁-C₄-alkyl, N,N-Di-C₁-C₄-alkylamino-C₁-C₄-alkyl, N-C₁-C₄-Alkanoylamino-C₁-C₄-alkyl, C₁-C₇-Alkoxycarbonylamino-C₂-C₇-alkyl, Amino-C₁-C₄-alkoxy, C₁-C₄-Alkylamino-C₁-C₄-alkoxy, N,N-Di-C₁-C₄-alkylamino-C₁-C₄-alkoxy, C₁-C₄-Alkanoylamino-C₁-C₄-alkoxy, C₁-C₇-Alkoxycarbonylamino-C₂-C₇-alkoxy, die Alkanoylgruppe in höherer als der α-Stellung tragendes C₁-C₇-Alkanoyl-C₂-C₄-alkoxy, C₁-C₇-Alkoxy, 3- bis 8-gliedriges Cycloalkoxy, C₂-C₇-Alkenyloxy-C₁-C₄-alkoxy, 3- bis 8-gliedriges Cycloalkoxy-C₁-C₄-alkoxy, C₁-C₄-Alkoxy-C₂-C₄-alkoxy, C₂-C₇-Alkenyloxy-C₁-C₄-alkyl, C₂-C₇-Alkenyloxy-C₁-C₄-alkoxy, C₁-C₄-Alkoxy-C₂-C₄-alkenyloxy, C₂-C₇-Alkenyloxy-C₁-C₄-alkyl, C₁-C₄-Alkylthio-C₁-C₄-alkoxy, C₁-C₇-Alkansulfonyl-C₁-C₄-alkoxy, C₁-C₄-Alkylthio-C₁-C₄-(hydroxy)alkoxy, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl- oder Naphthyl-C₁-C₄-alkoxy, Thiazolylthio-C₁-C₄-alkoxy, Thiazolinylthio-C₁-C₄-alkoxy, Imidazolylthio-C₁-C₄-alkoxy, gegebenenfalls N-oxidiertes Pyridylthio-C₁-C₄-alkoxy, Pyrimidinylthio-C₁-C₄-alkoxy, Cyano-C₁-C₄-alkoxy, C₁-C₄-Alkoxycarbonyl-C₁-C₄-alkoxy, Carbamoyl-C₁-C₄-alkoxy, N-C₁-C₇-Alkylcarbamoyl-C₁-C₄-alkoxy, N,N-Di-C₁-C₄-alkylcarbamoyl-C₁-C₄-alkoxy, Carboxy-C₁-C₄-alkyl, C₁-C₄-Alkoxycarbonyl-C₁-C₄-alkyl, Carbamoyl-C₁-C₇-alkyl oder N-Mono- oder N,N-Di-C₁-C₄-alkylcarbamoyl-C₁-C₄-alkyl bedeutet,
R₃ Wasserstoff, C₁-C₇-Alkyl, Polyhalogen-C₁-C₄-alkyl, C₁-C₄-Alkoxy-C₁-C₄-alkyl, 3- bis 8-gliedriges Cycloalkoxy-C₁-C₄-alkyl, Hydroxy-C₂-C₇-alkyl, C₁-C₄-Alkylthio-C₁-C₄-alkyl, C₁-C₇-Alkansulfonyl-C₁-C₄-alkyl, gegebenenfalls partiell hydriertes Pyridyl- oder N-Oxidopyridyl-C₁-C₄-alkyl, Thiazolylthio-C₁-C₄-alkyl bzw. Thiazolinylthio-C₁-C₄-alkyl, Imidazolylthio-C₁-C₄-alkyl, gegebenenfalls N-oxidiertes Pyridylthio-C₁-C₄-alkyl, Pyrimidinylthio-C₁-C₄-alkyl, Amino-C₁-C₄-alkyl, C₁-C₄-Alkylamino-C₁-C₄-alkyl, N,N-Di-C₁-C₄-alkylamino-C₁-C₄-alkyl, N-C₁-C₄-Alkanoylamino-C₁-C₄-alkyl, C₁-C₄-Alkansulfonylamino-C₁-C₄-alkyl, Trifluor-C₁-C₇-alkansulfonyl-C₁-C₄-alkyl, Pyrrolidino-C₁-C₄-alkyl, Piperidino-C₁-C₄-alkyl, Piperazino-C₁-C₄-alkyl, N'-C₁-C₄-Alkylpiperazino-C₁-C₄-alkyl, N'-C₂-C₇-Alkanoylpiperazino-C₁-C₄-alkyl, Morpholino-C₁-C₄-alkyl, Thiomorpholino-C₁-C₄-alkyl, S-Oxothiomorpholino-C₁-C₄-alkyl, S,S-Dioxothiomorpholino-C₁-C₄-alkyl, Cyano-C₁-C₄-alkyl, Carboxy-C₁-C₄-alkyl, C₁-C₄-Alkoxycarbonyl-C₁-C₄-alkyl, Carbamoyl-C₁-C₇-alkyl, N-Mono- oder N,N-Di-C₁-C₄-alkylcarbamoyl-C₁-C₄-alkyl, 3- bis 8-gliedriges Cycloalkyl, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl oder Naphthyl, Hydroxy, C₁-C₄-Alkoxy, 3- bis 8-gliedriges Cycloalkoxy, C₁-C₄-Alkoxy-C₂-C₄-alkoxy, 3- bis 8-gliedriges Cycloalkoxy-C₁-C₄-alkoxy, Hydroxy-C₂-C₇-alkoxy, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl-C₁-C₄-alkoxy oder Naphthyl-C₁-C₄-alkoxy, Polyhalogen-C₁-C₄-alkoxy, C₁-C₄-Alkylthio-C₁-C₄-alkoxy, C₁-C₇-Alkansulfonyl-C₁-C₄-alkoxy, gegebenenfalls partiell hydriertes Pyridyl- oder N-Oxidopyridyl-C₁-C₄-alkoxy, Thiazolylthio-C₁-C₄-alkoxy, Thiazolinylthio-C₁-C₄-alkoxy, Imidazolylthio-C₁-C₄-alkoxy, gegebenenfalls N-oxidierte Pyridylthio-C₁-C₄-alkoxy, Pyrimidinylthio-C₁-C₄-alkoxy, Amino-C₁-C₄-alkoxy, C₁-C₄-Alkylamino-C₁-C₄-alkoxy, N,N-Di-C₁-C₄-alkylamino-C₁-C₄-alkoxy, C₁-C₇-Alkanoylamino-C₁-C₄-alkoxy, C₁-C₇-Alkansulfonylamino-C₁-C₄-alkoxy, Trifluor-C₁-C₇-alkansulfonyl-C₁-C₄-alkoxy, Pyrrolidino-C₂-C₄-alkoxy, Piperidino-C₁-C₄-alkoxy, Piperazino-C₁-C₄-alkoxy, N'-C₁-C₄-Alkylpiperazino-C₁-C₄-alkoxy, N'-C₂-C₇-Alkanoylpiperazino-C₁-C₄-alkoxy, Morpholino-C₁-C₄-alkoxy, Thiomorpholino-C₁-C₄-alkoxy, S-Oxothiomorpholino-C₁-C₄-alkoxy, S,S-Dioxothiomorpholino-C₁-C₄-alkoxy, Cyano-C₁-C₄-alkoxy, Carboxy-C₁-C₄-alkoxy, C₁-C₄-Alkoxycarbonyl-C₁-C₄-alkoxy, Carbamoyl-C₁-C₇-alkoxy, N-C₁-C₄-alkylcarbamoyl-C₁-C₄-alkoxy oder N,N-Di-C₁-C₄-alkylcarbamoyl-C₁-C₄-alkoxy bedeutet oder gemeinsam mit R₄ C₁-C₄-Alkylendioxy oder einen ankondensierten Benzo- oder Cyclohexenoring darstellt,
R₄ gemeinsam mit R₃ C₁-C₄-Alkylendioxy oder einen ankondensierten Benzo- oder Cyclohexenoring darstellt oder für C₁-C₄-Alkyl, Hydroxy, C₁-C₄-Alkoxy oder 3- bis 8-gliedriges Cycloalkoxy steht,
X Methylen oder Hydroxymethylen bedeutet,
R₅ für C₁-C₇-Alkyl oder 3- bis 8-gliedriges Cycloalkyl steht,
R₇ C₁-C₇-Alkyl, C₂-C₇-Alkenyl, 3- bis 8-gliedriges oder unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl- oder Naphthyl-C₁-C₄-alkyl bedeutet
X₁ C₁-C₇-Alkoxycarbonyl, gegebenenfalls durch C₁-C₄-Alkyl, C₁-C₄-Alkoxy, Nitro und/oder Halogen substituiertes α-Phenyl- oder α,α-Diphenyl-C₁-C₄-alkoxycarbonyl, oder 2-Halogen-C₁-C₄-alkoxycarbonyl bedeutet,
X₂ Wasserstoff oder gemeinsam mit X₃ Carbonyl oder C₁-C₄-Alkyliden darstellt,
X₃ Wasserstoff, Tri-C₁-C₄-alkylsilyl oder gemeinsam mit X₂ Carbonyl oder C₁-C₄-Alkyliden darstellt bzw. gemeinsam mit X₄ für eine direkte Bindung steht und
X₄ C₁-C₄-Alkoxy, Phenyl-C₁-C₄-alkoxy oder Hydroxy ist oder gemeinsam mit X₃ eine direkte Bindung darstellt,
und ihre Salze.

Die Erfindung betrifft vor allem Verbindungen der Formel II, worin
R₁ Wasserstoff bedeutet,
R₂ C₁-C₇-Alkyl, C₁-C₄-Alkoxy-C₁-C₄-alkyl, C₁-C₄-Alkoxy-C₁-C₄-alkoxy, C₁-C₄-Alkoxy-C₁-C₄-alkoxy-C₁-C₄-alkyl, unsubstituiertes oder durch C₁-C₄-Alkyl, C₁-C₄-Alkoxy, Hydroxy, Halogen, Nitro und/oder Amino substituiertes Phenyl-C₁-C₄-alkoxy, Pyridyl- oder N-Oxidopyridyl-C₁-C₄-alkoxy, C₁-C₄-Alkylthio-C₁-C₄-alkoxy, C₁-C₇-Alkansulfonyl-C₁-C₄-alkoxy, C₁-C₇-Alkanoyl-C₂-C₄-alkoxy, Cyano-C₁-C₄-alkoxy, Carboxy-C₁-C₄-alkoxy, C₁-C₄-Alkoxycarbonyl-C₁-C₄-alkoxy, Carbamoyl-C₁-C₇-alkoxy oder N-Mono- oder N,N-Di-C₁-C₄-alkylcarbamoyl-C₁-C₄-alkoxy darstellt,
R₃ Wasserstoff, C₁-C₄-Alkyl, Hydroxy, C₁-C₄-Alkoxy oder Polyhalogen-C₁-C₄-alkoxy bedeutet oder gemeinsam mit R₄ C₁-C₄-Alkylendioxy darstellt,
R₄ Wasserstoff ist oder gemeinsam mit R₃ C₁-C₄-Alkylendioxy darstellt,
X Methylen oder Hydroxymethylen ist,
R₅ für C₁-C₇-Alkyl oder 3- bis 8-gliedriges Cycloalkyl steht,
R₇ C₁-C₇-Alkyl bedeutet,
X₁ C₁-C₇-Alkoxycarbonyl oder gegebenenfalls durch C₁-C₇-Alkyl, C₁-C₇-Alkoxy, Nitro und/oder Halogen substituiertes α-Phenyl-C₁-C₄-alkoxycarbonyl bedeutet,
X₂ Wasserstoff oder gemeinsam mit X₃ C₁-C₇-Alkyliden darstellt,
X₃ Wasserstoff oder gemeinsam mit X₂ C₁-C₇-Alkyliden darstellt bzw. gemeinsam mit X₄ für eine direkte Bindung steht und
X₄ Hydroxy oder gemeinsam mit X₃ eine direkte Bindung darstellt,
und ihre Salze.

Besonderer Gegenstand der Erfindung sind diejenigen Verbindungen der Formel II, worin mindestens ein, beispielsweise ein, zwei, oder vorzugsweise alle asymmetrischen C-Atome der Hauptkette die in der Formel IId 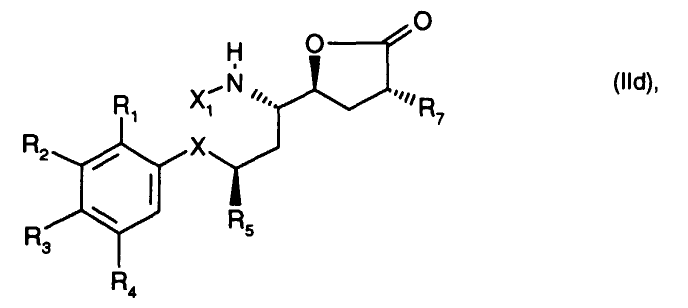 gezeigte Stereotaxie aufweist, wobei die Variablen jeweils die vorstehend angegebenen Bedeutungen haben, und ihre Salze. Die Erfindung betrifft in allererster Linie Verbindungen der Formel IId, worin
R₁ und R₄ Wasserstoff bedeuten,
R₂ für C₁-C₄-Alkoxy-C₁-C₄-alkoxy, wie 3-Methoxypropyloxy, oder C₁-C₄-Alkoxy-C₁-C₄-alkyl, wie 3-Methoxybutyl, steht,
R₃ C₁-C₄-Alkyl, wie Isopropyl oder Tertiärbutyl, oder C₁-C₄-Alkoxy, wie Methoxy, darstellt,
X Methylen ist,
R₅ und R₇ verzweigtes C₁-C₄-Alkyl, wie Isopropyl, darstellt und
X₁ C₁-C₇-Alkoxy-carbonyl, wie Tertärbutyloxycarbonyl, darstellt,
und ihre Salze.

Die Erfindung betrifft namentlich die in den Beispielen genannten Verbindungen der Formeln II und IId und ihre Salze.

Infolge der engen Beziehung zwischen den neuen Verbindungen in freier Form und in Form ihrer Salze sind vorstehend und nachfolgend unter den freien Verbindungen und ihren Salzen sinn- und zweckgemäß gegebenenfalls auch die entsprechenden Salze bzw. freien Verbindungen zu verstehen.

Die Erfindung betrifft ferner pharmazeutische Präparate enthaltende Verbindungen der Formel I.

Die pharmakologisch verwendbaren Verbindungen der vorliegenden Erfindung können z.B. zur Herstellung von pharmazeutischen Präparaten verwendet werden, welche eine wirksame Menge des Wirkstoffes zusammen oder im Gemisch mit einer signifikanten Menge von anorganischen oder organischen, festen oder flüssigen, pharmazeutisch verwendbaren Trägerstoffen enthalten.

Bei den erfindungsgemäßen pharmazeutischen Präparaten handelt es sich um solche zur enteralen, wie nasalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, dem Alter und dem individuellen Zustand, individuellen pharmakokinetischen Gegebenheiten, der zu behandelnden Krankheit sowie der Applikationsweise ab.

Die pharmazeutischen Präparate enthalten von etwa 1 % bis etwa 95 %, vorzugsweise von etwa 20 % bis etwa 90 % des Wirkstoffes. Erfindungsgemäße pharmazeutische Präparate können z.B. in Dosiseinheitsform, wie Ampullen, Vials, Suppositorien, Dragees, Tabletten oder Kapseln, vorliegen.

Die pharmazeutischen Präparate der vorliegenden Erfindung werden in an sich bekannter Weise, z.B. mittels konventioneller Lösungs-, Lyophilisierungs-, Misch-, Granulier- oder Dragierverfahren, hergestellt.

Vorzugsweise verwendet man Lösungen des Wirkstoffes, daneben auch Suspensionen, und zwar insbesondere isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Öl enthalten als ölige Komponente die für Injektionszwecke üblichen vegetabilen, synthetischen oder halbsynthetischen Öle. Als solche sind insbesondere flüssige Fettsäureester zu nennen, die als Säurekomponente eine langkettige Fettsäure mit 8-22, insbesondere 12-22, Kohlenstoffatomen, wie z.B. Laurinsäure, Tridecylsäure, Myristinsäure, Pentadecylsäure, Palmitinsäure, Margarinsäure, Stearinsäure, Arachidinsäure, Behensäure oder entsprechende ungesättigte Säuren, wie z.B. Ölsäure, Elaidinsäure, Erucasäure, Brasidinsäure oder Linolsäure, enthalten, gegebenenfalls unter Zusatz von Antioxidantien, wie z.B. Vitamin E, β-Carotin oder 3,5-Di-tert-butyl-4-hydroxytoluol. Die Alkoholkomponente dieser Fettsäureester hat maximal 6 Kohlenstoffatome und ist ein ein- oder mehrwertiger, z.B. ein-, zwei- oder dreiwertiger Alkohol, z.B. Methanol, Ethanol, Propanol, Butanol oder Pentanol oder deren Isomere, vor allem aber Glycol und Glycerin. Als Fettsäureester sind daher beispielsweise zu nennen: Ethyloleat, Isopropylmyristat, Isopropylpalmitat, "Labrafil M 2375" (Polyoxyethylenglycerintrioleat der Firma Gattefossé, Paris), "Myglyol 812" (Triglycerid gesättigter Fettsäuren der Kettenlänge C₈ bis C₁₂ der Firma Chemische Werke Witten/Ruhr, Deutschland), besonders aber vegetabile Öle wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingungen, ebenso das Abfüllen in Ampullen oder Vialen sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch, wenn erwünscht oder notwendig, nach Zugabe von geeigneten Hilfsstoffen, zu Tabletten, Dragée-Kernen oder Kapseln verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z.B. Lactose, Saccharose, Mannit oder Sorbit, Cellulosepräparate, und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die oben genannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglycol. Dragée-Keme werden mit geeigneten, gegebenenfalls magensaftresistenten Überzügen versehen, wobei man u.a. konzentrierte Zuckerlösungen, welche gegebenenfalls arabischen Gummi, Talk, Polyvinylpyrrolidon, Polyethylenglycol und/oder Titandioxid enthalten, Lacklosungen in geeigneten organischen Lösungsmitteln oder, zur Herstellung von magensaftresistenten Überzügen, Lösungen von geeigneten Cellulosepräparaten, wie Ethylcellulosephthalat oder Hydroxypropylmethylcellulosephthalat, verwendet. Kapseln sind Steckkapseln aus Gelatine sowie weiche, geschlossene Kapseln aus Gelatine und einem Weichmacher, wie Glycerin oder Sorbit. Die Steckkapseln können den Wirkstoff in Form eines Granulats, z.B. mit Füllstoffen, wie Lactose, Bindemitteln, wie Stärken, und/oder Gleitmitteln, wie Talk oder Magnesiumstearat, und gegebenenfalls mit Stabilisatoren, enthalten. In weichen Kapseln ist der Wirkstoff vorzugsweise in geeigneten öligen Hilfsstoffen, wie fetten Ölen, Paraffinöl oder flüssigen Polyethylenglykolen, gelöst oder suspendiert, wobei ebenfalls Stabilisatoren und/oder antibakterielle Mittel zugefügt sein können. Den Tabletten oder Diagée-Überzügen und den Kapselhüllen können Farbstoffe oder Pigmente, z.B. zur Identifizierung oder zur Kennzeichnung verschiedener Wirkstoffdosen, beigefügt werden.

Die Erfindung betrifft auch die Verwendung von Verbindungen der Formel I zur Behandlung von auf Reninhemmung ansprechenden Erkrankungen, wie der eingangs genannten, insbesondere von Bluthochdruck und/oder Glaukom.

Die am Warmblüter, z.B. Menschen, von beispielsweise etwa 70 kg Körpergewicht, zu verabreichenden Dosismengen, insbesondere die zur Hemmung des Enzyms Renin, zur Blutdrucksenkung und/oder zur Besserung der Glaukomsymptomatik wirksamen Dosen, liegen zwischen etwa 3 mg und etwa 3 g, vorzugsweise zwischen etwa 10 mg und etwa 1 g, z.B. bei ungefähr 20 bis 200 mg pro Person und Tag, verteilt auf vorzugsweise 1 bis 4 Einzeldosen, die z.B. gleich gross sein können. Üblicherweise erhalten Kinder etwa die halbe Dosis von Erwachsenen. Die individuell notwendige Dosierung kann z. B. durch Messung der Serumkonzentration des Wirkstoffes überwacht und optimal eingestellt werden.

Die nachfolgen Beispiele dienen zur Illustration der Erfindung; Temperaturen sind in Celsiusgraden, Drucke in mbar angegeben.
- HPLC - Säulendimension:: 250 x 4.6 mm
- HPLC - Säulenpackungen:: Nucleosil' 5C₁₈
- HPLC - Eluenten:: A) Wasser + 0.1 Vol.-% Trifluoressigsäure
B) Aceonitril + 0.1 Vol.-% Trifluoressigsäure
- HPLC - Gradient 0:: 20-100 % B in 20 Minuten + 8 Minuten 100%
- HPLC - Gradient I:: Linear in 60 Minuten von 30 Vol.-% B + 70 Vol.-% A bis 90 Vol.-% B + 10 Vol.-% A

Die Abkürzung "R_{f}(A)" bedeutet beispielsweise, dass der R_{f}-Wert im Lösungsmittelsystem A ermittelt wurde. Das Mengenverhältnis von Lösungsmitteln zueinander ist stets in Volumenanteilen angegeben.

Für die Bezeichnung der Fliessmittel-Systeme werden bei der Flash-Chromatographie und der Mitteldruckchromatographie die gleichen Abkürzungen verwendet.

Massenspektroskopische Messwerte werden entweder durch konventionelle MS oder nach der "Fast-Atom-Bombardment" (FAB-MS)-Methode erhalten. Die Massenangaben beziehen sich im ersten Fall auf das unprotonierte Molekülion (M)⁺ oder das protonierte Molekülion (M+H)⁺.

Die verwendeten Kurzbezeichnungen und Abkürzungen haben die folgenden Bedeutungen:
- C₁₈-Nucleosil': Handelsname für mit Octadecylresten belegtes "Reversed Phase"- Säulenmaterial für HPLC (Nucleosil' 5C₁₈, Macherey & Nagel, BRD)
- pFAB-MS: "Fast-Atom-Bombardment Mass-Spectroscopy"
- FC: "Flash-Chromatographie"
- HPLC: Hochleistungs-Flüssigchromatographie
- Hyflo': Handelsname für Filterhilfsmittel (Fluka, Buchs, Schweiz)
- IR: Infrarotspektroskopie
- Kp: beim in Torr angegebenen Druck
- ml: Milliliter
- MS: Massenspektroskopie
- R_{f}: Verhältnis von Laufstrecke einer Substanz zu Entfernung der Laufmittelfront vom Startpunkt bei DC
- Rₜ: Retentionszeit einer Substanz bei HPLC (in Minuten)
- Smp.: Schmelzpunkt (Temperatur).

### Beispiel 1: 2(R,S)-Methyl-4(S)-hydroxy-5(S)-amino-7(S)-isopropyl-8-(p-tertiärbutyl-phenyl)-octansäure(N-butyl)amid-hydrochlorid

111 mg N-Tertiärbutoxycarbonyl-2(R,S)-methyl-4(S)-hydroxy-5(S)-amino-7(S)-isopropyl-8-(p-tertiärbutyl-phenyl)-octansäure(N-butyl)amid werden in 2 ml 4N Salzsäure in Dioxan bei 0°C gelöst und für 60 Minuten bei 20°C gerührt. Das Reaktionsgemisch wird unter vermindertem Druck eingedampft und der Rückstand wird mittels FC (50 g Kieselgel, Dichlormethan-Methanol =9:1) gereinigt. Man erhat die Titelverbindung als Diastereomerengemisch: R_{f}(Dichlormethan-Methanol = 9:1) = 0.20; Rₜ(I) = 36.6 und 37.5 Minuten; FAB-MS (M+H)⁺ = 419.

Die Ausgangsmaterialien werden wie folgt hergestellt:

### a) N-Tertiärbutoxycarbonyl-2(R,S)-methyl-4(S)-hydroxy-5(S)-amino-7(S)-isopropyl-8-(p-tertiärbutyl-phenyl)-octansäure(N-butyl)amid

150 mg N-Tertiärbutoxycarbonyl-2-methylen-4(S)-hydroxy-5(S)-amino-7(S)-isopropyl-8-(p-tertiärbutyl-phenyl)-octansäure(N-butyl)amid (DiastereomerI) werden in Gegenwart von 150 mg Pd/C 10% in 20 ml Tetrahydrofuran für 2 Stunden bei Raumtemperatur und Normaldruck hydriert. Das Reaktionsgemisch wird filtriert und eingedampft. Der Rückstand wird mittels FC (50 g Kieselgel, Dichlormethan-Diethylether = 8:2) gereinigt. Man erhält die Titelverbindung als Diastereomerengemisch: R_{f}(Dichlormethan-Diethylether = 8:2) = 0.18.

### b) N-Tertiärbutoxycarbonyl-2-methylen-4(S)-hydroxy-5(S)-amino-7(S)-isopropyl-8-(p-tertiärbutyl-phenyl)-octansäure(N-butyl)amid

695 mg Methacrylsäurebutylamid werden in 30 ml Tetrahydrofuran gelöst und bei -75°C mit 6.2 ml 1.6 M n-Butyllithium in Hexan versetzt. Das Reaktionsgemisch wird 30 Minuten bei 0°C gerührt und anschliessend bei -75° mit 9.8 ml 1 M Chlortitantriisopropoxid in Hexan versetzt. Es wird 15 Minuten bei -75°C weitergerührt und danach bei der gleichen Temperatur die Lösung von 924 mg 2(S)-Tertiärbutoxycarbonyl-amino-4(S)-isopropyl-5-(p-tertiärbutyl-phenyl)-pentanal in 10 ml Tetrahydrofuran zugetropft. Das Reaktionsgemisch wird 15m in bei -75°C und 70 Minuten bei 0°C weitergerührt und dann hintereinander mit 15 ml 10%iger wässriger Citronensäurelösung, Wasser und Diethylether versetzt. Das Produkt wird durch mehrmalige Extraktion mit Diethylether extrahiert. Das Diastereomerengemisch wird durch FC (700 g Kieselgel, Laufmittel (Dichlormethan-Diethylether = 9:1) getrennt. Man erhält die Titelverbindung: DiastereomerI: R_{f}(Dichlormethan-Diethylether=9:1)=0.21; Diastereomer II: R_{f} (Dichlormethan-Diethylether=9:1) = 0.14.

### c) 2(S)-Tertiärbutoxycarbonylamino-4(S)-isopropyl-5-(p-tertiärbutyl-phenyl)-pentanal

Zu einer Lösung von 1 g 2(S)-tertiärbutoxycarbonylamino-4(S)-isopropyl-5-(p-tertiärbutylphenyl)-pentansäuremethylester in 20 ml Toluol wird bei -75°C 4.2 ml 1.2 M Diisobutylaluminiumhydrid-Lösung in Toluol langsam zugetropft. Das Reaktionsgemisch wird noch 30 Minuten bei -70°C weitergerührt, dann mit 10 ml Methanol versetzt, auf ein Gemisch von Eis und 10 ml 1N Salzsäure gegossen und mit Essigsäureethylester extrahiert. Man erhält die Titelverbindung: R_{f}(Dichlormethan) = 0.35.

### d) 2(S)-Tertiärbutoxycarbonylamino-4(S)-isopropyl-5-(p-tertiärbutyl-phenyl)-pentansäuremethylester

Zu einer Lösung von 2.6 g 2(S)-Amino-4(S)-isopropyl-5-(p-tertiärbutyl-phenyl)-pentansäuremethylester in 50 ml Dichlormethan werden bei 0°C 2 ml Ethyldiisopropylamin und anschliessend die Lösung von 2.4 g Di-tertiärbutyl-dicarbonat in 10 ml Dichlormethan zugetropft. Das Reaktionsgemisch wird 16 Stunden bei Raumtemperatur gerührt und dann eingedampft. Durch FC (240g Kieselgel, Laufmittel Dichlormethan) erhält man die Titelverbindung: R_{f}(Dichlormethan) = 0.50.

### e) 2(S)-Amino-4(S)-isopropyl-5-(p-tertiärbutyl-phenyl)-pentansäuremethylester Eine Lösung von 3.55 g 2(R)-isopropyl-5(S)-[2(S)isopropyl-3-(p-tertiärbutylphenyl)-propyl]-2.5-dihydro-3.6-dimethoxy-pyrazin in 36 ml Acetonitril wird unter Rühren bei Raumtemperatur mit 36 ml 1N Salzsäure versetzt und 3 Stunden weitergerührt. Danach wird die Reaktionslösung auf ein Gemisch von 45 ml gesättigter NaHCO₃-Lösung und Eis gegossen und die Suspension wird mit Dichlormethan extrahiert. Man reinigt die eingedampften Extrakte durch FC (700 g Kieselgel, Laufmittel Dichlormethan-Methanol-NH₃ = 200:10:1) und erhält die Titelverbindung: R_{f} (Dichlormethan-Methanol/Ammoniak konz.=200:10:1)=0.70.

### f) 2(R)-Isopropyl-5(S)-[2(S)-isopropyl-3-(p-tertiärbutyl-phenyl)-propyl]-2.5-dihydro-3.6-dimethoxypyrazin

Zu einer Lösung von 2.6 ml (2(R)-2.5-Dihydro-3.6-dimethoxy-2-isopropyl-pyrazin in 30ml Tetrahydrofuran werden unter Rühren bei -70°C 8.2 ml 1.6 M Butyllithium-Lösung in Hexan und nach weiteren 15 Minuten Rühren die Lösung von 2.8 g 1-Brom-2(R)-isopropyl-3-(p-tertiärbutyl-phenyl)-propen in 10 ml Tetrahydrofuran zugetropft. Das Reaktionsgemisch wird 2Stunden bei -70°C und 3 Stunden bei -25°C weitergerührt, dann 20 Stunden bei -10°C stehen gelassen und anschliessend eingedampft. Der Rückstand wird mit gesättigter Ammoniumchloridlösung und Wasser versetzt und mit Diethylether extrahiert. Die eingedampften Extrakte werden durch FC (200g Kieselgel, Laufmittel Dichlormethan-Hexan=1:1) gereinigt. Man erhält die Titelverbindung: R_{f} (Dichlormethan-Hexan=1:1) = 0.30.

### g) 1-Brom-2(R)-isopropyl-3-(p-tertiärbutyl-phenyl)-propan

Die Lösung von 2.3 g 2(R)-Isopropyl-3-(p-tertiärbutyl-phenyl)-propanol in 50 ml Dichlormethan wird unter Rühren bei 0°C mit 3.15g Triphenylphosphin und anschliessend portionenweise mit 2.14 g N-Bromsuccinimid versetzt. Danach wird das Reaktionsgemisch 16 Stunden bei Raumtemperatur gerührt und dann eingedampft. Aus dem Rückstand erhält man durch Reinigen mit FC (100g Kieselgel, Laufmittel Dichlormethan-Hexan=1:1) die Titelverbindung: R_{f} (Hexan) = 0.49.

### h) 2(R)-Isopropyl-3-(p-tertiärbutyl-phenyl)-propanol

Die Suspension von 2.41 g LiAlH₄ in 160 ml Tetrahydrofuran wird unter Rühren bei 0°C mit der Lösung von 8.63g 3-[2(R)-Isopropyl-3-(p-tertiärbutyl-phenyl)-propionyl]-4(R)-benzyloxazolidin-2-on in 40 ml Tetrahydrofuran tropfenweise versetzt. Das Reaktionsgemisch wird 4 Stunden bei 0°C weitergerührt und dann bei 0°C hintereinander mit 5 ml Essigsäureethylester, 30 ml eines Gemisches aus Tetrahydrofuran-Wasser =1:1 und danach mit 80 ml 2N Schwefelsäure versetzt.Die Suspension wird mit Essigsäureethylester extrahiert und die eingedampften Extrakte durch FC (700g Kieselgel, Laufmittel Dichlormethan) gereinigt. Man erhält die Titelverbindung: R_{f}(Dichlormethan) = 0.34; Smp.= 49°-51°C.

### i) 3-[2(R)-Isopropyl-3-(p-tertiärbutyl-phenyl)-propionyl]-4(R)benzyl-oxazolidin-2-on

Die Lösung von 31 ml 1M Lithiumhexamethyldisilazid wird mit 30 ml Tetrahydrofuran versetzt und bei -70 C gerührt. Nun wird die Lösung von 3-Isovaleroyl-4(R)-benzyloxazolidin-2-on in 20 ml Tetrahydrofuran zugetropft und das Reaktionsgemisch 1 Stunde bei -70°C weitergerührt. Danach wird die Lösung von 9.6 g p-tertiärbutylbenzylbromid in 20 ml Tetrahydrofuran zugetropft und noch 1Stunden bei -25°C und 4 Stunden bei 0°C weitergerührt. Nun wird das Reaktionsgemisch mit 6 ml gesättigter Ammoniumchoridlösung versetzt, durch Einengen vom Tetrahydrofuran befreit und mit Diethylether extrahiert. Aus dem Eindampfrückstand des Extraktes erhält man durch Reinigen mit FC (700 g Kieselgel, Laufmittel Dichlormethan-Hexan=1:1) die Titelverbindung: R_{f}(Dichlormethan-Hexan=1:1) = 0.30, Smp.= 123.5°-124°C.

### Beispiel 2: 2(R,S)-Methyl-4(S)-hydroxy-5(S)-amino-7(S)-ethyl-8-(p-tertiärbutyl-phenyl)-octansäure-(N-butyl)amid-hydrochlorid

Analog Beispiel 1 wird die Titelverbindung ausgehend von N-Tertiärbutoxycarbonyl-2(R,S)-methyl-4(S)-hydroxy-5(S)-amino-7(S)-ethyl-8(p-tertiärbutyl-phenyl)-octansäure-(N-butyl)-amid hergestellt und durch FC (20 g Kieselgel, Laufmittel Dichlormethan-Methanol =95:5) gereinigt. Titelverbindung: R_{f}(Dichlormethan-Methanol=95:5) = 0.09; Rₜ(I)=43.31 Minuten; FAB-MS (M+H)⁺ = 405.

Das Ausgangsmaterial wird analog Beispiel 1 hergestellt mit der Aenderung, dass in der Stufe i) an Stelle von 3-Isovaleroyl-4(R)-benzyl-oxazolidin-2-on das 3-Butyroyl-4(R)-benzyl-oxazolidin-2-on eingesetzt wird.

### Beispiel 3: 2(R,S)-Methyl-4(S)-hydroxy-5(S)-amino-7(S)-methyl-8-biphenyl-octansäure-(N-butyl)amid-hydrochlorid

Analog Beispiel 1 wird die Titelverbindung ausgehend von 100 mg N-Tertiärbutoxycarbonyl-2(R,S)-methyl-4(S)-hydroxy-5(S)-amino-7(S)-methyl-8-biphenyl-octansäure(N-butyl)amid hergestellt und durch FC (50 g Kieselgel, Laufmittel Dichlormethan-Methanol =9:1) gereinigt. Dies ergibt die reine Titelverbindung: R_{f} (Dichlormethan-Methanol = 9:1) = 0.11; Rₜ(I) = 29 Minuten; FAB-MS (M+H)⁺ = 411.

Das Ausgangsmaterial wird analog Beispiel 1 erhalten, mit der Aenderung, dass in Stufe i) an Stelle von 3-Isovaleroyl-4(R) benzyl-oxazolidin-2-on das 3-Propionyl-4(R)benzyl-oxazolidin-2-on und an Stelle von p-Tertiärbutyl-benzylbromid das p-Phenylbenzylbromid eingesetzt wird.

### Beispiel 4: 2(R,S)-Methyl-4(S)-hydroxy-5(S)-amino-7(S)-ethyl-8-(4-propyloxymethyl-naphth-2-yl)-octansäure(N-butyl)amid-hydrochlorid

Analog Beispiel 1 wird die Titelverbindung ausgehend von 51 mg N-Tertiärbutoxycarbonyl-2(R,S)-methyl-4(S)-hydroxy-5(S)-amino-7(S)-ethyl-8-(4-propyloxymethyl-naphth-2-yl)-octansäure(N-butyl)amid hergestellt und durch FC (15 g Kieselgel, Laufmittel Dichlormethan-Methanol = 8:2) gereinigt. Titelverbindung: R_{f} (Dichlormethan-Methanol=8:2) = 0.48; FAB-MS (M+H)⁺ = 471.

Das Ausgangsmaterial wird analog Beispiel 1 erhalten, wobei die Stufe i) wie folgt abgeändert wird:

### 3-[2(S)-Ethyl-3-(4-propyloxymethyl-naphth-2-yl)-propionyl]-4(R)-benzyl-oxazolidin-2-on:

Zu einer bei -75°C gerührten Lösung von 12 ml 1M Lithiumhexamethyldisilazid-Lösung werden nacheinander 30 ml Tetrahydrofuran und die Lösung von 2.97g 3-Butyroyl-4(R)-benzyl-oxazolidin-2-on in 15 ml Tetrahydrofuran zugetropft. Das Reaktionsgemisch wird 1Stunden bei -75°C gerührt, dann mit der Lösung von 3.52 g 4-Propoxymethyl-2-brommethylnaphthalin in 15ml Tetrahydrofuran tropfenweise versetzt und noch 1 Stunde bei -30°C und 3 Stunden bei 0°C weitergerührt. Nach dem Zutropfen von 2.7 ml gesättigter Ammoniumchloridlösung bei 0°C wird das Reaktionsgemisch eingedampft und der Rückstand zwischen Diethylether und Wasser verteilt. Aus dem Eindampfrückstand der organischen Extrakte erhält man durch Reinigen mit FC (1 kg Kieselgel, Laufmittel Dichlormethan - Hexan = 3:1) die Titelverbindung: R_{f} (Dichlormethan-Hexan = 3:1) = 0.24; FAB-MS(M+Na)⁺= 482.

### Beispiel 5: 2(R)-Methyl-4(S)-hydroxy-5(S)-amino-7(S)-isopropyl-8-(3-hydroxy-4-tertiärbutyl-phenyl)-octansäure(N-butyl)amid-hydrochlorid

30 mg N-Tertiärbutoxycarbonyl-2(R)-methyl-4(S)-hydroxy-5(S)-amino-7(S)-isopropyl-8-(3-hydroxy-4-tertiärbutyl-phenyl)-octansäure(N-butyl)amid werden mit 0.6 ml 4N Salzsäure in Dioxan analog Beispiel 1 behandelt und das Produkt mit FC (15 g Kieselgel, Dichlormethan-Methanol = 9:1) gereinigt. Man erhält die Titelverbindung: R_{f}(Dichlormethan-Methanol =9:1) = 0.17; Rₜ(I) = 28.54 Minuten; FAB-MS (M+H)⁺ = 435.

Die Ausgangsmaterialien werden wie folgt hergestellt:

### a) N-Tertiärbutoxycarbonyl-2(R)-methyl-4(S)-hydroxy-5(S)-amino-7(S)-isopropyl-8-(3-hydroxy-4-tertiärbutyl-phenyl)-octansäure(N-butyl)amid

860 mg N-Tertiärbutoxycarbonyl-2-methylen-4(S)-hydroxy-5(S)-amino-7(S)-isopropyl-8-(3-benzyloxy-4-tertiärbutyl-phenyl)-octansäure(N-butyl)amid werden in Gegenwart von 860 mg Pd/C 50% in 30 ml Methanol für 3 Stunden bei Raumtemperatur und Normaldruck hydriert. Das Reaktionsgemisch wird filtriert und eingedampft. Der Rückstand wird mittels FC (100g Kieselgel, Dichlormethan-Essigsäureethylester = 9:1) unter Trennung der Diastereomeren gereinigt. Man erhält die Titelverbindung: R_{f}(Dichlormethan-Essigsäureethylester = 8:2) = 0.23.

Das ungetrennte Diastereomerengemisch N-Tertiärbutoxycarbonyl-2(R,S)-methyl-4(S)-hydroxy-5(S)-amino-7(S)-isopropyl-8-(3-hydroxy-4-tertiärbutyl-phenyl)-octansäure(N-butyl)amid hat einen R_{f} (Essigsäureethylester-Hexan = 1:1) von 0.38.

### a') Das N-Tertiärbutoxycarbonyl-2(R)-methyl-4(S)-hydroxy-5(S)-amino-7(S)-isopropyl-8-(3-hydroxy-4-tertiärbutyl-phenyl)-octansäure-(N-butyl)amid kann auch wie folgt hergestellt werden:

175mg N-Tertiärbutoxycarbonyl-2-methylen-4(S)-hydroxy-5(S)-amino-7(S)-isopropyl-8-(3-benzyloxy-4-tertiärbutyl-phenyl)-octansäure(N-butyl)amid werden in Gegenwart von 12mg [Ru₂Cl₄(S-Binap)₂].(NEt₃) in 30ml Methanol während 20Stunden bei Raumtemperatur und 30bar hydriert. Das Reaktionsgemisch wird filtriert, eingedampft und durch FC (Hexan-Ethylacetat=1:1) gereinigt. Die so erhaltene Titelverbindung (R_{f} in Hexan/Ethylacetat=1:1)=0.15 wird durch Hydrieren mit 90mg Pd/C 10% in 10ml Methanol bei Raumtemperatur und Normaldruck zum N-Tertiärbutoxycarbonyl-2(R)-methyl-4(S)-hydroxy-5(S)-amino-7(S)-isopropyl-8-(3-hydroxy-4-tertiärbutyl-phenyl)-octansäure-(N-butyl)amid entschützt.

Das Ausgangsmaterial wird analog Beispiel 1, Stufen b) bis g) hergestellt, wobei das in Stufe g) eingesetzte 2(S)-Isopropyl-3-(3-benzyloxy-4-tertiärbutyl-phenyl)-propanol wie folgt hergestellt wird:

### h) 2(R)-Isopropyl-3-(3-benzyloxy-4-tertiärbutyl-phenyl)-propanol

Zu einer Lösung von 5.65 g 2(R)-Isopropyl-3-(3-hydroxy-4-tertiärbutyl-phenyl)-propanol in 100 ml Dimethylformamid werden bei Raumtemperatur unter Rühren 11 g Cäsiumcarbonat und tropfenweise die Lösung von 3.2 ml Benzylbromid in 20 ml Dimethylformamid zugegeben. Das Reaktionsgemisch wird 16 Stunden bei Raumtemperatur weitergerührt, eingedampft und der Rückstand zwischen Diethylether und Wasser verteilt. Aus dem Eindampfrückstand der organischen Phasen erhält man nach Reinigung mit FC (90 g Kieselgel, Dichlormethan-Hexan = 9:1) die Titelverbindung: R_{f}(Dichlormethan-Hexan = 9:1) = 0.44.

### i) 2(R)-Isopropyl-3-(3-hydroxy-4-tertiärbutyl-phenyl)-propanol

Zu einer bei 0°C gerührten Lösung von 12.3 ml Benzylmercaptan in 100 ml Tetrahydrofuran werden 49 ml einer 1.6 M Lösung von Butyllithiumin Hexan und nach 15 Minuten weiterrühren bei 0°C die Lösung von 12.1 g 3-[2(R)-Isopropyl-3-(3-acetoxy-4-tertiärbutyl-phenyl)-propanoyl]-4(R)-benzyl-oxazolidin-2-on in 100 ml Tetrahydrofuran zugetropft. Die Reaktionslösung wird 90Minuten bei 0°C weitergerührt und dann zu einer Suspension von 4.9 g LiAlH₄ in 100 ml Tetrahydrofuran bei 0°C unter Rühren zugetropft. Das Reaktionsgemisch wird 150 Minuten bei 0° C weitergerührt und dann nacheinander tropfenweise mit 26.8 ml Essigsäureethylester, 100 ml Tetrahydrofuran-Wasser=1:1 und 400 ml 2N H₂SO₄ versetzt. Das Tetrahydrofuran wird am Rotationsverdampfer entfernt und die verbleibende Suspension zwischen Diethylether und Wasser verteilt. Aus dem Eindampfrückstand der organischen Phasen erhält man nach Reinigung mit FC (300 Kieselgel, Dichlormethan-Essigsäureethylester = 9:1 und 200g Kieselgel, Essigsäureethylester-Hexan=1:2) die Titelverbindung: R_{f} (Essigsäureethylester-Hexan = 1:2) = 0.43.

### k) 3-[2(R)-Isopropyl-3-(3-Acetoxy-4-tertiärbutyl-phenyl)-propanoyl]-4(R)-benzyl-oxazolidin-2-on

Analog Beispiel 1i) erhält man die Titelverbindung ausgehend von 3-Acetoxy-4-tertiärbutyl-benzylbromid und durch Reinigung mittels FC (Kieselgel, Dichlormethan-Hexan=7:3) R_{f} (Dichlormethan-Hexan = 8:2) = 0.29.

### l) 3-Acetoxy-4-tertiärbutyl-benzylbromid

Zu einer bei 70°C gerührten Lösung von 19_g 3-Acetoxy-4-tertiärbutyl-toluol in 900 ml CCl₄ werden nacheinander 16.4 g N-Bromsuccinimid, 1g α,α'-Azoisobutyronitril und 1 g Dibenzoylperoxid zugegeben. Das Reaktionsgemisch wird 3 1/2 Stunden unter UV-Bestrahlung am Rückfluss gerührt, filtriert und das Filtrat eingedampft. Aus dem Rückstand erhält man mittels FC (900 g Kieselgel, Hexan-Essigsäureethylester=95:5) die Titelverbindung: R_{f}(Hexan-Essigsäureethylester =95:5) = 0.40.

### Beispiel 6: 2(R,S)-Methyl-4(S)-hydroxy-5(S)-amino-7(S)-isopropyl-8-(2-hydroxy-4-tertiärbutyl-phenyl)-octansäure(N-butyl)amid-hydrochlorid

Analog Beispiel 1 wird die Titelverbindung ausgehend von 20 mg N-Tertiärbutoxycarbonyl-2(R,S)-methyl-4(S)-hydroxy-5(S)-amino-7(S)-isopropyl-8-(2-hydroxy-4-tertiärbutyl-phenyl)-octansäure(N-butyl)amid durch Abspaltung der N-Tertiärbutyloxycarbonyl-Gruppe mit 4N Salzsäure in Dioxan hergestellt und mittels FC (8g Kieselgel, Dichlormethan-Methanol=9:1) gereinigt. R_{f}(Dichlormethan-Methanol = 8:2)=0.50; Rₜ(I) 28.47, 28.99 Minuten; FAB-MS (M+H)⁺=435.

Die Ausgangsmaterialien werden wie folgt hergestellt:

### a) N-Tertiärbutoxycarbonyl-2(R,S)-methyl-4(S)-hydroxy-5(S)-amino-7(S)-isopropyl-8-(2-hydroxy-4-tertiärbutyl-phenyl)-octansäure(N-butyl)amid

Die Titelverbindung wird analog Beispiel 5a) ausgehend von N-Tertiärbutoxycarbonyl-2-methylen-4(S)-hydroxy-5(S)-amino-7(S)-isopropyl-8-(2-benzyloxy-4-tertiärbutyl-phenyl)-octansäure(N-butyl)amid hergestellt und durch FC (Kieselgel, Hexan-Essigsäureethylester = 2:1, Essigsäureethylester) gereinigt: R_{f} (Hexan-Essigsäureethylester = 1:1) = 0.36.

Dieses Ausgangsmaterial wird analog Beispiel 5 erhalten, wobei das in Stufe k) einzusetzende 2-Benzyloxy-4-tertiärbutyl-benzylbromid wie folgt hergestellt wird:

### l) 2-Benzyloxy-4-tertiärbutyl-benzylbromid

Eine bei Raumtemperatur gerührte Lösung von 4 g 2-Benzyloxy-4-tertiärbutyl-benzylakohol in 100 ml Chloroform wird mit 2.9 ml Trimethylsilylbromid versetzt. Nach 1 Stunde Weiterrühren wird das Reaktionsgemisch zwischen Trichlormethan und Wasser verteilt. Aus den organischen Phasen erhält man nach Trocknen mit Na₂SO₄ und Eindampfen die Titelverbindung: R_{f}(Dichlormethan-Hexan = 8:2) = 0.95.

### m) 2-Benzyloxy-4-tertiärbutyl-benzylalkohol

Zu einer bei Raumtemperatur gerührten Suspension von 0.47 g LiAlH₄ in 40 ml Tetrahydrofuran wird die Lösung von 6.44 g 2-Benzyloxy-4-tertiärbutyl-benzoesäure-benzylester in 10 ml Tetrahydrofuran langsam zugetropft. Das Reaktionsgemisch wird 4 Stunden bei Raumtemperatur weitergerührt und dann hintereinander mit 0.96 ml Essigsäureethylester, 6.4 ml Tetrahydrofuran-Wasser = 1:1 und 9.6 ml 2N H₂SO₄ tropfenweise versetzt. Die Suspension wird zwischen Essigsäureethylester und Wasser/gesättigte Natriumchlorid-Lösung verteilt und der Eindampfrückstand der organischen Phasen mittels FC (150 g Kieselgel, Dichlormethan-Hexan=6:4) gereinigt. Titelverbindung: R_{f} (Dichlormethan-Hexan=8:2) = 0.24.

### n) 2-Benzyloxy-4-tertiärbutyl-benzoesäurebenzylester

Ein Gemisch von 5 g 2-Hydroxy-4-tertiärbutyl-benzoesäure, 9.1 ml Benzylbromid, 17 g Cesiumcarbonat, 0.3g Natriumjodid und 500 ml Aceton wird 20 Stunden am Rückfluss gerührt, dann filtriert und das Filtrat eingedampft. Der Eindampfrückstand wird zwischen Diethylether und Wasser verteilt und der Eindampfrückstand der organischen Phasen wird mittels FC (1000g Kieselgel, Dichlormethan-Hexan=1:1) gereinigt. Titelverbindung R_{f} (Dichlormethan-Hexan=1:1) = 0.47.

### Beispiel 7: 2(R)-Methyl-4(S)-hydroxy-5(S)-amino-7(S)-isopropyl-8-(3-ethoxycarbonylmethoxy-4-tertiärbutyl-phenyl)-octansäure(N-butyl)amid-hydrochlorid

Analog Beispiel 1 wird die Titelverbindung ausgehend von 62 mg N-Tertiärbutoxy-carbonyl-2(R)-methyl-4(S)-hydroxy-5(S)-amino-7(S)-isopropyl-8-(3-ethoxycarbonylmethoxy-4-tertiärbutyl-phenyl)-octansäure(N-butyl)amid hergestellt und durch FC (20 g Kieselgel, Dichlormethan -Methanol = 9:1) gereinigt. Dies ergibt die Titelverbindung: R_{f} (Dichlormethan-Methanol = 9:1) = 0.33 ; Rₜ(I) = 34.5 und 34.8 Minuten; FAB-MS (M+H)⁺ =521.

Die Ausgangsmaterialien werden wie folgt erhalten:

### a) N-Tertiärbutoxycarbonyl-2(R)-methyl-4(S)-hydroxy-5(S)-amino-7(S)-isopropyl-8-(3-ethoxycarbonylmethoxy-4-tertiärbutyl-phenyl)-octansäure(N-butyl)amid

Ein Gemisch aus 52 mg N-Tertiärbutoxycarbonyl-2(R)-methyl-4(S)-hydroxy-5(S)-amino-7(S)-isopropyl-8-(3-hydroxy-4-tertiärbutyl-phenyl)-octansäure(N-butyl)amid (Beispiel 5a), 47.5 mg Cäsiumcarbonat, 0.012 ml Jodessigsäureethylester und 5 ml Aceton wird 3 Stunden am Rückfluss gerührt und dann eingedampft. Der Rückstand wird zwischen Diethylether und Wasser verteilt. Der Eindampfrückstand der getrockneten und vereinigten organischen Phasen ergibt die Titelverbindung als Rohprodukt: R_{f} (Dichlormethan-Diethylether = 8:2) =0.28.

### Beispiel 8: 2(R)-Methyl-4(S)-hydroxy-5(S)-amino-7(S)-isopropyl-8-(3-allyloxy 4-tertiärbutyl-phenyl)-octansäure(N-butyl)amid-hydrochlorid

Analog Beispiel 1 wird die Titelverbindung ausgehend von 45 mg N-Tertiärbutoxy-carbonyl-2(R)-methyl-4(S)-hydroxy-5(S)-amino-7(S)-isopropyl-8-(3-allyloxy-4-tertiärbutyl-phenyl)-octansäure(N-butyl)amid hergestellt und durch FC (20 g Kieselgel, Dichlormethan-Methanol = 9:1) gereinigt. Dies ergibt die Titelverbindung: R_{f}(Dichlormethan-Methanol =9:1) =0.20; FAB-MS (M+H)⁺ = 475.

Das Ausgangsmaterial wird analog Beispiel 7a) unter Verwendung von Allyljodid hergestellt.

### Beispiel 9: 2(R,S)-Methyl-4(S)-hydroxy-5(S)-amino-7(S)-isopropyl-8-(3-methoxycarbonylallyloxy-4-tertiärbutyl-phenyl)-octansäure(N-butyl)amid-hydrochlorid

Analog Beispiel 1 wird die Titelverbindung ausgehend von 100 mg N-Tertiärbutoxycarbonyl-2(R,S)-methyl-4(S)-hydroxy-5(S)-amino-7(S)-isopropyl-8-(3-methoxycarbonylallyloxy-4-tertiärbutyl-phenyl)-octansäure-(N-butyl)amid hergestellt. Dies ergibt die Titelverbindung als Diastereomerengemisch: R_{f}(Dichlormethan-Methanol = 9:1) = 0.36; Rₜ(I) = 25.32 und 25.8 Minuten; FAB-MS(M+H)⁺ = 533.

Das Ausgangsmaterial wird analog Beispiel 7a) unter Verwendung von N-Tertiärbutoxycarbonyl-2(R,S)-methyl-4(S)-hydroxy-5(S)-amino-7(S)-isopropyl-8-(3-hydroxy-4-tertiärbutyl-phenyl)-octansäure(N-butyl)amid und von 4-Brom-2-butensäuremethylester hergestellt.

### Beispiel 10: 2(R,S)-Methyl-4(S)-hydroxy-5(S)-amino-7(S)-isopropyl-8-(3-methoxycarbonylmethoxy-4-tertiärbutyl-phenyl)-octansäure(N-butyl)amid-hydrochlorid

Analog Beispiel 1 wird die Titelverbindung ausgehend von 91 mg N-Tertiärbutoxy-carbonyl-2(R,S)-methyl-4(S)-hydroxy-5(S)-amino-7(S)-isopropyl-8-(3-methoxycarbonylmethoxy-4-tertiärbutyl-phenyl)-octansäure(N-butyl)amid hergestellt und durch FC (15 g Kieselgel, Essigsäureethylester-Methanol = 8:2) gereinigt. Dies ergibt die Titelverbindung als Diastereomerengemisch: R_{f}(Essigsäureethylester-Methanol = 8:2) = 0.45; Rₜ(I)=32.5 und 33.0 Minuten; FAB-MS(M+H)⁺ = 507.

Das Ausgangsmaterial wird analog Beispiel 7a) unter Verwendung von N-Tertiärbutoxy-carbonyl-2(R,S)-methyl-4(S)-hydroxy-5(S)-amino-7(S)-isopropyl-8-(3-hydroxy-4-tertiärbutyl-phenyl)-octansäure(N-butyl)amid und von Bromessigsäuremethylester hergestellt.

### Beispiel 11: 2(R,S)-Methyl-4(S)-hydroxy-5(S)-amino-7(S)-isopropyl-8-(3-carbamoyl-methoxy-4-tertiärbutyl-phenyl)-octansäure(N-butyl)amid

Analog Beispiel 1 wird die Titelverbindung ausgehend von 59 mg N-Tertiärbutoxy-carbonyl-2(R,S)-methyl-4(S)-hydroxy-5(S)-amino-7(S)-isopropyl-8-(3-carboxamidomethoxy-4-tertiärbutyl-phenyl)-octansäure(N-butyl)amid hergestellt und durch FC (20 g Kieselgel, Dichlormethan - Methanol/Ammoniak konz. = 140:10:1) gereinigt. Dies ergibt die Titelverbindung als Diastereomerengemisch: R_{f}(Dichlormethan-Methanol/Ammoniak konz. = 140:10:1) = 0.23 und 0.32; Rₜ(I) = 25.08 und 25.59 Minuten; FAB-MS(M+H)⁺ = 492.

Das Ausgangsmaterial wird analog Beispiel 7a) unter Verwendung von N-Tertiärbutoxycarbonyl-2(R,S)-methyl-4(S)-hydroxy-5(S)-amino-7(S)-isopropyl-8-(3-hydroxy-4-tertiärbutyl-phenyl)-octansäure(N-butyl)amid und von Jodacetamid hergestellt.

### Beispiel 12: 2(R,S)-Methyl-4(S)-hydroxy-5(S)-amino-7(S)-isopropyl-8-[3-(pyrid-2-yl-methoxy)-4-tertiärbutyl-phenyl]-octansäure(N-butyl)amid-hydrochlorid

Analog Beispiel 1 wird die Titelverbindung ausgehend von 40 mg N-Tertiärbutoxy-carbonyl-2(R,S)-methyl-4(S)-hydroxy-5(S)-amino-7(S)-isopropyl-8-[3-(pyrid-2-ylmethoxy)-4-tertiärbutyl-phenyl]-octansäure(N-butyl)amid hergestellt. Dies ergibt die Titelverbindung als Diastereomerengemisch: R_{f}(Dichlormethan-Methanol = 9:1) = 0.32; Rₜ(I) =24.52 und 25.19 Minuten; FAB-MS(M+H)⁺ = 526.

Das Ausgangsmaterial wird analog Beispiel 7a) unter Verwendung von N-Tertiärbutoxy-carbonyl-2(R,S)-methyl-4(S)-hydroxy-5(S)-amino-7(S)-isopropyl-8-(3-hydroxy-4-tertiärbutyl-phenyl)-octansäure(N-butyl)amid und von 2-Picolylchlorid-hydrochlorid hergestellt.

### Beispiel 13: 2(R,S)-Methyl-4(S)-hydroxy-5(S)-amino-7(S)-isopropyl-8-[3-(pyrid-4-yl-methoxy)-4-tertiärbutyl-phenyl]-octansäure(N-butyl)amid-hydrochlorid

Analog Beispiel 1 wird die Titelverbindung ausgehend von 46 mg N-Tertiärbutoxy-carbonyl-2(R,S)-methyl-4(S)-hydroxy-5(S)-amino-7(S)-isopropyl-8-[3-(pyrid-4-yl-methoxy)-4-tertiärbutyl-phenyl]-octansäure(N-butyl)amid hergestellt. Dies ergibt die Titelverbindung als Diastereomerengemisch: R_{f}(Dichlormethan-Methanol = 9:1) = 0.17; Rₜ(I) = 20.27 und 20.62Minuten; FAB-MS(M+H)⁺ = 526.

Das Ausgangsmaterial wird analog Beispiel 7a) unter Verwendung von N-Tertiärbutoxy-carbonyl-2(R,S)-methyl-4(S)-hydroxy-5(S)-amino-7(S)-isopropyl-8(3-hydroxy-4-tertiärbutyl-phenyl)-octansäure(N-butyl)amid und von 4-Picolylchlorid-hydrochlorld hergestellt.

### Beispiel 14: 2(R,S)-Methyl-4(S)-hydroxy-5(S)-amino-7(S)-isopropyl-8-[3-(N-oxido-pyrid-2-yl-methoxy)-4-tertiärbutyl-phenyl]-octansäure(N-butyl)amid-hydrochlorid

Analog Beispiel 1 wird die Titelverbindung ausgehend von 35 mg N-Tertiärbutoxy-carbonyl-2(R,S)-methyl-4(S)-hydroxy-5(S)-amino-7(S)-isopropyl-8-[3-(N-oxido-pyrid-2-yl-methoxy)-4-tertiärbutyl-phenyl]-octansäure(N-butyl)amid hergestellt. Dies ergibt die Titelverbindung als Diastereomerengemisch: R_{f}(Dichlormethan-Methanol = 9:1) = 0.14; Rₜ(I) =31.06 und 31.6 Minuten; FAB-MS(M+H)⁺ = 542.

Das Ausgangsmaterial wird analog Beispiel 7a) unter Verwendung von N-Tertiärbutoxycarbonyl-2(R,S)-methyl-4(S)-hydroxy-5(S)-amino-7(S)-isopropyl-8(3-hydroxy-4-tertiärbutyl-phenyl)-octansäure-N-(butyl)amid und von 2-Picolylchlorid-N-oxid hergestellt.

### Beispiel 15: 2(R,S)-Methyl-4(S)-hydroxy-5(S)-amino-7(S)-isopropyl-8-[3-(2-ethoxycarbonylallyloxy)-4-tertiärbutyl-phenyl]-octansäure(N-butyl)amid-hydrochlorid

Analog Beispiel 1 wird die Titelverbindung ausgehend von 30 mg N-Tertiärbutoxy-carbonyl-2(R,S)-methyl-4(S)-hydroxy-5(S)-amino-7(S)-isopropyl-8-[3-(2-ethoxycarbonylallyloxy)-4-tertiärbutyl-phenyl]-octansäure(N-butyl)amid hergestellt. Dies ergibt die Titelverbindung als Diastereomerengemisch: R_{f}(Dichlormethan-Methanol = 9:1) = 0.28; Rₜ(I) = 39.3 und 39.8 Minuten; FAB-MS(M+H)⁺ = 547.

Das Ausgangsmaterial wird analog Beispiel 7a) unter Verwendung von N-Tertiärbutoxy-carbonyl-2(R,S)-methyl-4(S)-hydroxy-5(S)-amino-7(S)-isopropyl-8-(3-hydroxy-4-tertiärbutyl-phenyl)-octansäure(N-butyl)amid und von Brommethylacrylsäureethylester hergestellt.

### Beispiel 16: 2(R,S)-Methyl-4(S)-hydroxy-5(S)-amino-7(S)-isopropyl-8-[3-(2-ethoxycarbonyl-propyloxy)-4-tertiärbutyl-phenyl]-octansäure(N-butyl)amid-hydrochlorid

Analog Beispiel 1 wird die Titelverbindung ausgehend von 9 mg N-Tertiärbutoxy-carbonyl-2(R,S)-methyl-4(S)-hydroxy-5(S)-amino-7(S)-isopropyl-8-[3-(2-ethoxycarbonyl-propyloxy)-4-tertiärbutyl-phenyl]-octansäure(N-butyl)amid hergestellt. Dies ergibt die Titelverbindung als Diastereomerengemisch: R_{f}(Dichlormethan-Methanol = 9:1) = 0.25; Rₜ(I) =38.5 ; 39.0 ; 39.6 und 40.2 Minuten; FAB-MS(M+H)⁺ = 549.

Das Ausgangsmaterial wird durch Hydrieren von N-Tertiärbutoxycarbonyl-2(R,S)-methyl-4(S)-hydroxy-5(S)-amino-7(S)-isopropyl-8-[3-(2-ethoxycarbonylallyloxy)-4-tertiärbutyl-phenyl]-octansäure(N-butyl)amid (Beispiel 15) mit Raney-Ni in Ethanol bei Raumtemperatur und 2 bar H₂ hergestellt: R_{f}(Essigsäureethylester-Hexan=1:2) = 0.16.

### Beispiel 17: 2(R,S)-Methyl-4(S)-hydroxy-5(S)-amino-7(S)-isopropyl-8-[3-(methylthio-methoxy)-4-tertiärbutyl-phenyl]-octansäure(N-butyl)amid-hydrochlorid

Analog Beispiel 1 wird die Titelverbindung ausgehend von 10 mg N-Tertiärbutoxycarbonyl-2(R,S)-methyl-4(S)-hydroxy-5(S)-amino-7(S)-isopropyl-8-[3-(methylthio-methoxy)-4-tertiärbutyl-phenyl]-octansäure-(N-butyl)amid hergestellt. Dies ergibt die Titelverbindung als Diastereomerengemisch: R_{f}(Dichlormethan-Methanol = 9:1) = 0.2; Rₜ(I) =29.32 und 29.56 Minuten; FAB-MS(M+H)⁺ = 495.

Das Ausgangsmaterial wird wie folgt hergestellt:

### a) N-Tertiärbutoxycarbonyl-2(R,S)-methyl-4(S)-hydroxy-5(S)-amino-7(S)-isopropyl-8-[3-(methylthio-methoxy)-4-tertiärbutyl-phenyl]-octansäure(N-butyl)amid

Zu einer bei Raumtemperatur gerührten Suspension von 7.6 mg einer 65%-igen NaH-Dispersion in 3 ml Dimethylformamid wird die Lösung von 100 mg N-Tertiärbutoxycarbonyl-2(R,S)-methyl-4(S)-hydroxy-5(S)-amino-7(S)-isopropyl-8-(3-hydroxy-4-tertiärbutyl-phenyl)-octansäure(N- butyl)amid (Beispiel 5a) in 5 ml Dimethylformamid zugetropft. Das Reaktionsgemisch wird 30 Minuten bei Raumtemperatur weitergerührt, dann mit der Lösung von 0.017 ml Chlordimethylsulfid in 2 ml Dimethylformamid versetzt, noch 24 Stunden weitergerührt und dann eingedampft. Der Rückstand wird zwischen Ether und Wasser verteilt. Aus dem Eindampfrückstand der organischen Phasen erhält man mit FC (12 g Kieselgel, Dichlormethan-Diethylether=2:1) die Titelverbindung: R_{f} (Dichlormethan-Diethylether = 2:1) = 0.33.

### Beispiel 18: 2(R,S)-Methyl-4(S)-hydroxy-5(S)-amino-7(S)-isopropyl-8-[3-(methylsulfonyl-methoxy)-4-tertiärbutyl-phenyl]-octansäure(N-butyl)amid-hydrochlorid

Analog Beispiel 1 wird die Titelverbindung ausgehend von 15 mg N-Tertiärbutoxy-carbonyl-2(R,S)-methyl-4(S)-hydroxy-5(S)-amino-7(S)-isopropyl-8-[3-(methylsulfonyl-methoxy)-4-tertiärbutyl-phenyl]-octansäure(N-butyl)amid hergestellt. Dies ergibt die Titelverbindung als Diastereomerengemisch: R_{f}(Dichlormethan-Methanol = 9:1) = 0.75; Rₜ(I) = 28.3 und 28.76 Minuten; FAB-MS(M+H)⁺ = 527.

Das Ausgangsmaterial wird wie folgt hergestellt:

### a) N-Tertiärbutoxycarbonyl-2(R,S)-methyl-4(S)-hydroxy-5(S)-amino-7(S)-isopropyl-8-[3-(methylsulfonyl-methoxy)-4-tertiärbutyl-phenyl)-octansäure(N-butyl)amid

Eine Lösung von 74 mg N-Tertiärbutoxycarbonyl-2(R,S)-methyl-4(S)-hydroxy-5(S)-amino-7(S)-isopropyl-8-[3-(methylthio-methoxy)-4-tertiärbutyl-phenyl]-octansäure(N-butyl)amid in 0.5 ml Methanol wird bei 0°C unter Rühren tropfenweise mit der Lösung von 115 mg Kaliummonopersulfat-tripelsalz in 0.5 ml Wasser versetzt und 20 Stunden bei Raumtemperatur weitergerührt. Das Reaktionsgemisch wird zwischen Dichlormethan und Wasser verteilt. Aus dem Eindampfrückstand der organischen Phasen erhält man durch FC (11 g Kieselgel, Essigsäureethylester-Hexan = 1:1) die Titelverbindung: R_{f} (Essigsäureethylester-Hexan = 1:1) = 0.26; FAB-MS (M+H)⁺ = 627.

### Beispiel 19: 2(R,S)-Methyl-4(S)-hydroxy-5(S)-amino-7(S)-isopropyl-8-[3-(carboxy-methoxy)-4-tertiärbutyl-phenyl]-octansäure-(N-butyl)amid-hydrochlorid

Analog Beispiel 1 wird die Titelverbindung ausgehend von 28 mg N-Tertiärbutoxy-carbonyl-2(R,S)-methyl-4(S)-hydroxy-5(S)-amino-7(S)-isopropyl-8-[3-(carboxy-methoxy)-4-tertiärbutyl-phenyl]-octansäure(N-butyl)amid hergestellt. Dies ergibt die Titelverbindung als Diastereomerengemisch: R_{f}(Dichlormethan-Methanol = 9:1) = 0.26; Rₜ(I) = 26.1 und 28.0 Minuten; FAB-MS(M+H)⁺ = 493.

Das Ausgangsmaterial wird analog Beispiel 7a) unter Verwendung von N-Tertiärbutoxy-carbonyl-2(R,S)-methyl-4(S)-hydroxy-5(S)-amino-7(S)-isopropyl-8-(3-hydroxy-4-tertiärbutyl-phenyl)-octansäure(N-butyl)amid und von Bromessigsäurebenzylester mit anschliessender hydrolytischer Abspaltung (Pd/C-Ethanol) der Benzylgruppe hergestellt.

### Beispiel 20: 2(R,S)-Methyl-4(S)-hydroxy-5(S)-amino-7(S)-isopropyl-8-[3-(3.3-dimethyl-2-oxo-butyloxy)-4-tertiärbutyl-phenyl]-octansäure(N-butyl)amid-hydrochlorid

Analog Beispiel 1 wird die Titelverbindung ausgehend von 42 mg N-Tertiärbutoxy-carbonyl-2(R,S)-methyl-4(S)-hydroxy-5(S)-amino-7(S)-isopropyl-8-[3-(3.3-dimethyl-2-oxo-butyloxy)-4-tertiärbutyl-phenyl]-octansäure(N-butyl)amid hergestellt. Dies ergibt die Titelverbindung als Diastereomerengemisch: R_{f}(Dichlormethan-Methanol = 9:1) = 0.3; Rₜ(I) =37.3 und 37.8 Minuten; FAB-MS(M+H)⁺ = 533.

Das Ausgangsmaterial wird analog Beispiel 17a) unter Verwendung von N-Tertiärbutoxy-carbonyl-2(R,S)-methyl-4(S)-hydroxy-5(S)-amino-7(S)-isopropyl-8(3-hydroxy-4-tertiärbutyl-phenyl)-octansäure(N-butyl)amid und von 1-Brompinakolin hergestellt.

### Beispiel 21: 2(R,S)-Methyl-4(S)-hydroxy-5(S)-amino-7(S)-isopropyl-8-[3-(2-nitrobenzyloxy)-4-tertiärbutyl-phenyl]-octansäure(N-butyl)amid-hydrochlorid

Analog Beispiel 1 wird die Titelverbindung ausgehend von 53 mg N-Tertiärbutoxy-carbonyl-2(R,S)-methyl-4(S)-hydroxy-5(S)-amino-7(S)-isopropyl-8-[3-(2-nitrobenzyloxy)-4-tertiärbutyl-phenyl]-octansäure(N-butyl)amid hergestellt. Dies ergibt die Titelverbindung als Diastereomerengemisch: R_{f}(Dichlormethan-Methanol = 9:1) = 0.35; Rₜ(I) = 52.0 und 52.4 Minuten; FAB-MS(M+H)⁺ = 570.

Das Ausgangsmaterial wird analog Beispiel 7a) unter Verwendung von N-Tertiärbutoxy-carbonyl-2(R,S)-methyl-4(S)-hydroxy-5(S)-amino-7(S)-isopropyl-8-(3-hydroxy-4-tertiärbutyl-phenyl)-octansäure(N-butyl)amid und von 2-Nitrobenzylchlorid hergestellt.

### Beispiel 22: 2(R,S)-Methyl-4(S)-hydroxy-5(S)-amino-7(S)-isopropyl-8-[3-(2-aminobenzyloxy)-4-tertiärbutyl-phenyl]-octansäure(N-butyl)amid-hydrochlorid

Die Titelverbindung wird ausgehend von 35 mg 2(R,S)-Methyl-4(S)-hydroxy-5(S)-amino-7(S)-isopropyl-8-[3-(2-nitrobenzyloxy)-4-tertiärbutyl-phenyl]-octansäure(N-butyl)amid (Beispiel 21) durch Hydrieren mit Pt/C in Tetrahydrofuran bei Raumtemperatur und Normaldruck hergestellt und durch FC (10 g Kieselgel, Dichlormethan-Methanol = 9:1) gereinigt. Dies ergibt die Titelverbindung als Diastereomerengemisch: R_{f}(Dichlormethan-Methanol = 9:1) = 0.27; Rₜ(I) = 30.5 und 3l,3 Minuten; FAB-MS(M+H)⁺ = 539.

### Beispiel 23: 2(R,S)-Methyl-4(S)-hydroxy-5(S)-amino-7(S)-isopropyl-8-[3-(3-chlor-2(R,S)-hydrox-propyloxy)-4-tertiärbutyl-phenyl]-octansäure(N-butyl)amid-hydrochlorid

Analog Beispiel 1 wird die Titelverbindung ausgehend von 31 mg N-Tertiärbutoxy-carbonyl-2(R,S)-methyl-4(S)-hydroxy-5(S)-amino-7(S)-isopropyl-8-[3-(2.3-epoxypropyloxy)-4-tertiärbutyl-phenyl]-octansäure(N-butyl)amid hergestellt. Dies ergibt die Titelverbindung als Diastereomerengemisch: R_{f}(Dichlormethan-Methanol = 9:1) = 0.18; Rₜ(I) = 31.9 und 32.3 Minuten; FAB-MS(M+H)⁺ = 527.

Das Ausgangsmaterial wird analog Beispiel 7a) unter Verwendung von N-Tertiärbutoxy-carbonyl-2(R,S)-methyl-4(S)-hydroxy-5(S)-amino-7(S)-isopropyl-8-(3-hydroxy-4-tertiärbutyl-phenyl)-octansäure(N-butyl)amid und von Epibromhydrin hergestellt.

### Beispiel 24: 2(R,S)-Methyl-4(S)-hydroxy-5(S)-amino-7(S)-isopropyl-8-[3-(3-methylthio-2(S,R)-hydroxypropyloxy)-4-tertiärbutyl-phenyl]-octansäure(N-butyl)amid-hydrochlorid

Analog Beispiel 1 wird die Titelverbindung ausgehend von 15 mg N-Tertiärbutoxy-carbonyl-2(R,S)-methyl-4(S)-hydroxy-5(S)-amino-7(S)-isopropyl-8-[3-(3-methylthio-2(S,R)-hydroxy-propyloxy)-4-tertiärbutyl-phenyl]-octansäure(N-butyl)amid hergestellt. Dies ergibt die Titelverbindung als Diastereomerengemisch: R_{f}(Dichlormethan-Methanol = 9:1) =0.32; Rₜ(I) = 32.6 und 32.9 Minuten; FAB-MS(M+H)⁺ = 539.

Das Ausgangsmaterial wird wie folgt hergestellt:

### a) N-Tertiärbutoxycarbonyl-2(R,S)-methyl-4(S)-hydroxy-5(S)-amino-7(S)-isopropyl-8-[3-(3-methylthio-2(S,R)-hydroxypropyloxy)-4-tertiärbutyl-phenyl]-octansäure(N-butyl)amid

Eine Lösung von 150 mg N-Tertiärbutoxycarbonyl-2(R,S)-methyl-4(S)-hydroxy-5(S)-amino-7(S)-isopropyl-8-[3-(2.3-epoxypropyloxy)-4-tertiärbutyl-phenyl]-octansäure(N-butyl)amid in 10 ml Methanol wird mit 18 mg Natriummethanthiolat versetzt und 7 Stunden am Rückfluss gehalten. Das Reaktionsgemisch wird eingedampft und der Rückstand zwischen Dichlormethan und Wasser verteilt. Aus dem Eindampfrückstand der organischen Phasen erhält man nach Reinigung mit FC (20 g Kieselgel, Dichlormethan-Diethylether = 1:1) die Titelverbindung: R_{f}(Dichlormethan-Diethylether = 1:1) = 0.33; FAB-MS (M+H)⁺ = 639.

### Beispiel 25: 2(R,S)-Methyl-4(S)-hydroxy-5(S)-amino-7(S)-isopropyl-8-[3-(3-methylsulfonyl-2(S,R)-hydroxypropyloxy)-4-tertiärbutyl-phenyl]-octansäure(N-butyl)amid-hydrochlorid

Analog Beispiel 1 wird die Titelverbindung ausgehend von 14 mg N-Tertiärbutoxy-carbonyl-2(R,S)-methyl-4(S)-hydroxy-5(S)-amino-7(S)-isopropyl-8-[3-(3-methylsulfonyl-2(S,R)-hydroxypropyloxy)-4-tertiärbutyl-phenyl]-octansäure(N-butyl)amid hergestellt. Dies ergibt die Titelverbindung als Diastereomerengemisch: R_{f}(Dichlormethan-Methanol = 9:1) =0.16; Rₜ(I) = 26.3 und 26.8 Minuten; FAB-MS(M+H)⁺ = 571.

Das Ausgangsmaterial wird analog Beispiel 18a) unter Verwendung von 62 mg N-Tertiärbutoxycarbonyl-2(R,S)-methyl-4(S)-hydroxy-5(S)-amino-7(S)-isopropyl-8-[3-(3-methylthio-2(S,R)-hydroxypropyloxy)-4-tertiärbutyl-phenyl]-octansäure(N-butyl)amid hergestellt: R_{f} (Essigsäureethylester) = 0.60; FAB-MS (M+H)⁺ = 671.

### Beispiel 26: 2(R)-Methyl-4(S)-hydroxy-5(S)-amino-7(S)-isopropyl-8-[3-(methylsulfonyl-methoxy)-4-tertiärbutyl-phenyl]-octansäure(N-3-morpholino-propyl)amid-hydrochlorid

Analog Beispiel 1 wird die Titelverbindung ausgehend von 18 mg N-Tertiärbutoxy-carbonyl-2(R)-methyl-4(S)-hydroxy-5(S)-amino-7(S)-isopropyl-8-(3-methylsulfonyl-methoxy)-4-tertiärbutyl-phenyl)-octansäure-(N-3-morpholino-propyl)amid hergestellt. Dies ergibt die Titelverbindung: R_{f}(Dichlormethan-Methanol = 8:2) = 0.16; Rₜ(I) = 17.61 Minuten; FAB-MS(M+H)⁺ = 598.

Das Ausgangsmaterial wird analog Beispiel 17a) und 18a) unter Verwendung von N-Tertiärbutoxycarbonyl-2(R)-methyl-4(S)-hydroxy-5(S)-amino-7(S)-isopropyl-8-(3-hydroxy-4-tertiärbutyl-phenyl)-octansäure(N-3-morpholino-propyl)amid und von Chlordimethylsulfid hergestellt.

Das N-Tertiärbutoxycarbonyl-2(R)-methyl-4(S)-hydroxy-5(S)-amino-7(S)-isopropyl-8-(3-hydroxy-4-tertiärbutyl-phenyl)-octansäure(N-3-morpholino-propyl)amid wird analog Beispiel 1) hergestellt, wobei in Stufe 1b) an Stelle von Methacrylsäurebutylamid das Methacrylsäure(N-3-morpholino-propyl)amid eingesetzt wird.

### Beispiel 27: 2(R)-Methyl-4(S)-hydroxy-5(S)-amino-7(S)-isopropyl-8-(3-methoxycarbonyl-methoxy-phenyl)-octansäure(N-butyl)amid-hydrochlorid

Analog Beispiel 1 wird die Titelverbindung ausgehend von 12 mg N-Tertiärbutoxy-carbonyl-2(R)-methyl-4(S)-hydroxy-5(S)-amino-7(S)-isopropyl-8-(3-methoxycarbonyl-methoxy-phenyl)-octansäure(N-butyl)amid hergestellt. Dies ergibt die Titelverbindung: R_{f}(Dichlormethan-Methanol = 9:1) = 0.18; Rₜ(I) = 21.74 Minuten; FAB-MS(M+H)⁺ = 451.

Das Ausgangsmaterial wird analog Beispiel 7a) unter Verwendung von N-Tertiärbutoxycarbonyl-2(R)-methyl-4(S)-hydroxy-5(S)-amino-7(S)-isopropyl-8-(3-hydroxy-phenyl)-octansäure(N-butyl)amid und von Bromessigsäuremethylester hergestellt.

Das als Ausgangsmaterial verwendete N-Tertiärbutoxycarbonyl-2(R)-methyl-4(S)-hydroxy-5(S)-amino-7(S)-isopropyl-8-(3-hydroxyphenyl)-octansäure-(N-butyl)amid wird analog Beispiel 5a) - 5l) hergestellt, wobei in Stufe k) an Stelle von 3-Acetoxy-4-tertiärbutyl-benzylbromid das 3-Benzyloxy-benzylbromid eingesetzt wird, wodurch in der stufe i) direkt das 2(R)-Isopropyl-3(3-benzyloxy-phenyl)-propanol, R_{f} (Dichlormethan-Hexan = 1:1) =0.19 erhalten wird.

### Beispiel 28: 2(R)-Methyl-4(S)-hydroxy-5(S)-amino-7(S)-isopropyl-8-[3-(methoxycarbonyl-methoxy)-4-methoxy-phenyl]-octansäure(N-butyl)amid-hydrochlorid

Analog Beispiel 1 wird die Titelverbindung ausgehend von 15 mg N-Tertiärbutoxy-carbonyl-2(R)-methyl-4(S)-hydroxy-5(S)-amino-7(S)-isopropyl-8-[3-(methoxycarbonylmethoxy)-4-methoxy-phenyl]-octansäure(N-butyl)amid hergestellt. Dies ergibt die Titelverbindung: R_{f}(Dichlormethan-Methanol = 9:1) = 0.16; Rₜ(I) = 19.33 Minuten; FAB-MS(M+H)⁺ = 481.

Das Ausgangsmaterial wird analog Beispiel 7a) unter Verwendung von N-Tertiärbutoxycarbonyl-2(R)-methyl-4(S)-hydroxy-5(S)-amino-7(S)-isopropyl-8-(3-hydroxy-4-methoxy-phenyl)-octansäure(N-butyl)amid und von Bromessigsäuremethylester hergestellt.

Das dazu als Ausgangsmaterial verwendete N-Tertiärbutoxycarbonyl-2(R)-methyl-4(S)-hydroxy-5(S)-amino-7(S)-isopropyl-8-(3-hydroxy-4-methoxy-phenyl)-octansäure(N-butyl)-amid wird wie folgt hergestellt:

### a1) N-Tertiärbutoxycarbonyl-2(R)-methyl-4(S)-hydroxy-5(S)-amino-7(S)-isopropyl-8-(3-benzyloxy-4-methoxy-phenyl)-octansäure(N-butyl)amid

3.5g N-Tertiärbutoxycarbonyl-2-methylen-4(S)-hydroxy-5(S)-amino-7(S)-isopropyl-8-(3-benzyloxy-4-methoxy-phenyl)-octansäure(N-butyl)amid werden in 30ml absolutem Methanol in Gegenwart von 20mg [Ru₂Cl₄((S)-Binap)₂].NEt₃ bei Raumtemperatur und 25bar während 5Stunden hydriert. Das Reaktionsgemisch wird filtriert und das Filtrat eingedampft. Der Rückstand wird durch FC (200g Kieselgel, Hexan-Essigsäureethylester=1:1) gereinigt. Titelverbindung: R_{f} (Hexan-Essigsäureethylester=1:1)=0.16; FAB-MS (M+H)⁺=599.

### a2) N-Tertiärbutoxycarbonyl-2(R)-methyl-4(S)-hydroxy-5(S)-amino-7(S)-isopropyl-8-(3-hydroxy-4-methoxy-phenyl)-octansäure(N-butyl)amid

4.7g N-Tertiärbutoxycarbonyl-2(R)-methyl-4(S)-hydroxy-5(S)-amino-7(S)-isopropyl-8-(3-benzyloxy-4-methoxy-phenyl)-octansäure(N-butyl)amid werden in 60ml Methanol in Gegenwart von 2.35g Pd/C 10% bei Raumtemperatur und Normaldruck während 1Stunden hydriert. Durch Filtrieren des Reaktionsgemisches und Eindampfen des Filtrats am Hochvakuum erhält man die Titelverbindung: R_{f} (Hexan-Essigsäureethylester=1:1)=0.15; FAB-MS (M+H)⁺=509.

Das als Ausgangsmaterial verwendete N-Tertiärbutoxycarbonyl-2-methylen-4(S)-hydroxy-5(S)-amino-7(S)-isopropyl-8-(3-benzyloxy-4-methoxy-phenyl)-octansäure(N-butyl)amid wird analog Beispiel1b) bis i) hergestellt, wobei in Stufei) an Stelle von p-tertiärbutyl-benzylbromid das 3-Benzyloxy-4-methoxybenzylbromid eingesetzt wird.

### Beispiel 29: 2(R)-Methyl-4(S)-hydroxy-5(S)-amino-7(S)-isopropyl-8-[3-(N-methyl-carbamoyl-methoxy)-4-methoxy-phenyl]-octansäure(N-butyl)amid-hydrochlorid

Analog Beispiel 1 wird die Titelverbindung ausgehend von 18 mg N-Tertiärbutoxy-carbonyl-2(R)-methyl-4(S)-hydroxy-5(S)-amino-7(S)-isopropyl-8-[3-(N-methyl-carbamoylmethoxy)-4-methoxy-phenyl]-octansäure(N-butyl)amid hergestellt. Dies ergibt die Titelverbindung: R_{f}(Dichlormethan-Methanol = 9:1) = 0.21; Rₜ(I) = 15.54 Minuten; FAB-MS(M+H)⁺ = 480.

Das Ausgangsmaterial wird wie folgt hergestellt:

Ein Gemisch aus 29 mg N-Tertiärbutoxycarbonyl-2(R)-methyl-4(S)-hydroxy-S(S)-amino-7(S)-isopropyl-8-[3-(methoxycarbonylmethoxy)-4-methoxy-phenyl]-octansäure(N-butyl)amid (Beispiel 32), 6 ml Dimethylformamid und 1 ml Methylamin wird 60 Stunden im Bombenrohr bei Raumtemperatur stehen gelassen. Durch Eindampfen und FC (5g Kieselgel, Dichlormethan-Methanol = 9:1) des Rückstandes erhält man die Titelverbindung: R_{f}(Dichlormethan-Methanol = 9:1) = 0.55.

### Beispiel 30: 2(R)-Methyl-4(S)-hydroxy-5(S)-amino-7(S)-isopropyl-8-[3-(3-methylsulfonyl-propyloxy)-4-methoxy-phenyl]-octansäure(N-butyl)amid-hydrochlorid

Analog Beispiel 1 wird die Titelverbindung ausgehend von 30 mg N-Tertiärbutoxy-carbonyl-2(R)-methyl-4(S)-hydroxy-5(S)-amino-7(S)-isopropyl-8-[3-(3-methylsulfonyl-propyloxy)-4- methoxy-phenyl]-octansäure(N-butyl)amid hergestellt. Dies ergibt die Titelverbindung: R_{f}(Dichlormethan-Methanol = 9:1) = 0.29; Rₜ(I) = 17.83 Minuten; FAB-MS(M+H)⁺ = 529.

Das Ausgangsmaterial wird analog Beispiel 17a) und 18a) unter Verwendung von N-Tertiärbutoxycarbonyl-2(R)-methyl-4(S)-hydroxy-5(S)-amino-7(S)-isopropyl-8-(3-hydroxy-4-methoxy-phenyl)-octansäure(N-butyl)amid und von 3-Methylthiopropyljodid mit anschliessender Oxydation zum Sulfon hergestellt.

### Beispiel 31: 2(R)-Methyl-4(S)-hydroxy-5(S)-amino-7(S)-isopropyl-8-[3-(methylsulfonyl-methoxy)-4-methoxy-phenyl]-octansäure(N-butyl)amid-hydrochlorid

Analog Beispiel 1 wird die Titelverbindung ausgehend von 100 mg N-Tertiärbutoxycarbonyl-2(R)-methyl-4(S)-hydroxy-5(S)-amino-7(S)-isopropyl-8[3-(methylsulfonyl-methoxy)-4-methoxy-phenyl]-octansäure(N-butyl)amid hergestellt. Dies ergibt die Titelverbindung: R_{f}(Dichlormethan-Methanol = 8:2) = 0.5; Rₜ(I) = 18.0 Minuten; FAB-MS(M+H)⁺ =501.

Das Ausgangsmaterial wird analog Beispiel 17a) und 18a) unter Verwendung von N-Tertiärbutoxycarbonyl-2(R)-methyl-4(S)-hydroxy-5(S)-amino-7(S)-isopropyl-8-(3-hydroxy-4-methoxy-phenyl)-octansäure(N-butyl)amid und von Chlordimethylsulfid mit anschliessender Oxydation zum Sulfon hergestellt.

### Beispiel 32: 2(R)-Methyl-4(S)-hydroxy-5(S)-amino-7(S)-isopropyl-8-[3-(3-methoxy-propyloxy)-4-methoxy-phenyl]-octansäure-(N-butyl)amid-hydrochlorid

Analog Beispiel 1 wird die Titelverbindung ausgehend von 27 mg N-Tertiärbutoxy-carbonyl-2(R)-methyl-4(S)-hydroxy-5(S)-amino-7(S)-isopropyl-8-[3-(3-methoxy-propyloxy)-4-methoxy-phenyl]-octansäure(N-butyl)amid hergestellt und durch FC (2g Kieselgel, Dichlormethan-Methanol = 95:5) gereinigt Dies ergibt die Titelverbindung: R_{f}(Dichlormethan-Methanol =9:1) = 0.15; Rₜ(I) = 21.9 Minuten; FAB-MS(M+H)⁺ = 481.

Das Ausgangsmaterial wird analog Beispiel 17a) unter Verwendung von N-Tertiärbutoxycarbonyl-2(R)-methyl-4(S)-hydroxy-5(S)-amino-7(S)-isopropyl-8-(3-hydroxy-4-methoxy-phenyl)-octansäure(N-butyl)amid und von 3-Methoxy-propyljodid hergestellt.

### Beispiel 33: 2(R)-Methyl-4(S)-hydroxy-5(S)-amino-7(S)-isopropyl-8-[3-(2-methoxy-ethoxy)-4-methoxy-phenyl]-octansäure(N-butyl)amid-hydrochlorid

Analog Beispiel 1 wird die Titelverbindung ausgehend von 68 mg N-Tertiärbutoxy-carbonyl-2(R)-methyl-4(S)-hydroxy-5(S)-amino-7(S)-isopropyl-8-[3-(2-methoxy-ethoxy)-4-methoxy-phenyl]-octansäure(N-butyl)amid hergestellt. Dies ergibt die Titelverbindung: R_{f}(Dichlormethan-Methanol = 9:1) = 0.32; Rₜ(I) = 19.84 Minuten; FAB-MS(M+H)⁺ = 467.

Das Ausgangsmaterial wird analog Beispiel 17a) unter Verwendung von N-Tertiärbutoxy-carbonyl-2(R)-methyl-4(S)-hydroxy-5(S)-amino-7(S)-isopropyl-8-(3-hydroxy-4-methoxy-phenyl)-octansäure(N-butyl)amid und von 2-Methoxy-ethyljodid hergestellt.

### Beispiel 34: 2(R)-Methyl-4(S)-hydroxy-5(S)-amino-7(S)-isopropyl-8-[3-(3-hydroxy-propyloxy)-4-methoxy-phenyl]-octansäure(N-butyl)amid-hydrochlorid

Analog Beispiel 1 wird die Titelverbindung ausgehend von 93 mg N-Tertiärbutoxy-carbonyl-2(R)-methyl-4(S)-hydroxy-5(S)-amino-7(S)-isopropyl-8-[3-(3-hydroxypropyloxy)4-methoxy-phenyl]-octansäure(N-butyl)amid hergestellt. Dies ergibt die Titelverbindung: R_{f}(Dichlormethan-Methanol = 9:1) = 0.24; Rₜ(I) = 16.13 Minuten; FAB-MS(M+H)⁺ = 467.

Das Ausgangsmaterial wird analog Beispiel 17a) unter Verwendung von N-Tertiärbutoxy-carbonyl-2(R)-methyl-4(S)-hydroxy-5(S)-amino-7(S)-isopropyl-8-(3-hydroxy-4-methoxy-phenyl)-octansäure(N-butyl)amid und von 3-Jodpropanol hergestellt.

### Beispiel 35: 2(R)-Methyl-4(S)-hydroxy-5(S)-amino-7(S)-isopropyl-8-[3-(carbamoylmethoxy)-4-methoxy-phenyl]-octansäure(N-butyl)amid-hydrochlorid

Analog Beispiel 1 wird die Titelverbindung ausgehend von 39 mg N-Tertiärbutoxy-carbonyl-2(R)-methyl-4(S)-hydroxy-5(S)-amino-7(S)-isopropyl-8-[3-(carbamoylmethoxy)-4-methoxy-phenyl]-octansäure(N-butyl)amid hergestellt Dies ergibt die Titelverbindung: R_{f}(Dichlormethan-Methanol = 8:2) = 0.38; Rₜ(I) = 13.86 Minuten; FAB-MS(M+H)⁺ =466.

Das Ausgangsmaterial wird analog Beispiel 7a) unter Verwendung von N-Tertiärbutoxy-carbonyl-2(R)-methyl-4(S)-hydroxy-5(S)-amino-7(S)-isopropyl-8-(3hydroxy-4-methoxy-phenyl)-octansäure(N-butyl)amid und von Jodacetamid hergestellt.

### Beispiel 36: 2(R)-Methyl-4(S)-hydroxy-5(S)-amino-7(S)-isopropyl-8-(3-cyanmethoxy-4-methoxy-phenyl)-octansäure(N-butyl)amid

Eine Lösung von 35 mg N-Tertiärbutoxycarbonyl-2(R)-methyl-4(S)-hydroxy-5(S)-amino-7(S)-isopropyl-8-(3-cyanmethoxy-4-methoxy-phenyl)-octansäure(N-butyl)amid in 1 ml Dichlormethan wird bei 0°C unter Rühren mit 1.5 ml eines Gemisches aus Trifluoressigsäure-Dichlormethan = 1:3 versetzt, 3 Stunden bei 0°C weitergerührt und dann eingedampft. Der Rückstand wird durch FC (5 g Kieselgel, Dichlormethan-Methanol = 9:1) gereinigt. Dies ergibt die Titelverbindung: R_{f}(Dichlormethan-Methanol = 9:1) = 0.19; Rₜ(I) = 19.59 Minuten; FAB-MS(M+H)⁺ =448.

Das Ausgangsmaterial wird analog Beispiel 7a) unter Verwendung von N-Tertiärbutoxy-carbonyl-2(R)-methyl-4(S)-hydroxy-5(S)-amino-7(S)-isopropyl-8-(3-hydroxy-4-methoxy-phenyl)-octansäure(N-butyl)amid und von Jodacetonitril hergestellt.

### Beispiel 37: 2(R)-Methyl-4(S)-hydroxy-5(S)-amino-7(S)-isopropyl-8-[3-(4-methoxy-butoxy)-4-methoxy-phenyl]-octansäure(N-butyl)amid-hydrochlorid

Analog Beispiel 1 wird die Titelverbindung ausgehend von 24 mg N-Tertiärbutoxy-carbonyl-2(R)-methyl-4(S)-hydroxy-5(S)-amino-7(S)-isopropyl-8-[3-(4-methoxy-butoxy)-4-methoxy-phenyl]-octansäure(N-butyl)amid hergestellt. Dies ergibt die Titelverbindung: R_{f}(Dichlormethan-Methanol = 9:1) = 0.29; Rₜ(I) = 22.51 Minuten; FAB-MS(M+H)⁺ = 495.

Das Ausgangsmaterial wird analog Beispiel 17a) unter Verwendung von N-Tertiärbutoxy-carbonyl-2(R)-methyl-4(S)-hydroxy-5(S)-amino-7(S)-isopropyl-8-(3-hydroxy-4-methoxy-phenyl)-octansäure(N-butyl)amid und von 4-Methoxy-propyljodid hergestellt.

### Beispiel 38: 2(R)-Methyl-4(S)-hydroxy-5(S)-amino-7(S)-isopropyl-8-[3-(2-ethoxy-ethoxy)-4-methoxy-phenyl]-octansäure(N-butyl)amid-hydrochlorid

Analog Beispiel 1 wird die Titelverbindung ausgehend von 24 mg N-Tertiärbutoxy-carbonyl-2(R)-methyl-4(S)-hydroxy-5(S)-amino-7(S)-isopropyl-8-[3-(2-ethoxy-ethoxy)-4-methoxy-phenyl]-octansäure(N-butyl)amid hergestellt. Dies ergibt die Titelverbindung: R_{f}(Dichlormethan-Methanol = 9:1) = 0.26; Rₜ(I) = 21.32 Minuten; FAB-MS(M+H)⁺ = 481.

Das Ausgangsmaterial wird analog Beispiel 17a) unter Verwendung von N-Tertiärbutoxy-carbonyl-2(R)-methyl-4(S)-hydroxy-5(S)-amino-7(S)-isopropyl-8-(3-hydroxy-4-methoxy-phenyl)-octansäure(N-butyl)amid und von 2-Joddiethylether hergestellt.

### Beispiel 39: 2(R)-Methyl-4(S)-hydroxy-5(S)-amino-7(S)-isopropyl-8-{3-[2-(2-methoxy-ethoxy)ethoxy]-4-methoxy-phenyl}-octansäure-(N-butyl)-amid-hydrochlorid

Analog Beispiel 1 wird die Titelverbindung ausgehend von 27 mg N-Tertiärbutoxy-carbonyl-2(R)-methyl-4(S)-hydroxy-5(S)-amino-7(S)-isopropyl-8-{3-[2-(2-methoxy-ethoxy)ethoxy]-4-methoxy-phenyl}-octansäure-(N-butyl)-amid hergestellt. Dies ergibt die Titelverbindung: R_{f}(Dichlormethan-Methanol = 1:1) = 0.19; Rₜ(I) = 18.93 Minuten; FAB-MS(M+H)⁺ =511.

Das Ausgangsmaterial wird analog Beispiel 17a) unter Verwendung von N-Tertiärbutoxy-carbonyl-2(R)-methyl-4(S)-hydroxy-5(S)-amino-7(S)-isopropyl-8-(3-hydroxy-4-methoxy-phenyl)-octansäure(N-butyl)amid und von 1-Jod-2-(2-methoxy-ethoxy)-ethan hergestellt.

### Beispiel 40: 2(R)-Methyl-4(S)-hydroxy-5(S)-amino-7(S)-isopropyl-8-(3-pentoxy-4-methoxy-phenyl)-octansäure(N-butyl)amid-hydrochlorid

Analog Beispiel 1 wird die Titelverbindung ausgehend von 53 mg N-Tertiärbutoxy-carbonyl-2(R)-methyl-4(S)-hydroxy-5(S)-amino-7(S)-isopropyl-8-(3-pentoxy-4-methoxy-phenyl)-octansäure(N-butyl)amid hergestellt. Dies ergibt die Titelverbindung: R_{f}(Dichlormethan-Methanol = 9:1) = 0.25; Rₜ(I) = 32.01 Minuten; FAB-MS(M+H)⁺ = 479.

Das Ausgangsmaterial wird analog Beispiel 17a) unter Verwendung von N-Tertiärbutoxy-carbonyl-2(R)-methyl-4(S)-hydroxy-5(S)-amino-7(S)-isopropyl-8-(3-hydroxy-4-methoxy-phenyl)-octansäure(N-butyl)amid und von Jodpentan hergestellt.

### Beispiel 41: 2(R)-Methyl-4(S)-hydroxy-5(S)-amino-7(S)-isopropyl-8-(3-benzyloxy-4-methoxy-phenyl)-octansäure(N-butyl)amid-hydrochlorid

Analog Beispiel 1 wird die Titelverbindung ausgehend von 100 mg N-Tertiärbutoxy-carbonyl-2(R)-methyl-4(S)-hydroxy-5(S)-amino-7(S)-isopropyl-8-(3-benzyloxy-4-methoxy-phenyl)-octansäure(N-butyl)amid hergestellt. Dies ergibt die Titelverbindung: R_{f}(Dichlormethan-Methanol = 9:1) = 0.31; Rₜ(I) = 44.21 Minuten; FAB-MS(M+H)⁺ = 499.

Das Ausgangsmaterial wird analog Beispiel 17a) unter Verwendung von N-Tertiärbutoxy-carbonyl-2(R)-methyl-4(S)-hydroxy-5(S)-amino-7(S)-isopropyl-8-(3-hydroxy-4-methoxy-phenyl)-octansäure(N-butyl)amid und von Benzylbromid hergestellt.

### Beispiel 42: 2(R)-Methyl-4(S)-hydroxy-5(S)-amino-7(S)-isopropyl-8-[3-(3-ethoxy-propyloxy)-4-methoxy-phenyl]-octansäure(N-butyl)amid-hydrochlorid

Analog Beispiel 1 wird die Titelverbindung ausgehend von 113 mg N-Tertiärbutoxy-carbonyl-2(R)-methyl-4(S)-hydroxy-5(S)-amino-7(S)-isopropyl-8-[3-(3-ethoxypropyloxy)-4-methoxy-phenyl]-octansäure(N-butyl)amid hergestellt. Dies ergibt die Titelverbindung: R_{f}(Dichlormethan-Methanol = 9:1) = 0.30; Rₜ(I) = 23.11 Minuten; FAB-MS(M+H)⁺ = 495.

Das Ausgangsmaterial wird analog Beispiel 7a) unter Verwendung von N-Tertiärbutoxy-carbonyl-2(R)-methyl-4(S)-hydroxy-5(S)-amino-7(S)-isopropyl-8-(3-hydroxy-4-methoxy-phenyl)-octansäure(N-butyl)amid und von 1-Ethoxy-3-jodpropan hergestellt.

### Beispiel 43: 2(R)-Methyl-4(S)-hydroxy-5(S)-amino-7(S)-isopropyl-8-[3-(pyrid-4-ylmethoxy)-4-methoxy-phenyl]-octansäure(N-butyl)amid-hydrochlorid

Analog Beispiel1 wird die Titelverbindung ausgehend von 71mg N-Tertiärbutoxycarbonyl-2(R)-methyl-4(S)-hydroxy-5(S)-amino-7(S)-isopropyl-8-[3-(pyrid-4-yl-methoxy)-4-methoxy-phenyl]-octansäure(N-butyl)amid hergestellt. Dies ergibt die Titelverbindung: R_{f}(Dichlormethan-Methanol=9:1)=0.19; Rₜ(I)=32.95Minuten; FAB-MS(M+H)⁺=500.

Das Ausgangsmaterial wird analog Beispiel7a) unter Verwendung von N-Tertiärbutoxy-carbonyl-2(R)-methyl-4(S)-hydroxy-5(S)-amino-7(S)-isopropyl-8-(3-hydroxy-4-methoxy-phenyl)-octansäure(N-butyl)amid und von 4-Picolylchlorid hergestellt.

### Beispiel 44: 2(R,S)-Methyl-4(S)-hydroxy-5(S)-amino-7(S)-isopropyl-8-(2-ethoxycarbonyl-methoxy-4-tertiärbutyl-phenyl)-octansäure(N-butyl)amid-hydrochlorid

Analog Beispiel 1 wird die Titelverbindung ausgehend von 67 mg N-Tertiärbutoxy-carbonyl-2(R,S)-methyl-4(S)-hydroxy-5(S)-amino-7(S)-isopropyl-8-(2-ethoxycarbonylmethoxy-4-tertiärbutyl-phenyl)-octansäure-(N-butyl)-amid hergestellt. Dies ergibt die Titelverbindung als Diastereomerengemisch: R_{f}(Dichlormethan-Methanol = 9:1) = 0.19; Rₜ(I) = 35.7 und 36.5 Minuten; FAB-MS(M+H)⁺ = 521.

Das Ausgangsmaterial wird analog Beispiel 7a) unter Verwendung von N-Tertiärbutoxy-carbonyl-2(R,S)-methyl-4(S)-hydroxy-5(S)-amino-7(S)-isopropyl-8-(2-hydroxy-4-tertiärbutyl-phenyl)-octansäure(N-butyl)amid (Beispiel 6a) und von Jodessigsäureethylester hergestellt.

### Beispiel 45: 2(R,S)-Methyl-4(S)-hydroxy-5(S)-amino-7(S)-isopropyl-8-(2-ethoxycarbonyl-4-tertiärbutyl-phenyl)-octansäure(N-butyl)amid-hydrochlorid

Analog Beispiel 1 wird die Titelverbindung ausgehend von 80 mg N-Tertiärbutoxy-carbonyl-2(R,S)-methyl-4(S)-hydroxy-5(S)-amino-7(S)-isopropyl-8-(2-ethoxycarbonylmethoxy-4-tertiärbutyl-phenyl)-octansäure(N-butyl)amid hergestellt. Dies ergibt die Titelverbindung als Diastereomerengemisch: R_{f}(Dichlormethan-Methanol = 9:1) = 0.21; Rₜ(I) = 27.8 und 28.39 Minuten; FAB-MS(M+H)⁺ = 492.

Das Ausgangsmaterial wird analog Beispiel 7a) unter Verwendung von N-Tertiärbutoxy-carbonyl-2(R,S)-methyl-4(S)-hydroxy-5(S)-amino-7(S)-isopropyl-8-(2-hydroxy-4-tertiärbutyl-phenyl)-octansäure(N-butyl)amid (Beispiel 6a) und von Jodacetamid hergestellt.

### Beispiel 46: 2(R)-Methyl-4(S)-hydroxy-5(S)-amino-7(S)-isopropyl-8-[3-(3-methoxy-propyl-oxy)-4,5-ethylendioxy-phenyl]-octansäure(N-butyl)amid-hydrochlorid

Analog Beispiel1 wird die Titelverbindung ausgehend von 34mg N-Tertiärbutoxycarbonyl-2(R)-methyl-4(S)-hydroxy-5(S)-amino-7(S)-isopropyl-8-[3-(3-methoxypropyloxy)-4.5-ethylendioxy-phenyl]-octansäure(N-butyl)amid hergestellt. Dies ergibt die Titelverbindung: R_{f} (Dichlormethan-Methanol=9:1)=0.16; Rₜ(I)=21.83Minuten; FAB-MS (M+H)⁺=509.

Das Ausgangsmaterial wird analog Beispiel17a) unter Verwendung von N-Tertiärbutoxy-carbonyl-2(R)-methyl-4(S)-hydroxy-5(S)-amino-7(S)-isopropyl-8-(3-hydroxy-4.5-ethylendioxy-phenyl)-octansäure(N-butyl)amid und von 3-Methoxy-propyljodid hergestellt.

Das N-Tertiärbutoxycarbonyl-2(R)-methyl-4(S)-hydroxy-5(S)-amino-7(S)-isopropyl-8-(3-hydroxy-4.5-ethylendioxy-phenyl)-octansäure(N-butyl)amid wird analog Beispiel28 hergestellt, wobei in Stufei) an Stelle von 3-Benzyloxy-4-methoxy-benzylbromid das 3-Benzyloxy-4.5-ethylendioxy-benzylbromid eingesetzt wird. Dieses wird wie folgt hergestellt:

### a) 5-Hydroxy-1.4-benzodioxan-7-carbonsäureethylester

Zu einer bei 100°C gerührten Lösung von 2g Gallussäureethylester und 6.5g Cäsiumcarbonat in 80ml Dimethylformamid wird im Abstand von jeweils 2Stunden viermal die Lösung von 0.2ml 1.2-Dibromethan in 4ml Dimethylformamid zugetropft. Nach weiteren 2Stunden Rühren bei 100°C wird das Reaktionsgemisch eingedampft und der Rückstand zwischen Diethylether und Wasser verteilt. Die organischen Phasen werden über Natriumsulfat getrocknet und eingedampft. Aus dem Rückstand erhält man durch FC (50g Kieselgel, Methylenchlorid-Methanol=8:2) die Titelverbindung: R_{f} (Methylenchlorid-Methanol=8:2)=0.39.

### b) 5-Benzyloxy-1.4-benzodioxan-7-carbonsäureethylester

Das Reaktionsgemisch aus 900ml Aceton, 17.4g Hydroxy-1.4-benzodioxan-7-carbonsäureethylester, 37.9g Cäsiumcarbonat, 11ml Benzylbromid und 7.7g Natriumjodid wird 3Stunden am Rückfluss gehalten und anschliessend eingedampft. Der Rückstand wird zwischen Diethylether und Wasser verteilt. Die organischen Phasen werden über Natriumsulfat getrocknet und eingedampft. Aus dem Rückstand erhält man durch FC (900g Kieselgel, Hexan-Essigsäureethylester=1:1) die Titelverbindung: R_{f} (Hexan-Essigsäureethylester=2:1)=0.36.

### c) 5-Benzyloxy-7-hydroxymethyl-1.4-benzodioxan

Zu einer Lösung von 110mg Lithiumaluminiumhydrid in 10ml Tetrahydrofuran wird bei Raumtemperatur die Lösung von 1.28g 5-Benzyloxy-1.4-benzodioxan-7-carbonsäureethylester in 5ml Tetrahydrofuran zugetropft und 30Minuten bei Raumtemperatur weitergerührt. Danach werden nacheinander 0.22ml Essigsäureethylester, 1.5ml eines Gemisches (Wasser-Tetrahydrofuran=1:1) und schliesslich 2.25ml 2N Schwefelsäure zugetropft. Das Reaktionsgemisch wird zwischen Diethylether und Wasser verteilt. Die organischen Phasen werden über Natriumsulfat getrocknet und eingedampft. Aus dem Rückstand erhält man durch FC (240g Kieselgel, Essigsäureethylester-Hexan=1:2) die Titelverbindung: R_{f} (Essigsäureethylester-Hexan=1:2)=0.18.

### d) 3-Benzyloxy-4.5-ethylendioxy-benzylbromid

Eine Lösung von 0.1g 5-Benzyloxy-7-hydroxymethyl-1.4-benzodioxan in 5ml Chloroform wird mit 0.07ml Trimethylsilylbromid versetzt, 15Minuten bei Raumtemperatur weitergerührt und am Rotationsverdampfer eingedampft. Der Rückstand wird sofort in wenig Ethylacetat gelöst, mit dem gleichen Volumen Hexan versetzt und durch 15g Kieselgel filtriert, wobei mit einem Gemisch (Hexan-Essigsäureethylester=4:1) nacheluiert wird. Durch Eindampfen der Eluate erhält man die Titelverbindung: R_{f} (Hexan-Essigsäureethylester=3:1)=0.48.

Beispiel 47: In analoger Weise wie in den Beispielen 1 bis 46 oder 62 bis 180 beschrieben kann man ferner 5(S)-Amino-4(S)-hydroxy-2(S),7(S)-diisopropyl-8-[4-(3-hydroxypropyloxy)-3-(3-methoxy-propyloxy)-phenyl]-octansäure-[N-(2-carbamoyl-2,2-dimethyl-ethyl)]-amid-hydrochlorid herstellen.

Beispiel 48: In analoger Weise wie in den Beispielen 1 bis 46 oder 62 bis 180 beschrieben kann man ferner 5(S)-Amino-4(S)-hydroxy-2(S),7(S)-diisopropyl-8-[4-isopropyl-3-(3-methoxypropyloxy)-phenyl]-octansäure-[N-(2-carbamoyl-2,2-dimethyl-ethyl)]-amid-hydrochlorid herstellen.

Beispiel 49: In analoger Weise wie in den Beispielen 1 bis 46 oder 62 bis 180 beschrieben kann man ferner 5(S)-Amino-4(S)-hydroxy-2(S),7(S)-diisopropyl-8-[4-tertiärbutyl-3-(3-methoxy-propyloxy)-phenyl]-octansäure-[N-(2-carbamoyl-2,2-dimethyl-ethyl)]-amid-hydrochlorid herstellen.

Beispiel 50: In analoger Weise wie in den Beispielen 1 bis 46 oder 62 bis 180 beschrieben kann man ferner 5(S)-Amino-4(S)-hydroxy-2(S),7(S)-diisopropyl-8-[4-(3-methylsulfonyl-propyloxy)-3-(3-methoxy-propyloxy)-phenyl]-octansäure-(N-2-morpholinoethyl)amid-dihydrochlorid herstellen.

Beispiel 51: In analoger Weise wie in den Beispielen 1 bis 46 oder 62 bis 180 beschrieben kann man ferner 5(S)-Amino-4(S)-hydroxy-2(S),7(S)-diisopropyl-8-[4-(3-methylsulfonyl-propyloxy)-3-(3-methoxy-propyloxy)-phenyl]-octansäure-[N-(2-carbamoyl-2,2-dimethyl-ethyl)]-amid-hydrochlorid herstellen.

Beispiel 52: In analoger Weise wie in den Beispielen 1 bis 46 oder 62 bis 180 beschrieben kann man ferner 5(S)-Amino-4(S)-hydroxy-2(S),7(S)-diisopropyl-8-[3,4-di-(3-hydroxypropyloxy)-phenyl]-octansäure-(N-2-morpholinoethyl)amid-dihydrochlorid herstellen.

Beispiel 53: In analoger Weise wie in den Beispielen 1 bis 46 oder 62 bis 180 beschrieben kann man ferner 5(S)-Amino-4(S)-hydroxy-2(S),7(S)-diisopropyl-8-[3,4-di-(3-hydroxypropyloxy)-phenyl]-octansäure-[N-(2-carbamoyl-2,2-dimethyl-ethyl)]-amid-hydrochlorid herstellen.

Beispiel 54: In analoger Weise wie in den Beispielen 1 bis 46 oder 62 bis 180 beschrieben kann man ferner 5(S)-Amino-4(S)-hydroxy-2(S),7(S)-diisopropyl-8-[4-(3-N-methylcarbamoyl-propyl)-3-(3-methoxy-propyloxy)-phenyl]-octansäure-(N-2-morpholinoethyl)amid-dihydrochlorid herstellen.

Beispiel 55: In analoger Weise wie in den Beispielen 1 bis 46 oder 62 bis 180 beschrieben kann man ferner 5(S)-Amino-4(S)-hydroxy-2(S),7(S)-diisopropyl-8-[4-(2-morpholinoethoxy)-3-(3-methoxy-propyloxy)-phenyl]-octansäure-[N-(2-carbamoyl-2,2-dimethyl-ethyl)]-amid-dihydrochlorid herstellen.

Beispiel 56: In analoger Weise wie in den Beispielen 1 bis 46 oder 62 bis 180 beschrieben kann man ferner 5(S)-Amino-4(S)-hydroxy-2(S),7(S)-diisopropyl-8-[3-(3-methoxy-propyloxy)-4,5-ethylendioxy-phenyl]-octansäure-(N-2-morpholinoethyl)amid-dihydrochlorid herstellen.

Beispiel 57: In analoger Weise wie in den Beispielen 1 bis 46 oder 62 bis 180 beschrieben kann man ferner 5(S)-Amino-4(S)-hydroxy-2(S),7(S)-diisopropyl-8-[3-(3-methoxy-propyloxy)-4,5-ethylendioxy-phenyl]-octansäure-[N-(2-carbamoyl-2,2-dimethyl-ethyl)]-amid-hydrochlorid herstellen.

Beispiel 58: In analoger Weise wie in den Beispielen 1 bis 46 oder 62 bis 180 beschrieben kann man ferner 5(S)-Amino-4(S)-hydroxy-2(S),7(S)-diisopropyl-8-[3-(3-methoxy-propyloxy)-4,5-methylendioxy-phenyl]-octansäure-(N-2-morpholinoethyl)amid-dihydrochlorid herstellen.

Beispiel 59: In analoger Weise wie in den Beispielen 1 bis 46 oder 62 bis 180 beschrieben kann man ferner 5(S)-Amino-4(S)-hydroxy-2(S),7(S)-diisopropyl-8-[3-(3-methoxypropyloxy)-4,5-methylendioxy-phenyl]-octansäure-[N-(2-carbamoyl-2,2-dimethyl-ethyl)]-amid-hydrochlorid herstellen.

Beispiel 60: In analoger Weise wie in den Beispielen 1 bis 46 oder 62 bis 180 beschrieben kann man ferner 5(S)-Amino-4(S)-hydroxy-2(S),7(S)-diisopropyl-8-[4-methoxy-3-(3-methoxy-propyloxy)-phenyl]-octansäure-[N-(2-carbamoyl-2,2-ethylen-ethyl)]-amid-hydrochlorid herstellen.

Beispiel 61: In analoger Weise wie in den Beispielen 1 bis 46 oder 62 bis 180 beschrieben kann man ferner 5(S)-Amino-4(S)-hydroxy-2(S),7(S)-diisopropyl-8-[4-methoxy-3-(3-methoxy-propyloxy)-phenyl]-octansäure-[N-(3(S)-2-oxo-pyrrolidin-3-yl-methyl)]-amid-hydrochlorid herstellen.

### Beispiel 62: 5(S)-Amino-4(S)-hydroxy-7(S)-isopropyl-2(R)-methyl-8-[4-methoxy-3-(4-methoxy-but-2-enoxy)-phenyl]-octansäure-(N-butyl)-amid-hydrochlorid

Analog Beispiel 1 wird die Titelverbindung ausgehend von 66 mg 5(S)-Tertiärbutoxycarbonylamino-4(S)-hydroxy-7(S)-isopropyl-2(R)-methyl-8-[4-methoxy-3(4- methoxy-but-2-enoxy)-phenyl]-octansäure-(N-butyl)-amid hergestellt und durch FC (30 g Kieselgel, Dichlormethan - Methanol = 9:1) gereinigt. Dies ergibt die Titelverbindung: R_{f}(Dichlormethan-Methanol = 9:1) = 0.26; HPLC Rₜ(I) = 40.4 Minuten; FAB-MS (M+H)⁺ = 493.

Das Ausgangsmaterial wird analog Beispiel 17a) unter Verwendung von 5(S)-Tertiärbutoxy-carbonylamino-4(S)-hydroxy-7(S)-isopropyl-2(R)-methyl-8-(3-hydroxy-4-methoxy-phenyl)-octansäure-(N-butyl)-amid (Beispiel 28) und von 4-Methoxy-but-2-enyljodid hergestellt.

### Beispiel 63: 5(S)-Amino-4(S)-hydroxy-7(S)-isopropyl-2(R)-methyl-8-[4-hydroxy-3-(3-methoxypropyloxy)-phenyl]-octansäure-(N-butyl)amid-hydrochlorid

Analog Beispiel 1 wird die Titelverbindung ausgehend von 20 mg 5(S)-Tertiärbutoxycarbonylamino-4(S)-hydroxy-7(S)-isopropyl-2(R)-methyl-8-[4-hydroxy-3-(3-methoxy-propyloxy)-phenyl]-octansäure-(N-butyl)-amid hergestellt. Dies ergibt die Titelverbindung: R_{f} (Dichlormethan - Methanol = 9:1) = 0.05; HPLC Rₜ(I) = 36.22 Minuten; FAB-MS (M+H)⁺ = 467.

Das Ausgangsmaterial wird wie folgt hergestellt:

### a) 5(S)-Tertiärbutoxycarbonylamino-4(S)-hydroxy-7(S)-isopropyl-2(R)-methyl-8-[4-hydroxy-3-(3-methoxypropyloxy)-phenyl]-octansäure-(N-butyl)-amid

1.34g 5(S)-Tertiärbutoxycarbonylamino-4(S)-hydroxy-7(S)-isopropyl-2(R)-methyl-8-[4-benzyloxy-3-(3-methoxypropyloxy)-phenyl]-octansäure-(N-butyl)-amid werden in Gegenwart von 400 mg Pd/C 5% in 50 ml Methanol für 10 Minuten bei Raumtemperatur und Normaldruck hydriert. Das Reaktionsgemisch wird filtriert und eingedampft. Der Rückstand wird mittels FC (50 g Kieselgel, Hexan - Essigsäureethylester = 1:1) gereinigt. Man erhält die Titelverbindung: R_{f} (Hexan - Essigsäureethylester = 1:1) = 0.16; HPLC Rₜ = 17.42 Minuten; FAB - MS: (M+H)⁺ = 567.

Das als Ausgangsmaterial verwendete 5(S)-Tertiärbutoxycarbonylamino-4(S)-hydroxy-7(S)-isopropyl-2(R)-methyl-8-[4-benzyloxy-3-(3-methoxy-propyloxy)-phenyl]-octansäure-(N-butyl)-amid wird analog Beispiel 28a1) und Beispiel 1b) bis 1g) hergestellt, wobei in Stufe g) an Stelle von 2(S)-Isopropyl-3-(p-tertiärbutyl-phenyl)-propanol das 2(S)-Isopropyl-3-[4-benzyloxy-3-(3-methoxypropyloxy)-phenyl]-propanol eingesetzt wird. Dieses wird analog Beispiel 124i) bis m) hergestellt, wobei in Stufe m) an Stelle von 4-Methoxy-3-(3-methoxy-propyloxy)-benzylalkohol der 4-Benzyloxy-3-(3-methoxypropyloxy)-benzylalkohol eingesetzt wird.

Dieser wird wie folgt hergestellt:

### b) 4-Benzyloxy-3-(3-methoxypropyloxy)-benzaldehyd

Zu einer Suspension von 5.54 g NaH (60%ige Dispersion in Mineralöl) in 150 ml Dimethylformamid abs. wird die Lösung von 28.8 g 4-Benzyloxy-3-hydroxy-benzaldehyd in 100 ml Dimethylformamid zugetropft. Das Reaktionsgemisch wird bei Raumtemperatur gerührt, nach 30 Minuten mit der Lösung aus 29 g 3-Methoxybrompropan in 120 ml Dimethylformamid versetzt, noch weitere 4 Stunden bei Raumtemperatur gerührt und dann unter vermindertem Druck eingedampft. Der Rückstand wird zwischen Diethylether und Wasser verteilt. Aus den vereinigten organischen Phasen erhält man nach Trocknen über Natriumsulfat, Eindampfen und Reinigen des Rückstandes durch FC (100g Kieselgel, Dichlormethan) die Titelverbindung, welche spontan kristallisiert: R_{f} (Dichlormethan - Diethylether) = 0.44.

### c) 4-Benzyloxy-3-(3-methoxypropyloxy)-benzylalkohol

Zu einer bei 0° C gerührten Suspension von 11.74 g Natriumboranat in 530 ml eines Gemisches aus Ethanol - Wasser = 8:2 wird die Lösung von 31 g 4-Benzyloxy-3-(3-methoxypropyloxy)-benzaldehyd in 530 ml Ethanol - Wasser = 8:2 zugetropft. Das Reaktionsgemisch wird 1 Stunde bei 0° C gerührt und dann eingedampft. Der Rückstand wird zwischen Diethylether und Wasser verteilt. Aus den vereinigten organischen Phasen erhält man nach Trocknen über Natriumsulfat, Eindampfen und Reinigen mit FC (100 g Kieselgel, Dichlormethan/Diethylether = 1:1) die Titelverbindung: R_{f} ( Dichlormethan - Diethylether = 1:1) = 0.43.

### Beispiel 64: 5(S)-Amino-4(S)-hydroxy-7(S)-isopropyl-2(R)-methyl-8-[4-benzyloxy-3-(3-methoxypropyloxy)-phenyl]-octansäure-(N-butyl)amid-hydrochlorid

Analog Beispiel 1 wird die Titelverbindung ausgehend von 60 mg 5(S)-Tertiärbutoxycarbonylamino-4(S)-hydroxy-7(S)-isopropyl-2(R)-methyl-8-[4-benzyloxy-3-(3-methoxy-propyloxy)-phenyl]-octansäure-(N-butyl)amid hergestellt. Dies ergibt die Titelverbindung: R_{f} (Dichlormethan - Methanol = 95:5) = 0.08; HPLC Rₜ(I) = 45.47 Minuten; FAB-MS (M+H)⁺ = 557.

### Beispiel 65: 5(S)-Amino-4(S)-hydroxy-7(S)-isopropyl-2(R)-methyl-8-[3.4-di-(3-methoxypropyloxy)-phenyl]-octansäure-(N-butyl)amid-hydrochlorid

Analog Beispiel 1 wird die Titelverbindung ausgehend von 66 mg 5(S)-Tertiärbutoxycarbonylamino-4(S)-hydroxy-7(S)-isopropyl-2(R)-methyl-8-[3.4-di-(3-methoxy-propyloxy)-phenyl]-octansäure-(N-butyl)amid hergestellt. Dies ergibt die Titelverbindung: R_{f} (Dichlormethan - Methanol = 9:1) = 0.21; Rₜ(I) = 40.0 Minuten; FAB-MS (M+H)⁺ = 539.

Das Ausgangsmaterial wird analog Beispiel 17a) unter Verwendung von 5(S)-Tertiärbutoxy-carbonylamino-4(S)-hydroxy-7(S)-isopropyl-2(R)-methyl-8-[4-hydroxy-3-(3-methoxypropyloxy)-phenyl]-octansäure-(N-butyl)amid und 3-Methoxy-brompropan hergestellt.

### Beispiel 66: 5(S)-Amino-4(S)-hydroxy-7(S)-isopropyl-2(R)-methyl-8-[4-(2.2,2-trifluorethoxy)-3-(3-methoxypropyloxy)-phenyl]-octansäure-(N-butyl)amid-hydrochlorid

Analog Beispiel 1 wird die Titelverbindung ausgehend von 14 mg 5(S)-Tertiärbutoxycarbonylamino-4(S)-hydroxy-7(S)-isopropyl-2(R)-methyl-8-[4-(2.2,2-trifluorethoxy)-3-(3-methoxypropyloxy)-phenyl]-octansäure-(N-butyl)amid hergestellt. Dies ergibt die Titelverbindung: R_{f} (Dichlormethan - Methanol = 9:1) = 0.31; HPLC Rₜ(I) = 28.7 Minuten; FAB-MS (M+H)⁺ = 549.

Das Ausgangsmaterial wird analog Beispiel 17a) unter Verwendung von 5(S)-Tertiärbutoxy-carbonylamino-4(S)-hydroxy-7(S)-isopropyl-2(R)-methyl-8-[4-hydroxy-3-(3-methoxypropyloxy)-phenyl]-octansäure-(N-butyl)amid und 2.2,2-Trifluorethyljodid hergestellt.

### Beispiel 67: 5(S)-Amino-4(S)-hydroxy-7(S)-isopropyl-2(R)-methyl-8-[4-(3-hydroxy-propyl-oxy)-3-(3-methoxypropyloxy)-phenyl]-octansäure-(N-butyl)amid-hydrochlorid

Analog Beispiel 1 wird die Titelverbindung ausgehend von 20 mg 5(S)-Tertiärbutoxycarbonylamino-4(S)-hydroxy-7(S)-isopropyl-2(R)-methyl-8-[4-(3-hydroxypropyloxy)-3-(3-methoxypropyloxy)-phenyl]-octansäure-(N-butyl)amid hergestellt und durch FC (2g Kieselgel, Dichlormethan - Methanol = 9:1) gereinigt. Dies ergibt die Titelverbindung: R_{f} (Dichlormethan - Methanol = 9:1) = 0.09; HPLC Rₜ = 11.03 Minuten; FAB-MS (M+H)⁺ = 525.

Das Ausgangsmaterial wird analog Beispiel 17a) unter Verwendung von 5(S)-Tertiärbutoxy-carbonylamino-4(S)-hydroxy-7(S)-isopropyl-2(R)-methyl-8-[4-hydroxy-3-(3-methoxypropyloxy)-phenyl]-octansäure-(N-butyl)amid und 3-Jodpropanol hergestellt.

### Beispiel 68: 5(S)-Amino-4(S)-hydroxy-7(S)-isopropyl-2(R)-methyl-8-[4-(2-amino-ethoxy)-3-(3-methoxypropyloxy)-phenyl]-octansäure-(N-butyl)amid-hydrochlorid.

Analog Beispiel 1 wird die Titelverbindung ausgehend von 7.5 mg 5(S)-Tertiärbutoxycarbonylamino-4(S)-hydroxy-7(S)-isopropyl-2(R)-methyl-8-[4-(2-amino-ethoxy)-3-(3-methoxypropyloxy)-phenyl]-octansäure-(N-butyl)amid hergestellt. Dies ergibt die Titelverbindung: R_{f} (Dichlormethan - Methanol/Ammoniak konz. = 100:50:1) = 0.28; HPLC Rₜ = 6.77 Minuten; FAB-MS (M+H)⁺ = 510.

Das Ausgangsmaterial wird analog Beispiel 17a) unter Verwendung von 5(S)-Tertiärbutoxy-carbonylamino-4(S)-hydroxy-7(S)-isopropyl-2(R)-methyl-8-[4-hydroxy-3-(3-methoxypropyloxy)-phenyl]-octansäure-(N-butyl)amid und Jodacetonitril, sowie anschliessender Reduktion der Nitrilfunktion zur Aminogruppe mit Raney-Nickel/H₂ bei Normaldruck und 40° C in Ethanol in Gegenwart von 4% Ammoniak hergestellt.

### Beispiel 69: 5(S)-Amino-4(S)-hydroxy-7(S)-isopropyl-2(R)-methyl-8-[4-(5-amino-pentyloxy)-3-(3-methoxypropyloxy)-phenyl]-octansäure-(N-butyl)amid-hydrochlorid

Analog Beispiel 1 wird die Titelverbindung ausgehend von 22 mg 5(S)-Tertiärbutoxycarbonylamino-4(S)-hydroxy-7(S)-isopropyl-2(R)-methyl-8-[4-(5-amino-pentyl-oxy)-3-(3-methoxypropyloxy)-phenyl]-octansäure-(N-butyl)amid hergestellt. Dies ergibt die Titelverbindung: R_{f} (Dichlormethan/Methanol/Ammoniak konz. = 100:50:1) = 0.11; HPLC Rₜ = 7.46 Minuten; FAB-MS (M+H)⁺ = 552.

Das Ausgangsmaterial wird analog Beispiel 17a) unter Verwendung von 5(S)-Tertiärbutoxy-carbonylamino-4(S)-hydroxy-7(S)-isopropyl-2(R)-methyl-8-[4-hydroxy-3-(3-methoxypropyloxy)-phenyl]-octansäure-(N-butyl)amid und 5-Jodvaleriansäurenitril, sowie anschliessender Reduktion der Nitrilfunktion zur Aminogruppe mit Raney-Nickel/H₂ bei Normaldruck und 40° C in Ethanol in Gegenwart von 4% Ammoniak hergestellt.

### Beispiel 70: 5(S)-Amino-4(S)-hydroxy-7(S)-isopropyl-2(R)-methyl-8-[4-(4-amino-butyloxy)-3-[3-methoxypropyloxy)-phenyl]-octansäure-(N-butyl)amid-hydrochlorid.

Analog Beispiel 1 wird die Titelverbindung ausgehend von 36 mg 5(S)-Tertiärbutoxycarbonylamino-4(S)-hydroxy-7(S)-isopropyl-2(R)-methyl-8[4-(4-amino-butyl-oxy)-3-(3-methoxypropyloxy)-phenyl]-octansäure-(N-butyl)amid hergestellt. Dies ergibt die Titelverbindung: R_{f} (Dichlormethan - Methanol - Ammoniak (konz.) = 100:50:1) = 0.15; HPLC Rₜ(I) = 33.3 Minuten; FAB-MS (M+H)⁺ = 538.

Das Ausgangsmaterial wird analog Beispiel 17a) unter Verwendung von 5(S)-Tertiärbutoxy-carbonylamino-4(S)-hydroxy-7(S)-isopropyl-2(R)-methyl-8-[4-hydroxy-3-(3-methoxypropyloxy)-phenyl]-octansäure-(N-butyl)amid und 4-Jodbutyronitril, sowie anschliessender Reduktion der Nitrilfunktion zur Aminogruppe mit Raney-Nickel/H₂ bei Normaldruck und 40° C in Ethanol in Gegenwart von 4% Ammoniak zum 5(S)-Tertiärbutoxycarbonylamino-4(S)-hydroxy-7(S)-isopropyl-2(R)-methyl-8[4-(4-amino-butyloxy)-3-(3-methoxypropyloxy)-phenyl]-octansäure-(N-butyl)amid, R_{f} (Dichlormethan/Methanol/Ammoniak konz. = 100:50:1) = 0.15, HPLC Rₜ = 13.55 Minuten hergestellt.

### Beispiel 71: 5(S)-Amino-4(S)-hydroxy-7(S)-isopropyl-2(R)-methyl-8-[4-(4-N,N-dimethyl-amino-butyloxy)-3-(3-methoxypropyloxy)-phenyl]-octansäure-(N-butyl)amid-hydrochlorid

Analog Beispiel 1 wird die Titelverbindung ausgehend von 30 mg 5(S)-Tertiärbutoxycarbonylamino-4(S)-hydroxy-7(S)-isopropyl-2(R)-methyl-8-[4-(4-N,N-dimethyl-amino-butyloxy)-3-(3-methoxypropyloxy)-phenyl]-octansäure-(N-butyl)amid hergestellt. Dies ergibt die Titelverbindung: R_{f} (Dichlormethan - Methanol - Ammoniak (konz.) = 100:50:1) = 0.21; HPLC Rₜ = 9.7 Minuten; FAB-MS (M+H)⁺ = 566.

Das Ausgangsmaterial wird durch Hydrieren von 80 mg 5(S)-Tertiärbutoxycarbonylamino-4(S)-hydroxy-7(S)-isopropyl-2(R)-methyl-8-[4-(4-amino-butyloxy)-3-(3-methoxy-propyloxy)-phenyl]-octansäure-(N-butyl)amid (Beispiel 70), gelöst in 6 ml Methanol und in Gegenwart von 25 ml 35%iger Formaldehydlösung mit 30 mg Pd/C 10% während 19 Stunden bei Raumtemperatur und Normaldruck hergestellt und durch FC (5g Kieselgel, Dichlormethan - Methanol - Ammoniak (konz.) = 350:50:1) gereinigt. R_{f} (Dichlormethan/Methanol/Ammoniak konz. = 350:50:1) = 0.21; HPLC Rₜ = 14.18 Minuten; FAB-MS (M+H)⁺ = 666.

### Beispiel 72: 5(S)-Amino-4(S)-hydroxy-7(S)-isopropyl-2(R)-methyl-8-{4-[4-N-(trifluormethansulfonylaminobutyloxy)-3-(3-methoxypropyloxy)-phenyl]}-octansäure-(N-butyl)amid-hydrochlorid

Analog Beispiel 1 wird die Titelverbindung ausgehend von 27 mg 5(S)-Tertiärbutoxycarbonylamino-4(S)-hydroxy-7(S)-isopropyl-2(R)-methyl-8-{4-[4-N-(trifluormethansulfonylaminobutyloxy)-3-(3-methoxypropyloxy)-phenyl]}-octansäure-(N-butyl)amid hergestellt. Dies ergibt die Titelverbindung: R_{f} (Dichlormethan/Methanol = 9:1) = 0.27; HPLC Rₜ = 14.67 Minuten; FAB-MS (M+H)⁺ = 670.

Das Ausgangsmaterial wird wie folgt hergestellt:

### a) 5(S-)Tertiärbutoxycarbonylamino-4(S)-hydroxy-7(S)-isopropyl-2(R)-methyl-8-[4-(4-N-trifluormethansulfonylamino-butyloxy)-3-(3-methoxypropyloxy)-phenyl]-octansäure-(N-butyl)-amid.

50 mg 5(S)-Tertiärbutoxycarbonylamino-4(S)-hydroxy-7(S)-isopropyl-2(R)-methyl-8-[4-(4-aminobutyloxy)-3-(3-methoxypropyloxy)-phenyl]-octansäure-(N-butyl)-amid werden in 4 ml Dichlormethan gelöst und bei 0°C mit 23 ml Triethylamin und 13 ml Trifluormethansulfonsäureanhydrid versetzt. Das Reaktionsgemisch wird 2 Stunden bei Raumtemperatur gerührt und dann zwischen Dichlormethan (3x) und gesättigter NaHCO₃-Lösung (1x) verteilt. Die organischen Phasen werden vereinigt, über Magnesiumsulfat getrocknet und eingedampft. Aus dem Rückstand erhält man nach Reinigung mit FC (15 g Kieselgel, Hexan - Essigsäureethylester= 1:1) die Titelverbindung: R_{f} (Hexan - Essigsäureethylester = 1:1) = 0.26; HPLC: Rₜ = 20.02 Minuten.

### Beispiel 73: 5(S)-Amino-4(S)-hydroxy-7(S)-isopropyl-2(R)-methyl-8-[4-carboxymethoxy-3-(3-methoxypropyloxy)-phenyl]-octansäure-(N-butyl)amid-hydrochlorid

Analog Beispiel 1 wird die Titelverbindung ausgehend von 70 mg 5(S)-Tertiärbutoxycarbonylamino-4(S)-hydroxy-7(S)-isopropyl-2(R)-methyl-8-[4-carboxymethoxy-3-(3-methoxypropyloxy)-phenyl]-octansäure-(N-butyl)amid hergestellt. Dies ergibt die Titelverbindung: R_{f} (Dichlormethan - Methanol = 7:3) = 0.35; HPLC Rₜ(I) = 37.18 Minuten; FAB-MS (M+H)⁺ = 525.

Das Ausgangsmaterial wird analog Beispiel 17a) unter Verwendung von 5(S)-Tertiärbutoxycarbonylamino-4(S)-hydroxy-7(S)-isopropyl-2(R)-methyl-8-[4-hydroxy-3-(3-methoxypropyloxy)-phenyl]-octansäure-(N-butyl)amid und Bromessigsäurebenzylester, sowie anschliessender Debenzylierung in Ethanol mit Pd/C 10% bei Raumtemperatur und Normaldruck hergestellt.

### Beispiel 74: 5(S)-Amino-4(S)-hydroxy-7(S)-isopropyl-2(R)-methyl-8-[4-(3-ethoxycarbonylpropyloxy)-3-(3-methoxy-propyloxy)-phenyl]-octansäure-(N-butyl)amid-hydrochlorid

Analog Beispiel 1 wird die Titelverbindung ausgehend von 27 mg 5(S)-Tertiärbutoxycarbonylamino-4(S)-hydroxy-7(S)-isopropyl-2(R)-methyl-8-[4-(3-ethoxycarbonylpropyloxy)-3-(3-methoxypropyloxy)-phenyl]-octansäure-(N-butyl)amid hergestellt. Dies ergibt die Titelverbindung: R_{f} (Dichlormethan - Methanol = 9:1) = 0.24; HPLC Rₜ = 18.18 Minuten; FAB-MS (M+H)⁺ = 581.

Das Ausgangsmaterial wird analog Beispiel 17a) unter Verwendung von 5(S)-Tertiärbutoxy-carbonylamino-4(S)-hydroxy-7(S)-isopropyl-2(R)-methyl-8-[4-hydroxy-3-(3-methoxypropyloxy)-phenyl]-octansäure-(N-butyl)amid und 4-Jodbuttersäureethylester hergestellt.

### Beispiel 75: 5(S)-Amino-4(S)-hydroxy-7(S)-isopropyl-2(R)-methyl-8-[4-(3-carboxy-propyl-oxy)-3-(3-methoxypropyloxy)-phenyl]-octansäure-(N-butyl)amid-hydrochlorid

Analog Beispiel 1 wird die Titelverbindung ausgehend von 41 mg 5(S)-Tertiärbutoxycarbonylamino-4(S)-hydroxy-7(S)-isopropyl-2(R)-methyl-8-[4-(3-carboxypropyl oxy)-3-(3-methoxypropyloxy)-phenyl]-octansäure-(N-butyl)amid hergestellt. Dies ergibt die Titelverbindung: R_{f} (Dichlormethan - Methanol = 9:1) = 0.20; HPLC Rₜ(I) = 37.65 Minuten; FAB-MS (M+H)⁺ = 553.

Das Ausgangsmaterial wird aus 5(S)Tertiärbutoxycarbonylamino-4(S)-hydroxy-7(S)-isopropyl-2(R)-methyl-8-[4-(3-ethoxycarbonylpropyloxy)-3-(3-methoxypropyloxy)-phenyl]-octansäure-(N-butyl)amid (Beispiel 74) durch Hydrolyse der Esterfunktion in methanolischer Lösung mit 2 Äquivalenten 1N Natriumhydroxid durch Rühren während 24 Stunden bei Raumtemperatur hergestellt. Das nach Eindampfen des Reaktionsgemisches durch Extraktion mit Essigsäureethylester aus einer auf pH 4 angesäuerten wässrigen Lösung des Rückstandes erhaltene Produkt wird durch FC (Dichlormethan - Methanol = 9:1) gereinigt. R_{f} (Dichlormethan - Methanol = 95 : 5) = 0.41.

### Beispiel 76: 5(S)-Amino-4(S)-hydroxy-7(S)-isopropyl-2(R)-methyl-8-[4-(4-methoxycarbonylbutyloxy)-3-(3-methoxypropyloxy)-phenyl]-octansäure-(N-butyl)amid-hydrochlorid

Analog Beispiel 1 wird die Titelverbindung ausgehend von 29 mg 5(S)-Tertiärbutoxycarbonylamino-4(S)-hydroxy-7(S)-isopropyl-2(R)-methyl-8-[4-(4-methoxy-carbonyl-butyloxy)-3-(3-methoxy-propyloxy)-phenyl]-octansäure-(N-butyl)amid hergestellt. Dies ergibt die Titelverbindung: R_{f} (Dichlormethan - Methanol = 9:1) = 0.24; HPLC Rₜ(I) = 42.55 Minuten; FAB-MS (M+H)⁺ = 581.

Das Ausgangsmaterial wird analog Beispiel 17a) unter Verwendung von 5(S)-Tertiärbutoxy-carbonylamino-4(S)-hydroxy-7(S)-isopropyl-2( R)-methyl-8-[4-hydroxy-3-(3-methoxypropyloxy)-phenyl]-octansäure-(N-butyl)amid und 5-Jodvaleriansäuremethylester hergestellt.

### Beispiel 77: 5(S)-Amino-4(S)-hydroxy-7(S)-isopropyl-2(R)-methyl-8-[4-(4-carboxy-butyloxy)-3-(3-methoxypropyloxy)-phenyl]-octansäure-(N-butyl)amid-hydrochlorid

Analog Beispiel 1 wird die Titelverbindung ausgehend von 10 mg 5(S)-Tertiärbutoxycarbonylamino-4(S)-hydroxy-7(S)-isopropyl-2(R)-methyl-8-[4-(4-carboxy-butyl-oxy)-3-(3-methoxypropyloxy)-phenyl]-octansäure-(N-butyl)amid hergestellt. Dies ergibt die Titelverbindung: R_{f} (Dichlormethan - Methanol = 8:2) = 0.34; HPLC Rₜ = 9.92 Minuten; FAB-MS (M+H)⁺ = 567.

Das Ausgangsmaterial wird aus 5(S)-Tertiärbutoxycarbonylamino-4(S)-hydroxy-7(S)-isopropyl-2(R)-methyl-8-[4-(4-methoxycarbonyl-butyloxy)-3-(3-methoxypropyloxy)-phenyl]-octansäure-(N-butyl)amid (Beispiel 76) durch Hydrolyse der Esterfunktion in methanolischer Lösung mit 2 Äquivalenten 1N Natriumhydroxid durch Rühren während 24 Stunden bei Raumtemperatur hergestellt. Das Reaktionsgemisch wird eingedampft, der Rückstand in Wasser gelöst, diese Lösung auf pH 4 angesäuert und mit Essigsäureethylester extrahiert. Die organischen Phasen werden über Magnesiumsulfat getrocknet und eingedampft. Aus dem Rückstand erhält man durch Reinigen mit FC (Kieselgel, Dichlormethan/Methanol = 9:1) die Tietelverbindung: R_{f} (Dichlormethan - Methanol - Ammoniak konz= 350 : 50 :1) = 0.14.

Beispiel 78: In analoger Weise wie in den Beispielen 1 bis 46 oder 62 bis 180 beschrieben kann man ferner 5(S)-Amino-4(S)-hydroxy-2(S),7(S)-diisopropyl-8-[4-methoxy-3-(3-methoxy-propyloxy)-phenyl]-octansäure-[N-(3(R)-2-oxo-pyrrolidin-3-yl-methyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(3(S)-2-oxo-piperidin-3-yl-methyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(3(R)-2-oxo-piperidin-3-yl-methyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(3-carbamoyl-3,3-dimethyl-propyl)]-amid-hydrochlorid,
5(S)-Amino-4(S)-hydroxy-2(S),7(S)-diisopropyl-8-[4-methoxy-3-(4-methoxy-butyl)-phenyl]-octansäure-[N-(5(S)-2-pyrrolidinon-5-yl-methyl)]-amid-hydrochlorid,
5(S)-Amino-4(S)-hydroxy-2(S),7(S)-diisopropyl-8-[4-methoxy-3-(4-methoxy-butyl)-phenyl]-octansäure-[N-(5(R)-2-pyrrolidinon-5-yl-methyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(6(S)-2-oxo-piperidin-6-yl-methyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(6(R)-2-oxo-piperidin-6-yl-methyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(2-thiazol-2-yl-ethyl)]-amid-dihydrochlorid,
5(S)-Amino-4(S)-hydroxy-2(S),7(S)-diisopropyl-8-[4-methoxy-3-(3-methoxy-propyloxy)-phenyl]-octansäure-[N-(4(S)-2-oxazolidinon-4-yl-methyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(4(R)-2-oxazolidinon-4-yl-methyl)]-amid-hydrochlorid, 5(S)-Amino-4(S)-hydroxy-2(S),7(S)-diisopropyl-8-[4-methoxy-3-(3-methoxy-propyloxy)-phenyl]-octansäure-[N-(3(S)-2,5-dioxo-pyrrolidin-3-yl-methyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(3(R)-2,5-dioxo-pyrrolidin-3-yl-methyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(2,6-dioxo-piperidin-4-yl-methyl)]-amid-hydrochlorid oder
5(S)-Amino-4(S)-hydroxy-2(S),7(S)-diisopropyl-8-[4-methoxy-3-(3-methoxy-propyloxy)-phenyl]-octansäure-[N-(4(S)-2-oxothiazolidin-4-yl-methyl)]-amid-hydrochlorid herstellen.
Beispiel 79: In analoger Weise wie in den Beispielen 1 bis 46 oder 62 bis 180 beschrieben kann man ferner

5(S)-Amino-4(S)-hydroxy-2(S),7(S)-diisopropyl-8-[3-(3-methoxy propoxy)-4,5-ethylendioxy-phenyl]-octansäure-N-(2-carbamoyl-2,2-dimethyl-ethyl)-amid,
5(S)-Amino-4(S)-hydroxy-2(S),7(S)-diisopropyl-8-[4-methoxy-3-(3-methoxy-propyloxy)-phenyl]-octansäure-[N-(4(R)-2-oxothiazolidin-4-yl-methyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(tetrahydro-2-pyrimidon-5-yl-methyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(N-acetyl-2-amino-2-methyl-propyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(N-formyl-2-amino-2-methyl-propyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(4-acetyl-piperazinyl-ethyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(2,4-imidazolindion-5-yl-methyl)]-amid-hydrochlorid,
5(S)-Amino-4(S)-hydroxy-2(S),7(S)-diisopropyl-8-[4-methoxy-3-(4-methoxy-butyl)-phenyl]-octansäure-[N-(2-hydroxy-pyridin-6-yl-methyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(2,2-dimethyl-2-sulfamoyl-ethyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(2,2-dimethyl-2(N,N-dimethyl)-sulfamoyl-ethyl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(2-oxo-piperidin-3(R)-yl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(2-oxo-piperidin-3(S)-yl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(2-oxo-piperidin-4-yl)]-amid-hydrochlorid,
5(S)-Amino-4(S)-hydroxy-2(S),7(S)-diisopropyl-8-[4-methoxy-3-(3-methoxy-propyloxy)-phenyl]-octansäure-[N-(N-acetyl-piperidin-4-yl)]-amid-hydrochlorid oder
5(S)-Amino-4(S)-hydroxy-2(S),7(S)-diisopropyl-8-[4-methoxy-3-(4-methoxy-but-1-en-yl)-phenyl]-octansäure-[N-(2-carbamoyl-2,2-dimethyl-ethyl)]-amid-hydrochlorid,

### Beispiel 80: 5(S)-Amino-4(S)-hydroxy-2(S),7(S)-diisopropyl-8-[4-methoxy-3-(3-methoxypropyloxy)-phenyl]-octansäure-(N-butyl)amid-hydrochlorid

Analog Beispiel 1 wird die Titelverbindung ausgehend von 82 mg 5(S)-Tertiärbutoxycarbonylamino-4(S)-hydroxy-2(S),7(S)-diisopropyl-8-[4-(4-methoxy-3-(3-methoxy-propyloxy)-phenyl]-octansäure-(N-butyl)amid hergestellt. Dies ergibt die Titelverbindung: R_{f} (Dichlormethan - Methanol = 9:1) = 0.32; HPLC Rₜ(I) = 42.32 Minuten; FAB-MS (M+H)⁺ = 509.

Das Ausgangsmaterial wird analog Beispiel 206a) und 200b) aus 3-Tertiärbutoxycarbonyl-5(S)-[2(S)-carboxy-3-methyl-butyl]-4(S)-{2(S)-isopropyl-3-[4-methoxy-3-(3-methoxypropyloxy)-phenyl]-propyl}-2.2-dimethyl-1.3-oxazolidin (Beispiel 200 c) und n-Butylamin hergestellt.

### Beispiel 81: 5(S)-Amino-4(S)-hydroxy-2(S),7(S)-diisopropyl-8-[4-methoxy-3-(2-methoxymethoxyethyl)-phenyl]-octansäure-(N-butyl)amid

50 mg 5(S)-Azido-4(S)-hydroxy-8(R,S)-isobutyroxy-2(S),7(S)-diisopropyl-8-[4-methoxy-3-(2-methoxymethoxyethyl)-phenyl]-octansäure-(N-butyl)amid werden in 10 ml Methanol in Gegenwart von 50 mg Pd/C 10% bei Raumtemperatur und Normaldruck hydriert. Das Reaktionsgemisch wird filtriert und eingedampft. Der Rückstand wird mittels FC (2 g Kieselgel, Dichlormethan/Methanol = 9:1) gereinigt. Man erhält die Titelverbindung: R_{f} (Dichlormethan - Methanol = 9:1) = 0.19; HPLC Rₜ = 13.42 Minuten.; FAB-MS (M+H)⁺ = 509. Das Ausgangsmaterial wird wie folgt hergestellt:

### a) 2-(2-Hydroxyethyl)-anisol

Zu einer Lösung von 10 g 2-(2-Hydroxyphenyl)-ethanol in 200 ml Aceton werden 35.3 g Cs₂CO₃ und anschliessend die Lösung von 6.5 ml Methyljodid in 40 ml Aceton zugegeben. Das Reaktionsgemisch wird 50 Minuten bei Raumtemperatur gerührt, filtriert und eingedampft. Der Rückstand wird zwischen Diethylether und Wasser verteilt. Aus den organischen Phasen erhält man nach Vereinigen, Trocknen über Magnesiumsulfat, Eindampfen und Reinigen des Rückstandes mittels FC (Dichlormethan-Diethylether = 97 : 3) die Titelverbindung: R_{f} (Dichlormethan -Diethylether = 97 : 3) = 0.34; HPLC Rₜ = 9.31 Minuten.

### b) 4-Brom-2-(2-hydroxyethyl)-anisol

Eine Lösung von 10.7 g 2-(2-Hydroxyethyl)-anisol in 195 ml Dichlormethan und 130 ml Methanol wird portionenweise mit 35.72 g Tetrabutylammoniumtribromid versetzt. Das Reaktionsgemisch wird 150 Minuten bei Raumtemperatur gerührt und dann am Rotavapor eingedampft. Der Rückstand wird zwischen Diethylether und Wasser verteilt. Aus den organischen Phasen erhält man durch Vereinigen, Trocknen über Magnesiumsulfat, Eindampfen und Reinigen des Rückstandes mittels FC (Dichlormethan) die Titelverbindung: R_{f} (Dichlormethan) = 0.26; HPLC Rₜ = 13.04 Minuten.

### c) 4-Brom-2-(2-methoxymethoxy-ethyl)-anisol

Eine Lösung von 948 mg 4-Brom-2-(2-hydroxyethyl)-anisol in 30 ml Dichlormethan wird bei Raumtemperatur mit 1.48 g N-Ethyl-diisopropylamin und 0.49 g Chlordimethyläther versetzt. Das Reaktionsgemisch wird 200 Minuten bei Raumtemperatur weitergerührt und dann mit 1 ml Wasser und 1 ml 25%iger Ammoniumhydroxidlösung versetzt. Das Zweiphasengemisch wird 15 Minuten kräftig weitergerührt und dann wird die organische Phase abgetrennt, über Natriumsulfat getrocknet und eingedampft. Aus dem Rückstand erhält man nach Reinigung mittels FC (Hexan/Dichlormethan = 1: 1) die Titelverbindung: R_{f} (Dichlormethan) = 0.6; HPLC Rₜ = 17.33 Minuten.

### d) 3(S)-Isopropyl-5(S)-{1(S)-azido-3(S)-isopropyl-4(R,S)-hydroxy-4-[4-methoxy-3-(2-methoxymethoxyethyl)-phenyl]-butyl}-tetrahydrofuran-2-on

Eine Suspension von 763 mg Magnesiumspänen in 0.5 ml Tetrahydrofuran wird mit einigen Jodkristallen versetzt und 30 Minuten im Ultraschallbad aktiviert. Danach werden 4 Tropfen 1.2-Dibromethan und anschliessend eine Lösung von 8.64 g 4-Brom-2-(2-methoxymethoxyethyl)-anisol in 30 ml Tetrahydrofuran derart zugetropft, dass das Reaktionsgemisch am Rückfluss siedet. Nach beendeter Zugabe wird noch 1 Stunde am Rückfluss gehalten. Dieses Reaktionsgemisch wird nun innert 45 Minuten zu einer auf -75° C gekühlten Lösung von 2.85 g 3(S)-Isopropyl-5(S)-[1(S)-azido-3(S)-isopropyl-4-oxobutyl]-tetrahydrofuran-2-on in 20 ml Tetrahydrofuran unter Rühren zugetropft. Das Reaktionsgemisch wird noch 150 Minuten bei -75° C weitergerührt und dann bei der gleichen Temperatur mit der Lösung von 1.4 ml Eisessig in 1 ml Tetrahydrofuran und danach mit 25 ml gesättigter Ammoniumchloridlösung versetzt. Anschliessend wird das RG auf Raumtemperatur gebracht, auf 60 ml Wasser gegossen und 3x mit 100 ml Essigsäureethylester extrahiert. Die organischen Phasen werden mit 50 ml gesättigter Kochsalzlösung gewaschen, vereinigt, über Magnesiumsulfat getrocknet und eingedampft. Durch Reinigen des Rückstandes mittels FC (400 g Kieselgel, Hexan/Essigsäureethylester = 8:2) erhält man die Titelverbindung: R_{f} (Hexan - Essigsäureethylester = 7:3) = 0.25; HPLC Rₜ = 48.10 und 50.29 Minuten;. (Diastereomerengemisch).

### e) 3(S)-Isopropyl-5(S)-{1(S)-azido-3(S)-isopropyl-4(R,S)-isobutyryloxy-4-[4-methoxy-3-(2-methoxymethoxyethyl)-phenyl]-butyl}-tetrahydrofuran-2-on

Eine Lösung von 300 mg 3(S)-Isopropyl-5(S)-{1(S)-azido-3(S)-isopropyl-4(R,S)-hydroxy-4-[4-methoxy-3-(2-methoxymethoxyethyl)-phenyl]-butyl}-tetrahydrofuran-2-on in 3.5 ml Dichlormethan wird mit 0.25 ml Pyridin, 0.31 ml Isobuttersäureanhydrid und 15 mg Dimethylaminopyridin versetzt und 80 Stunden bei Raumtemperatur weitergerührt. Danach wird das Reaktionsgemisch zwischen Dichlormethan (3x), Wasser (1x) und gesättigter Kochsalzlösung (2x) verteilt. Aus den vereinigten organischen Phasen erhält man nach Trocknen über Magnesiumsulfat, Eindampfen und Reinigen des Rückstandes durch FC (30 g Kieselgel, Hexan - Essigsäureethylester = 8:2) die Titelverbindung: R_{f} (Hexan - Essigsäureethylester = 8:2) = 0.26; HPLC Rₜ = 21.38 Minuten. und 21.76 Minuten. (Diastereomerengemisch).

### f) 5(S)-Azido-4(S)-hydroxy-2(S),7(S)-diisopropyl-8(R,S)-isobutyryloxy-8-[4-methoxy-3-(2-methoxymethoxyethyl)-phenyl]-octansäure(N-butyl)-amid

Eine Lösung von 170 mg 3(S)-Isopropyl-5(S)-{1(S)-azido-3(S)-isopropyl-4(R,S)-isobutyryloxy-4-[4-methoxy-3-(2-methoxymethoxyethyl)-phenyl]-butyl}-tetrahydrofuran-2-on in 1.4 ml Butylamin wird 16 Stunden bei Raumtemperatur gerührt und dann eingedampft. Aus dem Rückstand erhält man nach Reinigung mittels FC (Hexan/Essigsäureethylester = 7:3) die Titelverbindung: R_{f} (Hexan Essigsäureethylester = 7:3) = 0.25; HPLC Rₜ = 20.38 und 20.8 Minuten. (Diastereomerengemisch).

Das in Stufe d) eingesetzte 3(S)-Isopropyl-5(S)-[1(S)-azido-3(S)-isopropyl-4-oxo-butyl]-tetrahydrofuran-2-on wird wie folgt hergestellt.

### g) 2(S),7(S)-Diisopropyl-oct-4-en-dicarbonsäure-[bis-([4-(S)-benzyl-oxazolidin-2-on)]-amid.

Zu einer Lösung von 11.5 g 4(S)-Benzyl-3-isovaleroyl-oxazolidin-2-on in 32 ml Tetrahydrofuran wird unter Rühren bei -75° C 48 ml einer 1.0 M Lösung von Lithiumhexamethyldisilazid in Tetrahydrofuran innerhalb von 1 Stunde zugetropft. Es wird noch 2 Stunden bei -75° C und 20 Minuten bei -20° C weitergerührt, dann 10 ml 1,3-Dimethyl-3,4,5,6-tetrahydro-2-(1H)-pyrimidon (DMPU) und innert 45 Minuten die Lösung von 4.28 g 1.4-Dibrom-2-buten in 10 ml Tetrahydrofuran zugegeben. Das Reaktionsgemisch wird 15 Stunden bei -20° C weitergerührt, dann innert 1 Stunde auf 0° C gebracht, anschliessend bei -20° C mit 10 ml gesättigter Ammoniumchloridlösung versetzt und nach 15 Minuten auf Raumtemperatur gebracht. Nun wird das Raktionsgemisch zwischen Dichlormethan und gesättigter Kochsalzlösung/Wasser = 1:1 verteilt. Aus den organischen Phasen erhält man nach Vereinigen, Trocknen über Natriumsulfat, Eindampfen und Reinigen des Rückstandes mittels FC (Hexan/Essigsäureethylester = 4:1) die Titelverbindung: R_{f} (Hexan/Essigsäureethylester = 4 : 1) = 0.30; HPLC Rₜ = 21.6 Minuten.; FAB-MS (M+H)⁺ = 575; Smp. = 110-111° C (kristallisiert aus Essigsäureethylester - Hexan).

### h) 3(S)-Isopropyl-5(S)-{1(R)-brom-4-methyl-3(S)-[(4(S)-benzyloxazolidin-2-on-3-yl)-carbonyl]-pentyl}-tetrahydrofuran-2-on

Zu einer Lösung von 30 g 2(S),7(S)-Diisopropyl-oct-4-en-dicarbonsäure-[bis-(4(S)-benzyl-oxazolidin-2-on)]-amid in 360 ml Tetrahydrofuran und 120 ml Wasser werden unter Rühren 10.5 g N-Bromsuccinimid zugegeben, wobei die Temperatur mit einem Eisbad auf Raumtemperatur gehalten wird. Das Reaktionsgemisch wird 2 Stunden bei Raumtemperatur weitergerührt, dann wird am Rotavapor das Tetrahydrofuran abgedampft. Der wässrige Rückstand wird zwischen Diethylether (2x200 ml), Wasser (2x50 ml) und gesättigter Kochsalzlösung (1x 50 ml) verteilt. Aus den organischen Phasen erhält man nach Vereinigen, Trocknen über Magnesiumsulfat, Eindampfen und Reinigen des Rückstandes mittels FC (90 g Kieselgel, Hexan/ Essigsäureethylester = 3 : 1) die Titelverbindung als Rohprodukt. Durch Kristallisation aus Diisopropylether erhält man die reine Verbindung: Smp. = 91 - 92° C; R_{f} (Hexan - Essigsäureethylester = 8 : 2) = 0.28; HPLC Rₜ = 19.53 Minuten.; FAB-MS (M+H)⁺ = 494.

### i) 3(S)-Isopropyl-5(S)-{1(S)-azido-4-methyl-3(S)-[(4(S)-benzyl-oxazolidin-2-on-3-yl)-carbonyl]-pentyl}-tetrahydrofuran-2-on

Zu einer bei Raumtemperatur gerührten Lösung von 17.8 g 3(S)-Isopropyl-5(S)-{1(R)-brom-4-methyl-3(S)-[(4(S)-benzyl-oxazolidin-2-on-3-yl)-carbonyl]-pentyl}-tetrahydrofuran-2-on in 180 ml Toluol werden 13.6 g frisch getrocknetes Tetrabutylammoniumazid zugegeben und im Verlauf von 160 Stunden Rühren bei Raumtemperatur werden weitere 10 g des Azids zugefügt. Danach wird das Reaktionsgemisch zwischen Essigsäureethylester und Wasser (2x) und gesättigter Kochsalzlösung (1x) verteilt. Die organischen Phasen werden vereinigt, über Natriumsulfat getrocknet und eingeengt. Aus dem Eindampfrückstand erhält man mittels FC (Hexan/Essigsäureethylester = 8 : 2) und Kristallisation aus Diethylether - Hexan erhält man die Titelverbindung: Smp. = 102-103° C; R_{f} (Hexan/Essigsäureethylester = 8 : 2) = 0.2; HPLC Rₜ = 18.55 Minuten.; FAB-MS (M+H)⁺ = 457.

### k) 3(S)-Isopropyl-5(S)-(1(S)-azido-3(S)-carboxy-4-methyl-pentyl)-tetrahydrofuran-2-on

Zu einer bei -5° C gerührten Lösung von 55.3g 3(S)-Isopropyl-5(S)-{1(S)-azido-4-methyl-3(S)-[(4(S)-benzyl-oxazolidin-2-on-3-yl)-carbonyl]-pentyl}-tetrahydrofuran-2-on in 500 ml Tetrahydrofuran werden nacheinander 175 ml Wasser, 74 ml 30%ige Wasserstoffperoxidlösung und 5.9 g Lithiumhydroxid langsam zugegeben. Das Reaktionsgemisch wird 1 Stunde bei 5° C und 150 Minuten bei Raumtemperatur weitergerührt, dann bei 3° C während 30 Minuten. mit 750 ml wässriger 1M Natriumsulfitlösung versetzt und 30 Minuten bei Raumtemperatur weitergerührt. Durch Einengen wird dann das Reaktionsgemisch vom Tetrahydrofuran befreit und die wässrige Lösung wird 3x mit 1200 ml Essigsäureethylester gewaschen, wobei die organischen Phasen 3x mit 100 ml 0.1 N Natriumhydroxid zurückextrahiert werden. Die vereinigten wässrigen Phasen werden mit ca 200 ml 4N Salzsäure auf pH 1-2 gestellt und mit 3x 1200 ml Essigsäureethylester extrahiert. Aus den organischen Phasen erhält man durch Vereinigen, Trocknen über Magnesiumsulfat und Eindampfen das Rohprodukt, welches zur Cyclisierung von geöffnetem Lakton in 500 ml Toluol gelöst und zusammen mit ca. 1 g Molekularsieb und ca. 1 g p-Toluolsulfonsäure 2 Stunden bei 50° C gerührt wird. Filtrieren, Eindampfen und Reinigen des Rückstandes mittels FC (Hexan - Essigsäureethylester - Eisessig = 30 : 60 : 1) ergibt die Titelverbindung, welche spontan kristallisiert: Smp. = 56 - 58° C; R_{f} (Hexan - Essigsäureethylester - Eisessig = 30 : 60 : 1) = 0.62; HPLC Rₜ = 14.46 Minuten.; FAB MS (M+H)⁺ = 298.

### l) 3(S)-Isopropyl-5(S)-(1(S)-azido-3(S)-isopropyl-4-oxo-butyl)-tetrahydrofuran-2-on.

Zu einer Lösung von 1.7 g 3(S)-Isopropyl-5(S)-(1(S)-azido-3(S)-carboxy-4-methyl-pentyl)-tetrahydrofuran-2-on in 20 ml Toluol werden bei 0° C unter Rühren 1.45 ml Oxalylchlorid innert 10 Minuten. zugetropft. Danach werden noch 0.03 ml Dimethylformamid zugegeben und dann wird die Temperatur innert 30 Minuten auf 37° C erhöht. Das Reaktionsgemisch wird 2 Stunden bei 37° C gerührt, dann klarfiltriert und unter vermindertem Druck bei 30° C Badtemperatur eingedampft. Der Rückstand wird noch zweimal in 10 ml Toluol gelöst und in gleicher Weise wieder eingedampft. Das so erhaltene rohe Säurechlorid wird in 5 ml Tetrahydrofuran gelöst und bei -75° C mit 16 ml einer 0.34 M Lösung von NaAlH(O-tert.Bu)₃ in Diglyme (H.C. Brown et al.,J.Org.Chem. (1992) 58.472) innert 30 Minuten. versetzt. Das Reaktionsgemisch wird 70 Minuten. bei -75° C weitergerührt und dann wird bei gleicher Temperatur die Lösung von 0.385 ml Eisessig in 1 ml Tetrahydrofuran zugetropft, gefolgt von 2.1 ml gesättigter NH₄Cl-Lösung und anschliessend 20 ml Diethylether. Das Reaktionsgemisch wird auf Raumtemperatur gebracht und zwischen Diethylether und Wasser/gesättigter Kochsalzlösung verteilt. Aus den organischen Phasen erhält man nach Vereinigen, Trocknen über Magnesiumsulfat, Eindampfen und Reinigen des Rückstandes mittels FC (Hexan - Essigsäureethylester = 95 : 5) die Titelverbindung: R_{f} (Hexan - Essigsäureethylester = 2 : 1) = 0.55; HPLC Rₜ = 16.41 Minuten.

### Beispiel 82: 5(S)-Amino-4(S)-hydroxy-2(S),7(S)-diisopropyl-8-[4-(3-hydroxypropyloxy)-3-(3-methoxypropyloxy)-phenyl]-octansäure-N-(2-morpholinoethyl)amid-hydrochlorid

30 mg 5(S)-Tertiärbutoxycarbonylamino-4(S)-hydroxy-2(S),7(S)-diisopropyl-8-[4-(3-hydroxypropyloxy)-3-(3-methoxypropyloxy)-phenyl]-octansäure-(N-2-morpholinoethyl)amid werden in 1.5 ml einer auf 0° C gekühlten 4 N Lösung von Salzsäure in Dioxan gelöst und 10 Minuten. bei 0° C weitergerührt. Das Reaktionsgemisch wird unter vermindertem Druck bei Raumtemperatur zur Trockne eingedampft. Aus dem Rückstand erhält man durch Reinigen mittels FC (5 g Kieselgel, Dichlormethan - Methanol = 98 : 2) die Titelverbindung: R_{f} (Dichlormethan - Methanol = 8:2) = 0.20; Rₜ = 10.43 Minuten.; FAB-MS (M+H)⁺ = 610.

Das Ausgangsmaterial wird wie folgt hergestellt:

### a) 2-(3-Methoxypropyloxy)-phenol

Eine Suspension von 8.4 g NaH (60%ige Dispersion in Mineralöl) in 300 ml Dimethylformamid wird bei Raumtemperatur innert 30 Minuten. mit der Lösung von 22 g Brenzcatechin in 80 ml Dimethylformamid versetzt und 1 Stunde bei Raumtemperatur weitergerührt. Danach wird die Lösung von 49.3 g 3-Brompropyl-methylether in 80 ml Dimethylformamid zugetropft. Das Reaktionsgemisch wird 80 Stunden bei Raumtemperatur weitergerührt und dann unter vermindertem Druck bei 30° C Badtemperatur eingedampft. Der Rückstand wird zwischen Diethylether und Wasser verteilt. Aus den vereinigten organischen Phasen erhält man nach Trocknen über Magnesiumsulfat, Eindampfen und Reinigen des Rückstandes durch FC (100 g Kieselgel, Hexan -Dichlormethan = 5: 95) die Titelverbindung: R_{f} (Dichlormethan - Diethylether = 96 : 4) = 0.35; HPLC Rₜ = 11.2 Minuten.

### b) 4-Brom-2-(3-methoxypropyloxy)-phenol

Eine Lösung von 2.6 g 2-(3-Methoxypropyloxy)-phenol in 60 ml Dichlormethan und 40 ml Methanol wird bei Raumtemperatur mit 6.9 g Tetrabutylammoniumtribromid portionenweise versetzt und danach 30 Minuten weitergerührt. Das Reaktionsgemisch wird eingedampft und der Rückstand zwischen Diethylether und Wasser verteilt. Aus den vereinigten organischen Phasen erhält man nach Trocknen über Magnesiumsulfat, Eindampfen und Reinigen des Rückstandes durch FC (700 g Kieselgel, Dichlormethan - Diethylether = 98 : 2) die Titelverbindung: R_{f} (Dichlormethan - Diethylether = 97 : 3) = 0.50; HPLC Rₜ = 14.32 Minuten; FAB-MS (M+H)⁺ = 262.

### c) 4-(3-Benzyloxypropyloxy)-3-(3-methoxypropyloxy)-brombenzol

Ein Gemisch von 4 g 4-Brom-2-(3-methoxypropyloxy)-phenol, 2.3 g Kaliumcarbonat, 3.8 g Benzyl-(3-brompropyl)-ether, einer Spatelspitze Nal und 15 ml Acetonitril wird 30 Stunden am Rückfluss gerührt. Das Reaktionsgemisch wird filtriert und das Filtrat eingedampft. Der Rückstand wird zwischen Essigsäureethylester und Wasser verteilt. Aus den organischen Phasen erhält man durch Vereinigen, Trocknen über Magnesiumsulfat, Eindampfen und Reinigen des Rückstandes mittels FC (Hexan - Essigsäureethylester = 95 : 5) die Titelverbindung: R_{f} (Hexan - Essigsäureethylester = 9:1) = 0.15; HPLC Rₜ = 20.66 Minuten.

### d) 3(S)-Isopropyl-5(S)-{1(S)-azido-3(S)-isopropyl-4(R,S)-hydroxy-4-[4-(3-benzyloxy-propyloxy)-3-(3-methoxypropyloxy)-phenyl]-butyl}-tetrahydrofuran-2-on

Zu einer bei -75° C gerührten Lösung von 500 mg 4-(3-Benzyloxypropyloxy)-3-(3-methoxypropyloxy)-brombenzol in 2 ml Tetrahydrofuran werden 1.3 ml einer 0.9 M Lösung von Butyllithium in Hexan langsam zugetropft. Das Reaktionsgemisch wird 20 Minuten bei -75° C weitergerührt und dann mit einer aus 44.5 mg Magnesiumpulver und 0.158 ml 1.2-Dibromethan in 3 ml Tetrahydrofuran bei Raumtemperatur frisch hergestellten Suspension von Magnesiumbromid tropfenweise versetzt. Nachdem das Reaktionsgemisch 30 Minuten bei -75° C weitergerührt wird, wird eine Lösung von 172 mg 3(S)-Isopropyl-5(S)-[1(S)-azido-3(S)-isopropyl-4-oxo-butyl]-tetrahydrofuran-2-on in 2 ml Tetrahydrofuran tropfenweise zugefügt. Es wird nochmals 30 Minuten bei -75° C gerührt und dann werden bei der gleichen Temperatur 1.2 ml gesättigte Ammoniumchloridlösung zugetropft. Nachdem das Reaktionsgemisch auf Raumtemperatur gebracht wird, wird 3x mit Essigsäureethylester extrahiert. Aus den organischen Phasen erhält man nach Waschen mit Wasser (2x) und gesättigter Kochsalzlösung (1x) , Trocknen über Magnesiumsulfat, Vereinigen, Eindampfen und Reinigen des Rückstandes mittels FC (2x 30 g Kieselgel, Hexan/Essigsäureethylester = 6 : 2) die Titelverbindung: R_{f} (Hexan - Essigsäureethylester = 2 : 1) = 0.23; HPLC Rₜ = 20.27 und 21.07 Minuten. (Diastereomerengemisch); FAB-MS M⁺ = 611.

### e) 3(S)-Isopropyl-5(S)-{1(S)-azido-3(S)-isopropyl-4(R,S)-acetoxy-4-[4-(3-benzyloxy-propyloxy)-3-(3-methoxypropyloxy)-phenyl]-butyl}-tetrahydrofuran-2-on

Eine Lösung von 144 mg 3(S)-Isopropyl-5(S)-{1(S)-azido-3(S)-isopropyl-4(R,S)-hydroxy-4-[4-(3-benzyloxypropyloxy)-3-(3-methoxypropyloxy)-phenyl]-butyl} -tetrahydrofuran-2-on in 1.8 ml Essigsäureanhydrid und 0.057 ml Pyridin wird 30 Stunden bei Raumtemperatur gerührt und dann bei Raumtemperatur unter vermindertem Druck zur Trockne eingedampft. Der Rückstand wird zwischen Dichlormethan (3x) und Wasser-gesättigter Kochsalzlösung (3x) verteilt. Aus den organischen Phasen erhält man nach Vereinigen, Trocknen über Magnesiumsulfat, Eindampfen und Reinigen des Rückstandes mittels FC (Hexan - Essigsäureethylester = 4:1) die Titelverbindung: R_{f} (Hexan - Essigsäureethylester = 2:1) = 0.38 und 0.33; HPLC Rₜ = 21.76 und 21.82 Minuten. (Diastereomerengemisch); FAB-MS M⁺ = 653, (M+Na)⁺ = 676.

### f) 3(S)-Isopropyl-5(S)-{1(S)-amino-3(S)-isopropyl-4-[4-(3-hydroxypropyloxy)-3-(3-methoxypropyloxy)-phenyl]-butyl}-tetrahydrofuran-2-on

Eine Lösung von 151 mg 3(S)-Isopropyl-5(S)-{1(S)-azido-3(S)-isopropyl-4(R,S)-acetoxy-4-[4-(3-benzyloxypropyloxy)-3-(3-methoxypropyloxy)-phenyl]-butyl} -tetrahydrofuran-2-on in 10 ml Ethanol wird bei Normaldruck und Raumtemperatur in Gegenwart von 70 mg PdO während 170 Stunden hydriert. Das Reaktionsgemisch wird filtriert, eingedampft und der Rückstand in 10 ml Ethanol gelöst und nochmals 24 Stunden in Gegenwart von 140 mg PdO bei Normaldruck und Raumtemperatur hydriert. Filtrieren und Eindampfen ergibt die Titelverbindung als Rohprodukt: R_{f} (Dichlormethan/Methanol) = 0.32; HPLC Rₜ = 11.72 Minuten.; FAB-MS (M+H)⁺ = 480. Die Verbindung wird ohne Reinigung in der nächten Stufe eingesetzt.

### g) 3(S)-Isopropyl-5(S)-{1(S)-tertiärbutoxycarbonylamino-3(S)-isopropyl-4-[4-(3-hydroxy-propyloxy)-3-(3-methoxypropyloxy)-phenyl]-butyl}-tetrahydrofuran-2-on

Zu einer bei 0° C gerührten Lösung von 106 mg 3(S)-Isopropyl-5(S)-{1(S)-amino-3(S)-isopropyl-4-[4-(3-hydroxy-propyloxy)-3-(3-methoxypropyloxy)-phenyl]-butyl}-tetrahydrofuran-2-on in 4.5 ml Dichlormethan wird die Lösung von 0.07 ml N-Ethyldiisopropylamin in 0.1 ml Dichlormethan und anschliessend die Lösung von 77 mg Ditertiärbutyl-dicarbonat in 0.4 ml Dichlormethan zugetropft. Das Reaktionsgemisch wird danach auf Raumtemperatur gebracht, 20 Stunden bei Raumtemperatur weitergerührt und dann zur Trockne eingedampft. Aus dem Rückstand erhält man durch Reinigung mittels FC (50 g Kieselgel, Dichlormethan - Methanol = 98 : 2) die Titelverbindung: R_{f} (Dichlormethan - Methanol = 95 : 5) = 0.34; HPLC Rₜ = 19.07 Minuten.; FAB-MS M⁺ = 579, (M+Na)⁺ = 602.

### h) 5(S)-Tertiärbutoxycarbonylamino-4(S)-hydroxy-2(S),7(S)-diisopropyl-8-[4-(3-hydroxypropyloxy)-3-(3-methoxypropyloxy)-phenyl]-octansäure-(N-2-morpholinoethyl)amid

Ein Gemisch von 84 mg 3(S)-Isopropyl-5(S)-{1(S)-tertiärbutoxycarbonylamino-3(S)-isopropyl-4-[4-(3-hydroxypropyloxy)-3-(3-methoxypropyloxy)-phenyl]-butyl}-tetrahydrofuran-2-on, 0.6 ml 4-(2-Aminoethyl)-morpholin und 0.025 ml Eisessig wird 16 Stunden bei Raumtemperatur und 6 Stunden bei 45° C gerührt und dann zwischen Diethylether (2x) und gesättigter NaHCO₃-Lösung (1x) und Wasser (2x) verteilt. Aus den organischen Phasen erhält man durch Vereinigen, Trocknen über Magnesiumsulfat, Eindampfen und Reinigen mittels FC (5 g Kieselgel, Dichlormethan - Methanol = 98 : 2) die Titelverbindung: R_{f} (Dichlormethan - Methanol = 95 : 5) = 0.16; HPLC Rₜ = 14.49 Minuten.; FAB-MS (M+H)⁺ = 710.

### Beispiel 83: 5(S)-Amino-4(S)-hydroxy-2(S),7(S)-diisopropyl-8-[4-methoxy-3-(3-methoxypropyloxy)-phenyl]-octansäure-N-(2-carbamoyl-2,2-dimethyl-ethyl)-amid-semifumarat

Die gerührte Lösung von 2.35 g 5(S)-Amino-4(S)-hydroxy-2(S),7(S)-diisopropyl-8-[4-methoxy-3-(3-methoxypropyloxy)-phenyl]-octansäure-N-(2-carbamoyl-2,2-dimethyl-ethyl)-amid-hydrochlorid (Beispiel 137) in 20 ml Wasser wird nacheinander mit 20 g Eis und 12 ml 2N NaOH versetzt und anschließend mit 3x 50 ml Tertiärtbutylmethylether extrahiert. Die vereinigten organischen Phasen werden mit Magnesiumsulfat getrocknet und eingedampft. Der Eindampfrückstand wird in 25 ml Methanol mit 0.232 g Fumarsäure versetzt. Das Gemisch wird solange gerührt bis eine klare Lösung entstanden ist und anschließend eingedampft. Der Rückstand wird aus 100 ml Acetonitril/Ethanol = 95:5 kristallisiert. Die Kristalle werden abgenutscht und bei 60° C getrocknet. Man erhält die Titelverbindung als weißes Pulver vom Smp. 95 - 104° C.

### Beispiel 84: 5(S)-Amino-2(S),7(S)-diisopropyl-4(S)-hydroxy-8-[4-tertiärbutyl-3-(3-methoxy-propoxy)-phenyl]-octansäure[N-2-(morpholin-4-yl)-ethyl]-amid-dihydrochlorid

Zu 768 mg 5(S)-Tertiärbutoxycarbonylamino-4(S)-hydroxy-2(S),7(S)-diisopropyl-8-[4-tertiärbutyl-3-(3-methoxypropoxy)-phenyl]-octansäure[N-2-(morpholin-4-yl)-ethyl]-amid wird bei 0-5° C eine 4 N Salzsäure-Lösung in Dioxan (20 ml) addiert und die Mischung über 1 Stunde nachgerührt. Anschließend wird das Lösungsmittel durch Lyophilisation im Hochvakuum entfernt, der Rückstand in wasserfreiem Dichlormethan gelöst, über Watte filtriert und das Filtrat eingeengt. Der Rückstand wird erneut mit wenig 4 N Salzsäure in Dioxan versetzt, die erhaltene Lösung lyophilisiert und der Rückstand am Hochvakuum getrocknet. Man erhält die Titelverbindung als weiße amporphe Festsubstanz: R_{f} (Dichlormethan/Methanol/Ammoniak konz. = 9:1:0.1) = 0.23; HPLC Rₜ= 14.5 Minuten; FAB-MS (M+H)⁺= 592.

Die Ausgangsmaterialialien wird werden wie folgt hergestellt:

### a) 5(S)-Tertiärbutoxycarbonylamino-4(S)-hydroxy-2(S),7(S)-diisopropyl-8-[4-tertiärbutyl-3-(3-methoxypropoxy)-phenyl]-octansäure[N-2-(morpholin-4-yl)-ethyl]-amid

Eine Lösung von 756 mg 3(S)-Isopropyl-5(S)-{1(S)-tertiärbutoxycarbonylamino-3(S)-isopropyl-4-[4-tertiärbutyl-3-(3-methoxypropoxy)-phenyl]-butyl}-tetrahydrofuran-2-on in 4 ml 4-(2-Aminoethyl)-morpholin und 0.23 ml Eisessig wird während 4 Stunden bei 65° C gerührt und anschließend eingedampft. Der Rückstand wird zwischen Diethylether (30 ml) und einer gesättigten Natriumhydrogencarbonat-Lösung (10 ml) verteilt, die wässerige Phase mit Diethylether (2 x 30 ml) extrahiert, die vereinten organischen Phasen über Magnesiumsulfat getrocknet und eingeengt. Aus dem Rückstand erhält man nach Reinigung mittels FC (70 g Kieselgel, Dichlormethan/Methanol/Ammoniak konz. = 98:2:1 nach 96:4:1) die Titelverbindung als weißen Schaum: R_{f} (Dichlormethan/Methanol/Ammoniak konz. = 9:1:0.1) = 0.43; HPLC Rₜ= 19.8 Minuten.

### b) 3(S)-Isopropyl-5(S)-{1(S)-tertiärbutoxycarbonylamino-3(S)-isopropyl-4-[4-tertiärbutyl-3-(3-methoxypropoxy)-phenyl]-butyl}-tetrahydrofuran-2-on

Eine Lösung von 1.24 g 3(S)-Isopropyl-5(S)-{4(R,S)-acetoxy-1(S)-azido-3(S)-isopropyl-4(R,S)-[4-tertiärbutyl-3-(3-methoxypropoxy)-phenyl]-butyl}-tetrahydrofuran-2- on in 25 ml Ethanol wird in Gegenwart von 2.4 g PdO/C 5 % (Engelhardt) bei Raumtemperatur und Normaldruck über 28 Stunden hydriert. Das Reaktionsgemisch wird über Celite 545 filtriert, mit Ethanol nach-gewaschen und der Rückstand nach dem Einengen am Hochvakuum getrocknet. Das so erhaltene Produkt (843 mg) wird in 20 ml Dichlormethan gelöst und bei 0-5° C nacheinander mit 0.58 ml N-Diisopropylethylamin und einer Lösung von 638 mg Ditertiärbutyl-dicarbonat in 5 ml Dichlormethan versetzt. Der Ansatz wird bei Raumtemperatur über Nacht gerührt, anschließend das Lösungsmittel unter Vakuum entfernt und das Rohprodukt mittels FC (60 g Kieselgel, Hexan/Essigsäureethylester/Ammoniak konz. = 80:20:1) gereinigt. Man erhält die Titelverbindung als farbloses Öl: R_{f} (Hexan/Essigsäureethylester/Ammoniak konz. = 50:50:1) = 0.90; HPLC Rₜ= 26.2 Minuten.

### c) 3(S)-Isopropyl-5(S)-{4(R,S)-acetoxy-1(S)-azido-3(S)-isopropyl-4(R,S)-[4-tertiärbutyl-3-(3-methoxypropoxy)-phenyl]-butyl}-tetrahydrofuran-2-on

Die Mischung aus 1.15 g 3(S)-Isopropyl-5(S)-{4(R,S)-hydroxy-1(S)-azido-3(S)-isopropyl-4(R,S)-[4-tertiärbutyl-3-(3-methoxypropoxy)-phenyl]-butyl}-tetrahydrofuran-2- on, 11 ml Essigsäureanhydrid und 0.55 ml Pyridin wird bei Raumtemperatur über Nacht gerührt. Das Reaktionsgemisch wird eingeengt und der Rückstand zwischen 100 ml Dichlormethan und 20 ml Wasser verteilt. Das nach extraktiver Aufarbeitung erhaltene Rohprodukt wird durch FC (80 g Kieselgel, Hexan/Essigsäureethylester = 2:1) gereinigt. Man erhält die Titelverbindung als gelbliches Öl: R_{f}(Hexan/Essigsäureethylester = 2:1) = 0.66.

### d) 3(S)-Isopropyl-5(S)-{4(R,S)-hydroxy-1(S)-azido-3(S)-isopropyl-4(R,S)-[4-tertiärbutyl-3-(3-methoxypropoxy)-phenyl]-butyl}-tetrahydrofuran-2-on

In analoger Weise wie in Beispiel 185c) beschrieben wird 4-Tertiärbutyl-3-(3-methoxy-propoxy)-brombenzol (2.35 g), gelöst in 60 ml Tetrahydrofuran, mit 4.86 ml einer 1 N n-Butyllithium-Lösung (in Hexan) und anschließend mit einer Suspension von Magnesiumbromid in 20 ml Tetrahydrofuran (hergestellt aus 380 mg Magnesiumpulver und 1.35 ml 1,2-Dibromethan umgesetzt. Zu der erhaltenen Suspension wird eine Lösung von 1.46 g 3(S)-Isopropyl-5(S)-[1(S)-azido-3(S)-isopropyl-4-oxobutyl]-tetrahydrofuran-2-on in 6 ml Tetrahydrofuran bei - 70° C über 20 Minuten zugetropft und für eine weitere Stunde nachgerührt. Nach extraktiver Aufarbeitung wird das Rohprodukt durch FC (300 g Kieselgel, Hexan/Essigsäureethylester = 5:1 nach 3:1) gereinigt. Man erhält die Titelverbindung als blaßgelbes Öl: R_{f} (Hexan/Essigsäureethylester = 2:1) = 0.57; HPLC Rₜ= 22.9 und 24.1 Minuten (Diastereomerengemisch).

### e) 4-Tertiärbutyl-3-(3-methoxypropoxy)-brombenzol

Die Suspension aus 2.60 g 5-Brom-2-tertiärbutyl-phenol, 4.34 g 3-Methoxypropylbromid und 5.55 g Cesiumcarbonat in 40 ml Aceton wird bei 60° C über Nacht gerührt. Nach dem Abkühlen auf Raumtemperatur wird filtriert und das nach Einengen des Filtrats erhaltene Rohprodukt mittels FC (80 g Kieselgel, Hexan/Essigsäureethylester = 98:2) gereinigt. Man erhält die Titelverbindung als Öl: R_{f} (Hexan/Essigsäureethylester = 9:1) = 0.56.

### Beispiel 85: 5(S)-Amino-4(S)-hydroxy-7(S)-isopropyl-2(R)-methyl-8-[4-methoxy-3-(3-methoxypropyloxy)-phenyl]-octansäure-N-[2-(4-hydroxypiperidin-1-yl)ethyl]amid-dihydrochlorid

100 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-N-[2-(4-hydroxy-piperidin-1-yl)-ethyl]-amid werden in 3 ml 4N Salzsäure in Dioxan bei 0° C gelöst und für 2 Stunden bei 0° C gerührt. Das Reaktionsgemisch wird lyophilisiert und man erhält die Titelverbindung: R_{f} (Dichlormethan-Methanol = 8:2) = 0.08; HPLC Rₜ = 8.85 Minuten; FAB-MS (M+H)⁺ = 552.

Das Ausgangsmaterial wird wie folgt hergestellt:

### a) 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-N-[2-(4-hydroxypiperidin-1-yl)-ethyl]-amid

102 mg 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105e) und 0.5 g N-(2-Aminoethyl)-4-hydroxypiperidin werden während 2 Stunden bei 80° C verrührt. Das Reaktionsgemisch wird mittels FC (60 g Kieselgel, Dichlormethan-Methanol = 4:1) gereinigt. Man erhält die Titelverbindung: R_{f} (Dichlormethan-Methanol = 4:1) = 0.16

### Beispiel 86: 5(S)-Amino-4(S)-hydroxy-7(S)-isopropyl-2(R)-methyl-8-[4-methoxy-3-(3-methoxypropyloxy)-phenyl]-octansäure-N-(2,2-dimethyl-2-morpholino-ethyl)amid-dihydrochlorid

Analog Beispiel 85 erhält man die Titelverbindung ausgehend von 120 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-N-(2,2-dimethyl-2-morpholino-ethyl)amid: R_{f} (Dichlormethan-Methanol = 9:1) = 0.07; HPLC Rₜ = 9.22 Minuten; FAB-MS (M+H)⁺ = 566.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105 e) und 4-(2-Amino-1.1-dimethyl-ethyl)-morpholin hergestellt.

### Beispiel 87: 5(S)-Amino-4(S)-hydroxy-7(S)-isopropyl-2(R)-methyl-8-[4-methoxy-3-(3-methoxypropyloxy)-phenyl]-octansäure-N-[2-(trans-2.6-dimethyl-morpholino)ethyl]amid-dihydrochlorid

Analog Beispiel 85 erhält man die Titelverbindung ausgehend von 102 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-N-[2-(*trans*-2.6-dimethyl-morpholino)ethyl]amid : R_{f} (Dichlormethan-Methanol = 8:2) = 0.27; HPLC Rₜ = 9.56 Minuten; FAB-MS (M+H)⁺ = 566.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105 e) und 4-(2-Aminoethyl)-*trans*-2.6-dimethyl-morpholin hergestellt.

### a) 4-(2-Amino-ethyl)-2.6-(trans)-dimethyl-morpholin

8.20 g 4-(2-Phtaloylaminoethyl)-*trans*-2.6-dimethyl-morpholin werden in 250 ml Ethylalkohol mit 2.76 ml Hydrazin-hydrat während 2 Stunden am Rückfluss gerührt. Das Reaktionsgemisch wird verdünnt mit Diethylether und anschliessend klarfiltriert. Aus dem eingeengten Filtrat erhält man die rohe Titelverbindung: R_{f} (Dichlormethan-Methanol-Ammoniak konz. = 40:10:1) = 0.21

### b) 4-(2-Phtaloylaminoethyl)-trans-2.6-dimethyl-morpholin

10.16 g N-(2-Bromethyl)-phtalimid und 11.50 g *trans*-2.6-Dimethylmorpholin werden 30 Minuten bei 130° C gerührt. Das Reaktionsgemisch wird anschliessend zwischen Eis-Wasser und Essigsäureethylester verteilt. Die eingedampften organischen Phasen werden mittels FC (240g Kieselgel, Essigsäureethylester-Hexan = 1:2) gereinigt: Man erhält die Titelverbindung: R_{f} (Essigsäureethylester-Hexan = 1:2) = 0.39

### Beispiel 88: 5(S)-Amino-4(S)-hydroxy-7(S)-isopropyl-2(R)-methyl-8-[4-methoxy-3-(3-methoxypropyloxy)-phenyl]-octansäure-N-[2-(cis-2.6-dimethyl-morpholino)ethyl]amid-dihydrochlorid

Analog Beispiel 85 erhält man die Titelverbindung ausgehend von 97 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-N-[2-(*cis*-2.6-dimethyl-morpholino)ethyl]amid : R_{f} (Dichlormethan-Methanol = 8:2) = 0.21; HPLC Rₜ = 9.38 Minuten; FAB-MS (M+H)⁺ = 566.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl- tetrahydrofuran-5-on (Beispiel 105e) und 4-(2-Amino-ethyl)-*cis*-2.6-dimethyl-morpholin hergestellt.

Das 4-(2-Amino-ethyl)-*cis*-2.6-dimethyl-morpholin wird analog Beispiel 87 a) und 87 b) aus *cis*-2.6-Dimethylmorpholin hergestellt.

### Beispiel 89: 5(S)-Amino-4(S)-hydroxy-7(S)-isopropyl-2(R)-methyl-8-[4-methoxy-3-(3-methoxypropyloxy)-phenyl]-octansäure-N-(2-piperidinoethyl)amid-dihydrochlorid

Analog Beispiel 85 erhält man die Titelverbindung ausgehend von 74 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-N-(2-piperidinoethyl)amid : R_{f} (Dichlormethan-Methanol = 8:2) = 0.09; HPLC Rₜ = 9.55 Minuten; FAB-MS (M+H)⁺ = 536.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105 e) und N-(2-Piperidinoethyl)amin hergestellt.

### Beispiel 90: 5(S)-Amino-4(S)-hydroxy-7(S)-isopropyl-2(R)-methyl-8-[4-methoxy-3-(3-methoxypropyloxy)-phenyl]-octansäure-N-[2-(4-methoxypiperidino)-ethyl]-amid-dihydrochlorid

Analog Beispiel 85 erhält man die Titelverbindung ausgehend von 74 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-N-[2-(4-methoxy-piperidino)ethyl]-amid : R_{f} (Dichlormethan-Methanol = 8:2) = 0.12; HPLC Rₜ = 9.39 Minuten; FAB-MS (M+H)⁺ = 566.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105 e) und 1-(2-Aminoethyl)-4-methoxy-piperidin hergestellt.

Das 1-(2-Amino-ethyl)-4-methoxypiperidin wird analog Beispiel 87 a) und 87 b) aus 4-Methoxy-piperidin hergestellt.

### Beispiel 91: 5(S)-Amino-4(S)-hydroxy-7(S)-isopropyl-2(R)-methyl-8-[4-methoxy-3-(3-methoxypropyloxy)-phenyl]-octansäure-N-(2-thiomorpholinoethyl)amid-dihydrochlorid

Analog Beispiel 85 erhält man die Titelverbindung ausgehend von 110 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-N-(2-thiomorpholino-ethyl)amid: R_{f} (Dichlormethan-Methanol = 8:2) = 0.17; HPLC Rₜ = 9.53 Minuten; FAB-MS (M+H)⁺ = 554.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105e) und 4-(2-Aminoethyl)thiomorpholin hergestellt.

### Beispiel 92: 5(S)-Amino-4(S)-hydroxy-7(S)-isopropyl-2(R)-methyl-8-[4-methoxy-3-(3-methoxypropyloxy)-phenyl]-octansäure-[N-[3-hydroxypropyl)]amid-hydrochlorid

Analog Beispiel 85 erhält man die Titelverbindung ausgehend von 110 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(3-hydroxypropyl)]amid : R_{f} (Dichlormethan-Methanol = 9:1) = 0.07; HPLC Rₜ= 9.65 Minuten; FAB-MS (M+H)⁺ = 483.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyltetrahydrofuran-5-on (Beispiel 105 e) und 3-Amino-1-propanol hergestellt.

### Beispiel 93: 5(S)-Amino-4(S)-hydroxy-7(S)-isopropyl-2(R)-methyl-8-[4-methoxy-3-(3-methoxypropyloxy)-phenyl]-octansäure-[N-(4-hydroxybutyl)]amid-hydrochlorid

Analog Beispiel 85 erhält man die Titelverbindung ausgehend von 112 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(4-hydroxybutyl)]amid : R_{f} (Dichlormethan-Methanol = 9:1) = 0.07; HPLC Rₜ= 9.83 Minuten; FAB-MS (M+H)⁺ = 497.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105 e) und 4-Amino-1-butanol hergestellt.

### Beispiel 94: 5(S)-Amino-4(S)-hydroxy-7(S)-isopropyl-2(R)-methyl-8-[4-methoxy-3-(3-methoxypropyloxy)-phenyl]-octansäure-[N-(4-acetoxybutyl)]amid-hydrochlorid

Analog Beispiel 85 erhält man die Titelverbindung ausgehend von 27 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(4-acetoxybutyl)]amid : R_{f} (Dichlormethan-Methanol = 9:1) = 0.16; HPLC Rₜ= 11.53 Minuten; FAB-MS (M+H)⁺ = 539.

Das Ausgangsmaterial wird wie folgt hergestellt:

### a) 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(4-acetoxybutyl)]amid

116 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(4-hydroxybutyl)]amid (Beispiel 93) werden in 5 ml Tetrahydrofuran bei 0° C mit 30 ml Triethylamin, 2 mg 4-(N,N'-Dimethylamino)pyridin (DMAP) und 20 ml Essigsäureanhydrid versetzt. Die Reaktionslösung wird 18 Stunden bei Raumtemperatur weitergerührt. Das Reaktionsgemisch wird zwischen Diethylether und Wasser-gesättigter Kochsalzlösung verteilt. Die eingedampften organischen Phasen werden durch FC (40 g Kieselgel, Laufmiffel Dichlormethan-Methanol= 95:5) gereinigt. Man erhält die Titelverbindung: R_{f} (Dichlormethan-Methanol = 9:1) = 0.60

### Beispiel 95: 5(S)-Amino-4(S)-hydroxy-7(S)-isopropyl-2(R)-methyl-8-[4-methoxy-3-(3-methoxypropyloxy)-phenyl]-octansäure-[N-(3-cyanopropyl)]amid-hydrochlorid

Analog Beispiel 85 erhält man die Titelverbindung ausgehend von 107 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(3-cyanopropyl)]amid : R_{f} (Dichlormethan-Methanol = 9:1) = 0.07; HPLC Rₜ= 10.76 Minuten; FAB-MS (M+H)⁺ = 492.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105 e) und 4-Amino-butyronitril hergestellt.

### Beispiel 96: 5(S)-Amino-4(S)-hydroxy-7(S)-isopropyl-2(R)-methyl-8-[4-methoxy-3-(3-methoxypropyloxy)-phenyl]-octansäure-[N-(3-methoxypropyl)]amid-hydrochlorid

Analog Beispiel 85 erhält man die Titelverbindung ausgehend von 107 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(3-methoxypropyl)]amid : R_{f}(Dichlormethan-Methanol = 8:2) = 0.34; HPLC Rₜ= 10.70 Minuten; FAB-MS (M+H)⁺= 497.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105 e) und 3-Methoxy-propylamin hergestellt.

### Beispiel 97: 5(S)-Amino-4(S)-hydroxy-7(S)-isopropyl-2(R)-methyl-8-[4-methoxy-3-(3-methoxypropyloxy)-phenyl]-octansäure-[N-(2-acetylamino-ethyl)]amid-hydrochlorid

Analog Beispiel 85 erhält man die Titelverbindung ausgehend von 82 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(2-acetylamino-ethyl)]amid: R_{f}(Dichlormethan-Methanol = 8:2) = 0.17; HPLC Rₜ = 9.54 Minuten; FAB-MS (M+H)⁺= 510.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105 e) und N-Acetyl-ethylendiamin hergestellt.

### Beispiel 98: 5(S)-Amino-4(S)-hydroxy-7(S)-isopropyl-2(R)-methyl-8-[4-methoxy-3-(3-methoxypropyloxy)-phenyl]-octansäure-{N-[2-(2-pyridyl)-ethyl]}-amid-hydrochlorid

Analog Beispiel 85 erhält man die Titelverbindung ausgehend von 118 mg 5(S)-Tertiärbutoxycarbonylamino-4(S)-hydroxy-7(S)-isopropyl-2(R)-methyl-8-[4-methoxy-3-(3- methoxypropyloxy)-phenyl]-octansäure-{N-[2-(2-pyridyl)-ethyl]}-amid : R_{f} (Dichlormethan-Methanol = 9:1) = 0.09; HPLC Rₜ = 8.88 Minuten; FAB-MS (M+H)⁺= 530.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105 e) und 2-(2-Aminoethyl)-pyridin hergestellt.

### Beispiel 99: 5(S)-Amino-4(S)-hydroxy-7(S)-isopropyl-2(R)-methyl-8-[4-methoxy-3-(3-methoxypropyloxy)-phenyl]-octansäure-N-[2-(N'-oxomorpholino)ethyl]-amid-hydrochlorid

Analog Beispiel 85 erhält man die Titelverbindung ausgehend von 82 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-N-[2-(N'-oxomorpholino)ethyl]amid : R_{f} (Dichlormethan-Methanol = 8:2) = 0.07 HPLC Rₜ= 9.04 Minuten; FAB-MS (M+H)⁺= 554.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105 e) und 4-(2-Aminoethyl)-N-oxo-morpholin hergestellt.

Das Ausgangsmaterial wird wie folg hergestellt:

### a) 4-(2-Aminoethyl)-N-oxo-morpholin

2.8 g 4-(2-Benzyloxycarbonylaminoethyl)-N-oxo-morpholin werden in Gegenwart von 0.30 g Pd/C 10% in Methanol für 10 Minuten bei Raumtemperatur und Normaldruck hydriert. Das Reaktionsgemisch wird filtriert und eingedampft. Man erhält die rohe Titelverbindung: ¹H-NMR (CD₃OD), δ(ppm) = 4.90 (2H, s), 4.20 (1H, m), 3.87 - 2.80 (10H, m), 2.50 (1H, m)

### b) 4-(2-Benzyloxycarbonylaminoethyl)-N-oxo-morpholin

10.6 g 4-(2-Benzyloxycarbonylaminoethyl)-morpholin werden in 12 ml Methanol bei 60° C unter Rühren in 6 Portionen im Abstand von 12 Stunden mit jeweils 1.48 ml Wasserstoffperoxid 30% versetzt. Das abgekühlte Reaktionsgemisch wird vorsichtig mit gesättigter Natriumsulfitlösung versetzt, bis der Peroxidüberschuss vernichtet ist. Das Methanol wird abgedampft und die resultierende Suspension in Essigsäureethylester-Methanol 99:1 aufgenommen. Das Gemisch wird getrocknet mit Magnesiumsulfat und nach einer Filtration, das Filtrat eingedampft. Man erhält die Titelverbindung nach Kristallisation aus Essigsäureethylester: R_{f} (Dichlormethan-Methanol = 8:2) = 0.17; Smp. 163° C.

### Beispiel 100: 5(S)-Amino-4(S)-hydroxy-7(S)-isopropyl-2(R)-methyl-8-[4-methoxy-3-(3-methoxypropyloxy)-phenyl]-octansäure-{N-[3-(tertiärbutylsulfonyl)-propyl]}-amid-hydrochlorid

Analog Beispiel 85 erhält man die Titelverbindung ausgehend von 110 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-{N-[3-(tertiärbutylsulfonyl)-propyl]}-amid : R_{f} (Essigsäureethylester-Methanol-Ammoniak konz. = 80:15:5) = 0.45; HPLC Rₜ = 11.27 Minuten; FAB-MS (M+H)⁺ = 587.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105 e) und 3-Amino-1-(tertiärbutylsulfonyl)-propan hergestellt.

Das Ausgangsmaterial wird wie folgt hergestellt:

### a) 3-Amino-1-(tertiärbutylsulfonyl)-propan

1.0 g 3-Aminopropyl-(tertiärbutylsulfonyl)-propan werden bei 0° C in 30 ml Wasser gelöst. Nacheinander werden 2.14 g Kaliumpermanganat und 2 ml 4N Salzäure in 30 ml Wasser zugegeben und der Ansatz über Nacht bei 0° C gerührt. Die dunkle Suspension wird abfiltriert und mit 100 ml heissem Wasser nachgewaschen. Das Filtrat wird mit 50 ml Toluol versetzt und eingeengt. Die ausgefallenen weissen Kristalle werden mittels FC (10 g Kieselgel, Essigsäureethylester-Methanol-Ammoniak konz. = 80:15:5) gereinigt. Man erhält die Titelverbindung: R_{f} (Essigsäureethylester-Methanol-Ammoniak konz. = 80:15:5) = 0.20.

### Beispiel 101: 5(S)-Amino-4(S)-hydroxy-7(S)-isopropyl-2(R)-methyl-8-[4-methoxy-3-(3-methoxypropyloxy)-phenyl]-octansäure-{N-[3-(ethylsulfonyl)-propyl]}-amid-hydrochlorid

Analog Beispiel 85 erhält man die Titelverbindung ausgehend von 44 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-{N-[3-(ethylsulfonyl)-propyl]}-amid : R_{f} (Essigsäureethylester-Methanol-Ammoniak konz. = 80:15:5) = 0.26; HPLC Rₜ = 10.40 Minuten; FAB-MS (M+H)⁺ = 559.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105 e) und 3-Amino-1-(ethylsulfonyl)-propan hergestellt.

Das Ausgangsmaterial wird wie folgt hergestellt:

### a) 3-Amino-1-(ethylsulfonyl)-propan

1.0 g 3-Aminopropyl-ethyl-sulfid werden in 35 ml Methanol bei 0° C vorgelegt, 15.5 g Ozone wird in 35 ml Wasser addiert und 4 Stunden bei 0° C gerührt. 200 ml Wasser werden addiert und mit 3 x 150 ml Dichlormethan extrahiert. Die eingedampften organischen Ertrakte werden durch FC (10 g Kieselgel, Essigsäureethylester-Methanol-Ammoniak konz. = 80:15:5) gereinigt. Man erhält die Titelverbindung: R_{f} (Essigsäureethylester-Methanol-Ammoniak konz. = 80:15:5) = 0.15.

### Beispiel 102: 5(S)-Amino-4(S)-hydroxy-7(S)-isopropyl-2(R)-methyl-8-[4-methoxy-3-(3-methoxypropyloxy)-phenyl]-octansäure-{N-[2-(ethylsulfonyl)-ethyl]}-amid-hydrochlorid

Analog Beispiel 85 erhält man die Titelverbindung ausgehend von 90 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-{N-[2-(ethylsulfonyl)-ethyl]}-amid : R_{f} (Essigsäureethylester-Methanol-Ammoniak konz. = 80:15:5) = 0.39; HPLC Rₜ= 10.50 Minuten; FAB-MS (M+H)⁺ = 545.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105 e) und 2-Amino-1-(ethylsulfonyl)-ethan hergestellt.

### Beispiel 103: 5(S)-Amino-4(S)-hydroxy-7(S)-isopropyl-2(R)-methyl-8-[4-methoxy-3-(3-methoxypropyloxy)-phenyl]-octansäure-{N-[2-(N-butylsulfamoyl)-ethyl}-amid-hydrochlorid

Analog Beispiel 85 erhält man die Titelverbindung ausgehend von 67 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-{N-[2-(N-butylsulfamoyl)-ethyl]}-amid : R_{f} (Essigsäureethylester-Methanol-Ammoniak konz. = 80:15:5) = 0.41; HPLC Rₜ= 12.52 Minuten; FAB-MS (M+H)⁺ = 588.

Das Ausgangsmaterial wird analog Beispiel 85 a) aus 2-{1(S)-Tertiärbutoxycarbonylamino-3(S)-isopropyl-4-[4-methoxy-3-(3-methoxypropyloxy)-phenyl]-butyl}-4(R)-methyl-tetrahydrofuran-5-on (Beispiel 105 e) und 2-Aminoethyl-(N-butyl)-sulfonamid hergestellt.

### Beispiel 104: 5(S)-Amino-4(S)-hydroxy-7(S)-isopropyl-2(R)-methyl-8-[4-methoxy-3-(3-methoxypropyloxy)-phenyl]-octansäure-{N-[2-(N,N-dimethylsulfamoyl)-ethyl]}-amid-hydrochlorid

Analog Beispiel 85 erhält man die Titelverbindung ausgehend von 120 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-{N-[2-(N,N-dimethylsulfamoyl)-ethyl]}-amid : R_{f} (Essigsäureethylester-Methanol-Ammoniak konz. = 80:15:5) = 0.43; HPLC Rₜ = 11.03 Minuten; FAB-MS (M+H)⁺ = 560.

Das Ausgangsmaterial wird analog Beispiel 85 a) aus 2-{1(S)-Tertiärbutoxycarbonylamino-3(S)-isopropyl-4-[4-methoxy-3(3-methoxypropyloxy)-phenyl]-butyl}-4(R)-methyl-tetrahydrofuran-5-on (Beispiel 105 e) und 2-Aminoethyl-(N,N-dimethyl)-sulfonamid hergestellt.

### Beispiel 105: 5(S)-Amino-4(S)-hydroxy-7(S)-isopropyl-2(R)-methyl-8-[4-methoxy-3-(3-methoxypropyloxy)-phenyl]-octansäure-[N-(3-carbamoyl-propyl)]-amid-hydrochlorid

84 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(3-carbamoyl-propyl)]-amid werden in 3 ml 4N Salzsäure in Dioxan bei 0° C gelöst und für 2 Stunden bei 0° C gerührt. Das Reaktionsgemisch wird lyophilisiert und man erhält die Titel-verbindung: R_{f} (Dichlormethan-Methanol = 9:1) = 0.04; HPLC Rₜ = 9.44 Minuten; HR FAB-MS (M+H)⁺ = 510.

Die Ausgansmaterialien werden wie folgt hergestellt:

### a) 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(3-carbamoyl-propyl)]-amid

115 mg 5(S)-Tertiärbutoxycarbonylamino-4(S)-tertiärbutyldimethylsilyloxy-7(S)-isopropyl-2(R)-methyl-8-[4-methoxy-3-(3-methoxy-propyloxy)-phenyl]-octansäure-[N-(3-carbamoylpropyl)]-amid werden in 4 ml Dimethylformamid bei 0° C mit 50 mg Tetrabutylammoniumfluorid-trihydrat versetzt. Das Reaktionsgemisch wird noch 5 Stunden bei Raumtemperatur weitergerührt und anschliessend eingedampft. Der Eindampfrückstand wird mit 20 ml gesättigter Natriumhydrogencarbonatlösung versetzt und mehrmals mit Essigsäureethylester extrahiert. Die organischen Phasen werden mit gesättigter Kochsalzlösung gewaschen und eingedampft. Der Rückstand wird mittels FC (18 g Kieselgel, Dichlormethan-Methanol = 9:1) gereinigt. Man erhält die Titelverbindung: R_{f} (Dichlormethan-Methanol = 9:1) = 0.24

### b) 5(S)-Tertiärbutoxycarbonylamino-4(S)-tertiärbutyldimethylsilyloxy-7(S)-isopropyl-2(R)-methyl-8-[4-methoxy-3-(3-methoxypropyloxy)-phenyl]-octansäure-[N-(3-carbamoyl-propyl)]-amid

128 mg 5(S)-Tertiärbutoxycarbonylamino-4(S)-tertiärbutyldimethylsilyloxy-7(S)-isopropyl-2(R)-methyl-8-[4-methoxy-3-(3-methoxy-propyloxy)-phenyl]-octansäure werden in 8 ml Dimethylformamid bei 0° C nacheinander mit 67 µl Triethylamin, 34 mg 4-Aminobuttersäureamid-hydrochlorid und 38 µl Cyanphosphonsäure-diethylester versetzt. Das Reaktionsgemisch wird noch 18 Stunden bei Raumtemperatur gerührt. Das Reaktionsgemisch wird eingedampft und der Rückstand mit 20 ml Citronensäurelösung 10 % und Eis versetzt. Man extrahiert mehrmals mit Essigsäureethylester und wäscht anschliessend die organischen Phasen mit gesättigter Natriumhydrogen-carbonatlösung und gesättigter Kochsalzlösung. Der Eindampfrückstand wird mittels FC (70g Kieselgel, Dichlormethan-Methanol = 9:1) gereinigt. Man erhält die Titelverbindung: R_{f} (Dichlormethan-Methanol = 9:1) = 0.38

### c) 5(S)-Tertiärbutoxycarbonylamino-4(S)-tertiärbutyldimethylsilyloxy-7(S)-isopropyl-2(R)-methyl-8-[4-methoxy-3-(3-methoxypropyloxy)-phenyl]-octansäure

4.45 g 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure (roh) werden in 45 ml Dimethylformamid mit 2.36 g tert-Butyldimethylsilylchlorid und 2.03 g Imidazol während 6 Tagen bei Raumtemperatur gerührt. Der Eindampfrückstand wird zwischen Citronensäurelösung 10% und Essigsäureethylester verteilt. Die eingeengte organische Phase wird in 20 ml Tetrahydrofuran, 8 ml Wasser und 20 ml Essigsäure während 16 Stunden bei Raumtemperatur gerührt. Der Eindampfrückstand wird mit Eis/Wasser versetzt und anschliessend mit Essigsäureethylester extrahiert. Aus der organischen Phase erhält man nach FC (260 g Kieselgel, Essigsäureethylester-Hexan = 1:1) die Titelverbindung: R_{f} (Essigsäureethylester-Hexan =1:1) = 0.32

### d) 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure

3.6 g 2-{1(S)-Tertiärbutoxycarbonylamino-3(S)-isopropyl-4-[4-methoxy-3-(3-methoxy-propyl-oxy)-phenyl]-butyl}-4(R)-methyl-tetrahydrofuran-5-on werden in 210 ml 1.2-Dimethoxyethan/Wasser (2:1) bei Raumtemperatur mit 28.5 ml 1N Lithiumhydroxid-lösung versetzt. Das Reaktionsgemisch wird noch 1 Stunde bei Raumtemperatur weitergerührt. Der Eindampfrückstand wird mit Eis und wässriger Citronensäurelösung 10% versetzt. Durch mehrmalige Extraktion mit Chloroform erhält man die rohe Titelverbindung: R_{f} (Essigsäureethylester-Hexan = 1:1) = 0.05; HPLC Rₜ = 16.41 Minuten

### e) 2-{1(S)-Tertiärbutoxycarbonylamino-3(S)-isopropyl-4-[4-methoxy-3-(3-methoxy-propyloxy)-phenyl]-butyl}-4(R)-methyl-tetrahydrofuran-5-on

5.6 g 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-(N-butyl)-amid (Beispiel 32) werden in 240 ml Chloroform bei Raumtemperatur mit 2.02 g p-Toluolsufonsäure (Monohydrat) versetzt und noch 18 Stunden bei Raumtemperatur weitergerührt. Das Reaktionsgemisch wird eingedampft und der Rückstand zwischen Diethylether und 0.1 N Salzsäure verteilt. Aus dem Eindampfrückstand der organischen Phasen erhält man nach FC (160 g Kieselgel, Laufmittel Essigsäureethylester-Hexan 1:1) die Titelverbindung: R_{f} (Essigsäureethylester-Hexan = 2:1) = 0.47; Smp. 86 - 87° C

### Beispiel 106: 5(S)-Amino-4(S)-hydroxy-7(S)-isopropyl-2(R)-methyl-8-[4-methoxy-3-(3-methoxypropyloxy)-phenyl]-octansäure-{N-[3-(1H-tetrazol-5-yl)-propyl]}-amid-hydrochlorid

Analog Beispiel 105 erhält man die Titelverbindung ausgehend von 47 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-{N-[3-(1H-tetrazol-5-yl)-propyl]}-amid nach Lyophilisation: R_{f} (Dichlormethan-Methanol = 8:2) = 0.46; HPLC Rₜ= 9.97 Minuten; FAB-MS (M+H)⁺ = 535.

Das Ausgangsmaterial wird analog Beispiel 105 a) und 105 b) aus 5(S)-Tertiärbutoxycarbonylamino-4(S)-tertiärbutyldimethylsilyloxy-7(S)-isopropyl-2(R)-methyl-8-[4-methoxy-3-(3-methoxypropyloxy)-phenyl]-octansäure (Beispiel 105 c) und 3-(1H-Tetrazol-5-yl)-propylamin hergestellt.

### Beispiel 107: 5(S)-Amino-4(S)-hydroxy-7(S)-isopropyl-2(R)-methyl-8-[4-methoxy-3-(3-methoxypropyloxy)-phenyl]-octansäure-{N-[3-(1H-imidazol-5-yl)-propyl]}-amid-hydrochlorid

Analog Beispiel 105 erhält man die Titelverbindung ausgehend von 43 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-{N-[3-(1H-imidazol-5-yl)-propyl]}-amid nach Lyophilisation : R_{f} (Dichlormethan-Methanol = 8:2) = 0.13; HPLC Rₜ= 8.83 Minuten; FAB-MS (M+H)⁺ = 533.

Das Ausgangsmaterial wird analog Beispiel 105 a) und 105 b) aus 5(S)-Tertiärbutoxycarbonylamin-4(S)-tertiärbutyldimethylsilyloxy-7(S)-isopropyl-2(R)-methyl-8-[4-methoxy-3-(3-methoxypropyloxy)-phenyl]-octansäure (Beispiel 105 c) und 3-(1H-Imidazol-5-yl)-propylamin hergestellt.

### Beispiel 108: 5(S)-Amino-4(S)-hydroxy-7(S)-isopropyl-2(R)-methyl-8-[4-methoxy-3-(3-methoxypropyloxy)-phenyl]-octansäure-{N-[3-(3-methyl-1,2,4-oxadiazol-5-yl)-propyl]}-amid-hydrochlorid

Analog Beispiel 105 erhält man die Titelverbindung ausgehend von 140 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-{N-[3-(3-methyl-1.2,4-oxadiazol-5-yl)-propyl]}-amid nach Lyophilisation : R_{f} (Dichlormethan-Methanol = 9:1) = 0.12; HPLC Rₜ= 11.05 Minuten; FAB-MS (M+H)⁺ = 549.

Das Ausgangsmaterial wird analog Beispiel 105 a) und 105 b) aus 5(S)-Tertiärbutoxycarbonylamino-4(S)-tertiärbutyldimethylsilyloxy-7(S)-isopropyl-2(R)-methyl-8-[4-methoxy-3-(3-methoxypropyloxy)-phenyl]-octansäure (Beispiel 105 c) und 3-(3-Methyl-1.2,4-oxadiazol-5-yl)-propylamin hergestellt

### a) 3-(3-Methyl-1,2,4-oxadiazol-5-yl)-propylamin

272 mg 3-Methyl-5-[3-(N-phthaloylamino)propyl]--1,2,4-oxadiazol werden in 10 ml Ethylalkohol mit 146 ml Hydrazinhydrat während 2 Stunden am Rückfluss gerührt. Das Reaktionsgemisch wird mit Dietylether verdünnt und anschliessend klarfiltriert. Aus dem eingedampften Filtrat wird die rohe Titelverbindung erhalten: R_{f} (Dichlormethan-Methylalkohol-Ammoniak konz. = 40:10:1) = 0.37

### b) 3-Methyl-5-[3-(N-phthaloylamino)propyl]-1,2,4-oxadiazol

2.08 g Acetamidoxim werden in 200 ml Tetrahydrofuran bei Raumtemperatur mit 0.84 g Natriumhydrid-Dispersion (80%) versetzt und 2 Stunden bei 60° C gerührt. Anschliessend versetzt man mit einer Lösung aus 2.47 g 4-(N-Phthaloylamino)buttersäuremethylester in 30 ml Tetrahydrofuran und rührt weitere 3 Stunden bei 60° C. Das Reaktionsgemisch wird auf 1N Salzsäure/Eis gegossen und mehrmals mit Essigsäureethylester extrahiert. Die getrockneten organischen Phasen werden eingedampft und der Rückstand in 60 ml Xylol während 3 Stunden am Wasserabscheider gekocht. Das Lösungsmittel wird abgedampft und aus dem Rückstand nach FC (40 g Kieselgel, Essigsäureethylester-Hexan = 1:1) die Titelverbindung erhalten: R_{f} (Essigsäureethylester-Hexan = 1:1) = 0.26

### Beispiel 109: 5(S)-Amino-4(S)-hydroxy-7(S)-isopropyl-2(R)-methyl-8-[4-methoxy-3-(3-methoxypropyloxy)-phenyl]-octansäure-[N-(3-aminopropyl)]-amid-dihydrochlorid

Analog Beispiel 105 erhält man die Titelverbindung ausgehend von 125 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(3-tertiärbutoxycarbonylamino-propyl)]-amid nach Lyophilisation : R_{f} (Dichlormethan-Methanol-Ammoniak konz. = 40:10:1) = 0.08; HPLC Rₜ= 6.48 Minuten; FAB-MS (M+H)⁺ = 482.

Das Ausgangsmaterial wird analog Beispiel 105 a) und 105 b) aus 5(S)-Tertiärbutoxycarbonylamino-4(S)-tertiärbutyldimethylsilyloxy-7(S)-isopropyl-2(R)-methyl-8-[4-methoxy-3-(3-methoxypropyloxy)-phenyl]-octansäure (Beispiel 105 c) und 3-Tertiärbutoxycarbonylamino-propylamin hergestellt

### Beispiel 110: 5(S)-Amino-4(S)-hydroxy-7(S)-isopropyl-2(R)-methyl-8-[4-methoxy-3-(3-methoxypropyloxy)-phenyl]-octansäure-[N-[2-dimethylamino-ethyl)]-amid-dihydrochlorid

Analog Beispiel 105 erhält man die Titelverbindung ausgehend von 38 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-[2-dimethylamino-ethyl)]-amid nach Lyophilisation : R_{f} (Dichlormethan-Methanol-Ammoniak konz. = 350:50:1) = 0.03; HPLC Rₜ= 8.61 Minuten; FAB-MS (M+H)⁺ = 496.

Das Ausgangsmaterial wird analog Beispiel 105 a) und 105 b) aus 5(S)-Tertiärbutoxycarbonylamino-4(S)-tertiärbutyldimethylsilyloxy-7(S)-isopropyl-2(R)-methyl-8-[4-methoxy-3-(3-methoxypropyloxy)-phenyl]-octansäure (Beispiel 105 c) und 2-Dimethylaminoethylamin hergestellt.

### Beispiel 111: 5(S)-Amino-4(S)-hydroxy-7(S)-isopropyl-2(R)-methyl-8-[4-methoxy-3-(3-methoxypropyloxy)-phenyl]-octansäure-N-(2-morpholinoethyl)amid-dihydrochlorid

Analog Beispiel 105 erhält man die Titelverbindung ausgehend von 70 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-N-(2-morpholinoethyl)amid nach Lyophilisation : R_{f} (Dichlormethan-Methanol-Ammoniak konz. = 350:50:1) = 0.15; HPLC Rₜ= 8.74 Minuten; FAB-MS (M+H)⁺ = 538.

Das Ausgangsmaterial wird analog Beispiel 105 a) und 105 b) aus 5(S)-Tertiärbutoxycarbonylamino-4(S)-tertiärbutyldimethylsilyloxy-7(S)-isopropyl-2(R)-methyl-8-[4-methoxy-3-(3-methoxypropyloxy)-phenyl]-octansäure (Beispiel 105 c) und 4-(2-Aminoethyl)-morpholin hergestellt.

### Beispiel 112: 5(S)-Amino-4(S)-hydroxy-7(S)-isopropyl-2(R)-methyl-8-[4-methoxy-3-(3-methoxypropyloxy)-phenyl]-octansäure-N-(3-morpholinopropyl)amid-dihydrochlorid

Analog Beispiel 105 erhält man die Titelverbindung ausgehend von 37 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-N-(3-morpholinopropyl)amid nach Lyophilisation : R_{f} (Dichlormethan-Methanol-Ammoniak konz. = 350:50:1) = 0.11; HPLC Rₜ = 8.68 Minuten; FAB-MS (M+H)⁺ = 552.

Das Ausgangsmaterial wird analog Beispiel 105 a) und 105 b) aus 5(S)-Tertiärbutoxycarbonylamino-4(S)-tertiäbutyldimethylsilyloxy-7(S)-isopropyl-2(R)-methyl-8-[4-methoxy-3-(3-methoxypropyloxy)-phenyl]-octansäure (Beispiel 105 c) und 4-(3-Aminopropyl)-morpholin hergestellt

### Beispiel 113: 5(S)-Amino-4(S)-hydroxy-7(S)-isopropyl-2(R)-methyl-8-[4-methoxy-3-(3-methoxypropyloxy)-phenyl]-octansäure-N-[2-(1.1-dioxothiomorpholino)ethyl]amid-dihydrochlorid

Analog Beispiel 105 erhält man die Titelverbindung ausgehend von 100 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-N-[2-(1.1-dioxothiomorpholino)ethyl]amid nach Lyophilisation : R_{f} (Dichlormethan-Methanol = 8:2) = 0.30; HPLC Rₜ = 9.29 Minuten; FAB-MS (M+H)⁺ = 586.

Das Ausgangsmaterial wird analog Beispiel 105 a) und 105 b) aus 5(S)-Tertiärbutoxycarbonylamino-4(S)-tertiärbutyldimethylsilyloxy-7(S)-isopropyl-2(R)-methyl-8-[4-methoxy-3-(3-methoxypropyloxy)-phenyl]-octansäure (Beispiel 105 c) und 2-(1.1-Dioxothiomorpholino)-ethylamin hergestellt.

### Beispiel 114: 5(S)-Amino-4(S)-hydroxy-7(S)-isopropyl-2(R)-methyl-8-[4-methoxy-3-(3-methoxypropyloxy)-phenyl]-octansäure-N-(2-ethoxycarbonylethyl)amid-hydrochlorid

Analog Beispiel 105 erhält man die Titelverbindung ausgehend von 32 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(2-ethoxycarbonyl-ethyl)]-amid nach Lyophilisation : R_{f} (Dichlormethan-Methanol = 9:1) = 0.17; HPLC Rₜ= 11.31 Minuten; FAB-MS (M+H)⁺ = 525.

Das Ausgangsmaterial wird analog Beispiel 105 a) und 105 b) aus 5(S)-Tertiärbutoxycarbonylamino-4(S)-tertiärbutyldimethylsilyloxy-7(S)-isopropyl-2(R)-methyl-8-[4-methoxy-3-(3-methoxypropyloxy)-phenyl]-octansäure (Beispiel 105 c) und β-Alaninethylester-hydrochlorid hergestellt.

### Beispiel 115: 5(S)-Amino-4(S)-hydroxy-7(S)-isopropyl-2(R)-methyl-8-[4-methoxy-3-(3-methoxypropyloxy)-phenyl]-octansäure-[N-(2-carboxy-ethyl)]-amid-hydrochlorid

Analog Beispiel 105 erhält man die Titelverbindung ausgehend von 60 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(2-carboxy-ethyl)]-amid nach Lyophilisation : R_{f} (Dichlormethan-Methanol = 8:2) = 0.28; HPLC Rₜ= 9.74 Minuten; FAB-MS (M+H)⁺ = 497.

Das Ausgangsmaterial wird wie folgt hergestellt:

### a) 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(2-carboxyethyl)]-amid

70 mg 5(S)-Tertiärbutoxyamino-4(S)-hydroxy-7(S)-isopropyl-2(R)-methyl-8-[4-methoxy-3-(3-methoxypropyloxy)-phenyl]-octansäure-[N-(2-ethyloxy-carbonylethyl)]-amid (Beispiel 114) werden in 2 ml Methanol mit 224 µl 1N Natriumhydroxid während 18 Stunden bei Raumtemperatur gerührt. Nach dem Abdampfen des Methanols versetzt man mit 250 µl 1N Salzsäure und extrahiert das Produkt mit Essigsäureethylester. Der Eindampfrückstand der organischen Phase wird mittels FC (10 g Kieselgel, Laufmittel Dichlormethan-Methanol = 8:2) gereinigt. Man erhält die Titelverbindung: R_{f} (Dichlormethan-Methanol = 9:1) = 0.12

### Beispiel 116: 5(S)-Amino-4(S)-hydroxy-7(S)-isopropyl-2(R)-methyl-8-[4-methoxy-3-(3-methoxypropyloxy)-phenyl]-octansäure-[N-(3-methoxycarbonyl-ethyl)]-amidhydrochlorid

Analog Beispiel 105 erhält man die Titelverbindung ausgehend von 90 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(3-methoxycarbonyl-ethyl)]-amid nach Lyophilisation : R_{f} (Dichlormethan-Methanol = 9:1) = 0.13; HPLC Rₜ= 10.80 Minuten; FAB-MS (M+H)⁺ = 525.

Das Ausgangsmaterial wird analog Beispiel 105 a) und 105 b) aus 5(S)-Tertiärbutoxycarbonylamino-4(S)-tertiäbutyldimethylsilyl oxy-7(S)-isopropyl-2(R)-methyl-8-[4-methoxy-3-(3-methoxypropyloxy)-phenyl]-octansäure (Beispiel 105 c) und 4-Aminobuttersäuremethylester-hydrochlorid hergestellt.

### Beispiel 117: 5-(S)-Amino-4(S)-hydroxy-7(S)-isopropyl-2(R)-methyl-8-[4-methoxy -3-(3-methoxypropyloxy)-phenyl]-octansäure-[N-(3-carboxypropyl)]-amid-hydrochlorid

Analog Beispiel 105 erhält man die Titelverbindung ausgehend von 38 mg 5(S)-Tertiärbutoxycarbonylamino-4(S)-hydroxy-7(S)-isopropyl-2(R)-methyl-8-[4-methoxy-3-(3- methoxypropyloxy)-phenyl]-octansäure-[N-(3-carboxypropyl)]-amid nach Lyophilisation : R_{f} (Dichlormethan-Methanol = 8:2) = 0.55; HPLC Rₜ = 9.85 Minuten; FAB-MS (M+H)⁺ = 511.

Das Ausgangsmaterial wird wie folgt hergestellt:

### 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(3-carboxypropyl}]-amid

Analog Beispiel 115a wird die Titelverbindung aus 5(S)-Tertiärbutoxycarbonyl-amino-4(S)-hydroxy-7(S)-isopropyl-2(R)-methyl-8-[4-methoxy-3-(3-methoxypropyloxy)-phenyl]-octansäure-[N-(3-methyloxycarbonylpropyl)]-amid (Beispiel 116) hergestellt.

### Beispiel 118: 5(S)-Amino-4(S)-hydroxy-7(S)-isopropyl-2(R)-methyl-8-[4-methoxy-3-(3-methoxypropyloxy)-phenyl]-octansäure-[N-(2-carbamoylethyl)]-amid-hydrochloridM @ fw

Analog Beispiel 105 erhält man die Titelverbindung ausgehend von 93 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(2-carbamoylethyl)]-amid nach Lyophilisation : R_{f} (Dichlormethan-Methanol = 8:2) = 0.15; HPLC Rₜ= 9.33 Minuten; FAB-MS (M+H)⁺= 496.

Das Ausgangsmaterial wird analog Beispiel 105 a) und 105 b) aus 5(S)-Tertiärbutoxycarbonylamino-4(S)-teriärbutyldimethylsilyloxy-7(S)-isopropyl-2(R)-methyl-8-[4-methoxy-3-(3-methoxypropyloxy)-phenyl]-octansäure (Beispiel 105 c) und 3-Aminopropionsäureamid-hydrochlorid hergestellt.

### Beispiel 119: 5(S)-Amino-4(S)-hydroxy-7(S)-isopropyl-2(R)-methyl-8-[4-methoxy-3-(3-methoxypropyloxy)-phenyl]-octansäure-[N-(4-carbamoylbutyl)]-amid-hydrochloridM@ fw

Analog Beispiel 105 erhält man die Titelverbindung ausgehend von 85 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(4-carbamoylbutyl)]-amid nach Lyophilisation : R_{f} (Dichlormethan-Methanol = 8:2) = 0.20; HPLC Rₜ = 9.72 Minuten; FAB-MS (M+H)⁺ = 524.

Das Ausgangsmaterial wird analog Beispiel 105 a) und 105 b) aus 5(S)-Tertiärbutoxycarbonylamino-4(S)-tertiärbutyldimethylsilyloxy-7(S)-isopropyl-2(R)-methyl-8-[4-methoxy-3-(3-methoxypropyloxy)-phenyl]-octansäure (Beispiel 105 c) und 5-Aminopentansäureamid-hydrochlorid hergestellt.

### Beispiel 120: 5(S)-Amino-4(S)-hydroxy-7(S)-isopropyl-2(R)-methyl-8-[4-methoxy-3-(3-methoxypropyloxy)-phenyl]-octansäure-N-[3-(N-methylcarbamoyl)propyl]amid-hydrochlorid

Analog Beispiel 105 erhält man die Titelverbindung ausgehend von 89 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-N-[3-(N-methylcarbamoyl)propyl]amid nach Lyophilisation: R_{f} (Dichlormethan-Methanol = 9:1) = 0.04; HPLC Rₜ= 9.74 Minuten; FAB-MS (M+H)⁺ = 524.

Das Ausgangsmaterial wird analog Beispiel 105 a) und 105 b) aus 5(S)-Tertiärbutoxycarbonylamino-4(S)-tertiärbutyldimethylsilyloxy-7(S)-isopropyl-2(R)-methyl-8-[4-methoxy-3-(3-methoxypropyloxy)-phenyl]-octansäure (Beispiel 105 c) und 4-Amino-N-methyl-buttersäureamid-hydrochlorid hergestellt.

### Beispiel 121: 5(S)-Amino-4(S)-hydroxy-7(S)-isopropyl-2(R)-methyl-8-[4-methoxy-3-(3-methoxypropyloxy)-phenyl]-octansäure-N-{3-[N-(2-methoxyethyl)carbamoyl]propyl}amid-hydrochlorid

Analog Beispiel 105 erhält man die Titelverbindung ausgehend von 92 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-N-{3-[N-(2-methoxyethyl)carbamoyl]propyl}-amid nach Lyophilisation : R_{f} (Dichlormethan-Methanol = 8:2) = 0.28; HPLC Rₜ= 10.14 Minuten; FAB-MS (M+H)⁺ = 568.

Das Ausgangsmaterial wird analog Beispiel 105 a) und 105 b) aus 5(S)-Tertiärbutoxycarbonylamino-4(S)-tertiäbutyldimethylsilyloxy-7(S)-isopropyl-2(R)-methyl-8-[4-methoxy-3-(3-methoxypropyloxy)-phenyl]-octansäure (Beispiel 105 c) und 4-Amino-buttersäure-N-(2-methoxyethyl)amid-hydrochlorid hergestellt.

Das Ausgangsmaterial wird wie folgt hergestellt:

### 4-Aminobuttersäure-N-(2-methoxyethyl)amid-hydrochlorid

2.95 g 4-Benzyloxycarbonylaminobuttersäure-N-(2-methoxyethyl)amid werden in Gegenwart von 0.24 g Pd/C 10% in 150 ml Methanol und 100 ml 0.1 N Salzsäure während 2 Stunden bei Raumtemperatur und Normaldruck hydriert. Das Reaktionsgemisch wird filtriert und eingedampft. Man erhält die rohe Titelverbindung: ¹H NMR (CD₃OD), δ(ppm) = 4.92 (4H, s), 3.53 - 3.20 (4H, m), 3.34 (3H, s), 2.96 (2H, t, J=12 Hz), 2.37 (2H, t, J= 12 Hz), 1.93 (2H, m)

### b) 4-Benzyloxycarbonylaminobuttersäure-N-(2-methoxyethyl)amid

5.02 g 4-Benzyloxycarbonylaminobuttersäuremethylester werden in 35 ml Äthanol mit 15 ml 2-Methoxyethylamin während 5 Tagen am Rückfluss gerührt. Das Reaktionsgemisch wird eingedampft und der Rückstand mittels FC (240 g Kieselgel, Dichlormethan-Methanol = 95:5) gereinigt. Man erhält die Titelverbindung: R_{f} (Dichlormethan-Methanol = 95:5) = 0.33

### Beispiel 122: 5(S)-Amino-4(S)-hydroxy-7(S)-isopropyl-2(R)-methyl-8-[4-methoxy-3-(3-methoxypropyloxy)-phenyl]-octansäure-N-(4-morpholino-4-oxo-butyl)amid-hydrochlorid

Analog Beispiel 105 erhält man die Titelverbindung ausgehend von 110 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-N-(4-morpholino-4-oxo-butyl)amid nach Lyophilisation : R_{f} (Dichlormethan-Methanol= 9:1) = 0.06; HPLC Rₜ = 10.17 Minuten; FAB-MS (M+H)⁺ = 580.

Das Ausgangsmaterial wird analog Beispiel 105 a) und 105 b) aus 5(S)-Tertiärbutoxycarbonylamino-4(S)-tertiärbutyldimethylsilyloxy-7(S)-isopropyl-2(R)-methyl-8-[4-methoxy-3-(3-methoxypropyloxy)-phenyl]-octansäure (Beispiel 105 c) und 4-Aminobuttersäure-N'-(4-morpholino)amid-hydrochlorid hergestellt.

### Beispiel 123: 5(S)-Amino-4(S)-hydroxy-7(S)-isopropyl-2(R)-methyl-8-[4-methoxy-3-(3-methoxypropyloxy)-phenyl]-octansäure-[N-(2-carbamoyl-2,2-dimethyl-ethyl)]-amid-hydrochlorid

Analog Beispiel 105 erhält man die Titelverbindung ausgehend von 66 mg 5(S)-Tertiärbutoxycarbonylamino-4(S)-hydroxy-7(S)-isopropyl-2(R)-methyl-8-[4-methoxy-3-(3-methoxypropyloxy)-phenyl]-octansäure-[N-(2-carbamoyl-2.2-dimethyl-ethyl)]-amid nach Lyophilisation : R_{f} (Dichlormethan-Methanol = 8:2) = 0.27; HPLC Rₜ = 12.10 Minuten; FAB-MS (M+H)⁺ = 524.

Das Ausgangsmaterial wird analog Beispiel 105 a) und 105 b) aus 5(S)-Tertiärbutoxycarbonylamino-4(S)-tertiärbutyldimethylsilyloxy-7(S)-isopropyl-2(R)-methyl-8-[4-methoxy-3-(3-methoxypropyloxy)-phenyl]-octansäure (Beispiel 105 c) und 3-Amino-2.2-dimethyl-propionsäureamid-hydrochlorid hergestellt.

### Beispiel 124: 5(S)-Amino-4(S)-hydroxy-2(S),7(S)-diisopropyl-8-[4-methoxy-3-(3-methoxy-propyloxy)-phenyl]-octansäure-N-(2-morpholinoethyl)amid-dihydrochlorid

3.09 g 5(S)-Tertiärbutoxycarbonylamino-4(S)-hydroxy-2(S),7(S)-diisopropyl-8-[4-methoxy-3-(3-methoxypropyloxy)-phenyl]-octansäure-N-(2-morpholinoethyl)]amid werden in 40 ml 4N Salzsäure in Dioxan bei 0° C gelöst und für 2 Stunden bei 0° C gerührt. Das Reaktionsgemisch wird lyophilisiert und man erhält die Titelverbindung: R_{f} (Dichlormethan-Methanol = 8:2) = 0.27; HPLC Rₜ = 9.52 Minuten; HR FAB-MS (M+H)⁺ = 566.

Die Ausgangsmaterialien werden wie folgt hergestellt:

### a) 5(S)-Tertiärbutoxycarbonylamino-4(S)-hydroxy-2(S),7(S)-diisopropyl-8-[4-methoxy-3-(3-methoxypropyloxy)-phenyl]-octansäure-N-(2-morpholino-ethyl)amid

4.18 g 3-Tertiärbutoxycarbonyl-5(S)-{2(S)-[N-(2-morpholino-ethyl)carbamoyl]-(2(S)-isopropyl-ethyl}-4(S)-{2(S)-isopropyl-3-[4-methoxy-3-(3-methoxypropyloxy)-phenyl]-propyl}-2.2-dimethyl-1.3-oxazolidin werden in 160 ml Methanol bei 0° C mit 1.30 g p-Toluolsulfonsäure (Monohydrat) versetzt. Die Reaktionslösung wird 18 Stunden bei Raumtemperatur weitergerührt. Nach dem Abdampfen des Lösungsmittels wird der Rückstand mit 200 ml 0.1N Natriumhydroxid versetzt und mit Dichlormethan extrahiert. Die eingedampften organischen Extrakte werden durch FC (230 g Kieselgel, Dichlormethan/Methanol = 95:5) gereinigt. Man erhält die Titelverbindung: R_{f} (Dichlormethan-Methanol = 9:1) = 0.55

### b) 3-Tertiärbutoxycarbonyl-5(S)-{2(S)-[N-(2-morpholino-ethyl)-carbamoyl]-(2)S)-isopropyl-ethyl}-4(S)-{2(S)-isopropyl-3-[4-methoxy-3-(3-methoxypropyloxy)-phenyl]-propyl}-2.2-dimethyl-1,3-oxazolidin

3.88 g 3-Tertiärbutoxycarbonyl-5(S)-[2(S)-carboxy-3-methyl-butyl]-4(S)-{2(S)-isopropyl-3-[4-methoxy-3-(3-methoxypropyloxy)-phenyl]-propyl}-2.2-dimethyl-1.3-oxazolidin werden in 190 ml Dimethylformamid bei 0° C nacheinander mit 1.09 ml Triethylamin, 1.02 ml 4-(2-Aminoethyl)-morpholin und 1.19 ml Cyanphosphonsäurediethylester versetzt. Das Reaktionsgemisch wird noch 18 Stunden bei Raumtemperatur gerührt. Das Reaktionsgemisch wird eingedampft und der Rückstand zwischen Diethylether und gesättigter Natriumhydrogencarbonatlösung verteilt. Die organischen Phasen werden mit gesättigter Kochsalzlösung gewaschen und eingedampft. Der Eindampfrückstand wird durch FC (230 g Kieselgel, Dichlormethan-Methanol = 95:5) gereinigt. Man erhält die Titelverbindung: R_{f} (Dichlormethan-Methanol = 95:5) = 0.25

### c) 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1,3-oxazolidin

53 g 3-Tertiärbutoxycarbonyl-5(S)-(2(S)-formyl-3-methyl-butyl)-4(S) -{2(S)-isopropyl-3-[4-methoxy-3-(3-methoxypropyloxy)-phenyl]-propyl}-2.2-dimethyl-1.3-oxazolidin werden in 470 ml Toluol gelöst und bei 0° C nacheinander mit 470 ml Wasser, 79.1 g Kaliumpermanganat und 9.7 g Tetrabutylammoniumbromid versetzt. Das Reaktionsgemisch wird noch 48 Stunden bei 0-5° C weitergerührt, dann bei 10° C mit 1.2 Liter Natriumsulfitlösung 10% versetzt. Nach weiteren 30 Minuten werden 1.95 Liter Citronensäurelösung 10% und 1.2 Liter Wasser zugegeben. Das Produkt wird durch mehrmalige Extraktion mit Essigsäureethylester extrahiert. Die eingedampften Extrakte werden durch FC (2.3 kg Kieselgel, Essigsäureethylester-Hexan = 3:7) gereinigt. Man erhält die Titelverbindung: R_{f} (Essigsäureethylester-Hexan = 1:2) = 0.21

### d) 3-Tertiärbutoxycarbonyl-5(S)-(2(S)-formyl-3-methyl-butyl)-4(S)-{2(S)-isopropyl-3-[4-methoxy-3-(3-methoxypropyloxy)-phenyl]-propyl}-2.2-dimethyl-1,3-oxazolidin

53 g 3-Tertiärbutoxycarbonyl-5(S)-(3-hydroxy-2(S)-isopropyl-propyl)-4(S)-{2(S)-isopropyl-3-[4-methoxy-3-(3-methoxypropyloxy)-phenyl]-propyl}-2.2-dimethyl-1.3-oxazolidin werden in 1.8 Liter Dichlormethan bei Raumtemperatur mit 100 g Molekularsieb (0.3 nm) und 16.6 g N-Methylmorpholin-N-oxid versetzt. Das Reaktionsgemisch wird 10 Minuten gerührt und anschliessend mit 1.60 g Tetrapropylammonium-perruthenat versetzt. Das Reaktionsgemisch wird weitere 30 Minuten gerührt und anschliessend filtriert. Das mit Dichlormethan verdünnte Filtrat wird nacheinander mit 2M Natriumsulfitlösung, gesättigter Kochsalzlösung und 1M Kupfer-II-sulfat gewaschen. Die organische Phase wird eingedampft und man erhält die rohe Titelverbindung: R_{f} (Essigsäureethylester-Hexan = 1:2) = 0.43

### e) 3-Tertiärbutoxycarbonyl-5(S)-(3-hydroxy-2(S)-isopropyl-propyl)-4(S)-{2(S)-isopropyl-3-[4-methoxy-3-(3-methoxypropyloxy)-phenyl]-propyl}-2,2-dimethyl-1,3-oxazolidin

3.7 g 3-Tertiärbutoxycarbonyl-5(S)-(3-benzyloxy-2(S)-isopropyl-propyl)-4(S)-{2(S)-isopropyl-3-[4-methoxy-3-(3-methoxypropyloxy)-phenyl]-propyl}-2.2-dimethyl-1.3-oxazolidin werden in Gegenwart von 1.0 g Pd/C 5% in 50 ml Tetrahydrofuran für 15 Minuten bei Raumtemperatur und Normaldruck hydriert. Das Reaktionsgemisch wird filtriert und eingedampft. Der Rückstand wird mittels FC (140 g Kieselgel, Essigsäureethylester/Hexan = 1:2) gereinigt. Man erhält die Titelverbindung: R_{f} (Essigsäureethylester-Hexan = 1:2) = 0.28

### f) 3-Tertiärbutoxycarbonyl-5(S)-(3-benzyloxy-2(S)-isopropyl-propyl)-4(S)-{2(S)-isopropyl-3-[4-methoxy-3-(3-methoxypropyloxy)-phenyl]-propyl}-2.2-dimethyl-1,3-oxazolidin (A) und 3-Tertiärbutoxycarbonyl-5(R)-(3-benzyloxy-2(S)-isopropyl-propyl)-4(S)-{2(S)-isopropyl-3-[4-methoxy-3-(3-methoxypropyloxy)-phenyl]-propyl}-2.2-dimethyl-1,3-oxazolidin (B)

7.0 g 5(S)-Tertiärbutoxycarbonylamino-4(R,S)-hydroxy-2(S),7(S)-diisopropyl-8-[4-methoxy-3-(3-methoxypropyloxy)-phenyl]-octyl-benzylether werden in 1.86 Liter Dichlormethan bei Raumtemperatur mit 10.9 ml 2.2-Dimethoxypropan und 10 mg p-Toluolsulfonsäure (Monohydrat) versetzt. Das Reaktionsgemisch wird noch 24 Stunden bei Raumtemperatur weitergerührt. Der Eindampfrückstand wird durch FC (1 kg Kieselgel und Dichlormethan/Diethylether = 96:4) gereinigt. Man erhält die Titelverbindungen:
A) R_{f} (Dichlormethan/Tertiärbutylmethylether = 0.36
B) R_{f} (Dichlormethan/Tertiärbutylmethylether = 0.44

### g) 5(S)-Tertiärbutoxycarbonylamino-4(R,S)-hydroxy-2(S),7(S)-diisopropyl-8-[4-methoxy-3-(3-methoxypropyloxy)-phenyl]-octyl-benzylether

51.1g Magnesiumspäne werden in 1.4 Liter Tetrahydrofuran bei 55° C vorgelegt. Während 30 Minuten wird eine Lösung aus 380 g 2(S)-Brommethyl-3-methyl-butyl-benzylether, 30.2 ml 1.2-Dibromethan in 0.8 Liter Tetrahydrofuran bei 55° C zugetropft. Das Reaktionsgemisch wird noch 20 Minuten bei 55° C weitergerührt und anschliessend auf 5° C abgekühlt. Danach wird eine Lösung aus 190g 2(S)-Tertiärbutoxycarbonylamino-4(S)-isopropyl-5-[4-methoxy-3-(3-methoxypropyloxy)-phenyl]-pentanal in 0.7 Liter Tetrahydrofuran zugetropft. Das Reaktionsgemisch wird noch 3 Stunden bei Raumtemperatur weitergerührt, dann bei 5° C mit gesättigter Ammoniumchloridlösung versetzt und mit Diethylether extrahiert. Die eingedampften Ertrakte werden durch FC (4 kg Kieselgel, Essigsäureethylester-Hexan = 1:3) gereinigt. Man erhält die Titelverbindung als Diastereomerengemisch: R_{f} (Essigsäureethylester-Hexan = 1:2) = 0.26; HPLC Rₜ = 22.67 und 22.81 (40:60).

### h) 2(S)-Tertiärbutoxycarbonylamino-4(S)-isopropyl-5-[4-methoxy-3-(3-methoxy-propyloxy)-phenyl]-pentanal

Die Titelverbindung wird analog Beispiel 1 c) bis 1 g) hergestellt, wobei in Stufe 1 g) an Stelle von 2(S)-Isopropyl-3-(p-tertiärbutyl-phenyl)-propanol das 2(S)-Isopropyl-3-[4-methoxy-3-(3-methoxypropyloxy)-phenyl]-propanol eingesetzt wird. Dieses wird wie folgt hergestellt:

### i) 2(S)-Isopropyl-3-[4-methoxy-3-(3-methoxypropyloxy)-phenyl]-propanol

186 g 2(S)-Isopropyl-3-[4-methoxy-3-(3-methoxypropyloxy)-phenyl]-propionsäure in 0.5 Liter Tetrahydrofuran werden bei Raumtemperatur zu einer gerührten Mischung aus 27.2 g Natriumborhydrid in 1.5 Liter Tetrahydrofuran getropft. Nach 45 Minuten wird eine Lösung aus 76.2 g Iod in 1 Liter Tetrahydrofuran zugetropft. Das Reaktionsgemisch wird während 4 Tagen gerührt und anschliessend wird vorsichtig 1 Liter Methanol zugetropft. Nach dem Abdampfen der Lösungsmittel wird der Rückstand in 2 Liter 2N Salzsäure aufgenommen und mehrmals mit Essigsäureethylester extrahiert. Die organischen Extrakte werden nacheinander mit Wasser, gesättigter Natriumthiosulfatlösung, Wasser/gesättigter Kochsalzlösung (1:1), 0.1N Natronlauge und gesättigter Kochsalzlösung gewaschen. Die eingedampften organischen Extrakte werden durch FC (2.4 kg Kieselgel, Essigsäureethylester-Hexan = 1:4) gereinigt. Man erhält die Titelverbindung: R_{f} (Essigsäureethylester/Hexan = 1:1) = 0.28

### k) 2(S)-Isopropyl-3-[4-methoxy-3-(3-methoxypropyloxy)-phenyl]-pronionsäure

300 g 4(R)-Benzyl-3-{2(S)-isopropyl-3-[4-methoxy-3-(3-methoxypropyloxy)-phenyl]-propionyl}-oxazolidin-2-on werden in 4.8 Liter Tetrahydrofuran/Wasser (3:1) bei 0° C langsam mit 0.434 Liter Wasserstoffperoxid 30% versetzt. Nach der Zugabe von 31.2 g Lithiumhydroxid wird noch 3 Stunden bei 0-20° C gerührt. Das Reaktionsgemisch wird anschliessend bei 0-15° C mit 2.55 Liter 1.5M Natriumsulfitlösung versetzt und weitere 30 Minuten nachgerührt. Man versetzt mit 1 Liter gesättigter Natriumhydrogencarbonat-lösung und dampft das Tetrahydrofuran ab. Die wässrige Lösung wird mehrmals mit Dichlormethan gewaschen und anschliessend mit 2N Salzsäure angesäuert (pH 3.0). Durch Extraktion mit Dichlormethan und anschliessendem Abdampfen des Lösungsmittels wird die Titelverbindung erhalten: R_{f} (Essigsäureethylester-Hexan = 2:1) = 0.30; Smp. 43.5-44° C.

### l) 4(R)-Benzyl-3-{2(S)-isopropyl-3-[4-methoxy-3-(3-methoxypropyloxy)-phenyl]-propionyl}-oxazolidin-2-on

Die Lösung von 600 ml 1M Lithiumhexamethyldisilazid wird mit 600 ml Tetrahydrofuran versetzt und bei -70° C gerührt. Nun wird die Lösung von 156.6 g 4(R)-Benzyl-3-isovaleroyloxazolidin-2-on in 500 ml Tetrahydrofuran zugetropft und das Reaktionsgemisch 75 Minuten bei -70° C weitergerührt. Danach wird die Lösung von 145 g 4-Methoxy-3-(3-methoxypropyloxy)-benzylbromid in 500 ml Tetrahydrofuran zugetropft. Man läßt das Rekationsgemisch während 2 Stunden von -70° auf 0° C erwärmen. Das Reaktionsgemisch wird noch 18 Stunden bei 4° C stehen gelassen und anschliessend unter Rühren mit 250 ml gesättigter Ammoniumchloridlösung versetzt. Das Tetrahydrofuran wird abgedampft und der Rückstand mit Essigsäureethylester extrahiert. Aus dem Rückstand der Extrakte erhält man durch Reinigung mit FC (2.4 kg Kieselgel, Essigsäureethylester-Hexan = 1:1) die Titelverbindung: R_{f}(Essigsäureethylester-Hexan = 1:2) = 0.30; Smp.55-56° C.

### m) 4-Methoxy-3-(3-methoxypropyloxy)-benzylbromid

113.1 g 4-Methoxy-3-(3-methoxypropyloxy)-benzylalkohol werden in 1.31 Liter Chloroform unter Rühren bei Raumtemperatur mit 97 ml Trimethylbromsilan versetzt. Nach 10 Minuten wird das Lösungsmittel abgedampft und der Rückstand sofort mittels FC (900 g Kieselgel, Laufmittel Essigsäureethylester-Hexan 1:3) gereinigt. Man erhält die Titelverbindung: R_{f} (Essigsäureethylester-Hexan = 1:2) = 0.34; Smp.50-51° C

### n) 4-Methoxy-3-(3-methoxypropyloxy)-benzylalkohol

7.7 g 3-Hydroxy-4-methoxy-benzylalkohol, 10.35 g Kaliumcarbonat und 12.1 g 1-Brom-3-methoxy-propan werden in 150 ml Aceton während 3 Tagen am Rückfluss gerührt. Nach dem Abdampfen des Lösungsmittels wird der Rückstand mit Wasser versetzt und mit Essigsäureethylester extrahiert. Aus den organischen Extrakten wird nach dem Abdampfen des Lösungsmittels mittels FC (240 g Kieselgel, Dichlormethan-Methanol = 96:4) die Titelverbindung erhalten: R_{f} (Essigsäureethylester-Hexan = 2:1) = 0.31

### Beispiel 125: 5(S)-Amino-4(S)-hydroxy-2(S),7(S)-diisopropyl-8-[4-methoxy-3-(3-methoxypropyloxy)-phenyl]-octansäure-N-(3-morpholinopropyl)amid-dihydrochlorid

Analog Beispiel 124 erhält man die Titelverbindung ausgehend von 120 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-(3-morpholinopropyl)amid: R_{f} (Dichlormethan-Methanol-Ammoniak konz.= 350:50:1) = 0.12; HPLC Rₜ= 9.64 Minuten; FAB-MS (M+H)⁺ = 580.

Das Ausgangsmaterial wird analog Beispiel 124 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 4-(3-Aminopropyl)morpholin hergestellt.

### Beispiel 126: 5(S)-Amino-4(S)-hydroxy-2(S),7(S)-diisopropyl-8-[4-methoxy-3-(3-methoxypropyloxy)-phenyl]-octansäure-N-(2,2-dimethyl-2-morpholino-ethyl)amid-dihydrochlorid

Analog Beispiel 124 erhält man die Titelverbindung ausgehend von 110 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-(2.2-dimethyl-2-morpholino-ethyl)amid: R_{f} (Dichlormethan-Methanol = 9:1) = 0.05; HPLC Rₜ= 10.35 Minuten; FAB-MS (M+H)⁺ = 594.

Das Ausgangsmaterial wird analog Beispiel 124 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 4-(2-Amino-1.1-dimethyl-ethyl)-morpholin hergestellt.

### a) 4-(2-Amino-1.1-dimethyl-ethyl)-morpholin

3.33 g Lithiumaluminiumhydrid werden in 85 ml Tetrahydrofuran bei Raumtemperatur langsam mit einer Lösung aus 8.33 g 2-Methyl-2-morpholino-propionsäureamid in 50 ml Tetrahydrofuran versetzt. Das Reaktionsgemisch wird anschliessend noch 2 Stunden am Rückfluss gerührt. Das abgekühlte Reaktionsgemisch wird nacheinander mit 5 ml Wasser, 6.67 ml 2N Natriumhydroxid und nochmals mit 5 ml Wasser versetzt. Die Suspension wird klarfiltriert und aus dem eingeengten Filtrat erhält man die rohe Titelverbindung: ¹H NMR (CDCl₃), δ(ppm) = 3.67 (4H, m), 2.52 (2H, s), 2.48 (4H, m), 1.37 (2H, bs), 0.92 (6H, s)

### b) 2-Methyl-2-morpholino-propionsäureamid

57.9 g 2-Methyl-2-morpholino-propionitril werden unter Rühren langsam mit 272 ml Schwefelsäure konz. versetzt (exotherme Reaktion). Nach der Zugabe von 43 ml Wasser rührt man während 2 Stunden bei 100-110° C. Das auf 50° C abgekühlte Reaktionsgemisch wird bei 0° C zu einer Lösung aus 846 ml Ammoniak 20% in 242 ml Wasser getropft. Das Gemisch wird anschliessend mehrmals mit Dichlormethan extrahiert. Die organischen Phasen werden mit gesättigter Kochsalzlösung gewaschen und mit Natriumsulfat getrocknet. Aus dem eingeengten Filtrat erhält man die rohe Titelverbindung: ¹H NMR (CDCl₃), δ(ppm)= 7.08 (1H, bs), 5.38 (1H, bs), 3.72 (4H, m), 2.53 (4H, m), 1.22 (6H,s)

### Beispiel 127: 5(S)-Amino-4(S)-hydroxy-2(S),7(S)-diisopropyl-8-[4-methoxy-3-(3-methoxypropyloxy)-phenyl]-octansäure-N-(2-thiomorpholinoethyl)amid-dihydrochlorid

Analog Beispiel 124 erhält man die Titelverbindung ausgehend von 110 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-(2-thiomorpholinoethyl)amid: R_{f} (Dichlormethan-Methanol = 8:2) = 0.33; HPLC Rₜ= 10.39 Minuten; FAB-MS (M+H)⁺ = 582.

Das Ausgangsmaterial wird analog Beispiel 124 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 4-(2-Aminoethyl)thiomorpholin hergestellt.

### Beispiel 128: 5(S)-Amino-4(S)-hydroxy-2(S),7(S)-diisopropyl-8-[4-methoxy-3-(3-methoxypropyloxy)-phenyl]-octansäure-N-(1,1-dimethyl-2-morpholino-ethyl)amid-dihydrochlorid

Analog Beispiel 124 erhält man die Titelverbindung ausgehend von 95 mg 5(S)-Tertiärbutoxycarbonylamino-4(S)-hydroxy-2(S),7(S)-diisopropyl-8-[4-methoxy-3-(3-methoxy propyloxy)-phenyl]-octansäure-N-(1.1-dimethyl-2-morpholino-ethyl)amid: R_{f} (Dichlormethan-Methanol = 8:2) = 0.42; HPLC Rₜ= 10.37 Minuten; FAB-MS (M+H)⁺ = 594.

Das Ausgangsmaterial wird analog Beispiel 124 a) und 130 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 4-(2-Amino-2.2-dimethyl-ethyl)-morpholin hergestellt.

### Beispiel 129: 5(S)-Amino-4(S)-hydroxy-2(S),7(S)-diisopropyl-8-[4-methoxy-3-(3-methoxypropyloxy)-phenyl]-octansäure-N-[1(R,S)-methyl-2-morpholino-ethyl]amid-dihydrochlorid

Analog Beispiel 124 erhält man die Titelverbindung ausgehend von 73 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[1(R,S)-methyl-2-morpholino-ethyl]amid: R_{f} (Dichlormethan-Methanol = 8:2) = 0.43; HPLC Rₜ= 9.98/10.58 Minuten; FAB-MS (M+H)⁺ = 580.

Das Ausgangsmaterial wird analog Beispiel 124 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 4-(2-Amino-2(RS)-methyl-ethyl)-morpholin hergestellt.

### Beispiel 130: 5(S)-Amino-4(S)-hydroxy-2(S),7(S)-diisopropyl-8-[4-methoxy-3-(3-methoxypropyloxy)-phenyl]-octansäure-[N-(1-carbamoyl-1-methyl-ethyl)]-amid-hydrochlorid

56 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-[N-(1-carbamoyl-1-methyl-ethyl)]-amid werden in 1.5 ml Dichlormethan bei 0° C mit 1.5 ml Trifluoressigsäure versetzt. Man rührt noch 30 Minuten bei 0° C. Das Reaktionsgemisch wird auf gekühlte 1N Natriumhydroxid gegossen und das Produkt mehrmals mit Dichlormethan extrahiert. Die organischen Phasen werden getrocknet und mit etherischer Salzsäure versetzt. Nach dem Eindampfen erhält man die Titelverbindung: R_{f} (Dichlormethan-Methanol = 8:2) = 0.30; HPLC Rₜ = 11.25; FAB-MS (M+H)⁺ = 538.

Die Ausgangsmaterialien werden wie folgt hergestellt:

### a) 5(S)-Tertiärbutoxycarbonylamino-4(S)-hydroxy-2(S),7(S)-diisopropyl-8-[4-methoxy-3-(3-methoxypropyloxy)-phenyl]-octansäure-[N-(1-carbamoyl-1-methyl-ethyl)]-amid

82 mg 3-Tertiärbutoxycarbonyl-5(S)-{2-[N-(1-carbamoyl-1-methyl-ethyl)-carbamoyl]-2(S)-isopropyl-ethyl}-4(S)-{2(S)-isopropyl-3-[4-methoxy-3-(3-methoxypropyloxy)-phenyl]-propyl}-2.2-dimethyl-1,3-oxazolidin werden in 5 ml Methanol bei 0° C mit 5 mg p-Toluolsulfonsäure (Monohydrat) versetzt. Die Reaktionslösung wird 18 Stunden bei Raumtemperatur weitergerührt. Nach dem Abdampfen des Lösungsmittels wird der Rückstand mit 20 ml gesättigter Natriumhydrogencarbonatlösung versetzt und mehrmals mit Essigsäureethylester extrahiert. Die eingedampften organischen Extrakte werden durch FC (35 g Kieselgel, Dichlormethan-Methanol = 9:1) gereinigt. Man erhält die Titel-verbindung: R_{f} (Dichlormethan-Methanol = 9:1) = 0.47.

### b) 3-Tertiärbutoxycarbonyl-5(S)-{2-[N-(1-carbamoyl-1-methyl-ethyl)-carbamoyl]-2(S)-isopropyl-ethyl}-4(S)-{2(S)-isopropyl-3-[4-methoxy-3-(3-methoxypropyloxy)-phenyl]-propyl}-2.2-dimethyl-1,3-oxazolidin

119 mg 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124c) werden in 8 ml Dimethylformamid nacheinander mit 106 µl 4-Methyl-morpholin, 66 mg 2-Aminoisobuttersäureamid-hydrochlorid und 91 mg O-Benzotriazol-1-yl-N,N,N',N'-tetramethyl-uronium-hexafluorophosphat (HBTU) versetzt. Man rührt das Reaktionsgemisch während 8 Tagen bei 40° C. Die Mischung wird eingedampft und der Rückstand zwischen Essigsäureethylester und gesättigter Kochsalzlösung verteilt. Die organischen Phasen werden eingedampft und der Rückstand mittels FC (60 g Kieselgel, Dichlormethan-Methanol = 95:5) gereinigt. Man erhält die Titelverbindung: R_{f} (Dichlormethan-Methanol = 95:5) = 0.30.

### Beispiel 131: 5(S)-Amino-4(S)-hydroxy-2(S),7(S)-diisopropyl-8-[4-methoxy-3-(3-methoxypropyloxy)-phenyl]-octansäure-{N-[3-(N-methyl-carbamoyl)-propyl]}-amid-hydrochlorid

Analog Beispiel 124 erhält man die Titelverbindung ausgehend von 101 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-{N-[3-(N-methyl-carbamoyl)-propyl]}-amid: R_{f} (Dichlormethan-Methanol = 8:2) = 0.32; HPLC Rₜ = 10.11 Minuten; FAB-MS (M+H)⁺ = 552.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 4-Amino-N-methyl-buttersäureamid-hydrochlorid hergestellt.

### Beispiel 132: 5(S)-Amino-4(S)-hydroxy-2(S),7(S)-diisopropyl-8-[4-methoxy-3-(3-methoxypropyloxy)-phenyl]-octansäure-{N-[3-(N,N-dimethyl-carbamoyl)-propyl]}-amid-hydrochlorid

Analog Beispiel 124 erhält man die Titelverbindung ausgehend von 91 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-{N-[3-(N,N-dimethyl-carbamoyl)-propyl]}-amid: R_{f} (Dichlormethan-Methanol = 8:2) = 0.36; HPLC Rₜ= 10.38 Minuten; FAB-MS (M+H)⁺ = 566.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 4-Amino-N,N-dimethyl-buttersäureamid-hydrochlorid hergestellt.

### Beispiel 133: 5(S)-Amino-4(S)-hydroxy-2(S),7(S)-diisopropyl-8-[4-methoxy-3-(3-methoxypropyloxy)-phenyl]-octansäure-N-[2-(N,N-dimethyl-carbamoyl)ethyl]amid-hydrochlorid

Analog Beispiel 124 erhält man die Titelverbindung ausgehend von 87 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[2-(N,N-dimethylcarbamoyl)ethyl]-amid: R_{f} (Dichlormethan-Methanol = 8:2) = 0.38; HPLC Rₜ= 10.31 Minuten; FAB-MS (M+H)⁺ = 552.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3-Amino-N,N-dimethyl-propionsäureamid-hydrochlorid hergestellt.

### Beispiel 134: 5(S)-Amino-4(S)-hydroxy-2(S),7(S)-diisopropyl-8-[4-methoxy-3-(3-methoxypropyloxy)-phenyl]-octansäure-[N-(l-carbamoyl-methyl)]-amid-hydrochlorid

Analog Beispiel 124 erhält man die Titelverbindung ausgehend von 84 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-[N-(1-carbamoyl-methyl)]-amid: R_{f} (Dichlormethan-Methanol = 8:2) = 0.20; HPLC Rₜ= 9.73 Minuten; FAB-MS (M+H)⁺ = 510.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und Glycinamid-hydrochlorid hergestellt.

### Beispiel 135: 5(S)-Amino-4(S)-hydroxy-2(S),7(S)-diisopropyl-8-[4-methoxy-3-(3-methoxypropyloxy)-phenyl]-octansäure-[N-(2-carbamoyl-ethyl)]-amid-hydrochlorid

Analog Beispiel 124 erhält man die Titelverbindung ausgehend von 78 mg 5(S)-Tertiäbutoxycarbonylamino-4(S)-hydroxy-2(S),7(S)-diisopropyl-8-[4-methoxy-3-(3-methoxypropyloxy)-phenyl]-octansäure-N-(2-carbamoylethyl)amid: R_{f} (Dichlormethan-Methanol = 8:2) = 0.24; HPLC Rₜ= 9.87 Minuten; FAB-MS (M+H)⁺ = 524.

Das Ausgangsmaterial wird analog Beispiel 130 a) und 124 b) aus 3-Tertiärbutoxycarbonyl-5(S)-[2(S)-carboxy-3-methyl-butyl]-4(S)-{2(S)-isopropyl-3-[4-methoxy-3-(3-methoxypropyloxy)-phenyl]-propyl}-2.2-dimethyl-1.3-oxazolidin (Beispiel 124 c) und 3-Aminopropionsäureamid-hydrochlorid hergestellt.

### Beispiel 136: 5(S)-Amino-4(S)-hydroxy-2(S),7(S)-diisopropyl-8-[4-methoxy-3-(3-methoxypropyloxy)-phenyl]-octansäure-N-(3-carbamoylpropyl)amid-hydrochlorid

Analog Beispiel 124 erhält man die Titelverbindung ausgehend von 74 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-(3-carbamoylpropyl)amid: R_{f} (Dichlormethan-Methanol = 9:1) = 0.06; HPLC Rₜ= 10.27 Minuten; FAB-MS (M+H)⁺ = 538.

Das Ausgangsmaterial wird analog Beispiel 130 a) und 124 b) aus 3-Tertiärbutoxycarbonyl-5(S)-(2(S)-cardoxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 4-Aminobuttersäureamid-hydrochlorid hergestellt.

### Beispiel 137: 5(S)-Amino-4(S)-hydroxy-2(S),7(S)-diisopropyl-8-[4-methoxy-3-(3-methoxypropyloxy)-phenyl]-octansäure-N-(2-carbamoyl-2,2-dimethyl-ethyl)amid-hydrochlorid

Analog Beispiel 124 erhält man die Titelverbindung ausgehend von 94 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-(2-carbamoyl-2.2-dimethyl-ethyl)amid: R_{f} (Dichlormethan-Methanol = 8:2) = 0.33; HPLC Rₜ= 11.26 Minuten; FAB-MS (M+H)⁺ = 552.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3-Amino-2.2-dimethyl-propionsäureamid-hydrochlorid hergestellt.

### Beispiel 138: 5(S)-Amino-4(S)-hydroxy-2(S),7(S)-diisopropyl-8-[4-methoxy-3-(3-methoxypropyloxy)-phenyl]-octansäure-N-[2,2-dimethyl-2-(N-methyl-carbamoyl)-ethyl]amid-hydrochlorid

Analog Beispiel 124 erhält man die Titelverbindung ausgehend von 87 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-{N-[2.2-dimethyl-2-(N-methyl-carbamoyl)-ethyl]}-amid: R_{f} (Dichlormethan-Methanol = 8:2) = 0.40; HPLC Rₜ= 11.69 Minuten; FAB-MS (M+H)⁺ = 566.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3-Amino-2.2-dimethyl-N-methyl-propionsäureamid-hydrochlorid hergestellt.

### a) 3-Amino-2.2-dimethyl-N-methyl-propionsäureamid-hydrochlorid

Analog Beispiel 121 a) aus 3-Benzyloxycarbonylamino-2.2-dimethyl-N-methyl-propionsäureamid.

### b) 3-Benzyloxycarbonylamino-2.2-dimethyl-N-methyl-propionsäureamid

4.19 g 3-Benzyloxycarbonylamino-2.2-dimethylpropionsäureethylester und 50 ml Methylamin 33% (in Ethanol) werden während 8 Tagen bei 60° C im Bombenrohr gerührt. Das Reaktionsgemisch wird eingedampft und der Rückstand mittels FC (220g Kieselgel, Dichlormethan-Methanol = 95:5) gereinigt. Man erhält die Titelverbindung: R_{f} (Dichlormethan-Methanol = 9:1) = 0.51.

### c) 3-Benzyloxycarbonylamino-2.2-dimethylpropionsäureethylester

29.04 g 3-Amino-2.2-dimethylpropionsäureethylester werden in 500 ml Essigsäureethylester und 250 ml 1M Natriumhydrogencarbonatlösung bei 0-5° C langsam mit 31 ml Chlorameisensäure-benzylester 90% versetzt. Man rührt noch 2 Stunden bei 0-5° C und extrahiert das Reaktionsgemisch mit Essigsäureethylester. Die organischen Phasen werden mit gesättigter Kochsalzlösung gewaschen und anschliessend eingeengt. Der Eindampfrückstand wird mittels FC (1 kg Kieselgel, Laufmittel Essigsäureethylester-Hexan = 1:3) gereinigt. Man erhält die Titelverbindung: R_{f} (Essigsäureethylester-Hexan = 1:3) = 0.28.

### Beispiel 139: 5(S)-Amino-4(S)-hydroxy-2(S),7(S)-diisopropyl-8-[4-methoxy-3-(3-methoxypropyloxy)-phenyl]-octansäure-N-[2-(N-methyl-carbamoyl)ethyl]amid-hydrochlorid

Analog Beispiel 124 erhält man die Titelverbindung ausgehend von 92 mg 5(S)-Tertiärbutoxycarbonylamino-4(S)-hydroxy-2(S),7(S)-diisopropyl-8-[4-methoxy-3-(3-methoxy propyloxy)-phenyl]-octansäure-N-[2-(N-methyl-carbamoyl)-ethyl]amid: R_{f} (Dichlormethan-Methanol = 8:2) = 0.24; HPLC Rₜ= 10.40 Minuten; FAB-MS (M+H)⁺ = 538.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3-Amino-N-methyl-propionsäureamid-hydrochlorid hergestellt.

### Beispiel 140: 5(S)-Amino-4(S)-hydroxy-2(S),7(S)-diisopropyl-8-[4-methoxy-3-(3-methoxypropyloxy)-phenyl]-octansäure-N-(3-morpholino-3-oxo-propyl)amid-hydrochlorid

Analog Beispiel 124 erhält man die Titelverbindung ausgehend von 99 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-(3-morpholino-3-oxo-propyl)amid: R_{f} (Dichlormethan-Methanol = 8:2) = 0.51; HPLC Rₜ= 11.35 Minuten; FAB-MS (M+H)⁺ = 594.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3-Aminopropionsäuremorpholid-hydrochlorid hergestellt.

### Beispiel 141: 5(S)-Amino-4(S)-hydroxy-2(S),7(S)-diisopropyl-8-[4-methoxy-3-(3-methoxypropyloxy)-phenyl]-octansäure-N-(2-carbamoyl-1(R,S)-methyl-ethyl)amid-hydrochlorid

Analog Beispiel 124 erhält man die Titelverbindung ausgehend von 86 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-[N-(2-carbamoyl-1(R,S)-methyl-ethyl)]-amid: R_{f} (Dichlormethan-Methanol = 8:2) = 0.24; HPLC Rₜ = 10.43/11.16 Minuten; FAB-MS (M+H)⁺ = 538.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3[4-methoxy-3-(3-methoxypropyloxy)-phenyl]-propyl}-2.2-dimethyl-1.3-oxazolidin (Beispiel 124 c) und 3(R,S)-Aminobuttersäureamid-hydrochlorid hergestellt.

### Beispiel 142: 5(S)-Amino-4(S)-hydroxy-2(S),7(S)-diisopropyl-8-[4-methoxy-3-(3-methoxypropyloxy)-phenyl]-octansäure-{N-[2-(N-methyl-carbamoyl)-1(R,S)-methyl-ethyl]}-amid-hydrochlorid

Analog Beispiel 124 erhält man die Titelverbindung ausgehend von 95 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-{N-[2-(N-methyl-carbamoyl)-1(R,S)-methyl-ethyl]}-amid: R_{f} (Dichlormethan-Methanol = 8:2) = 0.33; HPLC Rₜ= 10.78/11.45 Minuten; FAB-MS (M+H)⁺ = 552.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3(R,S)-Amino-N-methyl-buttersäureamid-hydrochlorid hergestellt.

### Beispiel 143: 5(S)-Amino-4(S)-hydroxy-2(S),7(S)-diisopropyl-8-[4-methoxy-3-(3-methoxypropyloxy)-phenyl]-octansäure-{N-[2-(N,N-dimethyl-carbamoyl)-1(R,S)-methyl-ethyl]}-amid-hydrochlorid

Analog Beispiel 124 erhält man die Titelverbindung ausgehend von 95 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-{N-[2-(N,N-dimethyl-carbamoyl)-1(R,S)-methyl-ethyl]}-amid: R_{f} (Dichlormethan-Methanol = 8:2) = 0.39; HPLC Rₜ= 11.44/12.04 Minuten; FAB-MS (M+H)⁺ = 566.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3(R,S)-Amino-N,N-dimethyl-buttersäureamid-hydrochlorid hergestellt.

### Beispiel 144: 5-(S)-Amino-4(S)-hydroxy-2(S),7(S)-diisopropyl-8-[4-methoxy-3-(3-methoxypropyloxy)-phenyl]-octansäure-[N-(2-carbamoyl-1(R)-isopropyl-ethyl)]-amid-hydrochlorid

Analog Beispiel 124 erhält man die Titelverbindung ausgehend von 71 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-[N-(2-carbamoyl-1(R)-isopropyl-ethyl)]-amid: R_{f} (Dichlormethan-Methanol = 8:2) = 0.27; HPLC Rₜ= 10.64 Minuten; FAB-MS (M+H)⁺= 566.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3(S)-Amino-4-methyl-pentansäureamid-hydrochlorid hergestellt.

### a) 3(S)-Amino-4-methyl-pentansäureamid-hydrochlorid

wird analog Beispiel 121a aus 3(R)-Benzyloxycarbonylamino-4-methyl-pentansäureamid hergestellt.

### b) 3(S)-Benzyloxycarbonylamino-4-methyl-pentansäureamid

2.23 g 3(S)-Benzyloxycardonylamino-4-methyl-pentansäureethylester und 50 ml 6N Ammoniak (in Methanol) werden während 6 Tagen bei 75° C im Bombenrohr gerührt. Das Reaktionsgemisch wird eingedampft und der Rückstand aus Essigsäureethylester kristallisiert. Man erhält die Titelverbindung: R_{f} (Dichlormethan-Methanol = 95:5) = 0.20; Smp. 171-172° C

### Beispiel 145: 5(S)-Amino-4(S)-hydroxy-2(S),7(S)-diisopropyl-8-[4-methoxy-3-(3-methoxypropyloxy)-phenyl]-octansäure-{N-[2-(N-methylcarbamoyl)-1(R)-isopropyl-ethyl]}-amid-hydrochlorid

Analog Beispiel 124 erhält man die Titelverbindung ausgehend von 81 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[2-(N-methylcarbamoyl)-1(R)-isopropyl-ethyl]amid: R_{f} (Dichlormethan-Methanol = 8:2) = 0.37; HPLC Rₜ= 10.96 Minuten; FAB-MS (M+H)⁺ = 580.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3(R)-Amino-4-methyl-pentansäure-N-(methyl)amid-hydrochlorid hergestellt.

### a) 3(R)-Amino-4-methyl-pentansäure-N-(methyl)amid-hydrochlorid

Analog Beispiel 121 a) aus 3(R)-Benzyloxycarbonylamino-4-methyl-pentansäure-N-(methyl)amid hergestellt.

### b) 3(R)-Benzyloxycarbonylamino-4-methyl-pentansäure-N-(methyl)amid

2.23 g 3(R)-Benzyloxycarbonylamino-4-methyl-pentansäureethylester und 50 ml Methylamin 33% (in Äthanol) werden während 48 Stunden bei Raumtemperatur stehen lassen. Das Reaktionsgemisch wird eingedampft und der Rückstand aus Essigsäureethylester kristallisiert. Man erhält die Titelverbindung: R_{f} (Dichlormethan-Methanol = 95:5) = 0.24; Smp. 190-191° C.

### Beispiel 146: 5(S)-Amino-4(S)-hydroxy-2(S),7(S)-diisopropyl-8-[4-methoxy-3-(3-methoxypropyloxy)-phenyl]-octansäure-{N-[2-(N,N-dimethylcarbamoyl)-1(R)-isopropyl-ethyl]}-amid-hydrochlorid

Analog Beispiel 124 erhält man die Titelverbindung ausgehend von 72 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-{N-[2-(N,N-dimethylcarbamoyl)-1(R)-isopropyl-ethyl]}-amid: R_{f} (Dichlormethan-Methanol = 8:2) = 0.45; HPLC Rₜ= 11.76 Minuten; FAB-MS (M+H)⁺ = 594.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1,3-oxazolidin (Beispiel 124 c) und 3(R)-Amino-4-methyl-pentansäure-N,N-dimethylamid-hydrochlorid hergestellt.

### a) 3(R)-Amino-4-methyl-pentansäure-N,N-dimethylamid-hydrochlorid wird analog Beispiel 121 a) aus 3(R)-Benzyloxycarbonylamino-4-methyl-pentansäure-N,N-dimethylamid hergestellt.

### b) 3(R)-Benzyloxycarbonylamino-4-methyl-pentansäure-N,N-dimethylamid

2.23 g 3(R)-Benzyloxycarbonylamino-4-methyl-pentansäureethylester und 50 ml Dimethylamin 30% (in Methanol) werden während 6 Tagen bei 75° C im Bombenrohr gerührt. Das Reaktionsgemisch wird eingedampft und der Rückstand mittels FC (Dichlormethan-Methanol = 97:3) gereinigt. Man erhält die Titelverbindung: R_{f} (Dichlormethan-Methanol = 95:5) = 0.40

### Beispiel 147: 5(S)-Amino-4(S)-hydroxy-2(S),7(S)-diisopropyl-8-[4-methoxy-3-(3-methoxypropyloxy)-phenyl]-octansäure-[N-(1(S)-carbamoyl-2-hydroxy-ethyl)]-amid-hydrochlorid

Analog Beispiel 130 erhält man die Titelverbindung ausgehend von 82 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-[N-(1(S)-carbamoyl-2-hydroxy-ethyl)]-amid: R_{f} (Dichlormethan-Methanol = 8:2) = 0.16; HPLC Rₜ= 10.09 Minuten; FAB-MS (M+H)⁺ = 540.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und L-Serinamid-hydrochlorid hergestellt.

### Beispiel 148: 5(S)-Amino-4(S)-hydroxy-2(S),7(S)-diisopropyl-8-[4-methoxy-3-(3-methoxypropyloxy)-phenyl]-octansäure-[N-(1(S),2-dicarbamoyl-ethyl)]-amid-hydrochlorid

Analog Beispiel 130 erhält man die Titelverbindung ausgehend von 68 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-[N-(1(S),2-dicarbamoyl-ethyl)]-amid: R_{f} (Dichlormethan-Methanol = 8:2) = 0.12; HPLC Rₜ= 9.54 Minuten; FAB-MS (M+H)⁺ = 567.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und L-Asparaginsäurediamid-hydrochlorid hergestellt.

### Beispiel 149: 5(S)-Amino-4(S)-hydroxy-2(S),7(S)-diisopropyl-8-[4-methoxy-3-(3-methoxypropyloxy)-phenyl]-octansäure-[N-(1(S),3-dicarbamoyl-propyl)]-amid-hydrochlorid

Analog Beispiel 130 erhält man die Titelverbindung ausgehend von 83 mg 5(S)-Tertiärbutoxycarbonylamino-4(S)-hydroxy-2(S),7(S)-diisopropyl-8-[4-methoxy-3-(3-methoxy-propyloxy)-phenyl]-octansäure-[N-(1(S),3-dicarbamoyl-propyl)]-amid: R_{f} (Dichlormethan-Methanol = 8:2) = 0.13; HPLC Rₜ = 9.50 Minuten; FAB-MS (M+H)⁺ = 581.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und L-Glutarsäurediamid-hydrochlorid hergestellt.

### Beispiel 150: 5(S)-Amino-4(S)-hydroxy-2(S),7(S)-diisopropyl-8-[4-methoxy-3-(3-methoxypropyloxy)-phenyl]-octansäure-[N-(1(S)-carbamoyl-propyl)]-amid-hydrochlorid

Analog Beispiel 130 erhält man die Titelverbindung ausgehend von 90 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-[N-(1(S)-carbamoyl-propyl)]-amid: R_{f} (Dichlormethan-Methanol = 8:2) = 0.30; HPLC Rₜ= 10.73 Minuten; FAB-MS (M+H)⁺ = 538.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 2(S)-Aminobuttersäureamid-hydrochlorid hergestellt.

### Beispiel 151: 5(S)-Amino-4(S)-hydroxy-2(S),7(S)-diisopropyl-8-[4-methoxy-3-(3-methoxypropyloxy)-phenyl]-octansäure-[N-(1(S)-carbamoyl-2(S)-methyl-butyl)]-amid-hydrochlorid

Analog Beispiel 130 erhält man die Titelverbindung ausgehend von 73 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-[N-(1(S)-carbamoyl-2(S)-methyl-butyl)]-amid: R_{f} (Dichlormethan-Methanol = 8:2) = 0.36; HPLC Rₜ= 11.59 Minuten; FAB-MS (M+H)⁺ = 566.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1,3-oxazolidin (Beispiel 124 c) und L-Isoleucinamid-hydrochlorid hergestellt.

### Beispiel 152: 5(S)-Amino-4(S)-hydroxy-2(S),7(S)-diisopropyl-8-[4-methoxy-3-(3-methoxypropyloxy)-phenyl]-octansäure-N-[2(R,S)-carbamoyl-2(R,S)-methyl-ethyl]-amid-hydrochlorid

Analog Beispiel 124 erhält man die Titelverbindung ausgehend von 93 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[2(R,S)-carbamoyl-2(R,S)-methyl-ethyl]-amid: R_{f} (Dichlormethan-Methanol = 8:2) = 0.28; HPLC Rₜ= 10.19/10.31 Minuten; FAB-MS (M+H)⁺ = 538.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3-Amino-2(R,S)-methyl-propionsäureamid-hydrochlorid hergestellt.

### Beispiel 153: 5(S)-Amino-4(S)-hydroxy-2(S),7(S)-diisopropyl-8-[4-methoxy-3-(3-methoxypropyloxy)-phenyl]-octansäure-N-[2(R,S)-(N-methylcarbamoyl)-2(R,S)-methyl-ethyl]-amid-hydrochlorid

Analog Beispiel 124 erhält man die Titelverbindung ausgehend von 93 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[2(R,S)-(N-methyl-carbamoyl)-2(R,S)-methyl-ethyl]amid: R_{f} (Dichlormethan-Methanol = 8:2) = 0.31; HPLC Rₜ= 10.76/10.85 Minuten; FAB-MS (M+H)⁺ = 552.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3-Amino-2(R,S)-methyl-propionsäure-N-methyl-amid-hydrochlorid hergestellt.

### a) 3-Amino-2(R,S)-methyl-propionsäure-N-methyl-amid-hydrochlorid

wird analog Beispiel 121 a) aus 3-Benzyloxycarbonylamino-2(R,S)-methyl-propionsäure-N-methyl-amid hergestellt.

### b) 3-Benzyloxycarbonylamino-2(R,S)-methyl-propionsäure-N-methyl-amid

2.52 g 3-Benzyloxycarbonylamino-2(R,S)-methyl-propionsäuremethylester und 50 ml Methylamin 33% (in Ethanol) werden während 48 Stunden bei Raumtemperatur gerührt. Das Reaktionsgemisch wird eingedampft und aus dem Rückstand durch Kristallisation aus Essigigsäureethylester die Titelverbindung erhalten: R_{f} (Dichlormethan-Methanol = 95:5) = 0.42; Smp. 128-129° C.

### c) 3-Benzyloxycarbonylamino-2(R,S)-methyl-propionsäuremethylester

22.6 g 3-Benzyloxycarbonylamino-2(R,S)-methyl-propionsäure werden in 230 ml Methanol mit einigen Tropfen Schwefelsäure konz. während 24 Stunden stehen gelassen. Das Reaktionsgemisch wird eingedampft und der Rückstand mittels FC (220 g Kieselgel, Dichlormethan) gereinigt. Man erhält die Titelverbindung: R_{f} (Dichlormethan-Methanol = 95:5) = 0.60

### d) 3-Benzyloxycarbonylamino-2(R,S)-methyl-propionsäure

25 g 3-Amino-2(R,S)-methyl-propionsäure werden in 533 ml 1N Natriumhydroxid bei 0° C mit einer Lösung aus 41.7 ml Chlorameisensäurebenzylester (90%) in Toluol versetzt. Das Reaktionsgemisch wird noch 30 Minuten bei 0° C nachgerührt. Nach Zugabe von 400 ml Diethylether wird die wässrige Phase abgetrennt und mit 140 ml 4N Salzsäure versetzt.

Durch extrahieren mit Diethylether erhält man aus der organischen Phase die rohe Titelverbindung: R_{f} (Dichlormethan-Methanol = 8:2) = 0.41

### Beispiel 154: 5(S)-Amino-4(S)-hydroxy-2(S),7(S)-diisopropyl-8-[4-methoxy-3-(3-methoxypropyloxy)-phenyl}-octansäure-[N-(2-carbamoyl-1(S)-methyl-ethyl)]-amid-hydrochlorid

Analog Beispiel 124 erhält man die Titelverbindung ausgehend von 445 mg 5(S)-Tertiärbutoxycarbonylamino-4(S)-hydroxy-2(S),7(S)-diisopropyl-8-[4-methoxy-3-(3-methoxy-propyloxy)-phenyl]-octansäure-[N-(2-carbamoyl-1(S)-methyl-ethyl)]-amid: R_{f} (Dichlormethan-Methanol = 8:2) = 0.24; HPLC Rₜ= 10.27 Minuten; FAB-MS (M+H)⁺ = 538.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3(S)-Aminobuttersäureamid-hydrochlorid hergestellt.

### Beispiel 155: 5(S)-Amino-4(S)-hydroxy-2(S),7(S)-diisopropyl-8-[4-methoxy-3-(3-methoxypropyloxy)-phenyl]-octansäure-[N-(2-carbamoyl-1(R)-methyl-ethyl)]-amid-hydrochlorid

Analog Beispiel 124 erhält man die Titelverbindung ausgehend von 110 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-[N-(2-carbamoyl-1(R)-methyl-ethyl)]-amid:R_{f} (Dichlormethan-Methanol = 8:2) = 0.24; HPLC Rₜ= 10.92 Minuten; FAB-MS (M+H)⁺ = 538.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3(R)-Aminobuttersäureamid-hydrochlorid hergestellt.

### Beispiel 156: 5(S)-Amino-4(S)-hydroxy-2(S),7(S)-diisopropyl-8-[4-methoxy-3-(3-methoxypropyloxy)-phenyl]-octansäure-N-[2(S)-carbamoyl-2(S)-methyl-ethyl]-amid-hydrochlorid

Analog Beispiel 124 erhält man die Titelverbindung ausgehend von 350 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[2(S)-carbamoyl-2(S)-methyl-ethyl]-amid (Diastereomer A): R_{f} (Dichlormethan-Methanol = 8:2) = 0.19; HPLC Rₜ= 10.50 Minuten; FAB-MS (M+H)⁺ = 538.

Das Ausgangsmaterial wurde wie folgt hergestellt:

### 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[2(S)-carbamoyl-2(S)-methyl-ethyl]-amid (Diastereomer A) und 5(S)-Tertiärbutoxycarbonylamino-4(S)-hydroxy-2(S),7(S)-diisopropyl-8-[4-methoxy-3-(3-methoxypropyloxy)-phenyl]-octansäure-[N-[2(S)-carbamoyl-2(R)-methyl-ethyl]-amid [Diastereomer B)

1.29 g 3-Tertiärbutoxycarbonyl-5(S)-{2-[N-(2-carbamoyl-2(R,S)-methyl-ethyl)-carbamoyl]-2(S)-isopropyl-ethyl}-4(S)-{2(S)-isopropyl-3-[4-methoxy-3-(3-methoxypropyloxy)-phenyl]-propyl}-2.2-dimethyl-1.3-oxazolidin werden in 50 ml Methanol bei 0° C mit 40 mg p-Toluolsulfonsäure (Monohydrat) versetzt. Die Reaktionslösung wird 18 Stunden bei Raumtemperatur weitergerührt. Nach dem Abdampfen des Lösungsmittels wird der Rückstand mit 100 ml gesättigter Natriumhydrogencarbonatlösung versetzt und mehrmals mit Essigsäureethylester extrahiert. Die eingedampften organischen Extrakte werden durch FC (5 mal 60 g Kieselgel, Dichlormethan-Methanol = 9:1) aufgetrennt und gereinigt. Man erhält die Titelverbindungen:
Diastereomer A: R_{f} (Dichlormethan-Methanol = 95:5) = 0.19
Diastereomer B: R_{f} (Dichlormethan-Methanol = 95:5) = 0.14

Das Ausgangsmaterial wird analog Beispiel 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3-Amino-2(R,S)-methylpropionsäureamid-hydrochlorid hergestellt.

### Beispiel 157: 5(S)-Amino-4(S)-hydroxy-2(S),7(S)-diisopropyl-8-[4-methoxy-3-(3-methoxypropyloxy)-phenyl]-octansäure-N-[2(R)-carbamoyl-2(R)-methyl-ethyl]-amid-hydrochlorid

Analog Beispiel 124 erhält man die Titelverbindung ausgehend von 370 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[2(R)-carbamoyl-2(R)-methyl-ethyl]-amid Diastereomer B (Beispiel 156 a)): R_{f} (Dichlormethan-Methanol = 8:2) = 0.19; HPLC Rₜ= 10.39 Minuten; FAB-MS (M+H)⁺ = 538.

### Beispiel 158: 5(S)-Amino-4(S)-hydroxy-2(S),7(S)-diisopropyl-8-[4-methoxy-3-(3-methoxypropyloxy)-phenyl]-octansäure-{N-[2(R)-(N-methyl-carbamoyl)-2(R)-methyl-ethyl]}-amid-hydrochlorid

Analog Beispiel 124 erhält man die Titelverbindung ausgehend von 60 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[2(R)-(N-methyl-carbamoyl)-2(R)-methyl-ethyl]-amid: R_{f} (Dichlormethan-Methanol = 8:2) = 0.31; HPLC Rₜ= 10.33 Minuten; FAB-MS (M+H)⁺ = 552.

Das Ausgangsmaterial wird analog Beispiel 130 a) aus 3-Tertiärbutoxycarbonyl-5(S)-{2-[N-(2(R)-(N-methyl-carbamoyl)-2(R)-methyl-ethyl)-carbamoyl]-2(S)-isopropyl-ethyl}-4(S)-{2(S)-isopropyl-3-[4-methoxy-3-(3-methoxypropyloxy)-phenyl]-propyl}-2,2-dimethyl-1,3-oxazolidin hergestellt.

### a) 3-Tertiärbutoxycarbonyl-5(S)-{2(S)-[N-(2(R)-(N-methyl-carbamoyl)-2(R)-methyl-ethyl)-carbamoyl]-2(S)-isopropyl-ethyl}-4(S)-{2(S)-isopropyl-3-[4-methoxy-3-(3-methoxypropyloxy)-phenyl]-propyl}-2,2-dimethyl-1.3-oxazolidin

120 mg 3-Tertiärbutoxycarbonyl-5(S)-{2(S)-[N-(2(R)-methoxycarbonyl)-2(R)-methyl-ethyl)-carbamoyl]-2(S)-isopropyl-ethyl}-4(S)-{2(S)-isopropyl-3-[4-methoxy-3-(3-methoxypropyloxy)-phenyl]-propyl}-2.2-dimethyl-1.3-oxazolidin werden in 5 ml Methylaminlösung 33 % (in Ethanol) während 48 Stunden bei Raumtemperatur stehen gelassen. Das Reaktionsgemisch wird eingedampft und der Rückstand wird durch FC (30 g Kieselgel, Dichlormethan-Methanol = 95:5) gereinigt. Man erhält die Titelverbindung: R_{f} (Dichlormethan-Methanol = 95:5) = 0.30

### b) 3-Tertiärbutoxycarbonyl-5(S)-{2(S)-[N-(2(R)-methoxycarbonyl)-2(R)-methyl-ethyl)-carbamoyl]-2(S)-isopropyl-ethyl}-4(S)-{2(S)-isopropyl-3-[4-methoxy-3-(3-methoxypropyloxy)-phenyl]-propyl}-2,2-dimethyl-1.3-oxazolidin

Die Titelverbindung wird analog Beispiel 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3-Amino-2(R)-methylpropionsäuremethylester-hydrochlorid hergestellt.

### c) 3-Amino-2(R)-methylpropionsäuremethylester-hydrochlorid

2.7 g 3-Azido-2(R)-methylpropionsäuremethylester werden in Gegenwart von 1.4 g Pd/C 10% in 50 ml Tetrahydrofuran für 4 Stunden bei Raumtemperatur bei pH 6.0 (pH-Stat; 2N Salzsäure) hydriert. Das Reaktionsgemisch wird filtriert und eingedampft. Durch Kristallisation aus Isopropanol-Diethylether erhält man die Titelverbindeung: ¹H NMR (DMSO-d₆), δ(ppm) = 7.95 (3H, bs), 3.65 (3H, s), 3.12-2.78 (3H, m), 1.15 (3H, d); Smp. 122-125° C.

### Beispiel 159: 5(S)-Amino-4(S)-hydroxy-2(S),7(S)-diisopropyl-8-[4-methoxy-3-(3-methoxy-propyloxy)-phenyl]-octansäure-{N-[2(S)-(N-methyl-carbamoyl)-2(S)-methyl-ethyl]}-amid-hydrochlorid

Analog Beispiel 124 erhält man die Titelverbindung ausgehend von 81 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-{N-[2(S)-(N-methyl-carbamoyl)-2(S)-methyl-ethyl]}-amid: R_{f} (Dichlormethan-Methanol = 8:2) = 0.31; HPLC Rₜ= 10.50 Minuten; FAB-MS (M+H)⁺ = 552.

Das Ausgangsmaterial wird analog Beispiel 158 a) bis c) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3-Amino-2(S)-methylpropionsäuremethylester-hydrochlorid hergestellt.

### Beispiel 160: 5(S)-Amino-4(S)-hydroxy-2(S),7(S)-diisopropyl-8-[4-methoxy-3-(3-methoxypropyloxy)-phenyl]-octansäure-[N-(2-carboxy-2.2-dimethyl-ethyl)]-amid-hydrochlorid

Analog Beispiel 124 erhält man die Titelverbindung ausgehend von 71 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-[N-(2-carboxy-2.2-dimethyl-ethyl)]-amid: R_{f} (Dichlormethan-Methanol = 8:2) = 0.52; HPLC Rₜ = 10.95 Minuten; FAB-MS (M+H)⁺ = 553.

Das Ausgangsmaterial wird analog Beispiel 130 a) aus 3-Tertiärbutoxycarbonyl-5(S)-{2(S)-[N-(2-carboxy-2.2-dimethyl-ethyl)-carbamoyl]-2(S)-isopropyl-ethyl}-4(S)-{2(S)-isopropyl-3-[4-methoxy-3-(3-methoxypropyloxy)-phenyl]-propyl}-2.2-dimethyl-1.3-oxazolidin hergestellt.

### a) 3-Tertiärbutoxycarbonyl-5(S)-{2(S)-[N-(2-carboxy-2,2-dimethyl-ethyl)-carbamoyl]-2(S)-isopropyl-ethyl}-4(S)-{2(S)-isopropyl-3-[4-methoxy-3-(3-methoxypropyloxy)-phenyl]-propyl}-2.2-dimethyl-1.3-oxazolidin

36 mg 3-Tertiärbutoxycarbonyl-5(S)-{2(S)-[N-(2-ethyloxycarbonyl-2.2-dimethyl-ethyl)-carbamoyl]-2(S)-isopropyl-ethyl}-4(S)-{2(S)-isopropyl-3-[4-methoxy-3-(3-methoxypropyloxy)-phenyl]-propyl}-2,2-dimethyl-1.3-oxazolidin werden in 1 ml Ethanol und 0.1 ml 2N Kaliumhydroxid während 24 Stunden bei Raumtemperatur gerührt. Das Reaktionsgemisch wird eingedampft und nach Zugabe von 0.1 ml 2N Salzsäure mehrmals mit Diethylether extrahiert. Die eingedampften Extrakte werden durch FC (18 g Kieselgel, Dichlormethan-Methanol = 9:1) gereinigt. Man erhält die Titelverbindung R_{f} (Dichlormethan-Methanol = 9:1) = 0.45.

Das Ausgangsmaterial wird analog Beispiel 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3-Amino-2.2-dimethylpropionsäureethylester hergestellt.

### Beispiel 161: 5(S)-Amino-4(S)-hydroxy-2(S),7(S)-diisopropyl-8-[4-methoxy-3-(3-methoxypropyloxy)-phenyl]-octansäure-[N-(2-carboxy-2.2-diethyl-ethyl)]-amid-hydrochlorid

Analog Beispiel 124 erhält man die Titelverbindung ausgehend von 136 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-[N-(2-carboxy-2.2-diethyl-ethyl)]-amid: R_{f} (Dichlormethan-Methanol = 8:2) = 0.26; HPLC Rₜ= 12.53 Minuten; FAB-MS (M+H)⁺ = 581.

Das Ausgangsmaterial wird analog Beispiel 130 a) aus 3-Tertiärbutoxycarbonyl-5(S)-{2(S)-[N-(2-carboxy-2.2-diethyl-ethyl)-carbamoyl]-2(S)-isopropyl-ethyl}-4(S)-{2(S)-isopropyl-3-[4-methoxy-3-(3-methoxypropyloxy)-phenyl]-propyl}-2.2-dimethyl-1.3-oxazolidin hergestellt.

### a) 3-Tertiärbutoxycarbonyl-5(S)-{2-[N-(2-carboxy-2.2-diethyl-ethyl)-carbamoyl]-2(S)-isopropyl-ethyl}-4(S)-{2(S)-isopropyl-3-[4-methoxy-3-(3-methoxypropyloxy)-phenyl]-propyl}-2.2-dimethyl-1.3-oxazolidin

258 mg 3-Tertiärbutoxycarbonyl-5(S)-{2-[N-(2-(2-ethyloxycarbonyl-2.2- diethyl-ethyl)-carbamoyl]-2(S)-isopropyl-ethyl}-4(S)-{2(S)-isopropyl-3-[4-methoxy-3-(3-methoxypropyloxy)-phenyl]-propyl}-2.2 -dimethyl-1.3-oxazolidin werden in 6 ml Äthanol und 0.69 ml 2N Kaliumhydroxid während 24 Stunden bei 80° C gerührt. Das Reaktionsgemisch wird eingedampft und nach Zugabe von 0.69 ml 2N Salzsäure mehrmals mit Diethylether extrahiert. Die eingedampften Extrakte werden durch FC (35 g Kieselgel, Dichlormethan-Methanol = 9:1) gereinigt. Man erhält die Titelverbindung R_{f} (Dichlormethan-Methanol = 9:1) = 0.50

Das Ausgangsmaterial wird analog Beispiel 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 3-Amino-2.2-diethylpropionsäureethylester hergestellt.

### Beispiel 162: 5(S)-Amino-4(S)-hydroxy-2(S),7(S)-diisopropyl-8-[4-methoxy-3-(3-methoxypropyloxy)-phenyl]-octansäure-N-[{1-carboxy-cyclopentyl)-methyl]amid-hydrochlorid

Analog Beispiel 124 erhält man die Titelverbindung ausgehend von 142 mg 5(S)-Tertiärbutoxycarbonylamino-4(S)-hydroxy-2(S),7(S)-diisopropyl-8-[4-methoxy-3-(3-methoxy propyloxy)-phenyl]-octansäure-N-[(1-carboxy-cyclopentyl)-methyl]amid: R_{f} (Dichlormethan-Methanol = 8:2) = 0.26; HPLC Rₜ= 12.18 Minuten; FAB-MS (M+H)⁺ = 579.

Das Ausgangsmaterial wird analog Beispiel 130 a), 161 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 1-(Aminomethyl)cyclopentan-1-carbonsäureethylester hergestellt.

### Beispiel 163: 5(S)-Amino-4(S)-hydroxy-2(S),7(S)-diisopropyl-8-[4-methoxy-3-(3-methoxypropyloxy)-phenyl]-octansäure-{N-[2-(1H-tetrazol-5-yl)-ethyl]}-amid-hydrochlorid

Analog Beispiel 124 erhält man die Titelverbindung ausgehend von 100 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-{N-[2-(1H-tetrazol-5-yl)-ethyl]}-amid: R_{f} (Dichlormethan-Methanol = 8:2) = 0.19; HPLC Rₜ= 12.30 Minuten; FAB-MS (M+H)⁺ = 549.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 2-(1H-Tetrazol-5-yl)-ethylamin hergestellt.

### Beispiel 164: 5(S)-Amino-4(S)-hydroxy-2(S),7(S)-diisopropyl-8-[4-methoxy-3-(3-methoxypropyloxy)-phenyl]-octansäure-N-[1(S)-(5-oxopyrrolidin-2-yl)methyl]-amid-hydrochlorid

Analog Beispiel 124 erhält man die Titelverbindung ausgehend von 100 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[(S)-(5-oxopyrrolidin-2-yl)methyl]amid: R_{f} (Dichlormethan-Methanol = 8:2) = 0.27; HPLC Rₜ= 12.55 Minuten; FAB-MS (M+H)⁺ = 550.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 5(S)-(Aminomethyl)-2-pyrrolidon hergestellt.

### Beispiel 165: 5(S)-Amino-4(S)-hydroxy-2(S),7(S)-diisopropyl-8-[4-methoxy-3-(3-methoxypropyloxy)-phenyl]-octansäure-N-[1(R)-(5-oxopyrrolidin-2-yl)methyl]-amid-hydrochlorid

Analog Beispiel 124 erhält man die Titelverbindung ausgehend von 95 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[1(R)-(5-oxopyrrolidin-2-yl)methyl]-amid: R_{f} (Dichlormethan-Methanol = 8:2) = 0.3l; HPLC Rₜ= 12.24 Minuten; FAB-MS (M+H)⁺ = 550.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 5(R)-(Aminomethyl)-2-pyrrolidon hergestellt.

### Beispiel 166: 5(S)-Amino-4(S)-hydroxy-2(S),7(S)-diisopropyl-8-[4-methoxy-3-(3-methoxypropyloxy)-phenyl]-octansäure-{N-[(N,N-dimethyl)-carbamoyl-methyl}-amid-hydrochlorid

Analog Beispiel 130 erhält man die Titelverbindung ausgehend von 56 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-{N-[(N,N-dimethyl)-carbamoyl-methyl]}-amid nach Lyophilisation: R_{f} (Essigsäureethylester-Methanol-Ammoniak konz. = 80:15:5) = 0.42; HPLC Rₜ = 11.82 Minuten; FAB-MS (M+H)⁺ = 538.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 2(S)-Aminoessigsäure-(N,N-dimethyl)-amid-hydrochlorid hergestellt.

### Beispiel 167: 5(S)-Amino-4(S)-hydroxy-2(S),7(S)-diisopropyl-8-[4-methoxy-3-(3-methoxyprooyloxy)-phenyl]-octansäure-N-[(morpholin-4-yl)carbonyl-methyl]amid-hydrochlorid

Analog Beispiel 130 erhält man die Titelverbindung ausgehend von 76 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[(morpholin-4-yl)carbonyl-methyl]-amid nach Lyophilisation: R_{f} (Essigsäureethylester-Methanol-Ammoniak konz. = 80:15:5) = 0.43; HPLC Rₜ = 10.66 Minuten; FAB-MS (M+H)⁺ = 580.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 2-Aminoessigsäure-N-(morpholid-hydrochlorid hergestellt.

### Beispiel 168: 5(S)-Amino-4(S)-hydroxy-2(S),7(S)-diisopropyl-8-[4-methoxy-3-(3-methoxypropyloxy)-phenyl]-octansäure-[N-(1(S)-carbamoyl-ethyl)]-amid-hydrochlorid

Analog Beispiel 130 erhält man die Titelverbindung ausgehend von 64 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-[N-(1(S)-carbamoyl-ethyl)]-amid nach Lyophilisation: R_{f} (Essigsäureethylester-Methanol-Ammoniak konz. = 80:15:5) = 0.42; HPLC Rₜ= 10.48 Minuten; FAB-MS (M+H)⁺ = 524.

Das Ausgangsmaterial wird analog Beispiel 130 a) und 130 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 2(S)-Aminopropionsäureamid-hydrochlorid hergestellt.

### Beispiel 169: 5(S)-Amino-4(S)-hydroxy-2(S),7(S)-diisopropyl-8-[4-methoxy-3-(3-methoxypropyloxy)-phenyl]-octansäure-N-{1(S)-[(N-methyl)-carbamoyl]-ethyl}-amid-hydrochlorid

Analog Beispiel 130 erhält man die Titelverbindung ausgehend von 31 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-{1(S)-[(N-methyl)-cardamoyl]-ethyl}-amid nach Lyophilisation: R_{f} (Essigsäureethylester-Methanol-Ammoniak konz. = 80:15:5) = 0.38; HPLC Rₜ = 11.08 Minuten; FAB-MS (M+H)⁺ = 538.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyl oxy)-phenyl]-propyl}-2.2-dimethyl-1.3-oxazolidin (Beispiel 124 c) und 2(S)-Aminopropionsäure-(N-methyl)-amid-hydrochlorid hergestellt.

### Beispiel 170: 5(S)-Amino-4(S)-hydroxy-2(S),7(S)-diisopropyl-8-[4-methoxy-3-(3-methoxy-propyloxy)-phenyl]-octansäure-N-{1(S)-[(N,N-dimethyl)-carbamoyl]-ethyl}-amid-hydrochlorid

Analog Beispiel 130 erhält man die Titelverbindung ausgehend von 86 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-{1(S)-[(N,N-dimethyl)-carbamoyl]-ethyl}-amid nach Lyophilisation: R_{f} (Essigsäureethylester-Methanol-Ammoniak konz. = 80:15:5) = 0.50; HPLC Rₜ = 11.53 Minuten; FAB-MS (M+H)⁺ = 552.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 2(S)-Aminopropionsäure-(N,N-dimethyl)-amid-hydrochlorid hergestellt.

### Beispiel 171: 5(S)-Amino-4(S)-hydroxy-2(S),7(S)-diisopropyl-8-[4-methoxy-3-(3-methoxypropyloxy)-phenyl]-octansäure-N-{1(S)-N-[(morpholin-4-yl)-carbonyl]ethyl}amid-hydrochlorid

Analog Beispiel 130 erhält man die Titelverbindung ausgehend von 51 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-{1(S)-N-[(morpholin-4-yl)-carbonyl]ethyl}amid nach Lyophilisation: R_{f} (Essigsäureethylester-Methanol-Ammoniak konz. = 80:15:5) = 0.51; HPLC Rₜ = 11.29 Minuten; FAB-MS (M+H)⁺ = 594.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 2(S)-Aminopropionsäure-N-(morpholid-hydrochlorid hergestellt.

### Beispiel 172: 5(S)-Amino-4(S)-hydroxy-2(S),7(S)-diisopropyl-8-[4-methoxy-3-(3-methoxypropyloxy)-phenyl]-octansäure-N-[1(S)-carbamoyl-butyl]amid-hydrochlorid

Analog Beispiel 130 erhält man die Titelverbindung ausgehend von 49 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[1(S)-carbamoyl-butyl]amid nach Lyophilisation: R_{f} (Essigsäureethylester) = 0.38; HPLC Rₜ= 10.67 Minuten; FAB-MS (M+H)⁺ = 552.

Das Ausgangsmaterial wird analog Beispiel 130 a) und 130 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2,2-dimethyl-1.3-oxazolidin (Beispiel 124 c) und 2(S)-Aminopentansäureamid-hydrochlorid hergestellt.

### Beispiel 173: 5(S)-Amino-4(S)-hydroxy-2(S),7(S)-diisopropyl-8-[4-methoxy-3-(3-methoxypropyloxy)-phenyl]-octansäure-N-[1(S)-carbamoyl-2-methyl-propyl]-amid-hydrochlorid

Analog Beispiel 130 erhält man die Titelverbindung ausgehend von 65 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[1(S)-carbamoyl-2-methyl-propyl]-amid nach Lyophilisation: R_{f} (Essigsäureethylester-Methanol-Ammoniak konz. = 80:15:5) = 0.47; HPLC Rₜ = 11.22 Minuten; FAB-MS (M+H)⁺ = 552.

Das Ausgangsmaterial wird analog Beispiel 130 a) und 130 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 2(S)-Amino-3-methylbutteräureamid-hydrochlorid hergestellt.

### Beispiel 174: 5(S)-Amino-4(S)-hydroxy-2(S),7(S)-diisopropyl-8-[4-methoxy-3-(3-methoxypropyloxy)-phenyl]-octansäure-N-[1(S)-(N-methylcarbamoyl)-2-methyl-propyl]amid-hydrochlorid

Analog Beispiel 130 erhält man die Titelverbindung ausgehend von 58 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[1(S)-(N-methylcarbamoyl)-2-methyl-propyl]amidnach Lyophilisation: R_{f} (Essigsäureethylester-Methanol-Ammoniak konz. = 80:15:5) = 0.51; HPLC Rₜ = 11.87 Minuten; FAB-MS (M+H)⁺ = 566.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 2(S)-Amino-3-methylbuttersäure-(N-methyl)amid-hydrochlorid hergestellt.

### Beispiel 175: 5(S)-Amino-4(S)-hydroxy-2(S),7(S)-diisopropyl-8-[4-methoxy-3-(3-methoxypropyloxy)-phenyl]-octansäure-N-[1(S)-(N,N-dimethylcarbamoyl)-2-methyl-propyl]amid-hydrochlorid

Analog Beispiel 130 erhält man die Titelverbindung ausgehend von 80 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[1(S)-(N,N-dimethylcarbamoyl)-2-methyl-propyl]amid nach Lyophilisation: R_{f} (Essigsäureethylester-Methanol-Ammoniak konz. = 80:15:5) = 0.62; HPLC Rₜ= 12.36 Minuten; FAB-MS (M+H)⁺ = 580.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 2(S)-Amino-3-methyl-buttersäure-(N,N-dimethyl)amid-hydrochlorid hergestellt.

Das Ausgangsmaterial wird wie folgt hergestellt:

### a) 2(S)-Amino-3-methylbuttersäure-(N,N-dimethyl)amid-hydrochlorid

0.85 g 2(S)-Tertiärbutoxycarbonylamino-3-methylbutansäure-(N,N-dimethyl)amid werden in 10 ml 4N Salzsäure in Dioxan bei 0° C gelöst und für 7 Stunden bei 0° C gerührt. Das Reaktionsgemisch wird lyophilisiert und man erhält die Titelverbindung: R_{f} (Essigsäureethylester-Methanol-Ammoniak konz. = 80:15:5) = 0.23.

### Beispiel 176: 5(S)-Amino-4(S)-hydroxy-2(S),7(S)-diisopropyl-8-[4-methoxy-3-(3-methoxypropyloxy)-phenyl]-octansäure-N-{1(S)-[(morpholin-4-yl)carbonyl]-2-methyl-propyl}amid-hydrochlorid

Analog Beispiel 130 erhält man die Titelverbindung ausgehend von 74 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-{1(S)-[(morpholin-4-yl)carbonyl]-2-methyl-propyl}amid nach Lyophilisation: R_{f} (Essigsäureethylester-Methanol-Ammoniak konz. = 80:15:5) = 0.59; HPLC Rₜ = 11.81 Minuten; FAB-MS (M+H)⁺ = 622.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2,2-dimethyl-1.3-oxazolidin (Beispiel 124 c) und 2(S)-Amino-3-methylbutansäure-N-(morpholid-hydrochlorid hergestellt.

### Beispiel 177: 5(S)-Amino-4(S)-hydroxy-2(S),7(S)-diisopropyl-8-[4-methoxy-3-(3-methoxypropyloxy)-phenyl]-octansäure-N-[2-(N-methylsulfonyl)ethyl]amid-hydrochlorid

Analog Beispiel 124 erhält man die Titelverbindung ausgehend von 90 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[2-(N-methylsulfonyl)ethyl]amid nach Lyophilisation: R_{f} (Essigsäureethylester-Methanol-Ammoniak konz. = 80:15:5) = 0.52; HPLC Rₜ = 11.50 Minuten; FAB-MS (M+H)⁺ = 574.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 2-Aminoethyl-(N-methyl)-sulfonamid hergestellt.

### Beispiel 178: 5(S)-Amino-4(S)-hydroxy-2(S),7(S)-diisopropyl-8-[4-methoxy-3-(3-methoxypropyloxy)-phenyl]-octansäure-N-{2-[N-(morpholin-4-yl)-sulfonyl]ethyl}amid-hydrochlorid

Analog Beispiel 130 erhält man die Titelverbindung ausgehend von 98 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-{2-[N-(morpholin-4-yl)-sulfonyl]ethyl}amid nach Lyophilisation: R_{f} (Essigsäureethylester-Methanol-Ammoniak konz. = 80:15:5) = 0.53; HPLC Rₜ = 11.63 Minuten; FAB-MS (M+H)⁺ = 630.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 2-Aminoethyl-N-(morpholiny-4-l)-sulfonamid hergestellt.

Das Ausgangsmaterial wird wie folgt hergestellt:

### a) 2-Aminoethyl-N-(moroholin-4-yl)-sulfonamid

3.0 g 2-Phthaloylaminoethyl-N-(morpholin-4-yl)-sulfonamid werden in 20 ml Methanol, mit 20 ml Hydrazin-hydrat während 2 Stunden am Rückfluss gerührt. Das Rekationsgemisch wird abgekühlt und mit 1.0 ml konzentrierter Salzsäure und 15 ml Methanol versetzt. Das Reaktionsgemisch wird filtriert und das Filtrat eingeengt. Nach Zugabe von 10 ml Kaliumhydroxidlösung 10% erhält man ödurch Extraktion mit Dichlormethan die Titelverbindung: R_{f} (Essigsäureethylester-Methanol-Ammoniak konz. = 80:15:5) = 0.26.

### b) 2-Phthaloylaminoethyl-N-(morpholin-4-yl)-sulfonamid

5.0 g 2-Phthaloylaminoethylsulfonylchlorid werden in 40 ml Dichlormethan bei -12° C mit 4.77 ml Morpholin versetzt. Man rührt 30 Minuten bei 0° und wäscht das Reaktionsgemisch mit Wasser. Die organische Phase wird über Natriumsulfat getrocknet und eingeengt. Man erhält die Titelverbindung: R_{f} (Essigsäureethylester-Methanol-Ammoniak konz. = 80:15:5) = 0.68.

### Beispiel 179: 5(S)-Amino-4(S)-hydroxy-2(S),7(S)-diisopropyl-8-[4-methoxy-3-(3-methoxypropyloxy)-phenyl]-octansäure-N-[2-(N-acetyl)-piperidin-4-yl)ethyl]amid-hydrochlorid

Analog Beispiel 124 erhält man die Titelverbindung ausgehend von 42 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[2-(N-acetyl)-piperidin-4-yl)ethyl]amid nach Lyophilisation: R_{f} (Essigsäureethylester-Methanol-Ammoniak konz. = 80:15:5) = 0.51; HPLC Rₜ= 12.06 Minuten; FAB-MS (M+H)⁺ = 606.

Das Ausgangsmaterial wird analog Beispiel 130 a) und 124 b) aus 3-Tertiärbutoxycarbonyl-5(S)-(2(S)-carboxy-3-methyl-butyl)-4(S)-{2(S)-isopropyl-3-[4-methoxy-3-(3-methoxypropyloxy)-phenyl]-propyl}-2.2-dimethyl-1.3-oxazolidin (Beispiel 124 c) und 4-(2-Aminoethyl)-(N-acetyl)-piperidin-hydrochlorid hergestellt.

Das Ausgangsmaterial wird wie folgt hergestellt:

### a) 4-(2-Aminoethyl)-(N-acetyl)-piperidin-hydrochlorid

wird analog Beispiel 175 a) aus 4-(2-Tertiärbutoxycarbonylaminoethyl)-(N-acetyl)-piperidin hergestellt.

### b) N-Acetyl-4-(2-tertiärbutoxycarbonylaminoethyl)-piperidin

0.5 g 4-(2-Tertiärbutoxycarbonylaminoethyl)-piperidin und 0.61 ml Triethylamin werden in 5 ml Dichlormethan gelöst und bei 0° C mit 0.22 ml Acetylchlorid versetzt. Das Raktionsgemisch wird 7 Stunden bei Raumtemperatur gerührt und anschliessend mit Wasser gewaschen. Die eingedampften organische Phase wird eingedampft und durch FC (10 g Kieselgel, Essigsäureethylester/Methanol = 9:1) gereinigt. Man erhält die Titelverbindung R_{f} (Essigsäureethylester-Methanol = 9:1) = 0.39.

### Beispiel 180: 5(S)-Amino-4(S)-hydroxy-2(S),7(S)-diisopropyl-8-[4-methoxy-3-(3-methoxypropyloxy)-phenyl]-octansäure-N-[(N-acetyl-piperidin-4-yl)methyl]amid-hydrochlorid

Analog Beispiel 130 erhält man die Titelverbindung ausgehend von 71 mg 5(S)-Tertiärbutoxycarbonylamino-4(S)-hydroxy-2(S),7(S)-diisopropyl-8-[4-methoxy-3-(3-methoxypropyloxy)-phenyl]-octansäure-N-[(N-acetyl-piperidin-4-yl)methyl]amid nach Lyophilisation: R_{f} (Essigsäureethylester-Methanol-Ammoniak konz. = 80:15:5) = 0.44; HPLC Rₜ= 12.83 Minuten; FAB-MS (M+H)⁺ = 629.

Das Ausgangsmaterial wird analog Beispiel 130 a) und 130 b) aus 3-Tertiärbutoxycarbonyl-5(S)-(2(S)-carboxy-3-methyl-butyl)-4(S)-{2(S)-isopropyl-3-[4-methoxy-3-(3-methoxypropyl oxy)-phenyl]-propyl}-2.2-dimethyl-1.3-oxazolidin (Beispiel 124 c) und 4-Aminomethyl-(N-acetyl)-piperidin-hydrochlorid hergestellt.

### Beispiel 181: 5(S)-Amino-4(S)-hydroxy-2(S),7(S)-diisopropyl-8-[4-methoxy-3-(4-methoxy-butyl)-phenyl]-octansäure-N-(2-carbamoyl-2,2-dimethyl-ethyl)amid-hydrochlorid

Analog Beispiel 105 erhält man die Titelverbindung ausgehend von 25 mg 5(S)-Tertiärbutoxycarbonylamino-4(S)-hydroxy-2(S), 7(S)-diisopropyl-8-[4-methoxy-3-(4-methoxy-butyl)-phenyl]-octansäure-N-(2-carbamoyl-2,2-dimethyl-ethyl)amid: R_{f} (Dichlormethan/Methanol/Ammoniak konz. = 350:50:1) = 0.30; HPLC Rₜ = 13.31 Minuten; FAB-MS (M+H)⁺ = 550.

Das Ausgangsmaterial wird analog Beispiel 82 d), 82 e), Beispiel 83) und Beispiel 105 hergestellt, wobei in Stufe Beispiel 82 d) an Stelle von 4-(3-Benzyloxypropyloxy)-3-(3-methoxypropyloxy)-brombenzol das 4-Methoxy-3-(4-methoxy-butyl)-brombenzol eingesetzt wird.

Dieses wird wie folgt hergestellt.

### a) 4-Methoxy-3-(4-methoxybutyl)-brombenzol

Eine Lösung von 50 g 4-Methoxy-3-(4-methoxy-2-butenyl)-brombenzol in 700 ml Tetrahydrofuran wird bei Normaldruck und Raumtemperatur in Gegenwart von 2.5 g Pt/C 5% während 2 Stunden hydriert. Das Reaktionsgemisch wird abfiltriert. Das Filtrat wird eingedampft. Der aus dem Filtrat erhaltene Eindampfrückstand wird mittels FC (1.6 kg Kieselgel, Hexan/Essigsäureethylester = 20:1) gereinigt. Nach Destillation im Hochvakuum erhält man die Titelverbindung: R_{f} (Hexan/Essigsäureethylester = 10:1) = 0.38; HPLC Rₜ = 19.92 Minuten; FAB-MS (M+H)⁺ = 273.

### b) 4-Methoxy-3-(4-methoxy-2-butenyl)-brombenzol

Zur einer bei 5° gerührten Lösung von 110.8 g Natrium-bis-(trimethylsilyl)-amid in 1200 ml Tetrahydrofuran werden 251.1 g 3-Methoxy-propyl-triphenyl-phosphonium-bromid zugegeben. Das Reaktiongemisch wird 45 Minuten bei 0° weitergerührt und dann mit der Lösung von 100 g 5-Brom-o-anisaldehyd in 1000 ml Tetrahydrofuran tropfenweise versezt. Das Reaktionsgemisch wird noch 1 Stunde bei 0° weitergerührt. Dann wird bei 0° C 1 Liter einer gesättigten Ammoniumchloridlösung zugetropft. Nach dem Einengen wird der Rückstand 4x mit Essigsäureethylester extrahiert. Die organischen Phasen werden mit Wasser und gesättigter Kochsalzlösung gewaschen und eingedampft. Der Rückstand wird mittels FC (500 g Kieselgel, Hexan-Essigsäureethylester = 5:1) gereinigt. Nach Destillation im Hochvakuum erhält man die Titelverbindung: R_{f} (Hexan-Essigsäureethylester = 4:1) = 0.61.

### Beispiel 182: 5(S)-Amino-4(S)-hydroxy-2(S),7(S)-diisopropyl-8-[4-methoxy-3-(3-methoxypropyloxy)-phenyl]-octansäure-N-[2-(N,N-dimethylcarbamoyl)ethyl]amid-natriumdihydrogencitrat

768 mg 5(S)-Amino-4(S)-hydroxy-2(S),7(S)-diisopropyl-8-[4-methoxy-3-(3-methoxy-propyl-oxy)-phenyl]-octansäure-N-[2-(N,N-dimethylcarbamoyl)ethyl]amid-hydrochlorid (Beispiel 134) werden in 50 ml 0.1 N Natriumhydroxid verrührt und mehrmals mit Dichlormethan extrahiert. Die Extrakte werden eingeengt und der Rückstand in 50 ml Äthanol gelöst. Die gerührte Lösung wird nacheinander mit 274 mg Citronensäuremonohydrat, 50 ml Wasser und 1.30 ml 1N Natriumhydroxid versetzt. Die Lösung wird anschliessend zur Trockene eingedampft und der Rückstand in 100 ml Wasser aufgenommen und lyophilisiert. Das Lyophilisat wird in Methanol gelöst und klarfiltriert, das Filtrat eingeengt und der Rückstand im Hochvakuum bei Raumtemperatur getrocknet. Man erhält die Titelverbindung als weissen amorphen Festkörper mit dem Smp. 80° C.

### Beispiel 183: 5(S)-Amino-4(S)-hydroxy-2(S),7(S)-diisopropyl-8-[4-methoxy-3(4-methoxy-butyl)-phenyl]-octansäure-N-(2-morpholinoethyl)amid-dihydrochlorid Analog Beispiel 124 erhält man die Titelverbindung ausgehend von 100 mg 5(S)-Tertiärbutoxycarbonylamino-4(S)-hydroxy-2(S),7(S)-diisopropyl-8-[4-methoxy-3-(4-methoxybutyl)-phenyl]-octansäure-N-[2-(4-morpholino)ethyl]-amid: R_{f} (Dichlormethan/Methanol = 10:1) = 0.21; HPLC Rₜ = 12.69 Minuten; FAB-MS (M+H)⁺ = 564.

Das Ausgangsmaterial wird wie folgt hergestellt:

### a) 5(S)-Tertiärbutoxycarbonylamino-4(S)-hydroxy-2(S),7(S)-diisopropyl-8-[4-methoxy-3-(4-methoxybutyl)-phenyl]-octansäure-N-[2-(4-morpholino)-ethyl]-amid

Eine Lösung von 100 mg 3(S)-Isopropyl-5(S)-{1(S)-Tertiärbutoxycarbonylamino-3(S)-isopropyl-4-[4-methoxy-3-(4-methoxybutyl)-phenyl]-butyl}-tetrahydrofuran-2-on (Herstellung siehe Beispiel 181)) in 2 ml 4-(2-Aminoethyl)-morpholin wird mit 10 ml Essigsäure versetzt. Das Reaktiongemisch wird 39 Stunden bei 80 C gerührt und dann am Rotavapor eingedampft. Aus dem Rückstand erhält man nach Reinigung mittels FC (Dichlormethan-Methanol = 10:1) die Titelverbindung als Rohprodukt. Durch Kristallisation aus Diethylether-Hexan erhält man die Titelverbindung: Smp. = 94-96 C, R_{f} (Dichlormethan-Methanol = 10:1) = 0.35; HPLC Rₜ = 17.42 Minuten; FAB-MS (M+H)+ = 664.

### Beispiel 184: 5(S)-Amino-4(S),8(R,S)-dihydroxy-2(S),7(S)-diisopropyl-8-[4-methoxy-3-(2-methoxymethoxyethyl)-phenyl]-octansäure(N-butyl)-amid

40 mg 5(S)-Azido-4(S),8(R,S)-dihydroxy-2(S),7(S)-diisopropyl-8-[4-methoxy-3-(2-methoxymethoxyethyl)-phenyl]-octansäure(N-butyl)-amid werden in 10 ml Methanol/Essigsäure (9:1) in Gegenwart von 20 mg Pd/C 10% bei Raumtemperatur und Normaldruck hydriert. Das Reaktionsgemisch wird filtriert und eingedampft. Der Rückstand wird mittels FC (2.4 g Kieselgel, Dichlormethan/Methanol = 9:1) gereinigt. Man erhält die Titelverbindung: R_{f} (Dichlormethan/Methanol = 9:1) = 0.17; HPLC Rₜ= 11.44 und 12.63 Minuten (Diastereomerengemisch); FAB-MS (M+H)⁺= 525.

### a) 5(S)-Azido-4(S),8(R,S)-dihydroxy-2(S),7(S)-diisopropyl-8-[4-methoxy-3-(2-methoxy-methoxyethyl)-phenyl]-octansäure(N-butyl)-amid

Eine Lösung von 400 mg 3(S)-Isopropyl-5(S)-{1(S)-azido-4(R,S)-hydroxy-3(S)-isopropyl-4-[4-methoxy-3-(2-methoxy-methoxyethyl)-phenyl]-butyl}-tetrahydrofuran-2-on (Beispiel 81d) und 3.8 ml n-Butylamin wird während 16 Stunden bei 50° C gerührt und anschließend eingedampft. Aus dem Rückstand erhält man nach Reinigung mittels FC (50 g Kieselgel, Hexan/Essigsäureethyl-ester = 1:1) die Titelverbindung: R_{f} (Hexan/Essigsäureethylester = 1:1) = 0.44; HPLC Rₜ= 16.13 und 17.03 Minuten (Diastereomerengemisch)

### Beispiel185: 5(S)-Amino-4(S),8(S oder R)-dihydroxy-2(S),7(S)-diisopropyl-8-[4-methoxy-3-(3-methoxypropyloxy)-phenyl]-octansäure-N-(2-carbamoyl-2,2-dimethyl-ethyl)-amid-hydrochlorid

60 mg 5(S)-Azido-4(S),8(S oder R)-dihydroxy-2(S),7(S)-diisopropyl-8-[4-methoxy-3-(3-methoxypropyloxy)-phenyl]-octansäure-N-(2-carbamoyl-2,2-dimethyl-ethyl)-amid und 6 ml Ethanolamin werden in 8 ml Ethanol in Gegenwart von 120 mg PdO/C 5% bei Raumtemperatur und Normaldruck während 2 Stunden hydriert. Das Reaktionsgemisch wird filtriert und eingedampft. Der Rückstand wird gelöst in 0.5 ml Dioxan und mit 23 µl 4N Salzsäure in Dioxan versetzt. Nach Lyophilisation erhält man die Titelverbindung; HPLC Rₜ= 10.74; FAB-MS (M+H)⁺= 568.

Die Ausgangsmaterialien werden wie folgt hergestellt:

### a) 5(S)-Azido-4(S),8(S oder R)-dihydroxy-2(S),7(S)-diisopropyl-8-[4-methoxy-3-(3-methoxypropyloxy)-phenyl]-octansäure-N-(2-carbamoyl-2,2-dimethyl-ethyl)-amid

150 mg 3(S)-Isopropyl-5(S)-{1(S)-azido-4(S oder R)-hydroxy-3(S)-isopropyl-4-[4-methoxy-3-(3-methoxypropyloxy)-phenyl]-butyl}-tetrahydrofuran-2-on (Beispiel 185b) Diastereomer B) und 109 mg 3-Amino-2,2-dimethyl-propionsäureamid werden in 3 ml Triethylamin mit 30 mg 2-Hydroxypyridin während 24 Stunden bei Rückflußtemperatur gerührt. Nach dem Abdampfen des Lösungsmittels wird der Rückstand in Diethylether mehrmals mit Wasser gewaschen. Die eingedampften organischen Extrakte werden mittels FC (10 g Kieselgel, Dichlormethan/Methanol = 95:5) gereinigt. Man erhält die Titelverbindung: R_{f} (Dichlormethan/Methanol = 95:5) = 0.22; HPLC Rₜ= 14.88 Minuten.

### b) 3(S)-Isopropyl-5(S)-{1(S)-azido-4(S oder R)-hydroxy-3(S)-isopropyl-4-[4-methoxy-3-(3-methoxy-propyloxy)-phenyl]-butyl}-tetrahydrofuran-2-on (A) und 3(S)-Isopropyl-5(S)-{1(S)-azido-4(S oder R)-hydroxy-3(S)-isopropyl-4-[4-methoxy-3-(3-methoxy-propyloxy)-phenyl]-butyl}-tetrahydrofuran-2-on (B)

Trennung von 0.5 g 3(S)-Isopropyl-5(S)-{1(S)-azido-4(R,S)-hydroxy-3(S)-isopropyl-4-[4-methoxy-3-(3-methoxypropyloxy)-phenyl]-butyl}-tetrahydrofuran-2-on (Diastereomerengemisch) mittels präparativer HPLC an Kromasil 7 C18 (EKA-Nobel, A.B. Schweden); Mobile Phase: A) Wasser B) Acetonitril Gradient: 20 - 80% B in 40 Minuten. Man erhält die beiden reinen Diastereomeren A und B (zuerst eluiert Isomer A). Nach Eindampfen der Eluatfraktionen wird der wässrige Rückstand mit Essigsäureethylester extrahiert. Die organischen Extrakte werden über Magnesiumsulfat getrocknet und eingeengt. Man erhält die Titelverbindungen: Diastereomer A) HPLC Rₜ= 18.53 Minuten und B) HPLC Rₜ= 19.49 Minuten.

### c) 3(S)-Isopropyl-5(S)-{1(S)-azido-4(R,S)-hydroxy-3(S)-isopropyl-4-[4-methoxy-3-(3-methoxy-propyloxy)-phenyl]-butyl}-tetrahydrofuran-2-on

Zu einer Mischung von 12.1 g 4-Methoxy-3-(3-methoxypropyloxy)-brombenzol und 9.7 ml 4-Methyl-morpholin in 75 ml Tetrahydrofuran werden bei -75°C 45.1 ml einer 1N n-Butyllithiumlösung (in Hexan) zugetropft. Das Reaktionsgemisch wird noch 20 Minuten bei -75° C weitergerührt und anschließend bei -75° C bis -60° C mit einer Suspension von Magnesiumbromid in Tetrahydrofuran (frisch hergestellt aus 1.6 g Magnesiumpulver, 5.7 ml 1,2-Dibromethan in 150 ml Tetrahydrofuran) versetzt. Das Reaktionsgemisch wird noch 30 Minuten weitergerührt und dann bei -75°C mit einer Lösung von 8.84 g 3(S)-Isopropyl-5(S)-[1-(S)-azido-3(S)-isopropyl-4-oxobutyl]-tetrahydrofuran -2-on in 75 ml Tetrahydrofuran versetzt. Das Reaktionsgemisch wird noch 15 Minuten bei -75°C nachgerührt und dann mit 70 ml gesättigter Ammoniumchloridlösung versetzt. Anschließend wird das Reaktionsgemisch auf 180 ml gesättigte Kochsalzlösung : Wasser (1:1) gegossen und mit Essigsäureethylester (2x 360 ml) extrahiert. Die organischen Phasen werden über Magnesiumsulfat getrocknet und eingedampft. Durch Reinigen des Rückstandes mittels FC (240 g Kieselgel, Essigsäureethylester/Hexan = 1:2) erhält man die Titelverbindung: R_{f} (Essigsäureethylester/Hexan = 1:2) = 0.16; HPLC Rₜ= 18.53 und 19.49 Minuten (Diastereomerengemisch).

### d) 4-Methoxy-3-(3-methoxypropyloxy)-brombenzol

Zu einer Lösung von 64.6 g 5-Brom-2-methoxyphenol in 350 ml Acetonitril werden 66.0 g Kaliumcarbonat und 3-Methoxy-1-brompropan bei Raumtemperatur zugegeben. Das Reaktionsgemisch wird 14 Stunden am Rückfluß gerührt. Nach dem Abdampfen des Lösungsmittels wird der Rückstand mit 1200 ml Eis-Wasser versetzt und mit Ether extrahiert. Die organischen Extrakte werden mit gesättiger Kochsalzlösung gewaschen, über Magnesiumsulfat getrocknet und eingedampft. Nach Destillation im Hochvakuum erhält man die Titelverbindung: R_{f} (Hexan/Essigsäureethylester = 4:1) = 0.33; Kp = 126-129° C / 1.4 mbar; HPLC Rₜ= 16.38 Minuten; MS (M⁺) = 274, 276.

### Beispiel 186: 5(S)-Amino-4(S),8(R oder S)-dihydroxy-2(S),7(S)-diisopropyl-8-[4-methoxy-3-(3-methoxypropyloxy)-phenyl]-octansäure-N-(2-carbamoyl-2,2-dimethyl-ethyl)-amid-hydrochlorid

Analog Beispiel 185 erhält man die Titelverbindung ausgehend von 5(S)-Azido-4(S),8(R oder S)-dihydroxy-2(S),7(S)-diisopropyl-8-[4-methoxy-3-(3-methoxypropyloxy)-phenyl]-octansäure-N-(2-carbamoyl-2,2-dimethyl-ethyl)-amid : HPLC Rₜ= 10.68; FAB-MS (M+H)⁺= 568.

Das Ausgangsmaterial wird analog Beispiel 185a) aus 3(S)-Isopropyl-5(S)-{1(S)-azido-4(R oder S)-hydroxy-3(S)-isopropyl-4-[4-methoxy-3-(3-methoxy-propyloxy)-phenyl]-butyl}-tetrahydrofuran-2-on (Beispiel 185 b) Diastereomer A) hergestellt.

### Beispiel 187: Gelatine-Lösung:

Eine sterilfiltrierte wässrige Lösung mit 20 % Cyclodextrinen als Lösungsvermittler von einer der in den vorausgehenden Beispielen genannten Verbindungen der Formel I als Wirkstoff wird unter Erwärmen mit einer sterilen Gelatinelösung, welche als Konservierungsmittel Phenol enthält, unter aseptischen Bedingungen so vermischt, dass 1.0 ml Lösung die folgende Zusammensetzung hat:

| | |
|---|---|
| Wirkstoff | 3 mg |
| Gelatine | 150.0 mg |
| Phenol dest. Wasser mit 20 % Cyclodextrinen | 4.7 mg |
| als Lösungsvermittler | 1.0 ml |

### Beispiel 188: Sterile Trockensubstanz zur Injektion:

Man löst 5 mg einer der in den vorausgehenden Beispielen genannten Verbindungen der Formel I als Wirkstoff in 1 ml einer wässrigen Lösung mit 20 mg Mannit und 20 % Cyclodextrinen als Lösungsvermittler. Die Lösung wird sterilfiltriert und unter aseptischen Bedingungen in eine 2 ml-Ampulle gefüllt, tiefgekühlt und lyophilisiert. Vor dem Gebrauch wird das Lyophilisat in 1 ml destilliertem Wasser oder 1 ml physiologischer Kochsalzlösung gelöst. Die Lösung wird intramuskulär oder intravenös angewendet. Diese Formulierung kann auch in Doppelkammerspritzampullen abgefüllt werden.

### Beispiel 189: Nasenspray:

In einer Mischung von 3.5 ml "Myglyol 812" und 0.08 g Benzylalkohol werden 500 mg fein gemahlenes (<5.0 µm) Pulver einer der in den vorausgehenden Beispielen genannten Verbindungen der Formel I als Wirkstoff suspendiert. Diese Suspension wird in einen Behälter mit Dosierventil eingefüllt. Es werden 5.0 g "Freon 12" unter Druck durch das Ventil in einen Behälter abgefüllt. Durch Schütteln wird das "Freon" in der Myglyol-Benzyl-alkoholmischung gelöst. Dieser Spraybehälter enthält ca. 100 Einzeldosen, die einzeln appliziert werden können.

### Beispiel 190: Lacktabletten

Für die Herstellung von 10 000 Tabletten, enthaltend je 100 mg Wirkstoff werden folgende Bestandteile verarbeitet:

| | |
|---|---|
| Wirkstoff | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | quantum satis |

Ein Gemisch von einer der in den vorausgehenden Beispielen genannten Verbindungen der Formel I als Wirkstoff, 50 g Maisstärke und der kolloidalen Kieselsäure wird mit Stärkekleister aus 250 g Maisstärke und 2.2 kg entmineralisiertem Wasser zu einer feuchten Masse verarbeitet. Diese wird durch ein Sieb von 3 mm Maschenweite getrieben und bei 45 ° während 30 Minuten. im Wirbelschichttrockner getrocknet. Das getrocknete Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

## Patentansprüche

1. δ-Amino-γ-hydroxy-ω-aryl-alkansäureamide der Formel I 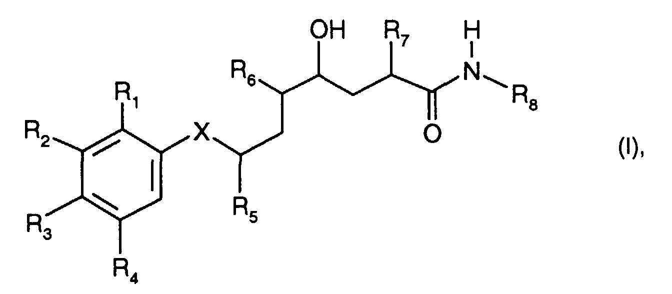 worin
R₁ Wasserstoff, Hydroxy, C₁-C₇-Alkoxy, 3- bis 8-gliedriges Cycloalkoxy, C₁-C₄-Alkoxy-C₂-C₄-alkoxy oder Carboxy-C₁-C₄-alkoxy, C₁-C₄-Alkoxycarbonyl-C₁-C₄-alkoxy, Carbamoyl-C₁-C₄-alkoxy, N-C₁-C₇-Alkylcarbamoyl-C₁-C₄-alkoxy oder N,N-Di-C₁-C₄-alkylcarbamoyl-C₁-C₄-alkoxy bedeutet,
R₂ Wasserstoff, C₁-C₇-Alkyl, 3- bis 8-gliedriges Cycloalkyl, C₁-C₄-Alkoxy-C₁-C₄-alkyl, C₁-C₄-Alkoxy-C₁-C₄-alkoxy-C₁-C₄-alkyl, 3- bis 8-gliedriges Cycloalkoxy-C₁-C₄-alkyl, Hydroxy, C₁-C₇-Alkanoyloxy-C₂-C₄-alkyl, Hydroxy-C₂-C₇-alkoxy, Halogen-C₂-C₇-(hydroxy)alkoxy, C₁-C₇-Alkansulfonyl-C₁-C₄-(hydroxy)alkoxy, Amino-C₁-C₄-alkyl, C₁-C₄-Alkylamino-C₁-C₄-alkyl, N,N-Di-C₁-C₄-alkylamino-C₁-C₄-alkyl, N-C₁-C₄-Alkanoylamino-C₁-C₄-alkyl, C₁-C₇-Alkoxycarbonylamino-C₂-C₇-alkyl, gegebenenfalls partiell hydriertes Pyridyl- oder N-Oxidopyridyl-C₁-C₄-alkyl, Amino-C₁-C₄-alkoxy, C₁-C₄-Alkylamino-C₁-C₄-alkoxy, N,N-Di-C₁-C₄-alkylamino-C₁-C₄-alkoxy, C₁-C₄-Alkanoylamino-C₁-C₄-alkoxy, C₁-C₇-Alkoxycarbonylamino-C₂-C₇-alkoxy, die Alkanoylgruppe in höherer als der α-Stellung tragendes C₁-C₇-Alkanoyl-C₂-C₄-alkoxy, C₁-C₇-Alkoxy, 3- bis 8-gliedriges Cycloalkoxy, C₂-C₇-Alkenyloxy, 3- bis 8-gliedriges Cycloalkoxy-C₁-C₄-alkoxy, C₁-C₄-Alkoxy-C₂-C₄-alkoxy, C₁-C₄-Alkoxy-C₂-C₄-alkenyl, C₂-C₇-Alkenyloxy-C₁-C₄-alkoxy, C₁-C₄-Alkoxy-C₂-C₄-alkenyloxy, C₂-C₇-Alkenyloxy-C₁-C₄-alkyl, C₁-C₄-Alkylthio-C₁-C₄-alkoxy, C₁-C₇-Alkansulfonyl-C₁-C₄-alkoxy, C₁-C₄-Alkylthio-C₁-C₄-(hydroxy)alkoxy, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl- oder Naphthyl-C₁-C₄-alkoxy, gegebenenfalls partiell hydriertes Pyridyl- oder N-Oxidopyridyl-C₁-C₄-alkoxy, Thiazolyl-C₁-C₄-alkoxy, gegebenenfalls N-oxidiertes Morpholino-C₁-C₄-alkoxy, Cyano-C₁-C₄-alkoxy, Carboxy-C₁-C₄-alkoxy, C₁-C₄-Alkoxycarbonyl-C₁-C₄-alkoxy, Carbamoyl-C₁-C₄-alkoxy, N-C₁-C₇-Alkylcarbamoyl-C₁-C₄-alkoxy oder N-Mono- oder N,N-Di-C₁-C₄-alkylcarbamoyl-C₁-C₄-alkoxy, Carboxy-C₁-C₄-alkyl, C₁-C₄-Alkoxycarbonyl-C₁-C₄-alkyl, Carbamoyl-C₁-C₇-alkyl oder N-Mono- oder N,N-Di-C₁-C₄-alkylcarbamoyl-C₁-C₄-alkyl bedeutet,
R₃ Wasserstoff, C₁-C₇-Alkyl, Polyhalogen-C₁-C₄-alkyl, C₁-C₄-Alkoxy-C₁-C₄-alkyl, 3- bis 8-gliedriges Cycloalkoxy-C₁-C₄-alkyl, Hydroxy-C₂-C₇-alkyl, C₁-C₄-Alkylthio-C₁-C₄-alkyl, C₁-C₇-Alkansulfonyl-C₁-C₄-alkyl, Thiazolylthio-C₁-C₄-alkyl, Thiazolinylthio-C₁-C₄-alkyl, Imidazolylthio-C₁-C₄-alkyl, gegebenenfalls N-oxidiertes Pyridylthio-C₁-C₄-alkyl, Pyrimidinylthio-C₁-C₄-alkyl, gegebenenfalls partiell hydriertes Pyridyl- oder N-Oxidopyridyl-C₁-C₄-alkyl, Amino-C₁-C₄-alkyl, C₁-C₄-Alkylamino-C₁-C₄-alkyl, N,N-Di-C₁-C₄-alkylamino-C₁-C₄-alkyl, N-C₁-C₄-Alkanoylamino-C₁-C₄-alkyl, C₁-C₄-Alkansulfonylamino-C₁-C₄-alkyl, Trifluor-C₁-C₇-alkansulfonylamino-C₁-C₄-alkyl, Pyrrolidino-C₁-C₄-alkyl, Piperidino-C₁-C₄-alkyl, Piperazino-C₁-C₄-alkyl, N'-C₁-C₄-Alkylpiperazino-C₁-C₄-alkyl, N'-C₂-C₇-Alkanoylpiperazino-C₁-C₄-alkyl, Morpholino-C₁-C₄-alkyl, Thiomorpholino-C₁-C₄-alkyl, S-Oxothiomorpholino-C₁-C₄-alkyl, S,S-Dioxothiomorpholino-C₁-C₄-alkyl, Cyano-C₁-C₄-alkyl, Carboxy-C₁-C₄-alkyl, C₁-C₄-Alkoxycarbonyl-C₁-C₄-alkyl, Carbamoyl-C₁-C₇-alkyl, N-Mono- oder N,N-Di-C₁-C₄-alkylcarbamoyl-C₁-C₄-alkyl, 3- bis 8-gliedriges Cycloalkyl, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl oder Naphthyl, Hydroxy, C₁-C₄-Alkoxy, 3- bis 8-gliedriges Cycloalkoxy, C₁-C₄-Alkoxy-C₂-C₄-alkoxy, 3- bis 8-gliedriges Cycloalkoxy-C₁-C₄-alkoxy, Hydroxy-C₂-C₇-alkoxy, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl-C₁-C₄-alkoxy oder Naphthyl-C₁-C₄-alkoxy, Polyhalogen-C₁-C₄-alkoxy, C₁-C₄-Alkylthio-C₁-C₄-alkoxy, C₁-C₇-Alkansulfonyl-C₁-C₄-alkoxy, gegebenenfalls partiell hydriertes Pyridyl- oder N-Oxidopyridyl-C₁-C₄-alkoxy, Thiazolyl-C₁-C₄-alkoxy, Morpholino-C₁-C₄-alkoxy, Thiazolylthio-C₁-C₄-alkoxy, Thiazolinylthio-C₁-C₄-alkoxy, Imidazolylthio-C₁-C₄-alkoxy, gegebenenfalls N-oxidiertes Pyridylthio-C₁-C₄-alkoxy, Pyrimidinylthio-C₁-C₄-alkoxy, Amino-C₁-C₄-alkoxy, C₁-C₄-Alkylamino-C₁-C₄-alkoxy, N,N-Di-C₁-C₄-alkylamino-C₁-C₄-alkoxy, C₁-C₇-Alkanoylamino-C₁-C₄-alkoxy, C₁-C₇-Alkansulfonylamino-C₁-C₄-alkoxy, Trifluor-C₁-C₇-alkansulfonyl-C₁-C₄-alkoxy, Pyrrolidino-C₂-C₄-alkoxy, Piperidino-C₁-C₄-alkoxy, Piperazino-C₁-C₄-alkyl, N'-C₁-C₄-Alkylpiperazino-C₁-C₄-alkyl, N'-C₂-C₇-Alkanoylpiperazino-C₁-C₄-alkyl, Morpholino-C₁-C₄-alkyl, Thiomorpholino-C₁-C₄-alkyl, S-Oxothiomorpholino-C₁-C₄-alkyl, S,S-Dioxothiomorpholino-C₁-C₄-alkyl, Cyano-C₁-C₄-alkoxy, Carboxy-C₁-C₄-alkoxy, C₁-C₄-Alkoxycarbonyl-C₁-C₄-alkoxy, Carbamoyl-C₁-C₇-alkoxy oder N-Mono- oder N,N-Di-C₁-C₄-alkylcarbamoyl-C₁-C₄-alkoxy bedeutet oder gemeinsam mit R₄ C₁-C₄-Alkylendioxy oder einen ankondensierten Benzo- oder Cyclohexenoring darstellt,
R₄ gemeinsam mit R₃ C₁-C₄-Alkylendioxy oder einen ankondensierten Benzo- oder Cyclohexenoring darstellt oder für Wasserstoff, C₁-C₇-Alkyl, Hydroxy, C₁-C₄-Alkoxy oder 3- bis 8-gliedriges Cycloalkoxy steht,
X Methylen oder Hydroxymethylen bedeutet,
R₅ für C₁-C₇-Alkyl oder 3- bis 8-gliedriges Cycloalkyl steht,
R₆ Amino, C₁-C₄-Alkylamino, Di-C₁-C₄-alkylamino oder N-C₁-C₇-Alkanoylamino bedeutet,
R₇ C₁-C₇-Alkyl, C₂-C₇-Alkenyl, 3- bis 8-gliedriges Cycloalkyl oder unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl- oder Naphthyl-C₁-C₄-alkyl bedeutet und
R₈ C₁-C₇-Alkyl, Cycloalkyl, Hydroxy-C₂-C₇-alkyl, C₁-C₇-Alkanoyloxy-C₂-C₄-alkyl, C₁-C₄-Alkoxy-C₁-C₄-alkyl, C₂-C₇-Alkenyloxy-C₁-C₄-alkyl, Amino-C₁-C₄-alkyl, N-C₁-C₄-alkylamino-C₁-C₄-alkyl, N,N-Di-C₁-C₄-alkylamino-C₁-C₄-alkyl, N-C₁-C₄-Alkanoylamino-C₁-C₄-alkyl, C₁-C₇-Alkansulfonylamino-C₁-C₄-alkyl, Trifluor-C₁-C₇-alkansulfonylamino-C₁-C₄-alkyl, Pyrrolidino-C₁-C₄-alkyl, Piperidino-C₁-C₄-alkyl, Hydroxypipendino-C₁-C₄-alkyl, C₁-C₄-Alkoxypiperidino-C₁-C₄-alkyl, Piperazino-C₁-C₄-alkyl, N'-C₁-C₄-Alkylpiperazino-C₁-C₄-alkyl, N'-C₂-C₇-Alkanoylpiperazino-C₁-C₄-alkyl, Moroholino-C₁-C₄-alkyl, Dimethylmorpholino-C₁-C₄-alkyl, Morpholinocarbonyl-C₁-C₄-alkyl, Thiomorpholino-C₁-C₄-alkyl, S,S-Dioxothiomoroholino-C₁-C₄-alkyl, Carboxy-C₁-C₄-alkyl, C₁-C₄-Alkoxycarbonyl-C₁-C₄-alkyl, Carbamoyl-C₁-C₇-alkyl, N-Mono- oder N,N-Di-C₁-C₄-alkylcarbamoyl-C₁-C₄-alkyl, Dicarboxy-C₁-C₄-alkyl, Di-C₁-C₄-alkoxycarbonyl-C₁-C₄-alkyl, Dicarbamoyl-C₁-C₄-alkyl, Di-(N-Mono- oder N,N-Di-C₁-C₄-alkylcarbamoyl-C₁-C₄-alkyl, Carboxy-C₁-C₇-(hydroxy)alkyl, C₁-C₄-Alkoxycarbonyl-C₁-C₇-(hydroxy)alkyl oder Carbamoyl-C₁-C₇-(hydroxy)alkyl, jeweils 5- oder 6-gliedriges Carboxycycloalkyl-C₁-C₄-alkyl, C₁-C₄-Alkoxycarbonylcycloalkyl-C₁-C₄-alkyl, Carbamoylcycloalkyl-C₁-C₄-alkyl oder N-Mono- oder N,N-Di-C₁-C₄-alkylcarbamoylcycloalkyl-C₁-C₄-alkyl, Cyano-C₁-C₄-alkyl, C₁-C₇-Alkansulfonyl-C₁-C₄-alkyl, Thiocarbamoyl-C₁-C₄-alkyl, C₁-C₄-Alkylthiocarbamoyl-C₁-C₄-alkyl, Di-C₁-C₄-alkylthiocarbamamoyl-C₁-C₄-alkyl, Sulfamoyl-C₁-C₄-alkyl, N-C₁-C₇-Alkylsulfamoyl-C₁-C₄-alkyl, N,N-Di-C₁-C₄-alkylsulfamoyl-C₁-C₄-alkyl, einen über ein C-Atom gebundenen, gegebenenfalls partiell hydrierten, oxosubstituierten und/oder benzokondensierten 5-gliedrigen Aza-, Diaza-, Triaza-, Oxadiaza- oder Tetra-aza-arylrest oder 6-gliedrigen Aza- oder Diaza-arylrest

2. Verbindungen gemäss Anspruch 1 der Formel I, worin
R₁ Wasserstoff bedeutet,
R₂ C₁-C₇-Alkyl, C₁-C₄-Alkoxy-C₁-C₄-alkyl, C₁-C₄-Alkoxy-C₁-C₄-alkoxy, C₁-C₄-Alkoxy-C₁-C₄-alkoxy-C₁-C₄-alkyl, unsubstituiertes oder durch C₁-C₄-Alkyl, C₁-C₄-Alkoxy, Hydroxy, Halogen, Nitro und/oder Amino substituiertes Phenyl-C₁-C₄-alkoxy, Pyridyl- oder N-Oxidopyridyl-C₁-C₄-alkoxy, C₁-C₄-Alkylthio-C₁-C₄-alkoxy, C₁-C₇-Alkansulfonyl-C₁-C₄-alkoxy, C₁-C₇-Alkanoyl-C₂-C₄-alkoxy, Cyano-C₁-C₄-alkoxy, Carboxy-C₁-C₄-alkoxy, C₁-C₄-Alkoxycarbonyl-C₁-C₄-alkoxy, Carbamoyl-C₁-C₇-alkoxy oder N-Mono- oder N,N-Di-C₁-C₄-alkylcarbamoyl-C₁-C₄-alkoxy darstellt,
R₃ Wasserstoff, C₁-C₇-Alkyl, Hydroxy, C₁-C₄-Alkoxy oder Polyhalogen-C₁-C₄-alkoxy bedeutet oder gemeinsam mit R₄ C₁-C₄-Alkylendioxy darstellt,
R₄ Wasserstoff ist oder gemeinsam mit R₃ C₁-C₄-Alkylendioxy darstellt,
X Methylen oder Hydroxymethylen bedeutet,
R₅ für C₁-C₇-Alkyl oder 3- bis 8-gliedriges Cycloalkyl steht,
R₆ Amino, C₁-C₄-Alkylamino, Di-C₁-C₄-alkylamino oder N-C₁-C₇-Alkanoylamino bedeutet,
R₇ C₁-C₇-Alkyl bedeutet und
R₈ C₁-C₇-Alkyl, Hydroxy-C₂-C₇-alkyl, C₁-C₇-Alkanoyloxy-C₂-C₄-alkyl, C₁-C₄-Alkoxy-C₁-C₄-alkyl, C₂-C₇-Alkenyloxy-C₁-C₄-alkyl, Amino-C₁-C₄-alkyl, N-C₁-C₄-Alkanoylamino-C₁-C₄-alkyl, N-C₁-C₄-alkylamino-C₁-C₄-alkyl, N,N-Di-C₁-C₄-alkylamino-C₁-C₄-alkyl,, Piperidino-C₁-C₄-alkyl, Hydroxypiperidino-C₁-C₄-alkyl, C₁-C₄-Alkoxypiperidino-C₁-C₄-alkyl, Morpholino-C₁-C₄-alkyl, Dimethylmorpholino-C₁-C₄-alkyl, Thiomorpholino-C₁-C₄-alkyl, S,S-Dioxothiomorpholino-C₁-C₄-alkyl, Carboxy-C₁-C₄-alkyl, C₁-C₄-Alkoxycarbonyl-C₁-C₄-alkyl, Carbamoyl-C₁-C₇-alkyl, N-Mono- oder N,N-Di-C₁-C₄-alkylcarbamoyl-C₁-C₄-alkyl, Carboxy-C₁-C₇-(hydroxy)alkyl, C₁-C₄-Alkoxycarbonyl-C₁-C₇-(hydroxy)alkyl oder Carbamoyl-C₁-C₇-(hydroxy)alkyl, 5- oder 6-gliedriges Carboxycycloalkyl-C₁-C₄-alkyl, 5- oder 6-gliedriges C₁-C₄-Alkoxycarbonylcycloalkyl-C₁-C₄-alkyl, 5- oder 6-gliedriges Carbamoylcycloalkyl-C₁-C₄-alkyl, 5- oder 6-gliedriges N-Mono- oder N,N-Di-C₁-C₄-alkylcarbamoylcycloalkyl-C₁-C₄-alkyl, Cyano-C₁-C₄-alkyl, C₁-C₇-Alkansulfonyl-C₁-C₄-alkyl, Sulfamoyl-C₁-C₄-alkyl, Imidazolyl-C₁-C₄-alkyl, Oxopyrrolidinyl-C₁-C₄-alkyl, Benzimidazolyl-C₁-C₄-alkyl, Oxadiazolyl-C₁-C₄-alkyl, Pyridyl-C₁-C₄-alkyl, Oxopiperidinyl-C₁-C₄-alkyl oder Chinolinyl-C₁-C₄-alkyl, Piperidin-4-yl-C₁-C₄-alkyl oder 1-C₁-C₇-Alkanoylpiperidin-4-yl-C₁-C₄-alkyl bedeutet,
und ihre Salze.

3. Verbindungen gemäss Anspruch 1 der Formel I, worin
R₁ und R₄ Wasserstoff bedeuten, R₂ für C₁-C₄-Alkoxy-C₁-C₄-alkoxy oder C₁-C₄-Alkoxy-C₁-C₄-alkyl steht,
R₃ C₁-C₄-Alkyl oder C₁-C₄-Alkoxy darstellt,
R₆ Amino ist,
X Methylen ist,
R₅ und R₇ verzweigtes C₁-C₄-Alkyl bedeuten und
R₈ Carbamoyl-C₁-C₄-alkyl, N-C₁-C₄-Alkylcarbamoyl-C₁-C₄-alkyl, N,N-Di-C₁-C₄-alkylcarbamoyl-C₁-C₄-alkyl, Morpholino-C₁-C₄-alkyl, Thiomorpholino-C₁-C₄-alkyl, 4-(1-C₁-C₄-Alkanoylpiperidyl)-C₁-C₄-alkyl oder 2-Oxopyrrolidinyl-C₁-C₄-alkyl bedeutet, und ihre Salze.

4. Verbindungen gemäss einem der Ansprüche 1 bis 3 der Formel I, worin mindestens ein asymmetrisches C-Atome der Hauptkette die in der Formel Ia 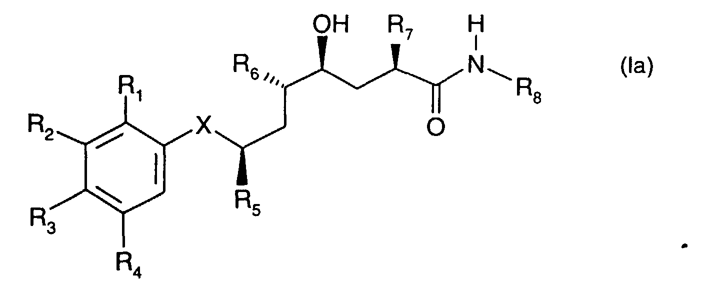 gezeigte Stereotaxie aufweisen, wobei die Variablen jeweils die in den Ansprüchen 1 bis 3 angegebenen Bedeutungen haben, und ihre pharmazeutisch verwendbaren Salze.

5. Verbindungen gemäss einem der Ansprüche 1 bis 4 der Formeln I und Ia, worin X Methylen bedeutet, und ihre pharmazeutisch verwendbaren Salze.

6. 5(S)-Amino-4(S)-hydroxy-2(S),7(S)-diisopropyl-8-[4-methoxy-3-(3-methoxypropyloxy)-phenyl]-octansäure-N-(2-carbamoyl-2,2-dimethyl-ethyl)-amid gemäss Anspruch 1 oder ein Salz davon.

7. 5(S)-Amino-4(S),8(R,S)-dihydroxy-2(S),7(S)-diisopropyl-8-[4-methoxy-3-(2-methoxymethoxyethyl)-phenyl]-octansäure(N-butyl)-amid;
5(S)-Amino-4(S),8(R)-dihydroxy-2(S),7(S)-diisopropyl-8-[4-methoxy-3-(3-methoxypropyloxy)-phenyl]-octansäure-N-(2-carbamoyl-2,2-dimethyl-ethyl)-amid;
5(S)-Amino-4(S)-hydroxy-2(S),7(S)-diisopropyl-8-[4-methoxy-3-(3-methoxypropyloxy)-phenyl]-octansäure-N-(2-carbamoyl-2,2-dimethyl-ethyl)-amid oder
5(S)-Amino-2(S),7(S)-diisopropyl-4(S)-hydroxy-8-[4-tertiärbutyl-3-(3-methoxypropoxy)-phenyl]-octansäure[N-2-(morpholin-4-yl)-ethyl]-amid gemäss Anspruch 1 oder jeweils ein Salz davon.

8. 2(R,S)-Methyl-4(S)-hydroxy-5(S)-amino-7(S)-isopropyl-8-(p-tertiärbutyl-phenyl)-octansäure(N-butyl)amid;
2(R,S)-Methyl-4(S)-hydroxy-5(S)-amino-7(S)-ethyl-8-(p-tertiärbutyl-phenyl)-octansäure-(N-butyl)amid;
2(R,S)-Methyl-4(S)-hydroxy-5(S)-amino-7(S)-methyl-8-biphenyl-octansäure-(N-butyl)amid;
2(R,S)-Methyl-4(S)-hydroxy-5(S)-amino-7(S)-ethyl-8-(4-propyloxymethyl-naphth-2-yl)-octansäure(N-butyl)amid;
2(R)-Methyl-4(S)-hydroxy-5(S)-amino-7(S)-isopropyl-8-(3-hydroxy-4-tertiärbutyl-phenyl)-octansäure(N-butyl)amid;
2(R,S)-Methyl-4(S)-hydroxy-5(S)-amino-7(S)-isopropyl-8-(2-hydroxy-4-tertiärbutyl-phenyl)-octansäure(N-butyl)amid;
2(R)-Methyl-4(S)-hydroxy-5(S)-amino-7(S)-isopropyl-8-(3-ethoxycarbonylmethoxy-4-tertiärbutyl-phenyl)-octansäure(N-butyl)amid;
2(R)-Methyl-4(S)-hydroxy-5(S)-amino-7(S)-isopropyl-8-(3-allyloxy 4-tertiärbutyl-phenyl)-octansäure(N-butyl)amid;
2(R,S)-Methyl-4(S)-hydroxy-5(S)-amino-7(S)-isopropyl-8-(3-methoxycarbonylallyloxy-4-tertiärbutyl-phenyl)-octansäure(N-butyl)amid;
2(R,S)-Methyl-4(S)-hydroxy-5(S)-amino-7(S)-isopropyl-8-(3-methoxycarbonylmethoxy-4-tertiärbutyl-phenyl)-octansäure(N-butyl)amid;
2(R,S)-Methyl-4(S)-hydroxy-5(S)-amino-7(S)-isopropyl-8-(3-carbamoyl-methoxy-4-tertiärbutyl-phenyl)-octansäure(N-butyl)amid;
2(R,S)-Methyl-4(S)-hydroxy-5(S)-amino-7(S)-isopropyl-8-[3-(pyrid-2-yl-methoxy)-4-tertiärbutyl-phenyl]-octansäure(N-butyl)amid;
2(R,S)-Methyl-4(S)-hydroxy-5(S)-amino-7(S)-isopropyl-8-[3-(pyrid-4-yl-methoxy)-4-tertiärbutyl-phenyl]-octansäure(N-butyl)amid;
2(R,S)-Methyl-4(S)-hydroxy-5(S)-amino-7(S)-isopropyl-8-[3-(N-oxido-pyrid-2-yl-methoxy)-4-tertiärbutyl-phenyl]-octansäure(N-butyl)amid;
2(R,S)-Methyl-4(S)-hydroxy-5(S)-amino-7(S)-isopropyl-8-[3-(2-ethoxycarbonylallyloxy)-4-tertiärbutyl-phenyl]-octansäure(N-butyl)amid;
2(R,S)-Methyl-4(S)-hydroxy-5(S)-amino-7(S)-isopropyl-8-[3-(2-ethoxycarbonylpropyloxy)-4-tertiärbutyl-phenyl]-octansäure(N-butyl)amid;
2(R,S)-Methyl-4(S)-hydroxy-5(S)-amino-7(S)-isopropyl-8-[3-(methylthio-methoxy)-4-tertiärbutyl-phenyl]-octansäure(N-butyl)amid;
2(R,S)-Methyl-4(S)-hydroxy-5(S)-amino-7(S)-isopropyl-8-[3-(methylsulfonyl-methoxy)-4-tertiärbutyl-phenyl]-octansäure(N-butyl)amid;
2(R,S)-Methyl-4(S)-hydroxy-5(S)-amino-7(S)-isopropyl-8-[3-(carboxy-methoxy)-4-tertiärbutyl-phenyl]-octansäure-(N-butyl)amid;
2(R,S)-Methyl-4(S)-hydroxy-5(S)-amino-7(S)-isopropyl-8-[3-(3,3-dimethyl-2-oxo-butyloxy)-4-tertiärbutyl-phenyl]-octansäure(N-butyl)amid;
2(R,S)-Methyl-4(S)-hydroxy-5(S)-amino-7(S)-isopropyl-8-[3-(2-nitrobenzyloxy)-4-tertiärbutyl-phenyl]-octansäure(N-butyl)amid;
2(R,S)-Methyl-4(S)-hydroxy-5(S)-amino-7(S)-isopropyl-8-[3-(2-aminobenzyloxy)-4-tertiärbutyl-phenyl]-octansäure(N-butyl)amid;
2(R,S)-Methyl-4(S)-hydroxy-5(S)-amino-7(S)-isopropyl-8-[3-(3-chlor-2(R,S)-hydroxypropyloxy)-4-tertiärbutyl-phenyl]-octansäure(N-butyl)amid;
2(R,S)-Methyl-4(S)-hydroxy-5(S)-amino-7(S)-isopropyl-8-[3-(3-methylthio-2(S,R)-hydroxypropyloxy)-4-tertiärbutyl-phenyl]-octansäure(N-butyl)amid;
2(R,S)-Methyl-4(S)-hydroxy-5(S)-amino-7(S)-isopropyl-8-[3-(3-methylsulfonyl-2(S,R)-hydroxypropyloxy)-4-tertiärbutyl-phenyl]-octansäure(N-butyl)amid;
2(R)-Methyl-4(S)-hydroxy-5(S)-amino-7(S)-isopropyl-8-[3-(methylsulfonyl-methoxy)-4-tertiärbutyl-phenyl]-octansäure(N-3-morpholino-propyl)amid;
2(R)-Methyl-4(S)-hydroxy-5(S)-amino-7(S)-isopropyl-8-(3-methoxycarbonylmethoxy-phenyl)-octansäure(N-butyl)amid;
2(R)-Methyl-4(S)-hydroxy-5(S)-amino-7(S)-isopropyl-8-[3-(methoxycarbonylmethoxy)-4-methoxy-phenyl]-octansäure(N-butyl)amid;
2(R)-Methyl-4(S)-hydroxy-5(S)-amino-7(S)-isopropyl-8-[3-(N-methyl-carbamoylmethoxy)-4-methoxy-phenyl]-octansäure(N-butyl)amid;
2(R)-Methyl-4(S)-hydroxy-5(S)-amino-7(S)-isopropyl-8-[3-(3-methylsulfonyl-propyloxy)-4-methoxy-phenyl]-octansäure(N-butyl)amid;
2(R)-Methyl-4(S)-hydroxy-5(S)-amino-7(S)-isopropyl-8-[3-(methylsulfonyl-methoxy)-4-methoxy-phenyl]-octansäure(N-butyl)amid;
2(R)-Methyl-4(S)-hydroxy-5(S)-amino-7(S)-isopropyl-8-[3-(3-methoxy-propyloxy)-4-methoxy-phenyl]-octansäure-(N-butyl)amid;
2(R)-Methyl-4(S)-hydroxy-5(S)-amino-7(S)-isopropyl-8-[3-(2-methoxy-ethoxy)-4-methoxy-phenyl]-octansäure(N-butyl)amid;
2(R)-Methyl-4(S)-hydroxy-5(S)-amino-7(S)-isopropyl-8-[3-(3-hydroxy-propyloxy)-4-methoxy-phenyl]-octansäure(N-butyl)amid;
2(R)-Methyl-4(S)-hydroxy-5(S)-amino-7(S)-isopropyl-8-[3-(carbamoylmethoxy)-4-methoxy-phenyl]-octansäure(N-butyl)amid;
2(R)-Methyl-4(S)-hydroxy-5(S)-amino-7(S)-isopropyl-8-(3-cyanmethoxy-4-methoxy-phenyl)-octansäure(N-butyl)amid;
2(R)-Methyl-4(S)-hydroxy-5(S)-amino-7(S)-isopropyl-8-[3-(4-methoxy-butoxy)-4-methoxy-phenyl]-octansäure(N-butyl)amid;
2(R)-Methyl-4(S)-hydroxy-5(S)-amino-7(S)-isopropyl-8-[3-(2-ethoxy-ethoxy)-4-methoxy-phenyl]-octansäure(N-butyl)amid;
2(R)-Methyl-4(S)-hydroxy-5(S)-amino-7(S)-isopropyl-8-{3-[2-(2-methoxy-ethoxy)ethoxy]-4-methoxy-phenyl}-octansäure-(N-butyl)-amid;
2(R)-Methyl-4(S)-hydroxy-5(S)-amino-7(S)-isopropyl-8-(3-pentoxy-4-methoxy-phenyl)-octansäure(N-butyl)amid;
2(R)-Methyl-4(S)-hydroxy-5(S)-amino-7(S)-isopropyl-8-(3-benzyloxy-4-methoxy-phenyl)-octansäure(N-butyl)amid;
2(R)-Methyl-4(S)-hydroxy-5(S)-amino-7(S)-isopropyl-8-[3-(3-ethoxy-propyloxy)-4-methoxy-phenyl]-octansäure(N-butyl)amid;
2(R)-Methyl-4(S)-hydroxy-5(S)-amino-7(S)-isopropyl-8-[3-(pyrid-4-ylmethoxy)-4-methoxy-phenyl]-octansäure(N-butyl)amid;
2(R,S)-Methyl-4(S)-hydroxy-5(S)-amino-7(S)-isopropyl-8-(2-ethoxycarbonylmethoxy-4-tertiärbutyl-phenyl)-octansäure(N-butyl)amid;
2(R,S)-Methyl-4(S)-hydroxy-5(S)-amino-7(S)-isopropyl-8-(2-ethoxycarbonyl-4-tertiärbutyl-phenyl)-octansäure(N-butyl)amid;
2(R)-Methyl-4(S)-hydroxy-5(S)-amino-7(S)-isopropyl-8-[3-(3-methoxy-propyloxy)-4,5-ethylendioxy-phenyl]-octansäure(N-butyl)amid;
5(S)-Amino-4(S)-hydroxy-2(S),7(S)-diisopropyl-8-[4-(3-hydroxypropyloxy)-3-(3-methoxypropyloxy)-phenyl]-octansäure-[N-(2-carbamoyl-2,2-dimethyl-ethyl)]-amid;
5(S)-Amino-4(S)-hydroxy-2(S),7(S)-diisopropyl-8-[4-isopropyl-3-(3-methoxypropyloxy)-phenyl]-octansäure-[N-(2-carbamoyl-2,2-dimethyl-ethyl)]-amid;
5(S)-Amino-4(S)-hydroxy-2(S),7(S)-diisopropyl-8-[4-tertiärbutyl-3-(3-methoxy-propyloxy)-phenyl]-octansäure-[N-(2-carbamoyl-2,2-dimethyl-ethyl)]-amid;
5(S)-Amino-4(S)-hydroxy-2(S),7(S)-diisopropyl-8-[4-(3-methylsulfonyl-propyloxy)-3-(3-methoxy-propyloxy)-phenyl]-octansäure-(N-2-morpholinoethyl)amid;
5(S)-Amino-4(S)-hydroxy-2(S),7(S)-diisopropyl-8-[4-(3-methylsulfonyl-propyloxy)-3-(3-methoxy-propyloxy)-phenyl]-octansäure-[N-(2-carbamoyl-2,2-dimethyl-ethyl)]-amid;
5(S)-Amino-4(S)-hydroxy-2(S),7(S)-diisopropyl-8-[3,4-di-(3-hydroxypropyloxy)-phenyl]-octansäure-(N-2-morpholinoethyl)amid;
5(S)-Amino-4(S)-hydroxy-2(S),7(S)-diisopropyl-8-[3,4-di-(3-hydroxypropyloxy)-phenyl]-octansäure-[N-(2-carbamoyl-2,2-dimethyl-ethyl)]-amid;
5(S)-Amino-4(S)-hydroxy-2(S),7(S)-diisopropyl-8-[4-(3-N-methylcarbamoyl-propyl)-3-(3-methoxy-propyloxy)-phenyl]-octansäure-(N-2-morpholinoethyl)amid;
5(S)-Amino-4(S)-hydroxy-2(S),7(S)-diisopropyl-8-[4-(2-morpholinoethoxy)-3-(3-methoxy-propyloxy)-phenyl]-octansäure-[N-(2-carbamoyl-2,2-dimethyl-ethyl)]-amid;
5(S)-Amino-4(S)-hydroxy-2(S),7(S)-diisopropyl-8-[3-(3-methoxypropyloxy)-4,5-ethylendioxyphenyl]-octansäure-(N-2-morpholinoethyl)amid;
5(S)-Amino-4(S)-hydroxy-2(S),7(S)-diisopropyl-8-[3-(3-methoxypropyloxy)-4,5-ethylendioxy-phenyl]-octansäure-[N-(2-carbamoyl-2,2-dimethyl-ethyl)]-amid;
5(S)-Amino-4(S)-hydroxy-2(S),7(S)-diisopropyl-8-[3-(3-methoxy-propyloxy)-4,5-methylendioxy-phenyl]-octansäure-(N-2-moroholinoethyl)amid;
5(S)-Amino-4(S)-hydroxy-2(S),7(S)-diisopropyl-8-[3-(3-methoxypropyloxy)-4,5-methylendioxy-phenyl]-octansäure-[N-(2-carbamoyl-2,2-dimethyl-ethyl)]-amid;
5(S)-Amino-4(S)-hydroxy-2(S),7(S)-diisopropyl-8-[4-methoxy-3-(3-methoxypropyloxy)-phenyl]-octansäure-[N-(2-carbamoyl-2,2-ethylen-ethyl)]-amid;
5(S)-Amino-4(S)-hydroxy-2(S),7(S)-diisopropyl-8-[4-methoxy-3-(3-methoxy-propyloxy)-phenyl]-octansäure-[N-(3(S)-2-oxo-pyrrolidin-3-yl-methyl)]-amid;
5(S)-Amino-4(S)-hydroxy-7(S)-isopropyl-2(R)-methyl-8-[4-methoxy-3-(4-methoxy-but-2-enoxy)-phenyl]-octansäure-(N-butyl)-amid;
5(S)-Amino-4(S)-hydroxy-7(S)-isopropyl-2(R)-methyl-8-[4-hydroxy-3-(3-methoxypropyloxy)-phenyl]-octansäure-(N-butyl)amid;
5(S)-Amino-4(S)-hydroxy-7(S)-isopropyl-2(R)-methyl-8-[4-benzyloxy-3-(3-methoxypropyloxy)-phenyl]-octansäure-(N-butyl)amid;
5(S)-Amino-4(S)-hydroxy-7(S)-isopropyl-2(R)-methyl-8-[3,4-di-(3-methoxypropyloxy)-phenyl]-octansäure-(N-butyl)amid;
5(S)-Amino-4(S)-hydroxy-7(S)-isopropyl-2(R)-methyl-8-[4-(2,2,2-trifluorethoxy)-3-(3-methoxypropyloxy)-phenyl]-octansäure-(N-butyl)amid;
5(S)-Amino-4(S)-hydroxy-7(S)-isopropyl-2(R)-methyl-8-[4-(3-hydroxy-propyloxy)-3-(3-methoxypropyloxy)-phenyl]-octansäure-(N-butyl)amid;
5(S)-Amino-4(S)-hydroxy-7(S)-isopropyl-2(R)-methyl-8-[4-(2-amino-ethoxy)-3-(3-methoxy-propyloxy)-phenyl]-octansäure-(N-butyl)amid;
5(S)-Amino-4(S)-hydroxy-7(S)-isopropyl-2(R)-methyl-8-[4-(5-amino-pentyloxy)-3-(3-methoxypropyloxy)-phenyl]-octansäure-(N-butyl)amid;
5(S)-Amino-4(S)-hydroxy-7(S)-isopropyl-2(R)-methyl-8-[4-(4-amino-butyloxy)-3-(3-methoxypropyloxy)-phenyl]-octansäure-(N-butyl)amid;
5(S)-Amino-4(S)-hydroxy-7(S)-isopropyl-2(R)-methyl-8-[4-(4-N,N-dimethylamino-butyloxy)-3-(3-methoxypropyloxy)-phenyl]-octansäure-(N-butyl)amid;
5(S)-Amino-4(S)-hydroxy-7(S)-isopropyl-2(R)-methyl-8-{4-[4-N-(trifluormethansulfonylamino-butyloxy)-3-(3-methoxypropyloxy)-phenyl]}-octansäure-(N-butyl)amid;
5(S)-Amino-4(S)-hydroxy-7(S)-isopropyl-2(R)-methyl-8-[4-carboxymethoxy-3-(3-methoxypropyloxy)-phenyl]-octansäure-(N-butyl)amid;
5(S)-Amino-4(S)-hydroxy-7(S)-isopropyl-2(R)-methyl-8-[4-(3-ethoxycarbonyl-propyloxy)-3-(3-methoxy-propyloxy)-phenyl]-octansäure-(N-butyl)amid;
5(S)-Amino-4(S)-hydroxy-7(S)-isopropyl-2(R)-methyl-8-[4-(3-carboxy-propyloxy)-3-(3-methoxypropyloxy)-phenyl]-octansäure-(N-butyl)amid;
5(S)-Amino-4(S)-hydroxy-7(S)-isopropyl-2(R)-methyl-8-[4-(4-methoxycarbonylbutyloxy)-3-(3-methoxypropyloxy)-phenyl]-octansäure-(N-butyl)amid;
5(S)-Amino-4(S)-hydroxy-7(S)-isopropyl-2(R)-methyl-8-[4-(4-carboxy-butyloxy)-3-(3-methoxypropyloxy)-phenyl]-octansäure-(N-butyl)amid;
5(S)-Amino-4(S)-hydroxy-2(S),7(S)-diisopropyl-8-[4-methoxy-3-(3-methoxypropyloxy)-phenyl]-octansäure-(N-butyl)amid;
5(S)-Amino-4(S)-hydroxy-2(S),7(S)-diisopropyl-8-[4-methoxy-3-(2-methoxymethoxyethyl)-phenyl]-octansäure-(N-butyl)amid;
5(S)-Amino-4(S)-hydroxy-2(S),7(S)-diisopropyl-8-[4-(3-hydroxypropyloxy)-3-(3-methoxypropyloxy)-phenyl]-octansäure-N-(2-morpholinoethyl)amid;
5(S)-Amino-4(S)-hydroxy-7(S)-isopropyl-2(R)-methyl-8-[4-methoxy-3-(3-methoxypropyloxy)-phenyl]-octansäure-N-[2-(4-hydroxypiperidin-1-yl)ethyl]amiddihydrochlorid;
5(S)-Amino-4(S)-hydroxy-7(S)-isopropyl-2(R)-methyl-8-[4-methoxy-3-(3-methoxypropyloxy)-phenyl]-octansäure-N-[2-(trans-2.6-dimethyl-morpholino)ethyl]amid;
5(S)-Amino-4(S)-hydroxy-7(S)-isopropyl-2(R)-methyl-8-[4-methoxy-3-(3-methoxypropyloxy)-phenyl]-octansäure-N-[2-(cis-2.6-dimethyl-morpholino)ethyl]amid;
5(S)-Amino-4(S)-hydroxy-7(S)-isopropyl-2(R)-methyl-8-[4-methoxy-3-(3-methoxypropyloxy)-phenyl]-octansäure-N-(2-piperidinoethyl)amid;
5(S)-Amino-4(S)-hydroxy-7(S)-isopropyl-2(R)-methyl-8-[4-methoxy-3-(3-methoxypropyloxy)-phenyl]-octansäure-N-[2-(4-methoxypiperidino)-ethyl]-amid;
5(S)-Amino-4(S)-hydroxy-7(S)-isopropyl-2(R)-methyl-8-[4-methoxy-3-(3-methoxypropyloxy)-phenyl]-octansäure-N-(2-thiomorpholinoethyl)amid;
5(S)-Amino-4(S)-hydroxy-7(S)-isopropyl-2(R)-methyl-8-[4-methoxy-3-(3-methoxypropyloxy)-phenyl]-octansäure-[N-(3-hydroxypropyl)]amid;
5(S)-Amino-4(S)-hydroxy-7(S)-isopropyl-2(R)-methyl-8-[4-methoxy-3-(3-methoxypropyloxy)-phenyl]-octansäure-[N-(4-acetoxybutyl)]amid;
5(S)-Amino-4(S)-hydroxy-7(S)-isopropyl-2(R)-methyl-8-[4-methoxy-3-(3-methoxypropyloxy)-phenyl]-octansäure-[N-(3-cyanopropyl)]amid;
5(S)-Amino-4(S)-hydroxy-7(S)-isopropyl-2(R)-methyl-8-[4-methoxy-3-(3-methoxypropyloxy)-phenyl]-octansäure-[N-(3-methoxypropyl)]amid;
5(S)-Amino-4(S)-hydroxy-7(S)-isopropyl-2(R)-methyl-8-[4-methoxy-3-(3-methoxypropyloxy)-phenyl]-octansäure-[N-(2-acetylamino-ethyl)]amid;
5(S)-Amino-4(S)-hydroxy-7(S)-isopropyl-2(R)-methyl-8-[4-methoxy-3-(3-methoxypropyloxy)-phenyl]-octansäure-{N-[2-(2-pyridyl)-ethyl]}-amid;
5(S)-Amino-4(S)-hydroxy-7(S)-isopropyl-2(R)-methyl-8-[4-methoxy-3-(3-methoxypropyloxy)-phenyl]-octansäure-N-[2-(N'-oxomorpholino)ethyl]-amid;
5(S)-Amino-4(S)-hydroxy-7(S)-isopropyl-2(R)-methyl-8-[4-methoxy-3-(3-methoxypropyloxy)-phenyl]-octansäure-{N-[3-(tertiärbutylsulfonyl)-propyl]}-amid;
5(S)-Amino-4(S)-hydroxy-7(S)-isopropyl-2(R)-methyl-8-[4-methoxy-3-(3-methoxypropyloxy)-phenyl]-octansäure-{N-[3-(ethylsulfonyl)-propyl]}-amid;
5(S)-Amino-4(S)-hydroxy-7(S)-isopropyl-2(R)-methyl-8-[4-methoxy-3-(3-methoxypropyloxy)-phenyl]-octansäure-{N-[2-(ethylsulfonyl)-ethyl]}-amid;
5(S)-Amino-4(S)-hydroxy-7(S)-isopropyl-2(R)-methyl-8-[4-methoxy-3-(3-methoxypropyloxy)-phenyl]-octansäure-{N-[2-(N-butylsulfamoyl)-ethyl]}-amid;
5(S)-Amino-4(S)-hydroxy-7(S)-isopropyl-2(R)-methyl-8-[4-methoxy-3-(3-methoxypropyloxy)-phenyl]-octansäure-{N-[2-(N,N-dimethylsulfamoyl)-ethyl]}-amid;
5(S)-Amino-4(S)-hydroxy-7(S)-isopropyl-2(R)-methyl-8-[4-methoxy-3-(3-methoxypropyloxy)-phenyl]-octansäure-{N-[3-(1H-tetrazol-5-yl)-propyl]}-amid;
5(S)-Amino-4(S)-hydroxy-7(S)-isopropyl-2(R)-methyl-8-[4-methoxy-3-(3-methoxypropyloxy)-phenyl]-octansäure-{N-[3-(1H-imidazol-5-yl)-propyl]}-amid;
5(S)-Amino-4(S)-hydroxy-7(S)-isopropyl-2(R)-methyl-8-[4-methoxy-3-(3-methoxypropyloxy)-phenyl]-octansäure-{N-[3-(3-methyl-1,2,4-oxadiazol-5-yl)-propyl]}-amid;
5(S)-Amino-4(S)-hydroxy-7(S)-isopropyl-2(R)-methyl-8-[4-methoxy-3-(3-methoxypropyloxy)-phenyl]-octansäure-[N-(3-aminopropyl)]-amid;
5(S)-Amino-4(S)-hydroxy-7(S)-isopropyl-2(R)-methyl-8-[4-methoxy-3-(3-methoxypropyloxy)-phenyl]-octansäure-[N-[2-dimethylamino-ethyl)]-amid;
5(S)-Amino-4(S)-hydroxy-7(S)-isopropyl-2(R)-methyl-8-[4-methoxy-3-(3-methoxypropyloxy)-phenyl]-octansäure-N-(2-morpholinoethyl)amid;
5(S)-Amino-4(S)-hydroxy-7(S)-isopropyl-2(R)-methyl-8-[4-methoxy-3-(3-methoxypropyloxy)-phenyl]-octansäure-N-(3-morpholinopropyl)amid;
5(S)-Amino-4(S)-hydroxy-7(S)-isopropyl-2(R)-methyl-8-[4-methoxy-3-(3-methoxypropyloxy)-phenyl]-octansäure-N-[2-(1,1-dioxothiomorpholino)ethyl]amid;
5(S)-Amino-4(S)-hydroxy-7(S)-isopropyl-2(R)-methyl-8-[4-methoxy-3-(3-methoxypropyloxy)-phenyl]-octansäure-N-(2-ethoxycarbonylethyl)amid;
5(S)-Amino-4(S)-hydroxy-7(S)-isopropyl-2(R)-methyl-8-[4-methoxy-3-(3-methoxypropyloxy)-phenyl]-octansäure-[N-(2-carboxy-ethyl)]-amid;
5(S)-Amino-4(S)-hydroxy-7(S)-isopropyl-2(R)-methyl-8-[4-methoxy-3-(3-methoxypropyloxy)-phenyl]-octansäure-[N-(3-methoxycarbonyl-ethyl)]-amid;
5(S)-Amino-4(S)-hydroxy-7(S)-isopropyl-2(R)-methyl-8-[4-methoxy-3-(3-methoxypropyloxy)-phenyl]-octansäure-[N-(3-carboxypropyl)]-amid;
5(S)-Amino-4(S)-hydroxy-7(S)-isopropyl-2(R)-methyl-8-[4-methoxy-3-(3-methoxypropyloxy)-phenyl]-octansäure-[N-(2-carbamoylethyl)]-amid;
5(S)-Amino-4(S)-hydroxy-7(S)-isopropyl-2(R)-methyl-8-[4-methoxy-3-(3-methoxypropyloxy)-phenyl]-octansäure-[N-(4-carbamoylbutyl)]-amid;
5(S)-Amino-4(S)-hydroxy-7(S)-isopropyl-2(R)-methyl-8-[4-methoxy-3-(3-methoxypropyloxy)-phenyl]-octansäure-N-{3-[N-(2-methoxyethyl)carbamoyl]propyl}amid;
5(S)-Amino-4(S)-hydroxy-7(S)-isopropyl-2(R)-methyl-8-[4-methoxy-3-(3-methoxypropyloxy)-phenyl]-octansäure-N-(4-morpholino-4-oxo-butyl)amid;
5(S)-Amino-4(S)-hydroxy-7(S)-isopropyl-2(R)-methyl-8-[4-methoxy-3-(3-methoxypropyloxy)-phenyl]-octansäure-[N-(2-carbamoyl-2,2-dimethyl-ethyl)]-amid;
5(S)-Amino-4(S)-hydroxy-2(S),7(S)-diisopropyl-8-[4-methoxy-3-(3-methoxypropyloxy)-phenyl]-octansäure-N-(1,1-dimethyl-2-morpholino-ethyl)amid;
5(S)-Amino-4(S)-hydroxy-2(S),7(S)-diisopropyl-8-[4-methoxy-3-(3-methoxypropyloxy)-phenyl]-octansäure-N-[1(R,S)-methyl-2-morpholino-ethyl]amid;
5(S)-Amino-4(S)-hydroxy-2(S),7(S)-diisopropyl-8-[4-methoxy-3-(3-methoxypropyloxy)-phenyl]-octansäure-[N-(1-carbamoyl-1-methyl-ethyl)]-amid;
5(S)-Amino-4(S)-hydroxy-2(S),7(S)-diisopropyl-8-[4-methoxy-3-(3-methoxypropyloxy)-phenyl]-octansäure-[N-(l-carbamoyl-methyl)]-amid;
5(S)-Amino-4(S)-hydroxy-2(S),7(S)-diisopropyl-8-[4-methoxy-3-(3-methoxypropyloxy)-phenyl]-octansäure-[N-(2-carbamoyl-ethyl)]-amid;
5(S)-Amino-4(S)-hydroxy-2(S),7(S)-diisopropyl-8-[4-methoxy-3-(3-methoxypropyloxy)-phenyl]-octansäure-N-[2-(N-methyl-carbamoyl)ethyl]amid;
5(S)-Amino-4(S)-hydroxy-2(S),7(S)-diisopropyl-8-[4-methoxy-3-(3-methoxypropyloxy)-phenyl]-octansäure-N-(3-morpholino-3-oxo-propyl)amid;
5(S)-Amino-4(S)-hydroxy-2(S),7(S)-diisopropyl-8-[4-methoxy-3-(3-methoxypropyloxy)-phenyl]-octansäure-{N-[2-(N,N-dimethyl-carbamoyl)-1(R,S)-methyl-ethyl]}-amid;
5(S)-Amino-4(S)-hydroxy-2(S),7(S)-diisopropyl-8-[4-methoxy-3-(3-methoxypropyloxy)-phenyl]-octansäure-[N-(2-carbamoyl-1(R)-isopropyl-ethyl)]-amid;
5(S)-Amino-4(S)-hydroxy-2(S),7(S)-diisopropyl-8-[4-methoxy-3-(3-methoxypropyloxy)-phenyl]-octansäure-{N-[2-(N-methylcarbamoyl)-1(R)-isopropyl-ethyl]}-amid;
5(S)-Amino-4(S)-hydroxy-2(S),7(S)-diisopropyl-8-[4-methoxy-3-(3-methoxypropyloxy)-phenyl]-octansäure-{N-[2-(N,N-dimethylcarbamoyl)-1(R)-isopropyl-ethyl]}-amid;
5(S)-Amino-4(S)-hydroxy-2(S),7(S)-diisopropyl-8-[4-methoxy-3-(3-methoxypropyloxy)-phenyl]-octansäure-[N-(1(S)-carbamoyl-2-hydroxy-ethyl)]-amid;
5(S)-Amino-4(S)-hydroxy-2(S),7(S)-diisopropyl-8-[4-methoxy-3-(3-methoxypropyloxy)-phenyl]-octansäure-[N-(1(S),2-dicarbamoyl-ethyl)]-amid;
5(S)-Amino-4(S)-hydroxy-2(S),7(S)-diisopropyl-8-[4-methoxy-3-(3-methoxypropyloxy)-phenyl]-octansäure-[N-(1(S),3-dicarbamoyl-propyl)]-amid;
5(S)-Amino-4(S)-hydroxy-2(S),7(S)-diisopropyl-8-[4-methoxy-3-(3-methoxypropyloxy)-phenyl]-octansäure-[N-(1(S)-carbamoyl-propyl)]-amid;
5(S)-Amino-4(S)-hydroxy-2(S),7(S)-diisopropyl-8-[4-methoxy-3-(3-methoxypropyloxy)-phenyl]-octansäure-[N-(1(S)-carbamoyl-2(S)-methyl-butyl)]-amid;
5(S)-Amino-4(S)-hydroxy-2(S),7(S)-diisopropyl-8-[4-methoxy-3-(3-methoxypropyloxy)-phenyl]-octansäure-N-[2(R,S)-carbamoyl-2(R,S)-methyl-ethyl]-amid;
5(S)-Amino-4(S)-hydroxy-2(S),7(S)-diisopropyl-8-[4-methoxy-3-(3-methoxypropyloxy)-phenyl]-octansäure-[N-(2-carbamoyl-1(S)-methyl-ethyl)]-amid;
5(S)-Amino-4(S)-hydroxy-2(S),7(S)-diisopropyl-8-[4-methoxy-3-(3-methoxypropyloxy)-phenyl]-octansäure-[N-(2-carbamoyl-1(R)-methyl-ethyl)]-amid;
5(S)-Amino-4(S)-hydroxy-2(S),7(S)-diisopropyl-8-[4-methoxy-3-(3-methoxypropyloxy)-phenyl]-octansäure-N-[2(S)-carbamoyl-2(S)-methyl-ethyl]-amid;
5(S)-Amino-4(S)-hydroxy-2(S),7(S)-diisopropyl-8-[4-methoxy-3-(3-methoxypropyloxy)-phenyl]-octansäure-{N-[2(S)-(N-methyl-carbamoyl)-2(S)-methyl-ethyl]}-amid;
5(S)-Amino-4(S)-hydroxy-2(S),7(S)-diisopropyl-8-[4-methoxy-3-(3-methoxypropyloxy)-phenyl]-octansäure-[N-(2-carboxy-2,2-dimethyl-ethyl)]-amid;
5(S)-Amino-4(S)-hydroxy-2(S),7(S)-diisopropyl-8-[4-methoxy-3-(3-methoxypropyloxy)-phenyl]-octansäure-[N-(2-carboxy-2,2-diethyl-ethyl)]-amid;
5(S)-Amino-4(S)-hydroxy-2(S),7(S)-diisopropyl-8-[4-methoxy-3-(3-methoxypropyloxy)-phenyl]-octansäure-N-[(1-carboxy-cyclopentyl)-methyl]amid;
5(S)-Amino-4(S)-hydroxy-2(S),7(S)-diisopropyl-8-[4-methoxy-3-(3-methoxypropyloxy)-phenyl]-octansäure-{N-[2-(1H-tetrazol-5-yl)-ethyl]}-amid;
5(S)-Amino-4(S)-hydroxy-2(S),7(S)-diisopropyl-8-[4-methoxy-3-(3-methoxypropyloxy)-phenyl]-octansäure-N-[1(S)-(5-oxopyrrolidin-2-yl)methyl]-amid;
5(S)-Amino-4(S)-hydroxy-2(S),7(S)-diisopropyl-8-[4-methoxy-3-(3-methoxypropyloxy)-phenyl]-octansäure-N-[1(R)-(5-oxopyrrolidin-2-yl)methyl]-amid;
5(S)-Amino-4(S)-hydroxy-2(S),7(S)-diisopropyl-8-[4-methoxy-3-(3-methoxypropyloxy)-phenyl]-octansäure-N-[(morpholin-4-yl)carbonyl-methyl]amid;
5(S)-Amino-4(S)-hydroxy-2(S),7(S)-diisopropyl-8-[4-methoxy-3-(3-methoxypropyloxy)-phenyl]-octansäure-[N-(1(S)-carbamoyl-ethyl)]-amid;
5(S)-Amino-4(S)-hydroxy-2(S),7(S)-diisopropyl-8-[4-methoxy-3-(3-methoxypropyloxy)-phenyl]-octansäure-N-{1(S)-[(N-methyl)-carbamoyl]-ethyl}-amid;
5(S)-Amino-4(S)-hydroxy-2(S),7(S)-diisopropyl-8-[4-methoxy-3-(3-methoxypropyloxy)-phenyl]-octansäure-N-{1(S)-[(N,N-dimethyl)-carbamoyl]-ethyl}-amid;
5(S)-Amino-4(S)-hydroxy-2(S),7(S)-diisopropyl-8-[4-methoxy-3-(3-methoxypropyloxy)-phenyl]-octansäure-N-{1(S)-N-[(morpholin-4-yl)-carbonyl]ethyl}amid;
5(S)-Amino-4(S)-hydroxy-2(S),7(S)-diisopropyl-8-[4-methoxy-3-(3-methoxypropyloxy)-phenyl]-octansäure-N-[1(S)-carbamoyl-butyl]amid;
5(S)-Amino-4(S)-hydroxy-2(S),7(S)-diisopropyl-8-[4-methoxy-3-(3-methoxypropyloxy)-phenyl]-octansäure-N-[1(S)-carbamoyl-2-methyl-propyl]-amid;
5(S)-Amino-4(S)-hydroxy-2(S),7(S)-diisopropyl-8-[4-methoxy-3-(3-methoxypropyloxy)-phenyl]-octansäure-N-[1(S)-(N-methylcarbamoyl)-2-methyl-propyl]amid;
5(S)-Amino-4(S)-hydroxy-2(S),7(S)-diisopropyl-8-[4-methoxy-3-(3-methoxypropyloxy)-phenyl]-octansäure-N-[1(S)-(N,N-dimethylcarbamoyl)-2-methyl-propyl]amid;
5(S)-Amino-4(S)-hydroxy-2(S),7(S)-diisopropyl-8-[4-methoxy-3-(3-methoxypropyloxy)-phenyl]-octansäure-N-{1(S)-[(morpholin-4-yl)carbonyl]-2-methyl-propyl}amid;
5(S)-Amino-4(S)-hydroxy-2(S),7(S)-diisopropyl-8-[4-methoxy-3-(3-methoxypropyloxy)-phenyl]-octansäure-N-[2-(N-methylsulfamoyl)ethyl]amid;
5(S)-Amino-4(S)-hydroxy-2(S),7(S)-diisopropyl-8-[4-methoxy-3-(3-methoxypropyloxy)-phenyl]-octansäure-N-{2-[N-(morpholin-4-yl)-sulfonyl]ethyl}amid;
5(S)-Amino-4(S)-hydroxy-2(S),7(S)-diisopropyl-8-[4-methoxy-3-(3-methoxypropyloxy)-phenyl]-octansäure-N-[(N-acetyl-piperidin-4-yl)methyl]amid;
5(S)-Amino-4(S)-hydroxy-2(S),7(S)-diisopropyl-8-[4-methoxy-3-(4-methoxy-butyl)-phenyl]-octansäure-N-(2-carbamoyl-2,2-dimethyl-ethyl)amid;
5(S)-Amino-4(S)-hydroxy-2(S),7(S)-diisopropyl-8-[4-methoxy-3-(3-methoxypropyloxy)-phenyl]-octansäure-N-[2-(N,N-dimethylcarbamoyl)ethyl]amid;
5(S)-Amino-4(S)-hydroxy-2(S),7(S)-diisopropyl-8-[4-methoxy-3-(4-methoxybutyl)-phenyl]-octansäure-N-(2-morpholinoethyl)amid;
5(S)-Amino-4(S)-hydroxy-2(S),7(S)-diisopropyl-8-[4-methoxy-3-(3-methoxy-propyloxy)-phenyl]-octansäure-[N-(3(R)-2-oxo-pyrrolidin-3-yl-methyl)]-amid;
5(S)-Amino-4(S)-hydroxy-2(S),7(S)-diisopropyl-8-[4-methoxy-3-(3-methoxy-propyloxy)-phenyl]-octansäure-[N-(3(S)-2-oxo-piperidin-3-yl-methyl)]-amid;
5(S)-Amino-4(S)-hydroxy-2(S),7(S)-diisopropyl-8-[4-methoxy-3-(3-methoxy-propyloxy)-phenyl]-octansäure-[N-(3(R)-2-oxo-piperidin-3-yl-methyl)]-amid;
5(S)-Amino-4(S)-hydroxy-2(S),7(S)-diisopropyl-8-[4-methoxy-3-(3-methoxy-propyloxy)-phenyl]-octansäure-[N-(3-carbamoyl-3,3-dimethyl-propyl)]-amid;
5(S)-Amino-4(S)-hydroxy-2(S),7(S)-diisopropyl-8-[4-methoxy-3-(4-methoxy-butyl)-phenyl]-octansäure-[N-(5(S)-2-pyrrolidinon-5-yl-methyl)]-amid;
5(S)-Amino-4(S)-hydroxy-2(S),7(S)-diisopropyl-8-[4-methoxy-3-(4-methoxy-butyl)-phenyl]-octansäure-[N-(5(R)-2-pyrrolidinon-5-yl-methyl)]-amid;
5(S)-Amino-4(S)-hydroxy-2(S),7(S)-diisopropyl-8-[4-methoxy-3-(3-methoxy-propyloxy)-phenyl]-octansäure-[N-(6(S)-2-oxo-piperidin-6-yl-methyl)]-amid;
5(S)-Amino-4(S)-hydroxy-2(S),7(S)-diisopropyl-8-[4-methoxy-3-(3-methoxy-propyloxy)-phenyl]-octansäure-[N-(6(R)-2-oxo-piperidin-6-yl-methyl)]-amid;
5(S)-Amino-4(S)-hydroxy-2(S),7(S)-diisopropyl-8-[4-methoxy-3-(3-methoxy-propyloxy)-phenyl]-octansäure-[N-(2-thiazol-2-yl-ethyl)]-amid;
5(S)-Amino-4(S)-hydroxy-2(S),7(S)-diisopropyl-8-[4-methoxy-3-(3-methoxy-propyloxy)-phenyl]-octansäure-[N-(4(S)-2-oxazolidinon-4-yl-methyl)]-amid;
5(S)-Amino-4(S)-hydroxy-2(S),7(S)-diisopropyl-8-[4-methoxy-3-(3-methoxy-propyloxy)-phenyl]-octansäure-[N-(4(R)-2-oxazolidinon-4-yl-methyl)]-amid;
5(S)-Amino-4(S)-hydroxy-2(S),7(S)-diisopropyl-8-[4-methoxy-3-(3-methoxy-propyloxy)-phenyl]-octansäure-[N-(3(S)-2,5-dioxo-pyrrolidin-3-yl-methyl)]-amid;
5(S)-Amino-4(S)-hydroxy-2(S),7(S)-diisopropyl-8-[4-methoxy-3-(3-methoxy-propyloxy)-phenyl]-octansäure-[N-(3(R)-2,5-dioxo-pyrrolidin-3-yl-methyl)]-amid;
5(S)-Amino-4(S)-hydroxy-2(S),7(S)-diisopropyl-8-[4-methoxy-3-(3-methoxy-propyloxy)-phenyl]-octansäure-[N-(2,6-dioxo-piperidin-4-yl-methyl)]-amid;
5(S)-Amino-4(S)-hydroxy-2(S),7(S)-diisopropyl-8-[4-methoxy-3-(3-methoxy-propyloxy)-phenyl]-octansäure-[N-(4(S)-2-oxothiazolidin-4-yl-methyl)]-amid;
5(S)-Amino-4(S)-hydroxy-2(S),7(S)-diisopropyl-8-[3-(3-methoxy propoxy)-4,5-ethylendioxyphenyl]-octansäure-N-(2-carbamoyl-2,2-dimethyl-ethyl)-amid;
5(S)-Amino-4(S)-hydroxy-2(S),7(S)-diisopropyl-8-[4-methoxy-3-(3-methoxy-propyloxy)-phenyl]-octansäure-[N-(4(R)-2-oxothiazolidin-4-yl-methyl)]-amid;
5(S)-Amino-4(S)-hydroxy-2(S),7(S)-diisopropyl-8-[4-methoxy-3-(3-methoxy-propyloxy)-phenyl]-octansäure-[N-(tetrahydro-2-pyrimidon-5-yl-methyl)]-amid;
5(S)-Amino-4(S)-hydroxy-2(S),7(S)-diisopropyl-8-[4-methoxy-3-(3-methoxy-propyloxy)-phenyl]-octansäure-[N-(N-acetyl-2-amino-2-methyl-propyl)]-amid;
5(S)-Amino-4(S)-hydroxy-2(S),7(S)-diisopropyl-8-[4-methoxy-3-(3-methoxy-propyloxy)-phenyl]-octansäure-[N-(N-formyl-2-amino-2-methyl-propyl)]-amid;
5(S)-Amino-4(S)-hydroxy-2(S),7(S)-diisopropyl-8-[4-methoxy-3-(3-methoxy-propyloxy)-phenyl]-octansäure-[N-(4-acetyl-piperazinyl-ethyl)]-amid;
5(S)-Amino-4(S)-hydroxy-2(S),7(S)-diisopropyl-8-[4-methoxy-3-(3-methoxy-propyloxy)-phenyl]-octansäure-[N-(2,4-imidazolindion-5-yl-methyl)]-amid;
5(S)-Amino-4(S)-hydroxy-2(S),7(S)-diisopropyl-8-[4-methoxy-3-(4-methoxy-butyl)-phenyl]-octansäure-[N-(2-hydroxy-pyridin-6-yl-methyl)]-amid;
5(S)-Amino-4(S)-hydroxy-2(S),7(S)-diisopropyl-8-[4-methoxy-3-(3-methoxy-propyloxy)-phenyl]-octansäure-[N-(2,2-dimethyl-2-sulfamoyl-ethyl)]-amid;
5(S)-Amino-4(S)-hydroxy-2(S),7(S)-diisopropyl-8-[4-methoxy-3-(3-methoxy-propyloxy)-phenyl]-octansäure-[N-(2,2-dimethyl-2(N,N-dimethyl)-sulfamoyl-ethyl)]-amid-hydrochlorid;
5(S)-Amino-4(S)-hydroxy-2(S),7(S)-diisopropyl-8-[4-methoxy-3-(3-methoxy-propyloxy)-phenyl]-octansäure-[N-(2-oxo-piperidin-3(R)-yl)]-amid;
5(S)-Amino-4(S)-hydroxy-2(S),7(S)-diisopropyl-8-[4-methoxy-3-(3-methoxy-propyloxy)-phenyl]-octansäure-[N-(2-oxo-piperidin-3(S)-yl)]-amid;
5(S)-Amino-4(S)-hydroxy-2(S),7(S)-diisopropyl-8-[4-methoxy-3-(3-methoxy-propyloxy)-phenyl]-octansäure-[N-(2-oxo-pipendin-4-yl)]-amid;
5(S)-Amino-4(S)-hydroxy-2(S),7(S)-diisopropyl-8-[4-methoxy-3-(3-methoxy-propyloxy)-phenyl]-octansäure-[N-(N-acetyl-piperidin-4-yl)]-amid;
5(S)-Amino-4(S)-hydroxy-2(S),7(S)-diisopropyl-8-[4-methoxy-3-(4-methoxy-but-1-en-yl)-phenyl]-octansäure-[N-(2-carbamoyl-2,2-dimethyl-ethyl)]-amid;
5(S)-Amino-4(S)-hydroxy-2(S),7(S)-diisopropyl-8-[4-methoxy-3-(3-methoxypropyloxy)-phenyl]-octansäure-N-(3-morpholinopropyl)amid;
5(S)-Amino-4(S)-hydroxy-2(S),7(S)-diisopropyl-8-[4-methoxy-3-(3-methoxypropyloxy)-phenyl]-octansäure-N-(2-morpholinoethyl)amid;
5(S)-Amino-4(S)-hydroxy-2(S),7(S)-diisopropyl-8-[4-methoxy-3-(3-methoxypropyloxy)-phenyl]-octansäure-{N-[2-(N-methyl-carbamoyl)-1(R,S)-methyl-ethyl]}-amid;
5(S)-Amino-4(S)-hydroxy-2(S),7(S)-diisopropyl-8-[4-methoxy-3-(3-methoxypropyloxy)-phenyl]-octansäure-N-(3-carbamoylpropyl)amid;
5(S)-Amino-4(S)-hydroxy-2(S),7(S)-diisopropyl-8-[4-methoxy-3-(3-methoxypropyloxy)-phenyl]-octansäure-{N-[2(R)-(N-methyl-carbamoyl)-2(R)-methyl-ethyl]}-amid;
5(S)-Amino-4(S)-hydroxy-2(S),7(S)-diisopropyl-8-[4-methoxy-3-(3-methoxypropyloxy)-phenyl]-octansäure-N-(2-thiomorpholinoethyl)amid;
5(S)-Amino-4(S)-hydroxy-2(S),7(S)-diisopropyl-8-[4-methoxy-3-(3-methoxypropyloxy)-phenyl]-octansäure-N-[2-(N,N-dimethyl-carbamoyl)ethyl]amid;
5(S)-Amino-4(S)-hydroxy-2(S),7(S)-diisopropyl-8-[4-methoxy-3-(3-methoxypropyloxy)-phenyl]-octansäure-N-(2-carbamoyl-1(R,S)-methyl-ethyl)amid;
5(S)-Amino-4(S)-hydroxy-2(S),7(S)-diisopropyl-8-[4-methoxy-3-(3-methoxypropyloxy)-phenyl]-octansäure-N-[2(R)-carbamoyl-2(R)-methyl-ethyl]-amid;
5(S)-Amino-4(S)-hydroxy-2(S),7(S)-diisopropyl-8-[4-methoxy-3-(3-methoxypropyloxy)-phenyl]-octansäure-N-(2-carbamoyl-2,2-dimethyl-ethyl)amid;
5(S)-Amino-4(S)-hydroxy-2(S),7(S)-diisopropyl-8-[4-methoxy-3-(3-methoxypropyloxy)-phenyl]-octansäure-N-[2-(N-acetyl)-piperidin-4-yl)ethyl]amid;
5(S)-Amino-4(S)-hydroxy-2(S),7(S)-diisopropyl-8-[4-methoxy-3-(3-methoxypropyloxy)-phenyl]-octansäure-{N-[(N,N-dimethyl)-carbamoyl-methyl]}-amid oder
5(S)-Amino-4(S)-hydroxy-2(S),7(S)-diisopropyl-8-[4-methoxy-3-(3-methoxypropyloxy)-phenyl]-octansäure-N-[2(R,S)-(N-methylcarbamoyl)-2(R,S)-methyl-ethyl]-amid gemäss Anspruch 1 oder jeweils ein Salz davon.

9. Verbindungen gemäss einem der Ansprüche 1 bis 8 zur Anwendung in einem Verfahren zur therapeutischen Behandlung des menschlichen oder tierischen Körpers.

10. Pharmazeutische Präparate, neben üblichen pharmazeutischen Hilfsstoffen als pharmazeutischen Wirkstoff enthaltend eine Verbindung gemäss einem der Ansprüche 1 bis 8 in freier Form oder in pharmazeutisch verwendbarer Salzform.

11. Verfahren zur Herstellung von δ-Amino-γ-hydroxy-ω-aryl-alkansäureamiden der Formel (I) in Anspruch 1, und ihrer Salze, dadurch gekennzeichnet, dass man
a) eine Verbindung der Formel II 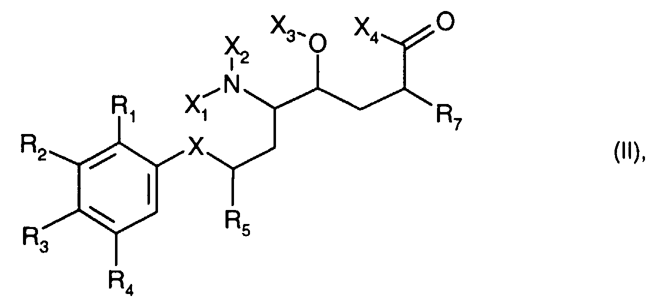 worin
X₁ C₁-C₄-Alkyl, C₁-C₄-Alkanoyl oder eine Aminoschutzgruppe bedeutet,
X₂ Wasserstoff oder gemeinsam mit X₃ eine bivalente Schutzgruppe darstellt,
X₃ Wasserstoff, eine Hydroxyschutzgruppe oder gemeinsam mit X₂ eine bivalente Schutzgruppe darstellt bzw. gemeinsam mit X₄ für eine direkte Bindung steht und
X₄ gegebenenfalls reaktionsfähig verethertes oder verestertes Hydroxy, oder gemeinsam mit X₃ eine direkte Bindung darstellt und R₁, R₂, R₃, R₄, X, R₅, R₆ und R₇ die unter der Formel I angegebenen Bedeutungen haben, mit einem Amin der Formel III
H₂N―R₈ (III),
worin R₈ eine der unter der Formel I angegebenen Bedeutungen hat, unter Bildung einer Amidbindung umsetzt und vorhandene Schutzgruppen abspaltet, oder
b) in einem Carbonsäureamid der Formel IV 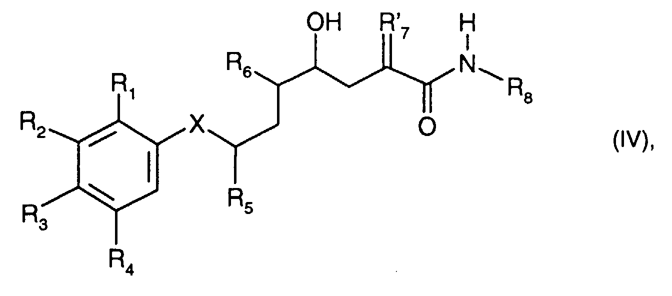 worin R₁, R₂, R₃, R₄, X, R₅, R₆, R₇ und R₈ die unter der Formel I angegebenen Bedeutungen haben und R'₇ eine der C₁-C₄-Alkyl- bzw. Aryl-C₁-C₄-alkylgruppe R₇ entsprechende C₁-C₄-Alkyliden- oder Aryl-C₁-C₄-alkylidengruppe bedeutet, wobei freie funktionelle Gruppen gegebenenfalls in geschützter Form vorliegen, oder ein Salz davon die Gruppe R'₇ durch Behandlung mit einem Hydrierungsmittel zu R₇ reduziert, oder
c) zur Herstellung von Verbindungen der Formel I, worin R₆ Amino bedeutet, in einem 5-Azidocarbonsäurederivat der Formel V 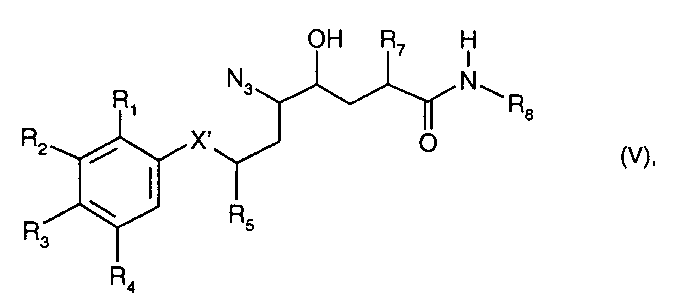 worin R₁, R₂, R₃, R₄, R₅, R₇ und R₈ die unter der Formel I angegebenen Bedeutungen haben, X' Methylen oder gegebenenfalls verestertes oder verethertes Hydroxymethyl bedeutet und freie funktionelle Gruppen gegebenenfalls in geschützter Form vorliegen, oder in einem Salz davon die Azidogruppe, gewünschtenfalls unter Freisetzung von Hydroxymethyl X oder Reduktion von X' zu Methylen X, zu Amino reduziert und vorhandene Schutzgruppen abspaltet und gewünschtenfalls eine nach einem der vorstehend genannten Verfahren a) bis c) erhältliche Verbindung der Formel I mit mindestens einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfinerfindungsgemässe Verbindung der Formel I in eine andere erfindungsgemässe Verbindung der Formel I umwandelt.

12. Verbindungen der Formel II 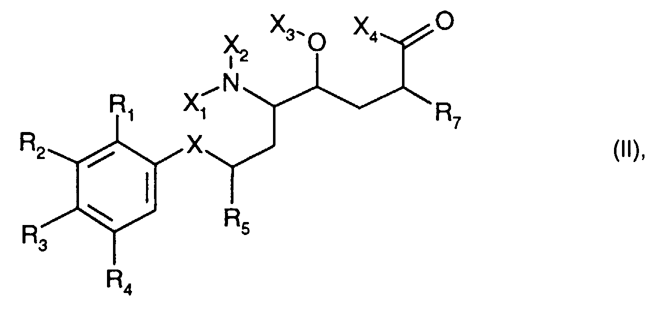 worin
R₁ Wasserstoff, Hydroxy, C₁-C₇-Alkoxy, 3- bis 8-gliedriges Cycloalkoxy, C₁-C₄-Alkoxy-C₂-C₄-alkoxy oder Carboxy-C₁-C₄-alkoxy, C₁-C₄-Alkoxycarbonyl-C₁-C₄-alkoxy, Carbamoyl-C₁-C₄-alkoxy, Carbamoyl-C₁-C₄-alkoxy, N-C₁-C₇-Alkylcarbamoyl-C₁-C₄-alkoxy oder N-Mono- oder N,N-Di-C₁-C₄-alkylcarbamoyl-C₁-C₄-alkoxy bedeutet,
R₂ Wasserstoff, C₁-C₇-Alkyl, 3- bis 8-gliedriges Cycloalkyl, C₁-C₄-Alkoxy-C₁-C₄-alkyl, C₁-C₄-Alkoxy-C₁-C₄-alkoxy-C₁-C₄-alkyl, 3- bis 8-gliedriges Cycloalkoxy-C₁-C₄-alkyl, Hydroxy, C₁-C₇-Alkanoyloxy-C₂-C₄-alkyl, Hydroxy-C₂-C₇-alkoxy, Halogen-C₂-C₇-(hydroxy)alkoxy, C₁-C₇-Alkansulfonyl-C₁-C₄-(hydroxy)alkoxy, Amino-C₁-C₄-alkyl, C₁-C₄-Alkylamino-C₁-C₄-alkyl, N,N-Di-C₁-C₄-alkylamino-C₁-C₄-alkyl, N-C₁-C₄-Alkanoylamino-C₁-C₄-alkyl, C₁-C₇-Alkoxycarbonylamino-C₂-C₇-alkyl, gegebenenfalls partiell hydriertes Pyridyl- oder N-Oxidopyridyl-C₁-C₄-alkyl, Amino-C₁-C₄-alkoxy, C₁-C₄-Alkylamino-C₁-C₄-alkoxy, N,N-Di-C₁-C₄-alkylamino-C₁-C₄-alkoxy, C₁-C₄-Alkanoylamino-C₁-C₄-alkoxy, C₁-C₇-Alkoxycarbonylamino-C₂-C₇-alkoxy, die Alkanoylgruppe in höherer als der α-Stellung tragendes C₁-C₇-Alkanoyl-C₂-C₄-alkoxy, C₁-C₇-Alkoxy, 3- bis 8-gliedriges Cycloalkoxy, C₂-C₇-Alkenyloxy, 3- bis 8-gliedriges Cycloalkoxy-C₁-C₄-alkoxy, C₁-C₄-Alkoxy-C₂-C₄-alkoxy, C₁-C₄-Alkoxy-C₂-C₄-alkenyl, C₂-C₇-Alkenyloxy-C₁-C₄-alkoxy, C₁-C₄-Alkoxy-C₂-C₄-alkenyloxy, C₂-C₇-Alkenyloxy-C₁-C₄-alkyl, C₁-C₄-Alkylthio-C₁-C₄-alkoxy, C₁-C₇-Alkansulfonyl-C₁-C₄-alkoxy, C₁-C₄-Alkylthio-C₁-C₄-(hydroxy)alkoxy, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl- oder Naphthyl-C₁-C₄-alkoxy, gegebenenfalls partiell hydriertes Pyridyl- oder N-Oxidopyridyl-C₁-C₄-alkoxy, Thiazolyl-C₁-C₄-alkoxy, gegebenenfalls N-oxidiertes Morpholino-C₁-C₄-alkoxy, Cyano-C₁-C₄-alkoxy, Carboxy-C₁-C₄-alkoxy, C₁-C₄-Alkoxycarbonyl-C₁-C₄-alkoxy, Carbamoyl-C₁-C₄-alkoxy, N-C₁-C₇-Alkylcarbamoyl-C₁-C₄-alkoxy oder N-Mono- oder N,N-Di-C₁-C₄-alkylcarbamoyl-C₁-C₄-alkoxy, Carboxy-C₁-C₄-alkyl, C₁-C₄-Alkoxycarbonyl-C₁-C₄-alkyl, Carbamoyl-C₁-C₇-alkyl oder N-Mono- oder N,N-Di-C₁-C₄-alkylcarbamoyl-C₁-C₄-alkyl bedeutet,
R₃ Wasserstoff, C₁-C₇-Alkyl, Polyhalogen-C₁-C₄-alkyl, C₁-C₄-Alkoxy-C₁-C₄-alkyl, 3- bis 8-gliedriges Cycloalkoxy-C₁-C₄-alkyl, Hydroxy-C₂-C₇-alkyl, C₁-C₄-Alkylthio-C₁-C₄-alkyl, C₁-C₇-Alkansulfonyl-C₁-C₄-alkyl, Thiazolylthio-C₁-C₄-alkyl bzw. Thiazolinylthio-C₁-C₄-alkyl, Imidazolylthio-C₁-C₄-alkyl, gegebenenfalls N-oxidiertes Pyridylthio-C₁-C₄-alkyl, Pyrimidinylthio-C₁-C₄-alkyl, gegebenenfalls partiell hydriertes Pyridyl- oder N-Oxidopyridyl-C₁-C₄-alkyl, Amino-C₁-C₄-alkyl, C₁-C₄-Alkylamino-C₁-C₄-alkyl, N,N-Di-C₁-C₄-alkylamino-C₁-C₄-alkyl, N-C₁-C₄-Alkanoylamino-C₁-C₄-alkyl, C₁-C₄-Alkansulfonylamino-C₁-C₄-alkyl, Trifluor-C₁-C₇-alkansulfonylamino-C₁-C₄-alkyl, Pyrrolidino-C₁-C₄-alkyl, Piperidino-C₁-C₄-alkyl, Piperazino-C₁-C₄-alkyl, N'-C₁-C₄-Alkylpiperazino-C₁-C₄-alkyl, N'-C₂-C₇-Alkanoylpiperazino-C₁-C₄-alkyl, Morpholino-C₁-C₄-alkyl, Thiomorpholino-C₁-C₄-alkyl, S-Oxothiomorpholino-C₁-C₄-alkyl, S,S-Dioxothiomorpholino-C₁-C₄-alkyl, Cyano-C₁-C₄-alkyl, Carboxy-C₁-C₄-alkyl, C₁-C₄-Alkoxycarbonyl-C₁-C₄-alkyl, Carbamoyl-C₁-C₇-alkyl, N-Mono- oder N,N-Di-C₁-C₄-alkylcarbamoyl-C₁-C₄-alkyl, 3- bis 8-gliedriges Cycloalkyl, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl oder Naphthyl, Hydroxy, C₁-C₄-Alkoxy, 3- bis 8-gliedriges Cycloalkoxy, C₁-C₄-Alkoxy-C₂-C₄-alkoxy, 3- bis 8-gliedriges Cycloalkoxy-C₁-C₄-alkoxy, Hydroxy-C₂-C₇-alkoxy, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl-C₁-C₄-alkoxy oder Naphthyl-C₁-C₄-alkoxy, C₁-C₄-Alkoxy, Polyhalogen-C₁-C₄-alkoxy, C₁-C₄-Alkylthio-C₁-C₄-alkoxy, C₁-C₇-Alkansulfonyl-C₁-C₄-alkoxy, gegebenenfalls partiell hydriertes Pyridyl- oder N-Oxidopyridyl-C₁-C₄-alkoxy, Thiazolyl-C₁-C₄-alkoxy, Morpholino-C₁-C₄-alkoxy, Thiazolylthio-C₁-C₄-alkoxy, Thiazolinylthio-C₁-C₄-alkoxy, Imidazolylthio-C₁-C₄-alkoxy, gegebenenfalls N-oxidiertes Pyridylthio-C₁-C₄-alkoxy, Pyrimidinylthio-C₁-C₄-alkoxy, Amino-C₁-C₄-alkoxy, C₁-C₄-Alkylamino-C₁-C₄-alkoxy, N,N-Di-C₁-C₄-alkylamino-C₁-C₄-alkoxy, C₁-C₇-Alkanoylamino-C₁-C₄-alkoxy, C₁-C₇-Alkansulfonylamino-C₁-C₄-alkoxy, Trifluor-C₁-C₇-alkansulfonyl-C₁-C₄-alkoxy, Pyrrolidino-C₂-C₄-alkoxy, Piperidino-C₁-C₄-alkoxy, Piperazino-C₁-C₄-alkyl, N'-C₁-C₄-Alkylpiperazino-C₁-C₄-alkyl, N'-C₂-C₇-Alkanoylpiperazino-C₁-C₄-alkyl, Morpholino-C₁-C₄-alkyl, Thiomorpholino-C₁-C₄-alkyl, S-Oxothiomorpholino-C₁-C₄-alkyl, S,S-Dioxothiomorpholino-C₁-C₄-alkyl, Cyano-C₁-C₄-alkoxy, Carboxy-C₁-C₄-alkoxy, C₁-C₄-Alkoxycarbonyl-C₁-C₄-alkoxy, Carbamoyl-C₁-C₇-alkoxy, N-Mono- oder N,N-Di-C₁-C₄-alkylcarbamoyl-C₁-C₄-alkoxy bedeutet oder gemeinsam mit R₄ C₁-C₄-Alkylendioxy oder einen ankondensierten Benzo- oder Cyclohexenoring darstellt,
R₄ gemeinsam mit R₃ C₁-C₄-Alkylendioxy oder einen ankondensierten Benzo- oder Cyclohexenoring darstellt oder für Wasserstoff, C₁-C₇-Alkyl, Hydroxy, C₁-C₄-Alkoxy oder 3- bis 8-gliedriges Cycloalkoxy steht,
X Methylen oder Hydroxymethylen ist,
R₅ für C₁-C₇-Alkyl oder 3- bis 8-gliedriges Cycloalkyl steht,
R₇ C₁-C₇-Alkyl, C₂-C₇-Alkenyl, 3- bis 8-gliedriges Cycloalkyl oder unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluormethyl mono-, di- oder trisubstituiertes Phenyl- oder Naphthyl-C₁-C₄-alkyl bedeutet,
X₁ eine Aminoschutzgruppe bedeutet, X₂ Wasserstoff oder gemeinsam mit X₃ eine bivalente Schutzgruppe darstellt,
X₃ Wasserstoff, eine Hydroxyschutzgruppe oder gemeinsam mit X₂ eine bivalente Schutzgruppe darstellt bzw. gemeinsam mit X₄ für eine direkte Bindung steht und
X₄ gegebenenfalls raktionsfähig verethertes oder verestertes Hydroxy, oder gemeinsam mit
X₃ eine direkte Bindung darstellt,
und ihre Salze.

13. Verbindungen gemäss Anspruch 12 der Formel IId 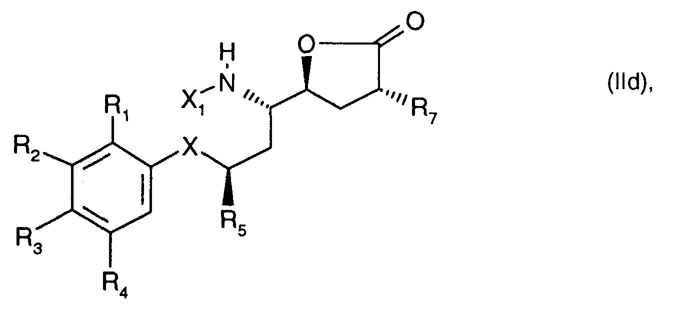 worin
R₁ und R₄ Wasserstoff bedeuten,
R₂ für C₁-C₄-Alkoxy-C₁-C₄-alkoxy oder C₁-C₄-Alkoxy-C₁-C₄-alkyl steht,
R₃ C₁-C₄-Alkyl oder C₁-C₄-Alkoxy darstellt, X Methylen ist,
R₅ und R₇ verzweigtes C₁-C₄-Alkyl bedeuten und
X₁ "C₁-C₇-Alkoxycarbonyl oder gegebenenfalls durch C₁-C₄-Alkyl, C₁-C₄-Alkoxy, Nitro und/oder Halogen substituiertes α-Phenyl-C₁-C₄-alkoxycarbonyl" darstellt,
und ihre Salze.

14. 3(S)-Isopropyl-5(S)-{1(S)-tertiärbutoxycarbonylamino-3(S)-isopropyl-4-[4-(3-hydroxypropyloxy)-3-(3-methoxypropyloxy)-phenyl]-butyl}-tetrahydrofuran-2-on;
3(S)-Isopropyl-5(S)-{1(S)-benzyloxycarbonylamino-3(S)-isopropyl-4-[4-methoxy-3-(3-methoxypropyloxy)-phenyl]-butyl}-tetrahydrofuran-2-on;
5(S)-Tertiärbutoxycarbonylamino-4(S)-tertiärbutyldimethylsilyloxy-7(S)-isopropyl-2(R)-methyl-8-[4-methoxy-3-(3-methoxypropyloxy)-phenyl]-octansäure;
5(S)-Tertiärbutoxycarbonylamino-4(S)-hydroxy-7(S)-isopropyl-2(R)-methyl-8-[4-methoxy-3-(3-methoxypropyloxy)-phenyl]-octansäure oder
2-{1(S)-Tertiärbutoxycarbonylamino-3(S)-isopropyl-4-[4-methoxy-3-(3-methoxy-propyloxy)-phenyl]-butyl}-4(R)-methyl-tetrahydrofuran-5-on gemäss Anspruch 12 oder jeweils ein Salz davon.

15. Verbindungen der Formel XVI 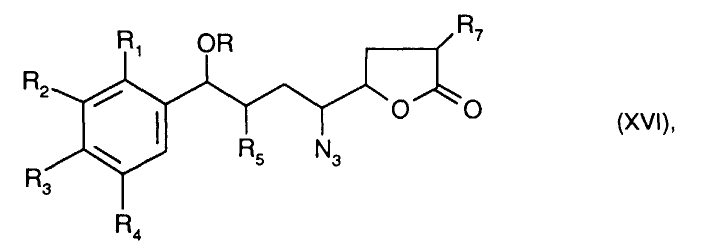
R₁ Wasserstoff, Hydroxy, C₁-C₇-Alkoxy, 3- bis 8-gliedriges Cycloalkoxy, C₁-C₄-Alkoxy-C₂-C₄-alkoxy oder Carboxy-C₁-C₄-alkoxy, C₁-C₄-Alkoxycarbonyl-C₁-C₄-alkoxy, Carbamoyl-C₁-C₄-alkoxy, Carbamoyl-C₁-C₄-alkoxy, N-C₁-C₇-Alkylcarbamoyl-C₁-C₄-alkoxy oder N-Mono- oder N,N-Di-C₁-C₄-alkylcarbamoyl-C₁-C₄-alkoxy bedeutet,
R₂ Wasserstoff, C₁-C₇-Alkyl, 3- bis 8-gliedriges Cycloalkyl, C₁-C₄-Alkoxy-C₁-C₄-alkyl, C₁-C₄-Alkoxy-C₁-C₄-alkoxy-C₁-C₄-alkyl, 3- bis 8-gliedriges Cycloalkoxy-C₁-C₄-alkyl, Hydroxy, C₁-C₇-Alkanoyloxy-C₂-C₄-alkyl, Hydroxy-C₂-C₇-alkoxy, Halogen-C₂-C₇-(hydroxy)alkoxy, C₁-C₇-Alkansulfonyl-C₁-C₄-(hydroxy)alkoxy, Amino-C₁-C₄-alkyl, C₁-C₄-Alkylamino-C₁-C₄-alkyl, N,N-Di-C₁-C₄-alkylamino-C₁-C₄-alkyl, N-C₁-C₄-Alkanoylamino-C₁-C₄-alkyl, C₁-C₇-Alkoxycarbonylamino-C₂-C₇-alkyl, gegebenenfalls partiell hydriertes Pyridyl- oder N-Oxidopyridyl-C₁-C₄-alkyl, Amino-C₁-C₄-alkoxy, C₁-C₄-Alkylamino-C₁-C₄-alkoxy, N,N-Di-C₁-C₄-alkylamino-C₁-C₄-alkoxy, C₁-C₄-Alkanoylamino-C₁-C₄-alkoxy, C₁-C₇-Alkoxycarbonylamino-C₂-C₇-alkoxy, die Alkanoylgruppe in höherer als der α-Stellung tragendes C₁-C₇-Alkanoyl-C₂-C₄-alkoxy, C₁-C₇-Alkoxy, 3- bis 8-gliedriges Cycloalkoxy, C₂-C₇-Alkenyloxy, 3- bis 8-gliedriges Cycloalkoxy-C₁-C₄-alkoxy, C₁-C₄-Alkoxy-C₂-C₄-alkoxy, C₁-C₄-Alkoxy-C₂-C₄-alkenyl, C₂-C₇-Alkenyloxy-C₁-C₄-alkoxy, C₁-C₄-Alkoxy-C₂-C₄-alkenyloxy, C₂-C₇-Alkenyloxy-C₁-C₄-alkyl, C₁-C₄-Alkylthio-C₁-C₄-alkoxy, C₁-C₇-Alkansulfonyl-C₁-C₄-alkoxy, C₁-C₄-Alkylthio-C₁-C₄-(hydroxy)alkoxy, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluoromethyl mono-, di- oder trisubstituiertes Phenyl- oder Naphthyl-C₁-C₄-alkoxy, gegebenenfalls partiell hydriertes Pyridyl- oder N-Oxidopyridyl-C₁-C₄-alkoxy, Thiazolyl-C₁-C₄-alkoxy, gegebenenfalls N-oxidiertes Morpholino-C₁-C₄-alkoxy, Cyano-C₁-C₄-alkoxy, Carboxy-C₁-C₄-alkoxy, C₁-C₄-Alkoxycarbonyl-C₁-C₄-alkoxy, Carbamoyl-C₁-C₄-alkoxy, N-C₁-C₇-Alkylcarbamoyl-C₁-C₄-alkoxy oder N-Mono- oder N,N-Di-C₁-C₄-alkylcarbamoyl-C₁-C₄-alkoxy, Carboxy-C₁-C₄-alkyl, C₁-C₄-Alkoxycarbonyl-C₁-C₄-alkyl, Carbamoyl-C₁-C₇-alkyl oder N-Mono- oder N,N-Di-C₁-C₄-alkylcarbamoyl-C₁-C₄-alkyl bedeutet,
R₃ Wasserstoff, C₁-C₇-Alkyl, Polyhalogen-C₁-C₄-alkyl, C₁-C₄-Alkoxy-C₁-C₄-alkyl, 3- bis 8-gliedriges Cycloalkoxy-C₁-C₄-alkyl, Hydroxy-C₂-C₇-alkyl, C₁-C₄-Alkylthio-C₁-C₄-alkyl, C₁-C₇-Alkansulfonyl-C₁-C₄-alkyl, Thiazolylthio-C₁-C₄-alkyl bzw. Thiazolinylthio-C₁-C₄-alkyl, Imidazolylthio-C₁-C₄-alkyl, gegebenenfalls N-oxidiertes Pyridylthio-C₁-C₄-alkyl, Pyrimidinylthio-C₁-C₄-alkyl, gegebenenfalls partiell hydriertes Pyridyl- oder N-Oxidopyridyl-C₁-C₄-alkyl, Amino-C₁-C₄-alkyl, C₁-C₄-Alkylamino-C₁-C₄-alkyl, N,N-Di-C₁-C₄-alkylamino-C₁-C₄-alkyl, N-C₁-C₄-Alkanoylamino-C₁-C₄-alkyl, C₁-C₄-Alkansulfonylamino-C₁-C₄-alkyl, Trifluor-C₁-C₇-alkansulfonylamino-C₁-C₄-alkyl, Pyrrolidino-C₁-C₄-alkyl, Piperidino-C₁-C₄-alkyl, Piperazino-C₁-C₄-alkyl, N'-C₁-C₄-Alkylpiperazino-C₁-C₄-alkyl, N'-C₂-C₇-Alkanoylpiperazino-C₁-C₄-alkyl, Morpholino-C₁-C₄-alkyl, Thiomorpholino-C₁-C₄-alkyl, S-Oxothiomorpholino-C₁-C₄-alkyl, S,S-Dioxothiomorpholino-C₁-C₄-alkyl, Cyano-C₁-C₄-alkyl, Carboxy-C₁-C₄-alkyl, C₁-C₄-Alkoxycarbonyl-C₁-C₄-alkyl, Carbamoyl-C₁-C₇-alkyl, N-Mono- oder N,N-Di-C₁-C₄-alkylcarbamoyl-C₁-C₄-alkyl, 3- bis 8-gliedriges Cycloalkyl, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluoromethyl mono-, di- oder trisubstituiertes Phenyl oder Naphthyl, Hydroxy, C₁-C₄-Alkoxy, 3- bis 8-gliedriges Cycloalkoxy, C₁-C₄-Alkoxy-C₂-C₄-alkoxy, 3- bis 8-gliedriges Cycloalkoxy-C₁-C₄-alkoxy, Hydroxy-C₂-C₇-alkoxy, unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluoromethyl mono-, di- oder trisubstituiertes Phenyl-C₁-C₄-alkoxy oder Naphthyl-C₁-C₄-alkoxy, C₁-C₄-Alkoxy, Polyhalogen-C₁-C₄-alkoxy, C₁-C₄-Alkylthio-C₁-C₄-alkoxy, C₁-C₇-Alkansulfonyl-C₁-C₄-alkoxy, gegebenenfalls partiell hydriertes Pyridyl-oder N-Oxidopyridyl-C₁-C₄-alkoxy, Thiazolyl-C₁-C₄-alkoxy, Morpholino-C₁-C₄-alkoxy, Thiazolylthio-C₁-C₄-alkoxy, Thiazolinylthio-C₁-C₄-alkoxy, Imidazolylthio-C₁-C₄-alkoxy, gegebenenfalls N-oxidiertes Pyridylthio-C₁-C₄-alkoxy, Pyrimidinylthio-C₁-C₄-alkoxy, Amino-C₁-C₄-alkoxy, C₁-C₄-Alkylamino-C₁-C₄-alkoxy, N,N-Di-C₁-C₄-alkylamino-C₁-C₄-alkoxy, C₁-C₇-Alkanoylamino-C₁-C₄-alkoxy, C₁-C₇-Alkansulfonylamino-C₁-C₄-alkoxy, Trifluor-C₁-C₇-alkansulfonyl-C₁-C₄-alkoxy, Pyrrolidino-C₂-C₄-alkoxy, Piperidino-C₁-C₄-alkoxy, Piperazino-C₁-C₄-alkyl, N'-C₁-C₄-Alkylpiperazino-C₁-C₄-alkyl, N'-C₂-C₇-Alkanoylpiperazino-C₁-C₄-alkyl, Morpholino-C₁-C₄-alkyl, Thiomorpholino-C₁-C₄-alkyl, S-Oxothiomorpholino-C₁-C₄-alkyl, S,S-Dioxothiomorpholino-C₁-C₄-alkyl, Cyano-C₁-C₄-alkoxy, Carboxy-C₁-C₄-alkoxy, C₁-C₄-Alkoxycarbonyl-C₁-C₄-alkoxy, Carbamoyl-C₁-C₇-alkoxy, N-Mono- oder N,N-Di-C₁-C₄-alkylcarbamoyl-C₁-C₄-alkoxy bedeutet oder gemeinsam mit R₄ C₁-C₄-Alkylendioxy oder einen ankondensierten Benzo- oder Cyclohexenoring darstellt,
R₄ gemeinsam mit R₃ C₁-C₄-Alkylendioxy oder einen ankondensierten Benzo- oder Cyclohexenoring darstellt oder für Wasserstoff, C₁-C₇-Alkyl, Hydroxy, C₁-C₄-Alkoxy oder 3- bis 8-gliedriges Cycloalkoxy steht,
R eine Hydroxyschutzgruppe darstellt und
R₇ C₁-C₇-Alkyl, C₂-C₇-Alkenyl, 3- bis 8-gliedriges Cycloalkyl oder unsubstituiertes oder durch C₁-C₄-Alkyl, C₁-C₄-Alkoxy, Hydroxy, C₁-C₄-Alkylamino, Di-C₁-C₄-alkylamino, Halogen und/oder Trifluoromethyl mono-, di- oder trisubstituiertes Phenyl- oder Naphthyl-C₁-C₄-alkyl bedeutet,
und ihre Salze.

16. 3(S)-Isopropyl-5(S)-{1(S)-azido-3(S)-isopropyl-4(R,S)-hydroxy-4-[4-methoxy-3-(2-methoxymethoxyethyl)-phenyl]-butyl}-tetrahydrofuran-2-on;
3(S)-Isopropyl-5(S)-{1(S)-azido-3(S)-isopropyl-4(R,S)-isobutyryloxy-4-[4-methoxy-3-(2-methoxymethoxyethyl)-phenyl]-butyl}-tetrahydrofuran-2-on;
3(S)-Isopropyl-5(S)-{1(S)-azido-3(S)-isopropyl-4(R,S)-hydroxy-4-[4-(3-benzyloxypropyloxy)-3-(3-methoxypropyloxy)-phenyl]-butyl}-tetrahydrofuran-2-on;
3(S)-Isopropyl-5(S)-{1(S)-azido-3(S)-isopropyl-4(R,S)-acetoxy-4-[4-(3-benzyloxypropyloxy)-3-(3-methoxypropyloxy)-phenyl]-butyl}-tetrahydrofuran-2-on;
3(S)-Isopropyl-5(S)-{4(R,S)-acetoxy-1(S)-azido-3(S)-isopropyl-4(R,S)-[4-tertiärbutyl-3-(3-methoxypropyloxy)-phenyl]-butyl}-tetrahydrofuran-2-on;
3(S)-Isopropyl-5(S)-{4(R,S)-hydroxy-1(S)-azido-3(S)-isopropyl-4(R,S)-[4-tertiärbutyl-3-(3-methoxypropyloxy)-phenyl]-butyl}-tetrahydrofuran-2-on oder
3(S)-Isopropyl-5(S)-(1(S)-azido-4(S,R)-hydroxy-3(S)-isopropyl-4-[4-methoxy-3-(3-methoxypropyloxy)-phenyl]-butyl}-tetrahydrofuran-2-on gemäss Anspruch 15.

## Claims

1. A δ-amino-γ-hydroxy-ω-aryl-alkanoic acid amide of formula I 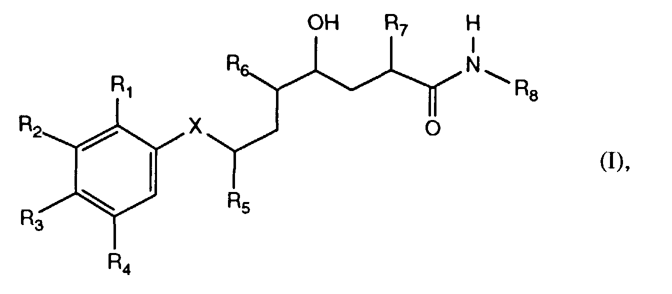 wherein
R₁ is hydrogen, hydroxy, C₁-C₇alkoxy, 3- to 8-membered cycloalkoxy, C₁-C₄alkoxy-C₂-C₄alkoxy or carboxy-C₁-C₄alkoxy, C₁-C₄alkoxycarbonyl-C₁-C₄alkoxy, carbamoyl-C₁-C₄alkoxy, N-C₁-C₇alkylcarbamoyl-C₁-C₄alkoxy or N,N-di-C₁-C₄alkylcarbamoyl-C₁-C₄alkoxy,
R₂ is hydrogen, C₁-C₇alkyl, 3- to 8-membered cycloalkyl, C₁-C₄alkoxy-C₁-C₄alkyl, C₁-C₄alkoxy-C₁-C₄alkoxy-C₁-C₄alkyl, 3- to 8-membered cycloalkoxy-C₁-C₄alkyl, hydroxy, C₁-C₇alkanoyloxy-C₂-C₄alkyl, hydroxy-C₂-C₇alkoxy, halo-C₂-C₇(hydroxy)alkoxy, C₁-C₇alkanesulfonyl-C₁-C₄(hydroxy)alkoxy, amino-C₁-C₄alkyl, C₁-C₄alkylamino-C₁-C₄alkyl, N,N-di-C₁-C₄alkylamino-C₁-C₄alkyl, N-C₁-C₄alkanoylamino-C₁-C₄alkyl, C₁-C₇alkoxycarbonylamino-C₂-C₇alkyl, optionally partially hydrogenated pyridyl- or N-oxidopyridyl-C₁-C₄alkyl, amino-C₁-C₄alkoxy, C₁-C₄alkylamino-C₁-C₄alkoxy, N,N-di-C₁-C₄alkylamino-C₁-C₄alkoxy, C₁-C₄alkanoylamino-C₁-C₄alkoxy, C₁-C₇alkoxycarbonylamino-C₂-C₇alkoxy, C₁-C₇alkanoyl-C₂-C₄alkoxy carrying the alkanoyl group in a position higher than the α-position, C₁-C₇alkoxy, 3- to 8-membered cycloalkoxy, C₂-C₇alkenyloxy, 3- to 8-membered cycloalkoxy-C₁-C₄-alkoxy, C₁-C₄alkoxy-C₂-C₄alkoxy, C₁-C₄alkoxy-C₂-C₄alkenyl, C₂-C₇alkenyloxy-C₁-C₄alkoxy, C₁-C₄alkoxy-C₂-C₄alkenyloxy, C₂-C₇alkenyloxy-C₁-C₄alkyl, C₁-C₄-alkylthio-C₁-C₄alkoxy, C₁-C₇alkanesulfonyl-C₁-C₄alkoxy, C₁-C₄alkylthio-C₁-C₄-(hydroxy)alkoxy; phenyl- or naphthyl-C₁-C₄alkoxy each unsubstituted or mono-, di- or tri-substituted by C₁-C₄alkyl, C₁-C₄alkoxy, hydroxy, C₁-C₄alkylamino, di-C₁-C₄-alkylamino, halogen and/or by trifluoromethyl; optionally partially hydrogenated pyridyl- or N-oxidopyridyl-C₁-C₄alkoxy, thiazolyl-C₁-C₄alkoxy, optionally N-oxidised morpholino-C₁-C₄alkoxy, cyano-C₁-C₄alkoxy, carboxy-C₁-C₄alkoxy, C₁-C₄-alkoxycarbonyl-C₁-C₄alkoxy, carbamoyl-C₁-C₄alkoxy, N-C₁-C₇alkylcarbamoyl-C₁-C₄alkoxy or N-mono- or N,N-di-C₁-C₄alkylcarbamoyl-C₁-C₄alkoxy, carboxy-C₁-C₄alkyl, C₁-C₄alkoxycarbonyl-C₁-C₄alkyl, carbamoyl-C₁-C₇alkyl or N-mono- or N,N-di-C₁-C₄alkylcarbamoyl-C₁-C₄alkyl,
R₃ is hydrogen, C₁-C₇alkyl, polyhalo-C₁-C₄alkyl, C₁-C₄alkoxy-C₁-C₄alkyl, 3- to 8-membered cycloalkoxy-C₁-C₄alkyl, hydroxy-C₂-C₇alkyl, C₁-C₄alkylthio-C₁-C₄alkyl, C₁-C₇alkanesulfonyl-C₁-C₄alkyl, thiazolylthio-C₁-C₄alkyl, thiazolinylthio-C₁-C₄-alkyl, imidazolylthio-C₁-C₄alkyl, optionally N-oxidised pyridylthio-C₁-C₄alkyl, pyrimidinylthio-C₁-C₄alkyl, optionally partially hydrogenated pyridyl- or N-oxidopyridyl-C₁-C₄alkyl, amino-C₁-C₄alkyl, C₁-C₄alkylamino-C₁-C₄alkyl, N,N-di-C₁-C₄-alkylamino-C₁-C₄alkyl, N-C₁-C₄alkanoylamino-C₁-C₄alkyl, C₁-C₄alkanesulfonylamino-C₁-C₄alkyl, trifluoro-C₁-C₇alkanesulfonylamino-C₁-C₄alkyl, pyrrolidino-C₁-C₄alkyl, piperidino-C₁-C₄alkyl, piperazino-C₁-C₄alkyl, N'-C₁-C₄alkylpiper-azino-C₁-C₄alkyl, N'-C₂-C₇alkanoylpiperazino-C₁-C₄alkyl, morpholino-C₁-C₄alkyl, thiomorpholino-C₁-C₄alkyl, S-oxothiomorpholino-C₁-C₄alkyl, S,S-dioxothiomorpholino-C₁-C₄alkyl, cyano-C₁-C₄alkyl, carboxy-C₁-C₄alkyl, C₁-C₄alkoxycarbonyl-C₁-C₄alkyl, carbamoyl-C₁-C₇alkyl, N-mono- or N,N-di-C₁-C₄alkylcarbamoyl-C₁-C₄-alkyl, 3- to 8-membered cycloalkyl; phenyl or naphthyl each unsubstituted or mono-, di- or tri-substituted by C₁-C₄alkyl, C₁-C₄alkoxy, hydroxy, C₁-C₄alkylamino, di-C₁-C₄alkylamino, halogen and/or by trifluoromethyl; hydroxy, C₁-C₄alkoxy, 3- to 8-membered cycloalkoxy, C₁-C₄alkoxy-C₂-C₄alkoxy, 3- to 8-membered cycloalkoxy-C₁-C₄alkoxy, hydroxy-C₂-C₇alkoxy; phenyl-C₁-C₄alkoxy or naphthyl-C₁-C₄-alkoxy each unsubstituted or mono-, di- or tri-substituted by C₁-C₄alkyl, C₁-C₄alkoxy, hydroxy, C₁-C₄alkylamino, di-C₁-C₄alkylamino, halogen and/or by trifluoromethyl; polyhalo-C₁-C₄alkoxy, C₁-C₄alkylthio-C₁-C₄alkoxy, C₁-C₇alkanesulfonyl-C₁-C₄alkoxy, optionally partially hydrogenated pyridyl- or N-oxidopyridyl-C₁-C₄alkoxy, thiazolyl-C₁-C₄alkoxy, morpholino-C₁-C₄alkoxy, thiazolylthio-C₁-C₄alkoxy, thiazolinylthio-C₁-C₄alkoxy, imidazolylthio-C₁-C₄alkoxy, optionally N-oxidised pyridylthio-C₁-C₄alkoxy, pyrimidinylthio-C₁-C₄alkoxy, amino-C₁-C₄alkoxy, C₁-C₄alkylamino-C₁-C₄alkoxy, N,N-di-C₁-C₄alkylamino-C₁-C₄alkoxy, C₁-C₇alkanoylamino-C₁-C₄alkoxy, C₁-C₇alkanesulfonylamino-C₁-C₄alkoxy, trifluoro-C₁-C₇alkanesulfonyl-C₁-C₄alkoxy, pyrrolidino-C₂-C₄alkoxy, piperidino-C₁-C₄alkoxy, piperazino-C₁-C₄alkyl, N'-C₁-C₄alkylpiperazino-C₁-C₄alkyl, N'-C₂-C₇-alkanoylpiperazino-C₁-C₄alkyl, morpholino-C₁-C₄alkyl, thiomorpholino-C₁-C₄alkyl, S-oxothiomorpholino-C₁-C₄alkyl, S,S-dioxothiomorpholino-C₁-C₄alkyl, cyano-C₁-C₄alkoxy, carboxy-C₁-C₄alkoxy, C₁-C₄alkoxycarbonyl-C₁-C₄alkoxy, carbamoyl-C₁-C₇alkoxy or N-mono- or N,N-di-C₁-C₄alkylcarbamoyl-C₁-C₄alkoxy, or, together with R₄, is C₁-C₄alkylenedioxy or a fused-on benzo or cyclohexeno ring,
R₄ together with R₃ is C₁-C₄alkylenedioxy or a fused-on benzo or cyclohexeno ring, or is hydrogen, C₁-C₇alkyl, hydroxy, C₁-C₄alkoxy or 3- to 8-membered cycloalkoxy,
X is methylene or hydroxymethylene,
R₅ is C₁-C₇alkyl or 3- to 8-membered cycloalkyl,
R₆ is amino, C₁-C₄alkylamino, di-C₁-C₄alkylamino or N-C₁-C₇alkanoylamino,
R₇ is C₁-C₇alkyl, C₂-C₇alkenyl, 3- to 8-membered cycloalkyl; or phenyl- or naphthyl-C₁-C₄alkyl each unsubstituted or mono-, di- or tri-substituted by C₁-C₄alkyl, C₁-C₄-alkoxy, hydroxy, C₁-C₄alkylamino, di-C₁-C₄alkylamino, halogen and/or by trifluoromethyl, and
R₈ is C₁-C₇alkyl, cycloalkyl, hydroxy-C₂-C₇alkyl, C₁-C₇alkanoyloxy-C₂-C₄alkyl, C₁-C₄alkoxy-C₁-C₄alkyl, C₂-C₇alkenyloxy-C₁-C₄alkyl, amino-C₁-C₄alkyl, N-C₁-C₄alkylamino-C₁-C₄alkyl, N,N-di-C₁-C₄alkylamino-C₁-C₄alkyl, N-C₁-C₄alkanoylamino-C₁-C₄alkyl, C₁-C₇alkanesulfonylamino-C₁-C₄alkyl, trifluoro-C₁-C₇alkanesulfonylamino-C₁-C₄alkyl, pyrrolidino-C₁-C₄alkyl, piperidino-C₁-C₄alkyl, hydroxypiperidino-C₁-C₄alkyl, C₁-C₄alkoxypiperidino-C₁-C₄alkyl, piperazino-C₁-C₄alkyl, N'-C₁-C₄alkylpiperazino-C₁-C₄alkyl, N'-C₂-C₇alkanoylpiperazino-C₁-C₄alkyl, morpholino-C₁-C₄alkyl, dimethylmorpholino-C₁-C₄alkyl, morpholinocarbonyl-C₁-C₄alkyl, thiomorpholino-C₁-C₄alkyl, S,S-dioxothiomorpholino-C₁-C₄alkyl, carboxy-C₁-C₄alkyl, C₁-C₄alkoxycarbonyl-C₁-C₄alkyl, carbamoyl-C₁-C₇alkyl, N-mono- or N,N-di-C₁-C₄alkylcarbamoyl-C₁-C₄alkyl, dicarboxy-C₁-C₄alkyl, di-C₁-C₄alkoxycarbonyl-C₁-C₄alkyl, dicarbamoyl-C₁-C₄alkyl, di-(N-mono- or N,N-di-C₁-C₄alkylcarbamoyl-C₁-C₄alkyl, carboxy-C₁-C₇(hydroxy)alkyl, C₁-C₄alkoxycarbonyl-C₁-C₇(hydroxy)alkyl or carbamoyl-C₁-C₇(hydroxy)alkyl; 5- or 6-membered carboxycycloalkyl-C₁-C₄alkyl, C₁-C₄alkoxycarbonylcycloalkyl-C₁-C₄alkyl, carbamoylcycloalkyl-C₁-C₄alkyl or N-mono- or N,N-di-C₁-C₄alkylcarbamoylcycloalkyl-C₁-C₄alkyl; cyano-C₁-C₄alkyl, C₁-C₇alkanesulfonyl-C₁-C₄alkyl, thiocarbamoyl-C₁-C₄alkyl, C₁-C₄alkylthiocarbamoyl-C₁-C₄alkyl, di-C₁-C₄alkylthiocarbamoyl-C₁-C₄alkyl, sulfamoyl-C₁-C₄alkyl, N-C₁-C₇alkylsulfamoyl-C₁-C₄alkyl, N,N-di-C₁-C₄alkylsulfamoyl-C₁-C₄alkyl, an optionally partially hydrogenated, oxo-substituted and/or benzo-fused 5-membered aza-, diaza-, triaza-, oxadiaza- or tetra-aza-aryl radical or 6-membered aza- or diaza-aryl radical each of which is bonded via a carbon atom, or an optionally partially hydrogenated, oxo-substituted and/or benzo-fused 5-membered aza-, diaza-, triaza-, oxadiaza- or tetra-aza-aryl-C₁-C₄alkyl radical or 6-membered aza- or diaza-aryl-C₁-C₄alkyl radical each of which is bonded via a carbon atom,
or a salt thereof.

2. A compound according to claim 1 of formula I wherein
R₁ is hydrogen,
R₂ is C₁-C₇alkyl, C₁-C₄alkoxy-C₁-C₄alkyl, C₁-C₄alkoxy-C₁-C₄alkoxy, C₁-C₄alkoxy-C₁-C₄alkoxy-C₁-C₄alkyl, unsubstituted or C₁-C₄alkyl-, C₁-C₄alkoxy-, hydroxy-, halo-, nitro- and/or amino-substituted phenyl-C₁-C₄alkoxy, pyridyl- or N-oxidopyridyl-C₁-C₄alkoxy, C₁-C₄alkylthio-C₁-C₄alkoxy, C₁-C₇alkanesulfonyl-C₁-C₄-alkoxy, C₁-C₇alkanoyl-C₂-C₄alkoxy, cyano-C₁-C₄alkoxy, carboxy-C₁-C₄alkoxy, C₁-C₄alkoxycarbonyl-C₁-C₄alkoxy, carbamoyl-C₁-C₇alkoxy or N-mono- or N,N-di-C₁-C₄alkylcarbamoyl-C₁-C₄alkoxy,
R₃ is hydrogen, C₁-C₇alkyl, hydroxy, C₁-C₄alkoxy or polyhalo-C₁-C₄alkoxy, or together with R₄ is C₁-C₄alkylenedioxy,
R₄ is hydrogen or together with R₃ is C₁-C₄alkylenedioxy
X is methylene or hydroxymethylene,
R₅ is C₁-C₇alkyl or 3- to 8-membered cycloalkyl,
R₆ is amino, C₁-C₄alkylamino, di-C₁-C₄alkylamino or N-C₁-C₇alkanoylamino,
R₇ is C₁-C₇alkyl, and
R₈ is C₁-C₇alkyl, hydroxy-C₂-C₇alkyl, C₁-C₇alkanoyloxy-C₂-C₄alkyl, C₁-C₄alkoxy-C₁-C₄alkyl, C₂-C₇alkenyloxy-C₁-C₄alkyl, amino-C₁-C₄alkyl, N-C₁-C₄alkanoylamino-C₁-C₄alkyl, N-C₁-C₄alkylamino-C₁-C₄alkyl, N,N-di-C₁-C₄alkylamino-C₁-C₄alkyl, piperidino-C₁-C₄alkyl, hydroxypiperidino-C₁-C₄alkyl, C₁-C₄alkoxypiperidino-C₁-C₄alkyl, morpholino-C₁-C₄alkyl, dimethylmorpholino-C₁-C₄alkyl, thiomorpholino-C₁-C₄alkyl, S,S-dioxothiomorpholino-C₁-C₄alkyl, carboxy-C₁-C₄alkyl, C₁-C₄-alkoxycarbonyl-C₁-C₄alkyl, carbamoyl-C₁-C₇alkyl, N-mono- or N,N-di-C₁-C₄alkylcarbamoyl-C₁-C₄alkyl, carboxy-C₁-C₇(hydroxy)alkyl, C₁-C₄alkoxycarbonyl-C₁-C₇(hydroxy)alkyl or carbamoyl-C₁-C₇(hydroxy)alkyl, 5- or 6-membered carboxycycloalkyl-C₁-C₄alkyl, 5- or 6-membered C₁-C₄alkoxycarbonylcycloalkyl-C₁-C₄alkyl, 5- or 6-membered carbamoylcycloalkyl-C₁-C₄alkyl, 5- or 6-membered N-mono- or N,N-di-C₁-C₄alkylcarbamoylcycloalkyl-C₁-C₄alkyl, cyano-C₁-C₄alkyl, C₁-C₇alkanesulfonyl-C₁-C₄alkyl, sulfamoyl-C₁-C₄alkyl, imidazolyl-C₁-C₄alkyl, oxopyrrolidinyl-C₁-C₄alkyl, benzimidazolyl-C₁-C₄alkyl, oxadiazolyl-C₁-C₄alkyl, pyridyl-C₁-C₄alkyl, oxopiperidinyl-C₁-C₄alkyl or quinolinyl-C₁-C₄alkyl, piperidin-4-yl-C₁-C₄alkyl or 1-C₁-C₇alkanoylpiperidin-4-yl-C₁-C₄alkyl,
or a salt thereof.

3. A compound according to claim 1 of formula I wherein
R₁ and R₄ are hydrogen,
R₂ is C₁-C₄alkoxy-C₁-C₄alkoxy or C₁-C₄alkoxy-C₁-C₄alkyl,
R₃ is C₁-C₄alkyl or C₁-C₄alkoxy,
R₆ is amino,
X is methylene,
R₅ and R₇ are branched C₁-C₄alkyl, and
R₈ is carbamoyl-C₁-C₄alkyl, N-C₁-C₄alkylcarbamoyl-C₁-C₄alkyl, N,N-di-C₁-C₄alkylcarbamoyl-C₁-C₄alkyl, morpholino-C₁-C₄alkyl, thiomorpholino-C₁-C₄alkyl, 4-(1-C₁-C₄alkanoylpiperidyl)-C₁-C₄alkyl or 2-oxopyrrolidinyl-C₁-C₄alkyl,
or a salt thereof.

4. A compound according to any one of claims 1 to 3 of formula I wherein at least one asymmetric carbon atom of the main chain has the stereochemical configuration shown in formula Ia 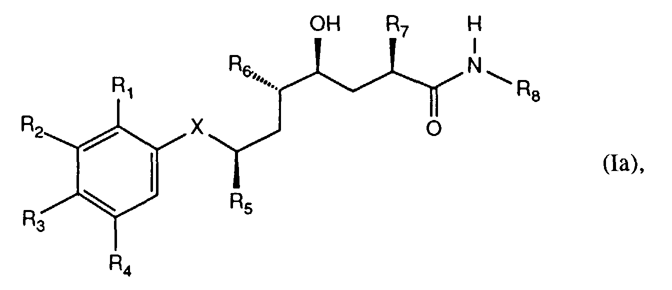 each of the variables being as defined in claims 1 to 3, or a pharmaceutically acceptable salt thereof.

5. A compound according to any one of claims 1 to 4 of formula I or Ia wherein X is methylene, or a pharmaceutically acceptable salt thereof.

6. 5(S)-Amino-4(S)-hydroxy-2(S),7(S)-diisopropyl-8-[4-methoxy-3-(3-methoxypropyloxy)-phenyl]-octanoic acid N-(2-carbamoyl-2,2-dimethyl-ethyl)amide according to claim 1, or a salt thereof.

7. 5(S)-Amino-4(S),8(R,S)-dihydroxy-2(S),7(S)-diisopropyl-8-[4-methoxy-3-(2-methoxymethoxyethyl)-phenyl]-octanoic acid (N-butyl)-amide;
5(S)-amino-4(S),8(R)-dihydroxy-2(S),7(S)-diisopropyl-8-[4-methoxy-3-(3-methoxypropyloxy)-phenyl]-octanoic acid N-(2-carbamoyl-2,2-dimethyl-ethyl)-amide;
5(S)-amino-4(S)-hydroxy-2(S),7(S)-diisopropyl-8-[4-methoxy-3-(3-methoxypropyloxy)-phenyl]-octanoic acid N-(2-carbamoyl-2,2-dimethyl-ethyl)-amide or
5(S)-amino-2(S),7(S)-diisopropyl-4(S)-hydroxy-8-[4-tert-butyl-3-(3-methoxypropoxy)-phenyl]-octanoic acid [N-2-(morpholin-4-yl)-ethyl]-amide
according to claim 1, or in each case a salt thereof.

8. 2(R,S)-Methyl-4(S)-hydroxy-5(S)-amino-7(S)-isopropyl-8-(p-tert-butyl-phenyl)-octanoic acid (N-butyl)amide;
2(R,S)-methyl-4(S)-hydroxy-5(S)-amino-7(S)-ethyl-8-(p-tert-butyl-phenyl)-octanoic acid (N-butyl)amide;
2(R,S)-methyl-4(S)-hydroxy-5(S)-amino-7(S)-methyl-8-biphenyl-octanoic acid (N-butyl)-amide;
2(R,S)-methyl-4(S)-hydroxy-5(S)-amino-7(S)-ethyl-8-(4-propyloxymethyl-naphth-2-yl)-octanoic acid (N-butyl)amide;
2(R)-methyl-4(S)-hydroxy-5(S)-amino-7(S)-isopropyl-8-(3-hydroxy-4-tert-butyl-phenyl)-octanoic acid (N-butyl)amide;
2(R,S)-methyl-4(S)-hydroxy-5(S)-amino-7(S)-isopropyl-8-(2-hydroxy-4-tert-butyl-phenyl)-octanoic acid (N-butyl)amide;
2(R)-methyl-4(S)-hydroxy-5(S)-amino-7(S)-isopropyl-8-(3-ethoxycarbonylmethoxy-4-tert-butyl-phenyl)-octanoic acid (N-butyl)amide;
2(R)-methyl-4(S)-hydroxy-5(S)-amino-7(S)-isopropyl-8-(3-allyloxy-4-tert-butyl-phenyl)-octanoic acid (N-butyl)amide;
2(R,S)-methyl-4(S)-hydroxy-5(S)-amino-7(S)-isopropyl-8-(3-methoxycarbonylallyloxy-4-tert-butyl-phenyl)-octanoic acid (N-butyl)amide;
2(R,S)-methyl-4(S)-hydroxy-5(S)-amino-7(S)-isopropyl-8-(3-methoxycarbonylmethoxy-4-tert-butyl-phenyl)-octanoic acid (N-butyl)amide;
2(R,S)-methyl-4(S)-hydroxy-5(S)-amino-7(S)-isopropyl-8-(3-carbamoyl-methoxy-4-tert-butyl-phenyl)-octanoic acid (N-butyl)amide;
2(R,S)-methyl-4(S)-hydroxy-5(S)-amino-7(S)-isopropyl-8-[3-(pyrid-2-yl-methoxy)-4-tert-butyl-phenyl]-octanoic acid (N-butyl)amide;
2(R,S)-methyl-4(S)-hydroxy-5(S)-amino-7(S)-isopropyl-8-[3-(pyrid-4-yl-methoxy)-4-tert-butyl-phenyl]-octanoic acid (N-butyl)amide;
2(R,S)-methyl-4(S)-hydroxy-5(S)-amino-7(S)-isopropyl-8-[3-(N-oxido-pyrid-2-yl-methoxy)-4-tert-butyl-phenyl]-octanoic acid (N-butyl)amide;
2(R,S)-methyl-4(S)-hydroxy-5(S)-amino-7(S)-isopropyl-8-[3-(2-ethoxycarbonylallyloxy)-4-tert-butyl-phenyl]-octanoic acid (N-butyl)amide;
2(R,S)-methyl-4(S)-hydroxy-5(S)-amino-7(S)-isopropyl-8-[3-(2-ethoxycarbonylpropyloxy)-4-tert-butyl-phenyl]-octanoic acid (N-butyl)amide;
2(R,S)-methyl-4(S)-hydroxy-5(S)-amino-7(S)-isopropyl-8-[3-(methylthio-methoxy)-4-tert-butyl-phenyl]-octanoic acid (N-butyl)amide;
2(R,S)-methyl-4(S)-hydroxy-5(S)-amino-7(S)-isopropyl-8-[3-(methylsulfonyl-methoxy)-4-tert-butyl-phenyl]-octanoic acid (N-butyl)amide;
2(R,S)-methyl-4(S)-hydroxy-5(S)-amino-7(S)-isopropyl-8-[3-(carboxy-methoxy)-4-tert-butyl-phenyl]-octanoic acid (N-butyl)amide;
2(R,S)-methyl-4(S)-hydroxy-5(S)-amino-7(S)-isopropyl-8-[3-(3,3-dimethyl-2-oxo-butyl-oxy)-4-tert-butyl-phenyl]-octanoic acid (N-butyl)amide;
2(R,S)-methyl-4(S)-hydroxy-5(S)-amino-7(S)-isopropyl-8-[3-(2-nitrobenzyloxy)-4-tert-butyl-phenyl]-octanoic acid (N-butyl)amide;
2(R,S)-methyl-4(S)-hydroxy-5(S)-amino-7(S)-isopropyl-8-[3-(2-aminobenzyloxy)-4-tert-butyl-phenyl]-octanoic acid (N-butyl)amide;
2(R,S)-methyl-4(S)-hydroxy-5(S)-amino-7(S)-isopropyl-8-[3-(3-chloro-2(R,S)-hydroxypropyloxy)-4-tert-butyl-phenyl]-octanoic acid (N-butyl)amide;
2(R,S)-methyl-4(S)-hydroxy-5(S)-amino-7(S)-isopropyl-8-[3-(3-methylthio-2(S,R)-hydroxypropyloxy)-4-tert-butyl-phenyl]-octanoic acid (N-butyl)amide;
2(R,S)-methyl-4(S)-hydroxy-5(S)-amino-7(S)-isopropyl-8-[3-(3-methylsulfonyl-2(S,R)-hydroxypropyloxy)-4-tert-butyl-phenyl]-octanoic acid (N-butyl)amide;
2(R)-methyl-4(S)-hydroxy-5(S)-amino-7(S)-isopropyl-8-[3-(methylsulfonyl-methoxy)-4-tert-butyl-phenyl]-octanoic acid (N-3-morpholino-propyl)amide;
2(R)-methyl-4(S)-hydroxy-5(S)-amino-7(S)-isopropyl-8-(3-methoxycarbonylmethoxyphenyl)-octanoic acid (N-butyl)amide;
2(R)-methyl-4(S)-hydroxy-5(S)-amino-7(S)-isopropyl-8-[3-(methoxycarbonylmethoxy)-4-methoxy-phenyl]-octanoic acid (N-butyl)amide;
2(R)-methyl-4(S)-hydroxy-5(S)-amino-7(S)-isopropyl-8-[3-(N-methyl-carbamoyl-mcthoxy)-4-methoxy-phenyl]-octanoic acid (N-butyl)amide;
2(R)-methyl-4(S)-hydroxy-5(S)-amino-7(S)-isopropyl-8-[3-(3-methylsulfonyl-propyl-oxy)-4-methoxy-phenyl]-octanoic acid (N-butyl)amide;
2(R)-methyl-4(S)-hydroxy-5(S)-amino-7(S)-isopropyl-8-[3-(methylsulfonyl-methoxy)-4-methoxy-phenyl]-octanoic acid (N-butyl)amide;
2(R)-methyl-4(S)-hydroxy-5(S)-amino-7(S)-isopropyl-8-[3-(3-methoxy-propyloxy)-4-methoxy-phenyl]-octanoic acid (N-butyl)amide;
2(R)-methyl-4(S)-hydroxy-5(S)-amino-7(S)-isopropyl-8-[3-(2-methoxy-ethoxy)-4-methoxy-phenyl]-octanoic acid (N-butyl)amide;
2(R)-methyl-4(S)-hydroxy-5(S)-amino-7(S)-isopropyl-8-[3-(3-hydroxy-propyloxy)-4-methoxy-phenyl]-octanoic acid (N-butyl)amide;
2(R)-methyl-4(S)-hydroxy-5(S)-amino-7(S)-isopropyl-8-[3-(carbamoylmethoxy)-4-methoxy-phenyl]-octanoic acid (N-butyl)amide;
2(R)-methyl-4(S)-hydroxy-5(S)-amino-7(S)-isopropyl-8-(3-cyanomethoxy-4-methoxy-phenyl)-octanoic acid (N-butyl)amide;
2(R)-methyl-4(S)-hydroxy-5(S)-amino-7(S)-isopropyl-8-[3-(4-methoxy-butoxy)-4-methoxy-phenyl]-octanoic acid (N-butyl)amide;
2(R)-methyl-4(S)-hydroxy-5(S)-amino-7(S)-isopropyl-8-[3-(2-ethoxy-ethoxy)-4-methoxy-phenyl]-octanoic acid (N-butyl)amide;
2(R)-methyl-4(S)-hydroxy-5(S)-amino-7(S)-isopropyl-8-{3-[2-(2-methoxy-ethoxy)-ethoxy]-4-methoxy-phenyl}-octanoic acid (N-butyl)-amide;
2(R)-methyl-4(S)-hydroxy-5(S)-amino-7(S)-isopropyl-8-(3-pentyloxy-4-methoxy-phenyl)-octanoic acid (N-butyl)amide;
2(R)-methyl-4(S)-hydroxy-5(S)-amino-7(S)-isopropyl-8-(3-benzyloxy-4-methoxy-phenyl)-octanoic acid (N-butyl)amide;
2(R)-methyl-4(S)-hydroxy-5(S)-amino-7(S)-isopropyl-8-[3-(3-ethoxy-propyloxy)-4-methoxy-phenyl]-octanoic acid (N-butyl)amide;
2(R)-methyl-4(S)-hydroxy-5(S)-amino-7(S)-isopropyl-8-[3-(pyrid-4-ylmethoxy)-4-methoxy-phenyl]-octanoic acid (N-butyl)amide;
2(R,S)-methyl-4(S)-hydroxy-5(S)-amino-7(S)-isopropyl-8-(2-ethoxycarbonylmethoxy-4-tert-butyl-phenyl)-octanoic acid (N-butyl)amide;
2(R,S)-methyl-4(S)-hydroxy-5(S)-amino-7(S)-isopropyl-8-(2-ethoxycarbonyl-4-tert-butyl-phenyl)-octanoic acid (N-butyl)amide;
2(R)-methyl-4(S)-hydroxy-5(S)-amino-7(S)-isopropyl-8-[3-(3-methoxy-propyloxy)-4,5-ethylenedioxy-phenyl]-octanoic acid (N-butyl)amide;
5(S)-amino-4(S)-hydroxy-2(S),7(S)-diisopropyl-8-[4-(3-hydroxypropyloxy)-3-(3-methoxy-propyloxy)-phenyl]-octanoic acid [N-(2-carbamoyl-2,2-dimethyl-ethyl)]-amide;
5(S)-amino-4(S)-hydroxy-2(S),7(S)-diisopropyl-8-[4-isopropyl-3-(3-methoxypropyloxy)-phenyl]-octanoic acid [N-(2-carbamoyl-2,2-dimethyl-ethyl)]-amide;
5(S)-amino-4(S)-hydroxy-2(S),7(S)-diisopropyl-8-[4-tert-butyl-3-(3-methoxy-propyl-oxy)-phenyl]-octanoic acid [N-(2-carbamoyl-2,2-dimethyl-ethyl)]-amide;
5(S)-amino-4(S)-hydroxy-2(S),7(S)-diisopropyl-8-[4-(3-methylsulfonyl-propyloxy)-3-(3-methoxy-propyloxy)-phenyl]-octanoic acid (N-2-morpholinoethyl)amide;
5(S)-amino-4(S)-hydroxy-2(S),7(S)-diisopropyl-8-[4-(3-methylsulfonyl-propyloxy)-3-(3-methoxy-propyloxy)-phenyl]-octanoic acid [N-(2-carbamoyl-2,2-dimethyl-ethyl)]-amide;
5(S)-amino-4(S)-hydroxy-2(S),7(S)-diisopropyl-8-[3,4-di-(3-hydroxypropyloxy)-phenyl]-octanoic acid (N-2-morpholinoethyl)amide;
5(S)-amino-4(S)-hydroxy-2(S),7(S)-diisopropyl-8-[3,4-di-(3-hydroxypropyloxy)-phenyl]-octanoic acid [N-(2-carbamoyl-2,2-dimethyl-ethyl)]-amide;
5(S)-amino-4(S)-hydroxy-2(S),7(S)-diisopropyl-8-[4-(3-N-methylcarbamoyl-propyl)-3-(3-methoxy-propyloxy)-phenyl]-octanoic acid (N-2-morpholinoethyl)amide;
5(S)-amino-4(S)-hydroxy-2(S),7(S)-diisopropyl-8-[4-(2-morpholinoethoxy)-3-(3-methoxy-propyloxy)-phenyl]-octanoic acid [N-(2-carbamoyl-2,2-dimethyl-ethyl)]-amide;
5(S)-amino-4(S)-hydroxy-2(S),7(S)-diisopropyl-8-[3-(3-methoxypropyloxy)-4,5-ethylene-dioxy-phenyl]-octanoic acid (N-2-morpholinoethyl)amide;
5(S)-amino-4(S)-hydroxy-2(S),7(S)-diisopropyl-8-[3-(3-methoxypropyloxy)-4,5-ethylene-dioxy-phenyl]-octanoic acid [N-(2-carbamoyl-2,2-dimethyl-ethyl)]-amide;
5(S)-amino-4(S)-hydroxy-2(S),7(S)-diisopropyl-8-[3-(3-methoxy-propyloxy)-4,5-methylenedioxy-phenyl]-octanoic acid (N-2-morpholinoethyl)amide;
5(S)-amino-4(S)-hydroxy-2(S),7(S)-diisopropyl-8-[3-(3-methoxypropyloxy)-4,5-methylenedioxy-phenyl]-octanoic acid [N-(2-carbamoyl-2,2-dimethyl-ethyl)]-amide;
5(S)-amino-4(S)-hydroxy-2(S),7(S)-diisopropyl-8-[4-methoxy-3-(3-methoxypropyloxy)-phenyl]-octanoic acid [N-(2-carbamoyl-2,2-ethylene-ethyl)]-amide;
5(S)-amino-4(S)-hydroxy-2(S),7(S)-diisopropyl-8-[4-methoxy-3-(3-methoxy-propyloxy)-phenyl]-octanoic acid [N-(3(S)-2-oxo-pyrrolidin-3-yl-methyl)]-amide;
5(S)-amino-4(S)-hydroxy-7(S)-isopropyl-2(R)-methyl-8-[4-methoxy-3-(4-methoxy-but-2-enoxy)-phenyl]-octanoic acid (N-butyl)-amide;
5(S)-amino-4(S)-hydroxy-7(S)-isopropyl-2(R)-methyl-8-[4-hydroxy-3-(3-methoxypropyloxy)-phenyl]-octanoic acid (N-butyl)amide;
5(S)-amino-4(S)-hydroxy-7(S)-isopropyl-2(R)-methyl-8-[4-benzyloxy-3-(3-methoxypropyloxy)-phenyl]-octanoic acid (N-butyl)amide;
5(S)-amino-4(S)-hydroxy-7(S)-isopropyl-2(R)-methyl-8-[3,4-di-(3-methoxypropyloxy)-phenyl]-octanoic acid (N-butyl)amide;
5(S)-amino-4(S)-hydroxy-7(S)-isopropyl-2(R)-methyl-8-[4-(2,2,2-trifluoroethoxy)-3-(3-methoxypropyloxy)-phenyl]-octanoic acid (N-butyl)amide;
5(S)-amino-4(S)-hydroxy-7(S)-isopropyl-2(R)-methyl-8-[4-(3-hydroxy-propyloxy)-3-(3-methoxypropyloxy)-phenyl]-octanoic acid (N-butyl)amide;
5(S)-amino-4(S)-hydroxy-7(S)-isopropyl-2(R)-methyl-8-[4-(2-amino-ethoxy)-3-(3-methoxypropyloxy)-phenyl]-octanoic acid (N-butyl)amide;
5(S)-amino-4(S)-hydroxy-7(S)-isopropyl-2(R)-methyl-8-[4-(5-amino-pentyloxy)-3-(3-methoxypropyloxy)-phenyl]-octanoic acid (N-butyl)amide;
5(S)-amino-4(S)-hydroxy-7(S)-isopropyl-2(R)-methyl-8-[4-(4-amino-butyloxy)-3-(3-methoxypropyloxy)-phenyl]-octanoic acid (N-butyl)amide;
5(S)-amino-4(S)-hydroxy-7(S)-isopropyl-2(R)-methyl-8-[4-(4-N,N-dimethylamino-butyloxy)-3-(3-methoxypropyloxy)-phenyl]-octanoic acid (N-butyl)amide;
5(S)-amino-4(S)-hydroxy-7(S)-isopropyl-2(R)-methyl-8-{4-[4-N-(trifluoromethane-sulfonylaminobutyloxy)-3-(3-methoxypropyloxy)-phenyl]}-octanoic acid (N-butyl)amide;
5(S)-amino-4(S)-hydroxy-7(S)-isopropyl-2(R)-methyl-8-[4-carboxymethoxy-3-(3-methoxypropyloxy)-phenyl]-octanoic acid (N-butyl)amide;
5(S)-amino-4(S)-hydroxy-7(S)-isopropyl-2(R)-methyl-8-[4-(3-ethoxycarbonyl-propyloxy)-3-(3-methoxy-propyloxy)-phenyl]-octanoic acid (N-butyl)amide;
5(S)-amino-4(S)-hydroxy-7(S)-isopropyl-2(R)-methyl-8-[4-(3-carboxy-propyloxy)-3-(3-methoxypropyloxy)-phenyl]-octanoic acid (N-butyl)amide;
5(S)-amino-4(S)-hydroxy-7(S)-isopropyl-2(R)-methyl-8-[4-(4-methoxycarbonylbutyloxy)-3-(3-methoxypropyloxy)-phenyl]-octanoic acid (N-butyl)amide;
5(S)-amino-4(S)-hydroxy-7(S)-isopropyl-2(R)-methyl-8-[4-(4-carboxy-butyloxy)-3-(3-methoxypropyloxy)-phenyl]-octanoic acid (N-butyl)amide;
5(S)-amino-4(S)-hydroxy-2(S),7(S)-diisopropyl-8-[4-methoxy-3-(3-methoxypropyloxy)-phenyl]-octanoic acid (N-butyl)amide;
5(S)-amino-4(S)-hydroxy-2(S),7(S)-diisopropyl-8-[4-methoxy-3-(2-methoxymethoxyethyl)-phenyl]-octanoic acid (N-butyl)amide;
5(S)-amino-4(S)-hydroxy-2(S),7(S)-diisopropyl-8-[4-(3-hydroxypropyloxy)-3-(3-methoxypropyloxy)-phenyl]-octanoic acid N-(2-morpholinoethyl)amide;
5(S)-amino-4(S)-hydroxy-7(S)-isopropyl-2(R)-methyl-8-[4-methoxy-3-(3-methoxypropyloxy)-phenyl]-octanoic acid N-[2-(4-hydroxypiperidin-1-yl)ethyl]amide dihydrochloride;
5(S)-amino-4(S)-hydroxy-7(S)-isopropyl-2(R)-methyl-8-[4-methoxy-3-(3-methoxypropyloxy)-phenyl]-octanoic acid N-[2-(trans-2,6-dimethyl-morpholino)ethyl]amide;
5(S)-amino-4(S)-hydroxy-7(S)-isopropyl-2(R)-methyl-8-[4-methoxy-3-(3-methoxypropyloxy)-phenyl]-octanoic acid N-[2-(cis-2,6-dimethyl-morpholino)ethyl]amide;
5(S)-amino-4(S)-hydroxy-7(S)-isopropyl-2(R)-methyl-8-[4-methoxy-3-(3-methoxypropyloxy)-phenyl]-octanoic acid N-(2-piperidinoethyl)amide;
5(S)-amino-4(S)-hydroxy-7(S)-isopropyl-2(R)-methyl-8-[4-methoxy-3-(3-methoxypropyloxy)-phenyl]-octanoic acid N-[2-(4-methoxypiperidino)-ethyl]-amide;
5(S)-amino-4(S)-hydroxy-7(S)-isopropyl-2(R)-methyl-8-[4-methoxy-3-(3-methoxypropyloxy)-phenyl]-octanoic acid N-(2-thiomorpholinoethyl)amide;
5(S)-amino-4(S)-hydroxy-7(S)-isopropyl-2(R)-methyl-8-[4-methoxy-3-(3-methoxypropyloxy)-phenyl]-octanoic acid [N-(3-hydroxypropyl)]amide;
5(S)-amino-4(S)-hydroxy-7(S)-isopropyl-2(R)-methyl-8-[4-methoxy-3-(3-methoxypropyloxy)-phenyl]-octanoic acid [N-(4-acetoxybutyl)]amide;
5(S)-amino-4(S)-hydroxy-7(S)-isopropyl-2(R)-methyl-8-[4-methoxy-3-(3-methoxypropyloxy)-phenyl]-octanoic acid [N-(3-cyanopropyl)]amide;
5(S)-amino-4(S)-hydroxy-7(S)-isopropyl-2(R)-methyl-8-[4-methoxy-3-(3-methoxypropyloxy)-phenyl]-octanoic acid [N-(3-methoxypropyl)]amide;
5(S)-amino-4(S)-hydroxy-7(S)-isopropyl-2(R)-methyl-8-[4-methoxy-3-(3-methoxypropyloxy)-phenyl]-octanoic acid [N-(2-acetylamino-ethyl)]amide;
5(S)-amino-4(S)-hydroxy-7(S)-isopropyl-2(R)-methyl-8-[4-methoxy-3-(3-methoxypropyloxy)-phenyl]-octanoic acid {N-[2-(2-pyridyl)-ethyl]}-amide;
5(S)-amino-4(S)-hydroxy-7(S)-isopropyl-2(R)-methyl-8-[4-methoxy-3-(3-methoxypropyloxy)-phenyl]-octanoic acid N-[2-(N'-oxomorpholino)ethyl]-amide;
5(S)-amino-4(S)-hydroxy-7(S)-isopropyl-2(R)-methyl-8-[4-methoxy-3-(3-methoxypropyloxy)-phenyl]-octanoic acid {N-[3-(tert-butylsulfonyl)-propyl]}-amide;
5(S)-amino-4(S)-hydroxy-7(S)-isopropyl-2(R)-methyl-8-[4-methoxy-3-(3-methoxypropyloxy)-phenyl]-octanoic acid {N-[3-(ethylsulfonyl)-propyl]}-amide;
5(S)-amino-4(S)-hydroxy-7(S)-isopropyl-2(R)-methyl-8-[4-methoxy-3-(3-methoxypropyloxy)-phenyl]-octanoic acid {N-[2-(ethylsulfonyl)-ethyl]}-amide;
5(S)-amino-4(S)-hydroxy-7(S)-isopropyl-2(R)-methyl-8-[4-methoxy-3-(3-methoxypropyloxy)-phenyl]-octanoic acid {N-[2-(N-butylsulfamoyl)-ethyl]}-amide;
5(S)-amino-4(S)-hydroxy-7(S)-isopropyl-2(R)-methyl-8-[4-methoxy-3-(3-methoxypropyloxy)-phenyl}-octanoic acid {N-[2-(N,N-dimethylsulfamoyl)-ethyl]}-amide;
5(S)-amino-4(S)-hydroxy-7(S)-isopropyl-2(R)-methyl-8-[4-methoxy-3-(3-methoxypropyloxy)-phenyl]-octanoic acid {N-[3-(1H-tetrazol-5-yl)-propyl]}-amide;
5(S)-amino-4(S)-hydroxy-7(S)-isopropyl-2(R)-methyl-8-[4-methoxy-3-(3-methoxypropyloxy)-phenyl]-octanoic acid {N-[3-(1H-imidazol-5-yl)-propyl]}-amide;
5(S)-amino-4(S)-hydroxy-7(S)-isopropyl-2(R)-methyl-8-[4-methoxy-3-(3-methoxypropyloxy)-phenyl]-octanoic acid {N-[3-(3-methyl-1,2,4-oxadiazol-5-yl)-propyl]}-amide;
5(S)-amino-4(S)-hydroxy-7(S)-isopropyl-2(R)-methyl-8-[4-methoxy-3-(3-methoxypropyloxy)-phenyl]-octanoic acid [N-(3-aminopropyl)]-amide;
5(S)-amino-4(S)-hydroxy-7(S)-isopropyl-2(R)-methyl-8-[4-methoxy-3-(3-methoxypropyloxy)-phenyl]-octanoic acid [N-[2-dimethylamino-ethyl)]-amide;
5(S)-amino-4(S)-hydroxy-7(S)-isopropyl-2(R)-methyl-8-[4-methoxy-3-(3-methoxypropyloxy)-phenyl]-octanoic acid N-(2-morpholinoethyl)amide;
5(S)-amino-4(S)-hydroxy-7(S)-isopropyl-2(R)-methyl-8-[4-methoxy-3-(3-methoxypropyloxy)-phenyl]-octanoic acid N-(3-morpholinopropyl)amide;
5(S)-amino-4(S)-hydroxy-7(S)-isopropyl-2(R)-methyl-8-[4-methoxy-3-(3-methoxypropyloxy)-phenyl]-octanoic acid N-[2-(1,1-dioxothiomorpholino)ethyl]amide;
5(S)-amino-4(S)-hydroxy-7(S)-isopropyl-2(R)-methyl-8-[4-methoxy-3-(3-methoxypropyloxy)-phenyl]-octanoic acid N-(2-ethoxycarbonylethyl)amide;
5(S)-amino-4(S)-hydroxy-7(S)-isopropyl-2(R)-methyl-8-[4-methoxy-3-(3-methoxypropyloxy)-phenyl]-octanoic acid [N-(2-carboxy-ethyl)]-amide;
5(S)-amino-4(S)-hydroxy-7(S)-isopropyl-2(R)-methyl-8-[4-methoxy-3-(3-methoxypropyloxy)-phenyl]-octanoic acid [N-(3-methoxycarbonyl-ethyl)]-amide;
5(S)-amino-4(S)-hydroxy-7(S)-isopropyl-2(R)-methyl-8-[4-methoxy-3-(3-methoxypropyloxy)-phenyl]-octanoic acid [N-(3-carboxypropyl)]-amide;
5(S)-amino-4(S)-hydroxy-7(S)-isopropyl-2(R)-methyl-8-[4-methoxy-3-(3-methoxypropyloxy)-phenyl]-octanoic acid [N-(2-carbamoylethyl)]-amide;
5(S)-amino-4(S)-hydroxy-7(S)-isopropyl-2(R)-methyl-8-[4-methoxy-3-(3-methoxypropyloxy)-phenyl]-octanoic acid [N-(4-carbamoylbutyl)]-amide;
5(S)-amino-4(S)-hydroxy-7(S)-isopropyl-2(R)-methyl-8-[4-methoxy-3-(3-methoxypropyloxy)-phenyl]-octanoic acid N-{3-[N-(2-methoxyethyl)carbamoyl]propyl}amide;
5(S)-amino-4(S)-hydroxy-7(S)-isopropyl-2(R)-methyl-8-[4-methoxy-3-(3-methoxypropyloxy)-phenyl]-octanoic acid N-(4-morpholino-4-oxo-butyl)amide;
5(S)-amino-4(S)-hydroxy-7(S)-isopropyl-2(R)-methyl-8-[4-methoxy-3-(3-methoxypropyloxy)-phenyl]-octanoic acid [N-(2-carbamoyl-2,2-dimethyl-ethyl)]-amide;
5(S)-amino-4(S)-hydroxy-2(S),7(S)-diisopropyl-8-[4-methoxy-3-(3-methoxypropyloxy)-phenyl]-octanoic acid N-(1,1-dimethyl-2-morpholino-ethyl)amide;
5(S)-amino-4(S)-hydroxy-2(S),7(S)-diisopropyl-8-[4-methoxy-3-(3-methoxypropyloxy)-phenyl]-octanoic acid N-[1(R,S)-methyl-2-morpholino-ethyl]amide;
5(S)-amino-4(S)-hydroxy-2(S),7(S)-diisopropyl-8-[4-methoxy-3-(3-methoxypropyloxy)-phenyl]-octanoic acid [N-(1-carbamoyl-1-methyl-ethyl)]-amide;
5(S)-amino-4(S)-hydroxy-2(S),7(S)-diisopropyl-8-[4-methoxy-3-(3-methoxypropyloxy)-phenyl]-octanoic acid [N-(1-carbamoyl-methyl)]-amide;
5(S)-amino-4(S)-hydroxy-2(S),7(S)-diisopropyl-8-[4-methoxy-3-(3-methoxypropyloxy)-phenyl]-octanoic acid [N-(2-carbamoyl-ethyl)]-amide;
5(S)-amino-4(S)-hydroxy-2(S),7(S)-diisopropyl-8-[4-methoxy-3-(3-methoxypropyloxy)-phenyl]-octanoic acid N-[2-(N-methyl-carbamoyl)ethyl]amide;
5(S)-amino-4(S)-hydroxy-2(S),7(S)-diisopropyl-8-[4-methoxy-3-(3-methoxypropyloxy)-phenyl]-octanoic acid N-(3-morpholino-3-oxo-propyl)amide;
5(S)-amino-4(S)-hydroxy-2(S),7(S)-diisopropyl-8-[4-methoxy-3-(3-methoxypropyloxy)-phenyl]-octanoic acid [N-[2-(N,N-dimethyl-carbamoyl)-1(R,S)-methyl-ethyl]}-amide;
5(S)-amino-4(S)-hydroxy-2(S),7(S)-diisopropyl-8-[4-methoxy-3-(3-methoxypropyloxy)-phenyl]-octanoic acid [N-(2-carbamoyl-1(R)-isopropyl-ethyl)]-amide;
5(S)-amino-4(S)-hydroxy-2(S),7(S)-diisopropyl-8-[4-methoxy-3-(3-methoxypropyloxy)-phenyl]-octanoic acid {N-[2-(N-methylcarbamoyl)-1(R)-isopropyl-ethyl]}-amide;
5(S)-amino-4(S)-hydroxy-2(S),7(S)-diisopropyl-8-[4-methoxy-3-(3-methoxypropyloxy)-phenyl]-octanoic acid {N-[2-(N,N-dimethylcarbamoyl)-1(R)-isopropyl-ethyl]}-amide;
5(S)-amino-4(S)-hydroxy-2(S),7(S)-diisopropyl-8-[4-methoxy-3-(3-methoxypropyloxy)-phenyl]-octanoic acid [N-(1(S)-carbamoyl-2-hydroxy-ethyl)]-amide;
5(S)-amino-4(S)-hydroxy-2(S),7(S)-diisopropyl-8-[4-methoxy-3-(3-methoxypropyloxy)-phenyl]-octanoic acid [N-(1(S),2-dicarbamoyl-ethyl)]-amide;
5(S)-amino-4(S)-hydroxy-2(S),7(S)-diisopropyl-8-[4-methoxy-3-(3-methoxypropyloxy)-phenyl]-octanoic acid [N-(1(S),3-dicarbamoyl-propyl)]-amide;
5(S)-amino-4(S)-hydroxy-2(S),7(S)-diisopropyl-8-[4-methoxy-3-(3-methoxypropyloxy)-phenyl]-octanoic acid [N-(1(S)-carbamoyl-propyl)]-amide;
5(S)-amino-4(S)-hydroxy-2(S),7(S)-diisopropyl-8-[4-methoxy-3-(3-methoxypropyloxy)-phenyl]-octanoic acid [N-(1(S)-carbamoyl-2(S)-methyl-butyl)]-amide;
5(S)-amino-4(S)-hydroxy-2(S),7(S)-diisopropyl-8-[4-methoxy-3-(3-methoxypropyloxy)-phenyl]-octanoic acid N-[2(R,S)-carbamoyl-2(R,S)-methyl-ethyl]-amide;
5(S)-amino-4(S)-hydroxy-2(S),7(S)-diisopropyl-8-[4-methoxy-3-(3-methoxypropyloxy)-phenyl]-octanoic acid [N-(2-carbamoyl-1(S)-methyl-ethyl)]-amide;
5(S)-amino-4(S)-hydroxy-2(S),7(S)-diisopropyl-8-[4-methoxy-3-(3-methoxypropyloxy)-phenyl]-octanoic acid [N-(2-carbamoyl-1(R)-methyl-ethyl)]-amide;
5(S)-amino-4(S)-hydroxy-2(S),7(S)-diisopropyl-8-[4-methoxy-3-(3-methoxypropyloxy)-phenyl]-octanoic acid N-[2(S)-carbamoyl-2(S)-methyl-ethyl]-amide;
5(S)-amino-4(S)-hydroxy-2(S),7(S)-diisopropyl-8-[4-methoxy-3-(3-methoxypropyloxy)-phenyl]-octanoic acid {N-[2(S)-(N-methyl-carbamoyl)-2(S)-methyl-ethyl]}-amide;
5(S)-amino-4(S)-hydroxy-2(S),7(S)-diisopropyl-8-[4-methoxy-3-(3-methoxypropyloxy)-phenyl]-octanoic acid [N-(2-carboxy-2,2-dimethyl-ethyl)]-amide;
5(S)-amino-4(S)-hydroxy-2(S),7(S)-diisopropyl-8-[4-methoxy-3-(3-methoxypropyloxy)-phenyl]-octanoic acid [N-(2-carboxy-2,2-diethyl-ethyl)]-amide;
5(S)-amino-4(S)-hydroxy-2(S),7(S)-diisopropyl-8-[4-methoxy-3-(3-methoxypropyloxy)-phenyl]-octanoic acid N-[(1-carboxy-cyclopentyl)-methyl]amide;
5(S)-amino-4(S)-hydroxy-2(S),7(S)-diisopropyl-8-[4-methoxy-3-(3-methoxypropyloxy)-phenyl]-octanoic acid {N-[2-(1H-tetrazol-5-yl)-ethyl]}-amide;
5(S)-amino-4(S)-hydroxy-2(S),7(S)-diisopropyl-8-[4-methoxy-3-(3-methoxypropyloxy)-phenyl]-octanoic acid N-[1(S)-(5-oxopyrrolidin-2-yl)methyl]-amide;
5(S)-amino-4(S)-hydroxy-2(S),7(S)-diisopropyl-8-[4-methoxy-3-(3-methoxypropyloxy)-phenyl]-octanoic acid N-[1(R)-(5-oxopyrrolidin-2-yl)methyl]-amide;
5(S)-amino-4(S)-hydroxy-2(S),7(S)-diisopropyl-8-[4-methoxy-3-(3-methoxypropyloxy)-phenyl]-octanoic acid N-[(morpholin-4-yl)carbonyl-methyl]amide;
5(S)-amino-4(S)-hydroxy-2(S),7(S)-diisopropyl-8-[4-methoxy-3-(3-methoxypropyloxy)-phenyl]-octanoic acid [N-(1(S)-carbamoyl-ethyl)]-amide;
5(S)-amino-4(S)-hydroxy-2(S),7(S)-diisopropyl-8-[4-methoxy-3-(3-methoxypropyloxy)-phenyl]-octanoic acid N-{1(S)-[(N-methyl)-carbamoyl]-ethyl}-amide;
5(S)-amino-4(S)-hydroxy-2(S),7(S)-diisopropyl-8-[4-methoxy-3-(3-methoxypropyloxy)-phenyl]-octanoic acid N-{1(S)-[(N,N-dimethyl)-carbamoyl]-ethyl}-amide;
5(S)-amino-4(S)-hydroxy-2(S),7(S)-diisopropyl-8-[4-methoxy-3-(3-methoxypropyloxy)-phenyl]-octanoic acid N-{1(S)-N-[(morpholin-4-yl)-carbonyl]ethyl}amide;
5(S)-amino-4(S)-hydroxy-2(S),7(S)-diisopropyl-8-[4-methoxy-3-(3-methoxypropyloxy)-phenyl]-octanoic acid N-[1(S)-carbamoyl-butyl]amide;
5(S)-amino-4(S)-hydroxy-2(S),7(S)-diisopropyl-8-[4-methoxy-3-(3-methoxypropyloxy)-phenyl]-octanoic acid N-[1(S)-carbamoyl-2-methyl-propyl]-amide;
5(S)-amino-4(S)-hydroxy-2(S),7(S)-diisopropyl-8-[4-methoxy-3-(3-methoxypropyloxy)-phenyl]-octanoic acid N-[1(S)-(N-methylcarbamoyl)-2-methyl-propyl]amide;
5(S)-amino-4(S)-hydroxy-2(S),7(S)-diisopropyl-8-[4-methoxy-3-(3-methoxypropyloxy)-phenyl]-octanoic acid N-[1(S)-(N,N-dimethylcarbamoyl)-2-methyl-propyl]amide;
5(S)-amino-4(S)-hydroxy-2(S),7(S)-diisopropyl-8-[4-methoxy-3-(3-methoxypropyloxy)-phenyl]-octanoic acid N-{1(S)-[(morpholin-4-yl)carbonyl]-2-methyl-propyl}amide;
5(S)-amino-4(S)-hydroxy-2(S),7(S)-diisopropyl-8-[4-methoxy-3-(3-methoxypropyloxy)-phenyl]-octanoic acid N-[2-(N-methylsulfamoyl)ethyl]amide;
5(S)-amino-4(S)-hydroxy-2(S),7(S)-diisopropyl-8-[4-methoxy-3-(3-methoxypropyloxy)-phenyl]-octanoic acid N-{2-[N-(morpholin-4-yl)-sulfonyl]ethyl}amide;
5(S)-amino-4(S)-hydroxy-2(S),7(S)-diisopropyl-8-[4-methoxy-3-(3-methoxypropyloxy)-phenyl]-octanoic acid N-[(N-acetyl-piperidin-4-yl)methyl]amide;
5(S)-amino-4(S)-hydroxy-2(S),7(S)-diisopropyl-8-[4-methoxy-3-(4-methoxy-butyl)-phenyl]-octanoic acid N-(2-carbamoyl-2,2-dimethyl-ethyl)amide;
5(S)-amino-4(S)-hydroxy-2(S),7(S)-diisopropyl-8-[4-methoxy-3-(3-methoxypropyloxy)-phenyl]-octanoic acid N-[2-(N,N-dimethylcarbamoyl)ethyl]amide;
5(S)-amino-4(S)-hydroxy-2(S),7(S)-diisopropyl-8-[4-methoxy-3-(4-methoxybutyl)-phenyl]-octanoic acid N-(2-morpholinoethyl)amide;
5(S)-amino-4(S)-hydroxy-2(S),7(S)-diisopropyl-8-[4-methoxy-3-(3-methoxy-propyloxy)-phenyl]-octanoic acid [N-(3(R)-2-oxo-pyrrolidin-3-yl-methyl)]-amide;
5(S)-amino-4(S)-hydroxy-2(S),7(S)-diisopropyl-8-[4-methoxy-3-(3-methoxy-propyloxy)-phenyl]-octanoic acid [N-(3(S)-2-oxo-piperidin-3-yl-methyl)]-amide;
5(S)-amino-4(S)-hydroxy-2(S),7(S)-diisopropyl-8-[4-methoxy-3-(3-methoxy-propyloxy)-phenyl]-octanoic acid [N-(3(R)-2-oxo-piperidin-3-yl-methyl)]-amide;
5(S)-amino-4(S)-hydroxy-2(S),7(S)-diisopropyl-8-[4-methoxy-3-(3-methoxy-propyloxy)-phenyl]-octanoic acid [N-(3-carbamoyl-3,3-dimethyl-propyl)]-amide;
5(S)-amino-4(S)-hydroxy-2(S),7(S)-diisopropyl-8-[4-methoxy-3-(4-methoxy-butyl)-phenyl]-octanoic acid [N-(5(S)-2-pyrrolidinon-5-yl-methyl)]-amide;
5(S)-amino-4(S)-hydroxy-2(S),7(S)-diisopropyl-8-[4-methoxy-3-(4-methoxy-butyl)-phenyl]-octanoic acid [N-(5(R)-2-pyrrolidinon-5-yl-methyl)]-amide;
5(S)-amino-4(S)-hydroxy-2(S),7(S)-diisopropyl-8-[4-methoxy-3-(3-methoxy-propyloxy)-phenyl]-octanoic acid [N-(6(S)-2-oxo-piperidin-6-yl-methyl)]-amide;
5(S)-amino-4(S)-hydroxy-2(S),7(S)-diisopropyl-8-[4-methoxy-3-(3-methoxy-propyloxy)-phenyl]-octanoic acid [N-(6(R)-2-oxo-piperidin-6-yl-methyl)]-amide;
5(S)-amino-4(S)-hydroxy-2(S),7(S)-diisopropyl-8-[4-methoxy-3-(3-methoxy-propyloxy)-phenyl]-octanoic acid [N-(2-thiazol-2-yl-ethyl)]-amide;
5(S)-amino-4(S)-hydroxy-2(S),7(S)-diisopropyl-8-[4-methoxy-3-(3-methoxy-propyloxy)-phenyl]-octanoic acid [N-(4(S)-2-oxazolidinon-4-yl-methyl)]-amide;
5(S)-amino-4(S)-hydroxy-2(S),7(S)-diisopropyl-8-[4-methoxy-3-(3-methoxy-propyloxy)-phenyl]-octanoic acid [N-(4(R)-2-oxazolidinon-4-yl-methyl)]-amide;
5(S)-amino-4(S)-hydroxy-2(S),7(S)-diisopropyl-8-[4-methoxy-3-(3-methoxy-propyloxy)-phenyl]-octanoic acid [N-(3(S)-2,5-dioxo-pyrrolidin-3-yl-methyl)]-amide;
5(S)-amino-4(S)-hydroxy-2(S),7(S)-diisopropyl-8-[4-methoxy-3-(3-methoxy-propyloxy)-phenyl]-octanoic acid [N-(3(R)-2,5-dioxo-pyrrolidin-3-yl-methyl)]-amide;
5(S)-amino-4(S)-hydroxy-2(S),7(S)-diisopropyl-8-[4-methoxy-3-(3-methoxy-propyloxy)-phenyl]-octanoic acid [N-(2,6-dioxo-piperidin-4-yl-methyl)]-amide;
5(S)-amino-4(S)-hydroxy-2(S),7(S)-diisopropyl-8-[4-methoxy-3-(3-methoxy-propyloxy)-phenyl]-octanoic acid [N-(4(S)-2-oxothiazolidin-4-yl-methyl)]-amide;
5(S)-amino-4(S)-hydroxy-2(S),7(S)-diisopropyl-8-[3-(3-methoxypropoxy)-4,5-ethylene-dioxy-phenyl]-octanoic acid N-(2-carbamoyl-2,2-dimethyl-ethyl)-amide;
5(S)-amino-4(S)-hydroxy-2(S),7(S)-diisopropyl-8-[4-methoxy-3-(3-methoxy-propyloxy)-phenyl]-octanoic acid [N-(4(R)-2-oxothiazolidin-4-yl-methyl)]-amide;
5(S)-amino-4(S)-hydroxy-2(S),7(S)-diisopropyl-8-[4-methoxy-3-(3-methoxy-propyloxy)-phenyl]-octanoic acid [N-(tetrahydro-2-pyrimidon-5-yl-methyl)]-amide;
5(S)-amino-4(S)-hydroxy-2(S),7(S)-diisopropyl-8-[4-methoxy-3-(3-methoxy-propyloxy)-phenyl]-octanoic acid [N-(N-acetyl-2-amino-2-methyl-propyl)]-amide;
5(S)-amino-4(S)-hydroxy-2(S),7(S)-diisopropyl-8-[4-methoxy-3-(3-methoxy-propyloxy)-phenyl]-octanoic acid [N-(N-formyl-2-amino-2-methyl-propyl)]-amide;
5(S)-amino-4(S)-hydroxy-2(S),7(S)-diisopropyl-8-[4-methoxy-3-(3-methoxy-propyloxy)-phenyl]-octanoic acid [N-(4-acetyl-piperazinyl-ethyl)]-amide;
5(S)-amino-4(S)-hydroxy-2(S),7(S)-diisopropyl-8-[4-methoxy-3-(3-methoxy-propyloxy)-phenyl]-octanoic acid [N-(2,4-imidazolinedion-5-yl-methyl)]-amide;
5(S)-amino-4(S)-hydroxy-2(S),7(S)-diisopropyl-8-[4-methoxy-3-(4-methoxy-butyl)-phenyl]-octanoic acid [N-(2-hydroxy-pyridin-6-yl-methyl)]-amide;
5(S)-amino-4(S)-hydroxy-2(S),7(S)-diisopropyl-8-[4-methoxy-3(3-methoxy-propyloxy)-phenyl]-octanoic acid [N-(2,2-dimethyl-2-sulfamoyl-ethyl)]-amide;
5(S)-amino-4(S)-hydroxy-2(S),7(S)-diisopropyl-8-[4-methoxy-3-(3-methoxy-propyloxy)-phenyl]-octanoic acid [N-(2,2-dimethyl-2-(N,N-dimethyl)-sulfamoyl-ethyl)]-amide hydrochloride;
5(S)-amino-4(S)-hydroxy-2(S),7(S)-diisopropyl-8-[4-methoxy-3-(3-methoxy-propyloxy)-phenyl]-octanoic acid [N-(2-oxo-piperidin-3(R)-yl)]-amide;
5(S)-amino-4(S)-hydroxy-2(S),7(S)-diisopropyl-8-[4-methoxy-3-(3-methoxy-propyloxy)-phcnyl]-octanoic acid [N-(2-oxo-piperidin-3(S)-yl)]-amide;
5(S)-amino-4(S)-hydroxy-2(S),7(S)-diisopropyl-8-[4-methoxy-3-(3-methoxy-propyloxy)-phenyl]-octanoic acid [N-(2-oxo-piperidin-4-yl)]-amide;
5(S)-amino-4(S)-hydroxy-2(S),7(S)-diisopropyl-8-[4-methoxy-3-(3-methoxy-propyloxy)-phenyl]-octanoic acid [N-(N-acetyl-piperidin-4-yl)]-amide;
5(S)-amino-4(S)-hydroxy-2(S),7(S)-diisopropyl-8-[4-methoxy-3-(4-methoxy-but-1-en-yl)-phenyl]-octanoic acid [N-(2-carbamoyl-2,2-dimethyl-ethyl)]-amide;
5(S)-amino-4(S)-hydroxy-2(S),7(S)-diisopropyl-8-[4-methoxy-3-(3-methoxypropyloxy)-phenyl]-octanoic acid N-(3-morpholinopropyl)amide;
5(S)-amino-4(S)-hydroxy-2(S),7(S)-diisopropyl-8-[4-methoxy-3-(3-methoxypropyloxy)-phenyl]-octanoic acid N-(2-morpholinoethyl)amide;
5(S)-amino-4(S)-hydroxy-2(S),7(S)-diisopropyl-8-[4-methoxy-3-(3-methoxypropyloxy)-phenyl]-octanoic acid {N-[2-(N-methyl-carbamoyl)-1(R,S)-methyl-ethyl]}-amide;
5(S)-amino-4(S)-hydroxy-2(S),7(S)-diisopropyl-8-[4-methoxy-3-(3-methoxypropyloxy)-phenyl]-octanoic acid N-(3-carbamoylpropyl)amide;
5(S)-amino-4(S)-hydroxy-2(S),7(S)-diisopropyl-8-[4-methoxy-3-(3-methoxypropyloxy)-phenyl]-octanoic acid {N-[2(R)-(N-methyl-carbamoyl)-2(R)-methyl-ethyl]}-amide;
5(S)-amino-4(S)-hydroxy-2(S),7(S)-diisopropyl-8-[4-methoxy-3-(3-methoxypropyloxy)-phenyl]-octanoic acid N-(2-thiomorpholinoethyl)amide;
5(S)-amino-4(S)-hydroxy-2(S),7(S)-diisopropyl-8-[4-methoxy-3-(3-methoxypropyloxy)-phenyl]-octanoic acid N-[2-(N,N-dimethyl-carbamoyl)ethyl]amide;
5(S)-amino-4(S)-hydroxy-2(S),7(S)-diisopropyl-8-[4-methoxy-3-(3-methoxypropyloxy)-phenyl]-octanoic acid N-(2-carbamoyl-1(R,S)-methyl-ethyl)amide;
5(S)-amino-4(S)-hydroxy-2(S),7(S)-diisopropyl-8-[4-methoxy-3-(3-methoxypropyloxy)-phenyl]-octanoic acid N-[2(R)-carbamoyl-2(R)-methyl-ethyl]-amide;
5(S)-amino-4(S)-hydroxy-2(S),7(S)-diisopropyl-8-[4-methoxy-3-(3-methoxypropyloxy)-phenyl]-octanoic acid N-(2-carbamoyl-2,2-dimethyl-ethyl)amide;
5(S)-amino-4(S)-hydroxy-2(S),7(S)-diisopropyl-8-[4-methoxy-3-(3-methoxypropyloxy)-phenyl]-octanoic acid N-[2-(N-acetyl)-piperidin-4-yl)ethyl]amide;
5(S)-amino-4(S)-hydroxy-2(S),7(S)-diisopropyl-8-[4-methoxy-3-(3-methoxypropyloxy)-phenyl]-octanoic acid {N-[(N,N-dimethyl)-carbamoyl-methyl]}-amide or
5(S)-amino-4(S)-hydroxy-2(S),7(S)-diisopropyl-8-[4-methoxy-3-(3-methoxypropyloxy)-phenyl]-octanoic acid N-[2(R,S)-(N-methylcarbamoyl)-2(R,S)-methyl-ethyl]-amide
according to claim 1, or in each case a salt thereof.

9. A compound according to any one of claims 1 to 8 for use in a method for the therapeutic treatment of the human or animal body.

10. A pharmaceutical composition comprising as pharmaceutical active ingredient a compound according to any one of claims 1 to 8 in free form or in pharmaceutically acceptable salt form, together with customary pharmaceutical excipients.

11. A process for the preparation of a δ-amino-γ-hydroxy-ω-aryl-alkanoic acid amide of formula (I) in claim 1, or a salt thereof, which comprises
a) reacting a compound of formula II 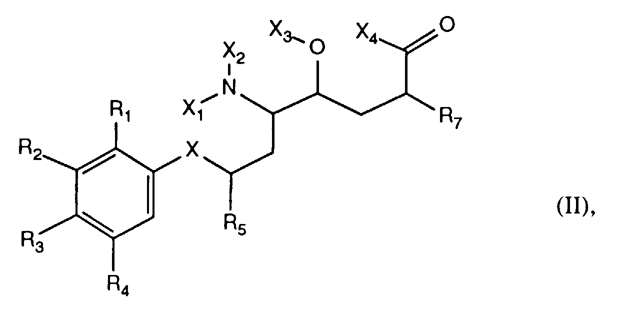 wherein
X₁ is C₁-C₄alkyl, C₁-C₄alkanoyl or an amino-protecting group,
X₂ is hydrogen or together with X₃ is a bivalent protecting group,
X₃ is hydrogen or a hydroxy-protecting group or together with X₂ is a bivalent protecting group or together with X₄ is a direct bond, and
X₄ is free or reactively etherified or esterified hydroxy or together with X₃ is a direct bond, and
R₁, R₂, R₃, R₄, X, R₅, R₆ and R₇ are as defined for formula I, with an amine of formula III
H₂N―R₈ (III),
wherein
R₈ has one of the meanings given for formula I,
with the formation of an amide bond, and removing any protecting groups present, or
b) in a carboxylic acid amide of formula IV 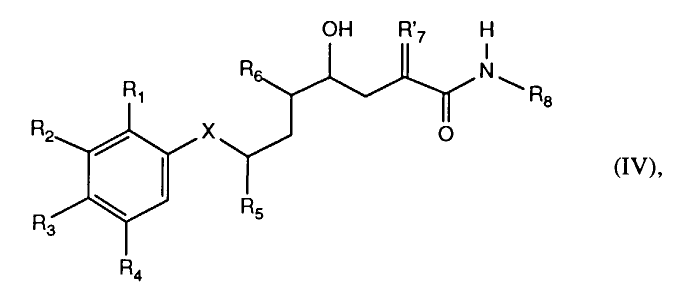 wherein R₁, R₂, R₃, R₄, X, R₅, R₆, R₇ and R₈ are as defined for formula I and
R'₇ is a C₁-C₄alkylidene or aryl-C₁-C₄alkylidene group corresponding to the C₁-C₄alkyl or aryl-C₁-C₄alkyl group R₇,
free functional groups being present, if desired, in protected form, or in a salt thereof, reducing the group R'₇ to R₇ by treatment with a hydrogenating agent, or
c) for the preparation of a compound of formula I wherein R₆ is amino, in a 5-azidocarboxylic acid derivative of formula V 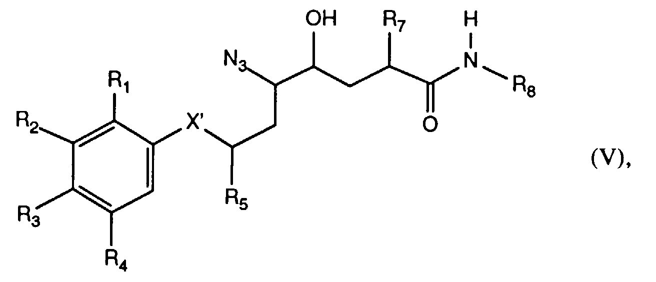 wherein
R₁, R₂, R₃, R₄, R₅, R₇ and R₈ are as defined for formula I, X' is methylene or free or esterified or etherified hydroxymethyl, and free functional groups are present, if desired, in protected form,
or in a salt thereof, reducing the azido group to amino, if desired with the freeing of hydroxymethyl X or the reduction of X' to methylene X, and removing any protecting groups present,
and, if desired, converting a compound of formula I having at least one salt-forming group obtainable by one of the above-mentioned processes a) to c) into its salt, or converting an obtainable salt into the free compound or into a different salt and/or separating mixtures of isomers that may be obtainable and/or converting a compound of formula I according to the invention into a different compound of formula I according to the invention.

12. A compound of formula II 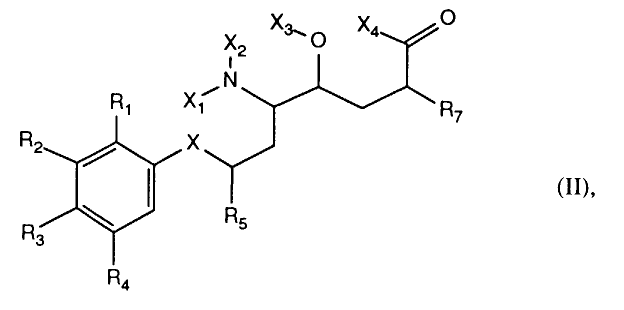 wherein
R₁ is hydrogen, hydroxy, C₁-C₇alkoxy, 3- to 8-membered cycloalkoxy, C₁-C₄alkoxy-C₂-C₄alkoxy or carboxy-C₁-C₄alkoxy, C₁-C₄alkoxycarbonyl-C₁-C₄alkoxy, carbamoyl-C₁-C₄alkoxy, carbamoyl-C₁-C₄alkoxy, N-C₁-C₇alkylcarbamoyl-C₁-C₄alkoxy or N-mono- or N,N-di-C₁-C₄alkylcarbamoyl-C₁-C₄alkoxy,
R₂ is hydrogen, C₁-C₇alkyl, 3- to 8-membered cycloalkyl, C₁-C₄alkoxy-C₁-C₄alkyl, C₁-C₄alkoxy-C₁-C₄alkoxy-C₁-C₄alkyl, 3- to 8-membered cycloalkoxy-C₁-C₄alkyl, hydroxy, C₁-C₇alkanoyloxy-C₂-C₄alkyl, hydroxy-C₂-C₇alkoxy, halo-C₂-C₇(hydroxy)alkoxy, C₁-C₇alkanesulfonyl-C₁-C₄(hydroxy)alkoxy, amino-C₁-C₄alkyl, C₁-C₄alkylamino-C₁-C₄alkyl, N,N-di-C₁-C₄alkylamino-C₁-C₄alkyl, N-C₁-C₄alkanoylamino-C₁-C₄alkyl, C₁-C₇alkoxycarbonylamino-C₂-C₇alkyl, optionally partially hydrogenated pyridyl- or N-oxidopyridyl-C₁-C₄alkyl, amino-C₁-C₄alkoxy, C₁-C₄alkylamino-C₁-C₄alkoxy, N,N-di-C₁-C₄alkylamino-C₁-C₄alkoxy, C₁-C₄alkanoylamino-C₁-C₄-alkoxy, C₁-C₇alkoxycarbonylamino-C₂-C₇alkoxy, C₁-C₇alkanoyl-C₂-C₄alkoxy carrying the alkanoyl group in a position higher than the α-position, C₁-C₇alkoxy, 3- to 8-membered cycloalkoxy, C₂-C₇alkenyloxy, 3- to 8-membered cycloalkoxy-C₁-C₄-alkoxy, C₁-C₄alkoxy-C₂-C₄alkoxy, C₁-C₄alkoxy-C₂-C₄alkenyl, C₂-C₇alkenyloxy-C₁-C₄alkoxy, C₁-C₄alkoxy-C₂-C₄alkenyloxy, C₂-C₇alkenyloxy-C₁-C₄alkyl, C₁-C₄-alkylthio-C₁-C₄alkoxy, C₁-C₇alkanesulfonyl-C₁-C₄alkoxy, C₁-C₄alkylthio-C₁-C₄(hydroxy)alkoxy; phenyl- or naphthyl-C₁-C₄alkoxy each unsubstituted or mono-, di- or tri-substituted by C₁-C₄alkyl, C₁-C₄alkoxy, hydroxy, C₁-C₄alkylamino, di-C₁-C₄-alkylamino, halogen and/or by trifluoromethyl; optionally partially hydrogenated pyridyl- or N-oxidopyridyl-C₁-C₄alkoxy, thiazolyl-C₁-C₄alkoxy, optionally N-oxidised morpholino-C₁-C₄alkoxy, cyano-C₁-C₄alkoxy, carboxy-C₁-C₄alkoxy, C₁-C₄-alkoxycarbonyl-C₁-C₄alkoxy, carbamoyl-C₁-C₄alkoxy, N-C₁-C₇alkylcarbamoyl-C₁-C₄alkoxy or N-mono- or N,N-di-C₁-C₄alkylcarbamoyl-C₁-C₄alkoxy, carboxy-C₁-C₄alkyl, C₁-C₄alkoxycarbonyl-C₁-C₄alkyl, carbamoyl-C₁-C₇alkyl or N-mono- or N,N-di-C₁-C₄alkylcarbamoyl-C₁-C₄alkyl,
R₃ is hydrogen, C₁-C₇alkyl, polyhalo-C₁-C₄alkyl, C₁-C₄alkoxy-C₁-C₄alkyl, 3- to 8-membered cycloalkoxy-C₁-C₄alkyl, hydroxy-C₂-C₇alkyl, C₁-C₄alkylthio-C₁-C₄alkyl, C₁-C₇alkanesulfonyl-C₁-C₄alkyl, thiazolylthio-C₁-C₄alkyl or thiazolinylthio-C₁-C₄alkyl, imidazolylthio-C₁-C₄alkyl, optionally N-oxidised pyridylthio-C₁-C₄alkyl, pyrimidinylthio-C₁-C₄alkyl, optionally partially hydrogenated pyridyl- or N-oxidopyridyl-C₁-C₄alkyl, amino-C₁-C₄alkyl, C₁-C₄alkylamino-C₁-C₄alkyl, N,N-di-C₁-C₄alkylamino-C₁-C₄alkyl, N-C₁-C₄alkanoylamino-C₁-C₄alkyl, C₁-C₄alkanesulfonylamino-C₁-C₄alkyl, trifluoro-C₁-C₇alkanesulfonylamino-C₁-C₄alkyl, pyrrolidino-C₁-C₄alkyl, piperidino-C₁-C₄alkyl, piperazino-C₁-C₄alkyl, N'-C₁-C₄-alkylpiperazino-C₁-C₄alkyl, N'-C₂-C₇alkanoylpiperazino-C₁-C₄alkyl, morpholino-C₁-C₄alkyl, thiomorpholino-C₁-C₄alkyl, S-oxothiomorpholino-C₁-C₄alkyl, S,S-dioxothiomorpholino-C₁-C₄alkyl, cyano-C₁-C₄alkyl, carboxy-C₁-C₄alkyl, C₁-C₄alkoxycarbonyl-C₁-C₄alkyl, carbamoyl-C₁-C₇alkyl, N-mono- or N,N-di-C₁-C₄alkylcarbamoyl-C₁-C₄alkyl, 3- to 8-membered cycloalkyl; phenyl or naphthyl each unsubstituted or mono-, di- or tri-substituted by C₁-C₄alkyl, C₁-C₄alkoxy, hydroxy, C₁-C₄alkylamino, di-C₁-C₄alkylamino, halogen and/or by trifluoromethyl; hydroxy, C₁-C₄alkoxy, 3- to 8-membered cycloalkoxy, C₁-C₄alkoxy-C₂-C₄alkoxy, 3- to 8-membered cycloalkoxy-C₁-C₄alkoxy, hydroxy-C₂-C₇alkoxy; phenyl-C₁-C₄alkoxy or naphthyl-C₁-C₄alkoxy each unsubstituted or mono-, di- or tri-substituted by C₁-C₄alkyl, C₁-C₄alkoxy, hydroxy, C₁-C₄alkylamino, di-C₁-C₄alkylamino, halogen and/or by trifluoromethyl; C₁-C₄alkoxy, polyhalo-C₁-C₄alkoxy, C₁-C₄alkylthio-C₁-C₄alkoxy, C₁-C₇alkanesulfonyl-C₁-C₄alkoxy, optionally partially hydrogenated pyridyl- or N-oxidopyridyl-C₁-C₄alkoxy, thiazolyl-C₁-C₄alkoxy, morpholino-C₁-C₄-alkoxy, thiazolylthio-C₁-C₄alkoxy, thiazolinylthio-C₁-C₄alkoxy, imidazolylthio-C₁-C₄alkoxy, optionally N-oxidised pyridylthio-C₁-C₄alkoxy, pyrimidinylthio-C₁-C₄-alkoxy, amino-C₁-C₄alkoxy, C₁-C₄alkylamino-C₁-C₄alkoxy, N,N-di-C₁-C₄alkylamino-C₁-C₄alkoxy, C₁-C₇alkanoylamino-C₁-C₄alkoxy, C₁-C₇alkanesulfonylamino-C₁-C₄alkoxy, trifluoro-C₁-C₇alkanesulfonyl-C₁-C₄alkoxy, pyrrolidino-C₂-C₄alkoxy, piperidino-C₁-C₄alkoxy, piperazino-C₁-C₄alkyl, N'-C₁-C₄alkylpiperazino-C₁-C₄alkyl, N'-C₂-C₇alkanoylpiperazino-C₁-C₄alkyl, morpholino-C₁-C₄alkyl, thiomorpholino-C₁-C₄alkyl, S-oxothiomorpholino-C₁-C₄alkyl, S,S-dioxothiomorpholino-C₁-C₄alkyl, cyano-C₁-C₄alkoxy, carboxy-C₁-C₄alkoxy, C₁-C₄alkoxycarbonyl-C₁-C₄alkoxy, carbamoyl-C₁-C₇alkoxy or N-mono- or N,N-di-C₁-C₄alkylcarbamoyl-C₁-C₄alkoxy, or, together with R₄, is C₁-C₄alkylenedioxy or a fused-on benzo or cyclohexeno ring,
R₄ together with R₃ is C₁-C₄alkylenedioxy or a fused-on benzo or cyclohexeno ring, or is hydrogen, C₁-C₇alkyl, hydroxy, C₁-C₄alkoxy or 3- to 8-membered cycloalkoxy,
X is methylene or hydroxymethylene,
R₅ is C₁-C₇alkyl or 3- to 8-membered cycloalkyl,
R₇ is C₁-C₇alkyl, C₂-C₇alkenyl, 3- to 8-membered cycloalkyl; or phenyl- or naphthyl-C₁-C₄alkyl each unsubstituted or mono-, di- or tri-substituted by C₁-C₄alkyl, C₁-C₄alkoxy, hydroxy, C₁-C₄alkylamino, di-C₁-C₄alkylamino, halogen and/or by trifluoromethyl,
X₁ is an amino-protecting group,
X₂ is hydrogen or together with X₃ is a bivalent protecting group,
X₃ is hydrogen, a hydroxy-protecting group or together with X₂ is a bivalent protecting group or together with X₄ is a direct bond, and
X₄ is free or reactively etherified or esterified hydroxy or together with X₃ is a direct bond,
or a salt thereof.

13. A compound according to claim 12 of formula IId 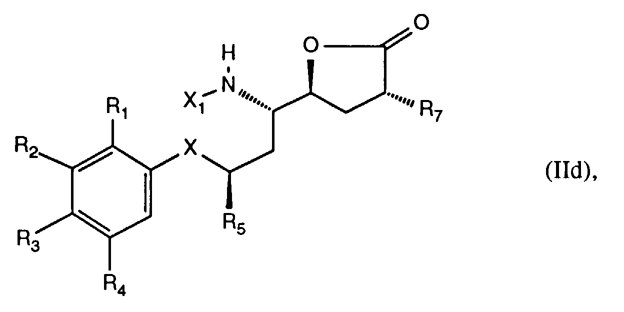 wherein
R₁ and R₄ are hydrogen,
R₂ is C₁-C₄alkoxy-C₁-C₄alkoxy or C₁-C₄alkoxy-C₁-C₄alkyl,
R₃ is C₁-C₄alkyl or C₁-C₄alkoxy,
X is methylene,
R₅ and R ₇ are branched C₁-C₄alkyl, and
X₁ is C₁-C₇alkoxycarbonyl, or α-phenyl-C₁-C₄alkoxycarbonyl unsubstituted or substituted by C₁-C₄alkyl, C₁-C₄alkoxy, nitro and/or by halogen,
or a salt thereof.

14. 3(S)-Isopropyl-5(S)-{1(S)-tert-butoxycarbonylamino-3(S)-isopropyl-4-[4-(3-hydroxypropyloxy)-3-(3-methoxypropyloxy)-phenyl]-butyl}-tetrahydrofuran-2-one;
3(S)-isopropyl-5(S)-{1(S)-benzyloxycarbonylamino-3(S)-isopropyl-4-[4-methoxy-3-(3-methoxypropyloxy)-phenyl]-butyl}-tetrahydrofuran-2-one;
5(S)-tert-butoxycarbonylamino-4(S)-tert-butyldimethylsilyloxy-7(S)-isopropyl-2(R)-methyl-8-[4-methoxy-3-(3-methoxypropyloxy)-phenyl]-octanoic acid;
5(S)-tert-butoxycarbonylamino-4(S)-hydroxy-7(S)-isopropyl-2(R)-methyl-8-[4-methoxy-3-(3-methoxypropyloxy)-phenyl]-octanoic acid or
2-{1(S)-tert-butoxycarbonylamino-3(S)-isopropyl-4-[4-methoxy-3-(3-methoxy-propyloxy)-phenyl]-butyl}-4(R)-methyl-tetrahydrofuran-5-one
according to claim 12, or in each case a salt thereof.

15. A compound of formula XVI 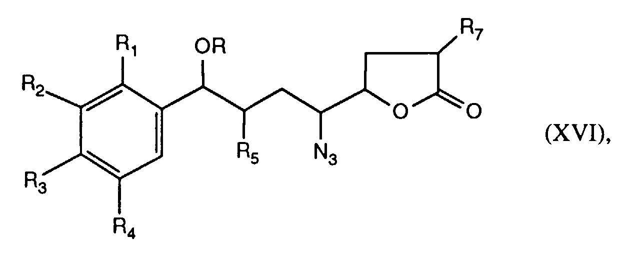 wherein
R₁ is hydrogen, hydroxy, C₁-C₇alkoxy, 3- to 8-membered cycloalkoxy, C₁-C₄alkoxy-C₂-C₄alkoxy or carboxy-C₁-C₄alkoxy, C₁-C₄alkoxycarbonyl-C₁-C₄alkoxy, carbamoyl-C₁-C₄alkoxy, carbamoyl-C₁-C₄alkoxy, N-C₁-C₇alkylcarbamoyl-C₁-C₄alkoxy or N-mono- or N,N-di-C₁-C₄alkylcarbamoyl-C₁-C₄alkoxy,
R₂ is hydrogen, C₁-C₇alkyl, 3- to 8-membered cycloalkyl, C₁-C₄alkoxy-C₁-C₄alkyl, C₁-C₄alkoxy-C₁-C₄alkoxy-C₁-C₄alkyl, 3- to 8-membered cycloalkoxy-C₁-C₄alkyl, hydroxy, C₁-C₇alkanoyloxy-C₂-C₄alkyl, hydroxy-C₂-C₇alkoxy, halo-C₂-C₇(hydroxy)alkoxy, C₁-C₇alkanesulfonyl-C₁-C₄(hydroxy)alkoxy, amino-C₁-C₄alkyl, C₁-C₄alkylamino-C₁-C₄alkyl, N,N-di-C₁-C₄alkylamino-C₁-C₄alkyl, N-C₁-C₄alkanoylamino-C₁-C₄alkyl, C₁-C₇alkoxycarbonylamino-C₂-C₇alkyl, optionally partially hydrogenated pyridyl- or N-oxidopyridyl-C₁-C₄alkyl, amino-C₁-C₄alkoxy, C₁-C₄alkylamino-C₁-C₄alkoxy, N,N-di-C₁-C₄alkylamino-C₁-C₄alkoxy, C₁-C₄alkanoylamino-C₁-C₄alkoxy, C₁-C₇alkoxycarbonylamino-C₂-C₇alkoxy, C₁-C₇alkanoyl-C₂-C₄alkoxy carrying the alkanoyl group in a position higher than the α-position, C₁-C₇alkoxy, 3- to 8-membered cycloalkoxy, C₂-C₇alkenyloxy, 3- to 8-membered cycloalkoxy-C₁-C₄-alkoxy, C₁-C₄alkoxy-C₂-C₄alkoxy, C₁-C₄alkoxy-C₂-C₄alkenyl, C₂-C₇alkenyloxy-C₁-C₄alkoxy, C₁-C₄alkoxy-C₂-C₄alkenyloxy, C₂-C₇alkenyloxy-C₁-C₄alkyl, C₁-C₄-alkylthio-C₁-C₄alkoxy, C₁-C₇alkanesulfonyl-C₁-C₄alkoxy, C₁-C₄alkylthio-C₁-C₄-(hydroxy)alkoxy; phenyl- or naphthyl-C₁-C₄alkoxy each unsubstituted or mono-, di- or tri-substituted by C₁-C₄alkyl, C₁-C₄alkoxy, hydroxy, C₁-C₄alkylamino, di-C₁-C₄-alkylamino, halogen and/or by trifluoromethyl; optionally partially hydrogenated pyridyl- or N-oxidopyridyl-C₁-C₄alkoxy, thiazolyl-C₁-C₄alkoxy, optionally N-oxidised morpholino-C₁-C₄alkoxy, cyano-C₁-C₄alkoxy, carboxy-C₁-C₄alkoxy, C₁-C₄-alkoxycarbonyl-C₁-C₄alkoxy, carbamoyl-C₁-C₄alkoxy, N-C₁-C₇alkylcarbamoyl-C₁-C₄alkoxy or N-mono- or N,N-di-C₁-C₄alkylcarbamoyl-C₁-C₄alkoxy, carboxy-C₁-C₄alkyl, C₁-C₄alkoxycarbonyl-C₁-C₄alkyl, carbamoyl-C₁-C₇alkyl or N-mono- or N,N-di-C₁-C₄alkylcarbamoyl-C₁-C₄alkyl,
R₃ is hydrogen, C₁-C₇alkyl, polyhalo-C₁-C₄alkyl, C₁-C₄alkoxy-C₁-C₄alkyl, 3- to 8-membered cycloalkoxy-C₁-C₄alkyl, hydroxy-C₂-C₇alkyl, C₁-C₄alkylthio-C₁-C₄alkyl, C₁-C₇alkanesulfonyl-C₁-C₄alkyl, thiazolylthio-C₁-C₄alkyl or thiazolinylthio-C₁-C₄alkyl, imidazolylthio-C₁-C₄alkyl, optionally N-oxidised pyridylthio-C₁-C₄alkyl, pyrimidinylthio-C₁-C₄alkyl, optionally partially hydrogenated pyridyl- or N-oxidopyridyl-C₁-C₄alkyl, amino-C₁-C₄alkyl, C₁-C₄alkylamino-C₁-C₄alkyl, N,N-di-C₁-C₄alkylamino-C₁-C₄alkyl, N-C₁-C₄alkanoylamino-C₁-C₄alkyl, C₁-C₄aikanesulfonylamino-C₁-C₄alkyl, trifluoro-C₁-C₇alkanesulfonylamino-C₁-C₄alkyl, pyrrolidino-C₁-C₄alkyl, piperidino-C₁-C₄alkyl, piperazino-C₁-C₄alkyl, N'-C₁-C₄-alkylpiperazino-C₁-C₄alkyl, N'-C₂-C₇alkanoylpiperazino-C₁-C₄alkyl, morpholino-C₁-C₄alkyl, thiomorpholino-C₁-C₄alkyl, S-oxothiomorpholino-C₁-C₄alkyl, S,S-dioxothiomorpholino-C₁-C₄alkyl, cyano-C₁-C₄alkyl, carboxy-C₁-C₄alkyl, C₁-C₄-alkoxycarbonyl-C₁-C₄alkyl, carbamoyl-C₁-C₇alkyl, N-mono- or N,N-di-C₁-C₄alkylcarbamoyl-C₁-C₄alkyl, 3- to 8-membered cycloalkyl; phenyl or naphthyl each unsubstituted or mono-, di- or tri-substituted by C₁-C₄alkyl, C₁-C₄alkoxy, hydroxy, C₁-C₄alkylamino, di-C₁-C₄alkylamino, halogen and/or by trifluoromethyl; hydroxy, C₁-C₄alkoxy, 3- to 8-membered cycloalkoxy, C₁-C₄alkoxy-C₂-C₄alkoxy, 3- to 8-membered cycloalkoxy-C₁-C₄alkoxy, hydroxy-C₂-C₇alkoxy; phenyl-C₁-C₄alkoxy or naphthyl-C₁-C₄alkoxy each unsubstituted or mono-, di- or tri-substituted by C₁-C₄-alkyl, C₁-C₄alkoxy, hydroxy, C₁-C₄alkylamino, di-C₁-C₄alkylamino, halogen and/or by trifluoromethyl; C₁-C₄alkoxy, polyhalo-C₁-C₄alkoxy, C₁-C₄alkylthio-C₁-C₄-alkoxy, C₁-C₇alkanesulfonyl-C₁-C₄alkoxy, optionally partially hydrogenated pyridyl- or N-oxidopyridyl-C₁-C₄alkoxy, thiazolyl-C₁-C₄alkoxy, morpholino-C₁-C₄alkoxy, thiazolylthio-C₁-C₄alkoxy, thiazolinylthio-C₁-C₄alkoxy, imidazolylthio-C₁-C₄alkoxy, optionally N-oxidised pyridylthio-C₁-C₄alkoxy, pyrimidinylthio-C₁-C₄alkoxy, amino-C₁-C₄alkoxy, C₁-C₄alkylamino-C₁-C₄alkoxy, N,N-di-C₁-C₄alkylamino-C₁-C₄alkoxy, C₁-C₇alkanoylamino-C₁-C₄alkoxy, C₁-C₇alkanesulfonylamino-C₁-C₄alkoxy, trifluoro-C₁-C₇alkanesulfonyl-C₁-C₄alkoxy, pyrrolidino-C₂-C₄alkoxy, piperidino-C₁-C₄alkoxy, piperazino-C₁-C₄alkyl, N'-C₁-C₄alkylpiperazino-C₁-C₄alkyl, N'-C₂-C₇-alkanoylpiperazino-C₁-C₄alkyl, morpholino-C₁-C₄alkyl, thiomorpholino-C₁-C₄alkyl, S-oxothiomorpholino-C₁-C₄alkyl, S,S-dioxothiomorpholino-C₁-C₄alkyl, cyano-C₁-C₄alkoxy, carboxy-C₁-C₄alkoxy, C₁-C₄alkoxycarbonyl-C₁-C₄alkoxy, carbamoyl-C₁-C₇alkoxy or N-mono- or N,N-di-C₁-C₄alkylcarbamoyl-C₁-C₄alkoxy, or, together with R₄, is C₁-C₄alkylenedioxy or a fused-on benzo or cyclohexeno ring,
R₄ together with R₃ is C₁-C₄alkylenedioxy or a fused-on benzo or cyclohexeno ring, or is hydrogen, C₁-C₇alkyl, hydroxy, C₁-C₄alkoxy or 3- to 8-membered cycloalkoxy,
R is a hydroxy-protecting group, and
R₇ is C₁-C₇alkyl, C₂-C₇alkenyl, 3- to 8-membered cycloalkyl; or phenyl- or naphthyl-C₁-C₄alkyl each unsubstituted or mono-, di- or tri-substituted by C₁-C₄alkyl, C₁-C₄alkoxy, hydroxy, C₁-C₄alkylamino, di-C₁-C₄alkylamino, halogen and/or by trifluoromethyl,
or a salt thereof.

16. 3(S)-Isopropyl-5(S)-{1(S)-azido-3(S)-isopropyl-4(R,S)-hydroxy-4-[4-methoxy-3-(2-methoxymethoxyethyl)-phenyl]-butyl}-tetrahydrofuran-2-one;
3(S)-isopropyl-5(S)-{1(S)-azido-3(S)-isopropyl-4(R,S)-isobutyryloxy-4-[4-methoxy-3-(2-methoxymethoxyethyl)-phenyl]-butyl}-tetrahydrofuran-2-one;
3(S)-isopropyl-5(S)-{1(S)-azido-3(S)-isopropyl-4(R,S)-hydroxy-4-[4-(3-benzyloxypropyloxy)-3-(3-methoxypropyloxy)-phenyl]-butyl}-tetrahydrofuran-2-one;
3(S)-isopropyl-5(S)-{1(S)-azido-3(S)-isopropyl-4(R,S)-acetoxy-4-[4-(3-benzyloxypropyloxy)-3-(3-methoxypropyloxy)-phenyl]-butyl}-tetrahydrofuran-2-one;
3(S)-isopropyl-5(S)-{4(R,S)-acetoxy-1(S)-azido-3(S)-isopropyl-4(R,S)-[4-tert-butyl-3-(3-methoxypropyloxy)-phenyl]-butyl}-tetrahydrofuran-2-one;
3(S)-isopropyl-5(S)-{4(R,S)-hydroxy-1(S)-azido-3(S)-isopropyl-4(R,S)-[4-tert-butyl-3-(3-methoxypropyloxy)-phenyl]-butyl}-tetrahydrofuran-2-one or
3(S)-isopropyl-5(S)-{1(S)-azido-4(S,R)-hydroxy-3(S)-isopropyl-4-[4-methoxy-3-(3-methoxypropyloxy)-phenyl]-butyl}-tetrahydrofuran-2-one
according to claim 15.

## Revendications

1. Amides d'acide δ-amino-γ-hydroxy-ω-aryl-alcanoïque de formule I 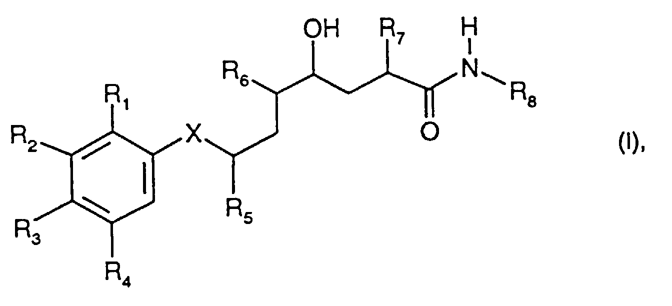 dans laquelle
R₁ représente un hydrogène, un hydroxy, un alcoxy en C₁ à C₇, un cycloalcoxy à 3 à 8 chaînons, un alcoxy en C₁ à C₄-alcoxy en C₂ à C₄ ou un carboxy-alcoxy en C₁ à C₄, un alcoxy en C₁ à C₄-carbonyl-alcoxy en C₁ à C₄, un carbamoyl-alcoxy en C₁ à C₄, un N-alkyle en C₁ à C₇-carbamoyl-alcoxy en C₁ à C₄ ou un N,N-di-alkyle en C₁ à C₄-carbamoyle-alcoxy en C₁ à C₄,
R₂ représente un hydrogène, un alkyle en C₁ à C₇, un cycloalkyle à 3 à 8 chaînons, un alcoxy en C₁ à C₄-alkyle en C₁ à C₄, un alcoxy en C₁ à C₄-alcoxy en C₁ à C₄-alkyle en C₁ à C₄, un cycloalcoxy à 3 à 8 chaînons-alkyle en C₁ à C₄, un hydroxy, un alcanoyloxy en C₁ à C₇-alkyle en C₂ à C₄, un hydroxy-alcoxy en C₂ à C₇, un halogène-(hydroxy)alcoxy en C₂ à C₇, un alcane en C₁ à C₇-sulfonyl-(hydroxy)alcoxy en C₁ à C₄, un amino-alkyle en C₁ à C₄, un alkylamino en C₁ à C₄-alkyle en C₁ à C₄, un N,N-di-alkylamino en C₁ à C₄-alkyle en C₁ à C₄, un N-alcanoylamino en C₁ à C₄-alkyle en C₁ à C₄, un alcoxy en C₁ à C₇-carbonylamino-alkyle en C₂ à C₇, un pyridyl- ou N-oxydopyridyl-alkyle en C₁ à C₄ éventuellement partiellement hydrogéné, un amino-alcoxy en C₁ à C₄, un alkylamino en C₁ à C₄-alcoxy en C₁ à C₄, un N,N-di-alkylamino en C₁ à C₄-alcoxy en C₁ à C₄, un alcanoylamino en C₁ à C₄-alcoxy en C₁ à C₄, un alcoxy en C₁ à C₇-carbonylamino-alcoxy en C₂ à C₇, un alcanoyle en C₁ à C₇-alcoxy en C₂ à C₄ portant le groupe alcanoyle en une position supérieure à la position α, un alcoxy en C₁ à C₇, un cycloalcoxy à 3 à 8 chaînons, un alcényloxy en C₂ à C₇, un cycloalcoxy à 3 à 8 chaînons-alcoxy en C₁ à C₄, un alcoxy en C₁ à C₄-alcoxy en C₂ à C₄, un alcoxy en C₁ à C₄-alcényle en C₂ à C₄, un alcényloxy en C₂ à C₇-alcoxy en C₁ à C₄, un alcoxy en C₁ à C₄-alcényloxy en C₂ à C₄, un alcényloxy en C₂ à C₇-alkyle en C₁ à C₄, un alkylthio en C₁ à C₄-alcoxy en C₁ à C₄, un alcane sulfonyle en C₁ à C₇-alcoxy en C₁ à C₄, un alkylthio en C₁ à C₄-(hydroxy)alcoxy en C₁ à C₄, un phényl- ou naphtyl-alcoxy en C₁ à C₄ non substitué ou mono-, di- ou trisubstitué par un alkyle en C₁ à C₄, un alcoxy en C₁ à C₄, un hydroxy, un alkylamino en C₁ à C₄, un di-alkylamino en C₁ à C₄, un halogène et/ou un trifluorométhyle, un pyridyl- ou N-oxydopyridylalcoxy en C₁ à C₄ éventuellement partiellement hydrogéné, un thiazolylalcoxy en C₁ à C₄, un morpholino-alcoxy en C₁ à C₄ éventuellement N-oxydé, un cyano-alcoxy en C₁ à C₄, un carboxy-alcoxy en C₁ à C₄, un alcoxy en C₁ à C₄-carbonyl-alcoxy en C₁ à C₄, un carbamoyle-alcoxy en C₁ à C₄, un N-alkyle en C₁ à C₇-carbamoyl-alcoxy en C₁ à C₄ ou un N-mono- ou N,N-di-alkyle en C₁ à C₄-carbamoyl-alcoxy en C₁ à C₄, un carboxy-alkyle en C₁ à C₄, un alcoxy en C₁ à C₄-carbonyl-alkyle en C₁ à C₄, un carbamoyl-alkyle en C₁ à C₇ ou un N-mono- ou N,N-di-alkyle en C₁ à C₄-carbamoyl-alkyle en C₁ à C₄,
R₃ représente un hydrogène, un alkyle en C₁ à C₇, un polyhalogène-alkyle en C₁ à C₄, un alcoxy en C₁ à C₄-alkyle en C₁ à C₄, un cycloalcoxy à 3 à 8 chaînons-alkyle en C₁ à C₄, un hydroxy-alkyle en C₂ à C₇, un alkylthio en C₁ à C₄-alkyle en C₁ à C₄, un alcanesulfonyle en C₁ à C₇-alkyle en C₁ à C₄, un thiazolylthio-alkyle en C₁ à C₄, un thiazolinylthio-alkyle en C₁ à C₄, un imidazolylthio-alkyle en C₁ à C₄, un pyridylthio-alkyle en C₁ à C₄ éventuellement N-oxydé, un pyrimidinylthio-alkyle en C₁ à C₄, un pyridyl- ou N-oxydopyridyl-alkyle en C₁ à C₄ éventuellement partiellement hydrogéné, un amino-alkyle en C₁ à C₄, un alkylamino en C₁ à C₄-alkyle en C₁ à C₄, un N,N-di-alkylamino en C₁ à C₄-alkyle en C₁ à C₄, un N-alcanoylamino en C₁ à C₄-alkyle en C₁ à C₄, un alcanesulfonylamino en C₁ à C₄-alkyle en C₁ à C₄, un trifluoro-alcane-sulfonylamino en C₁ à C₇-alkyle en C₁ à C₄, un pyrrolidino-alkyle en C₁ à C₄, un pipéridino-alkyle en C₁ à C₄, un pipérazino-alkyle en C₁ à C₄, un N'-alkyle en C₁ à C₄-pipérazino-alkyle en C₁ à C₄, un N'-alcanoyle en C₂ à C₇-pipérazino-alkyle en C₁ à C₄, un morpholino-alkyle en C₁ à C₄, un thiomorpholino-alkyle en C₁ à C₄, un S-oxothiomorpholino-alkyle en C₁ à C₄, un S,S-dioxo-thiomorpholino-alkyle en C₁ à C₄, un cyano-alkyle en C₁ à C₄, un carboxy-alkyle en C₁ à C₄, un alcoxy en C₁ à C₄-carbonyl-alkyle en C₁ à C₄, un carbamoyl-alkyle en C₁ à C₇, un N-mono- ou N,N-di-alkyle en C₁ à C₄-carbamoyl-alkyle en C₁ à C₄, un cycloalkyle à 3 à 8 chaînons, un phényle ou naphtyle non substitué ou mono-, di- ou trisubstitué par un alkyle en C₁ à C₄, un alcoxy en C₁ à C₄, un hydroxy, un alkylamino en C₁ à C₄, un di-alkylamino en C₁ à C₄, un halogène et/ou un trifluorométhyle, un hydroxy, un alcoxy en C₁ à C₄, un cycloalcoxy à 3 à 8 chaînons, un alcoxy en C₁ à C₄-alcoxy en C₂ à C₄, un cycloalcoxy à 3 à 8 chaînons-alcoxy en C₁ à C₄, un hydroxy-alcoxy en C₂ à C₇, un phényl-alcoxy en C₁ à C₄ ou naphtyl-alcoxy en C₁ à C₄ non substitué ou mono-, di- ou trisubstitué par un alkyle en C₁ à C₄, un alcoxy en C₁ à C₄, un hydroxy, un alkylamino en C₁ à C₄, un di-alkylamino en C₁ à C₄, un halogène et/ou un trifluorométhyle, un polyhalogène-alcoxy en C₁ à C₄, un alkylthio en C₁ à C₄-alcoxy en C₁ à C₄, un alcane sulfonyle en C₁ à C₇-alcoxy en C₁ à C₄, un pyridyl- ou un N-oxydo-pyridyl-alcoxy en C₁ à C₄ éventuellement partiellement hydrogéné, un thiazolyl-alcoxy en C₁ à C₄, un morpholino-alcoxy en C₁ à C₄, un thiazolylthio-alcoxy en C₁ à C₄, un thiazolinylthio-alcoxy en C₁ à C₄, un imidazolylthio-alcoxy en C₁ à C₄, un pyridylthio-alcoxy en C₁ à C₄ éventuellement N-oxydé, un pyrimidinylthio-alcoxy en C₁ à C₄, un amino-alcoxy en C₁ à C₄, un alkylamino en C₁ à C₄-alcoxy en C₁ à C₄, un N,N-dialkylamino en C₁ à C₄-alcoxy en C₁ à C₄, un alcanoylamino en C₁ à C₇-alcoxy en C₁ à C₄, un alcanesulfonylamino en C₁ à C₇-alcoxy en C₁ à C₄, un trifluoro-alcane sulfonyle en C₁ à C₇-alcoxy en C₁ à C₄, un pyrrolidino-alcoxy en C₂ à C₄, un pipéridino-alcoxy en C₁ à C₄, un pipérazino-alkyle en C₁ à C₄, un N'-alkyle en C₁ à C₄-pipérazino-alkyle en C₁ à C₄, un N'-alcanoyle en C₂ à C₇-pipérazino-alkyle en C₁ à C₄, un morpholino-alkyle en C₁ à C₄, un thiomorpholino-alkyle en C₁ à C₄, un S-oxothiomorpholino-alkyle en C₁ à C₄, un S,S-dioxothiomorpholino-alkyle en C₁ à C₄, un cyano-alcoxy en C₁ à C₄, un carboxy-alcoxy en C₁ à C₄, un alcoxy en C₁ à C₄-carbonyl-alcoxy en C₁ à C₄, un carbamoyl-alcoxy en C₁ à C₇ ou un N-mono- ou N,N-di-alkyle en C₁ à C₄-carbamoyl-alcoxy en C₁ à C₄ ou représentent ensemble avec R₄ un alkylène-dioxy en C₁ à C₄ ou un noyau benzo- ou cyclohexéno condensé,
R₄ représente ensemble avec R₃ un alkylène dioxy en C₁ à C₄ ou un noyau benzo- ou cyclohexéno condensé, ou représente un hydrogène, un alkyle en C₁ à C₇, un hydroxy, un alcoxy en C₁ à C₄ ou un cycloalcoxy à 3 à 8 chaînons,
X représente un méthylène ou un hydroxyméthylène,
R₅ représente un alkyle en C₁ à C₇ ou un cycloalkyle à 3 à 8 chaînons,
R₆ représente un amino, un alkylamino en C₁ à C₄, un di-alkylamino en C₁ à C₄ ou un N-alcanoylamino en C₁ à C₇,
R₇ représente un alkyle en C₁ à C₇, un alcényle en C₂ à C₇, un cycloalkyle à 3 à 8 chaînons ou un phényl- ou naphtyl-alkyle en C₁ à C₄ non substitué ou mono-, di- ou trisubstitué par un alkyle en C₁ à C₄, un alcoxy en C₁ à C₄, un hydroxy, un alkylamino en C₁ à C₄, un di-alkylamino en C₁ à C₄ , un halogène et/ou un trifluorométhyle, et
R₈ représente un alkyle en C₁ à C₇, un cycloalkyle, un hydroxy-alkyle en C₂ à C₇, un alcanoyloxy en C₁ à C₇-alkyle en C₂ à C₄, un alcoxy en C₁ à C₄-alkyle en C₁ à C₄, un alcényloxy en C₂ à C₇-alkyle en C₁ à C₄, un aminoalkyle en C₁ à C₄, un N-alkylamino en C₁ à C₄-alkyle en C₁ à C₄, un N,N-dialkylamino en C₁ à C₄-alkyle en C₁ à C₄, un N-alcanoylamino en C₁ à C₄-alkyle en C₁ à C₄, un alcane en C₁ à C₇-sulfonylamino-alkyle en C₁ à C₄, un trifluoro-alcane en C₁ à C₇-sulfonylamino-alkyle en C₁ à C₄, un pyrrolidino-alkyle en C₁ à C₄, un pipéridino-alkyle en C₁ à C₄, un hydroxypipéridino-alkyle en C₁ à C₄, un alcoxy en C₁ à C₄-pipéridino-alkyle en C₁ à C₄, un pipérazino-alkyle en C₁ à C₄, un N'-alkyle en C₁ à C₄-pipérazino-alkyle en C₁ à C₄, un N'-alcanoyle en C₂ à C₇-pipérazino-alkyle en C₁ à C₄, un morpholino-alkyle en C₁ à C₄, un diméthylmorpholino-alkyle en C₁ à C₄, un morpholinocarbonyl-alkyle en C₁ à C₄, un thiomorpholino-alkyle en C₁ à C₄, un S,S-dioxo-thiomorpholino-alkyle en C₁ à C₄, un carboxy-alkyle en C₁ à C₄, un alcoxy en C₁ à C₄-carbonyl-alkyle en C₁ à C₄, un carbamoyl-alkyle en C₁ à C₇, un N-mono- ou N,N-di-alkyle en C₁ à C₄-carbamoyl-alkyle en C₁ à C₄, un dicarboxy-alkyle en C₁ à C₄, un di-alcoxy en C₁ à C₄-carbonyl-alkyle en C₁ à C₄, un dicarbamoyl-alkyle en C₁ à C₄, un di-(N-mono- ou N,N-di-alkyle en C₁ à C₄-carbamoyl-alkyle en C₁ à C₄, un carboxy-(hydroxy)alkyle en C₁ à C₇, un alcoxy en C₁ à C₄-carbonyl-(hydroxy)-alkyle en C₁ à C₇ ou un carbamoyl-(hydroxy)alkyle en C₁ à C₇, un carboxycycloalkyle ayant toujours 5 ou 6 chaînons-alkyle en C₁ à C₄, un alcoxy en C₁ à C₄-carbonyl-cycloalkyl-alkyle en C₁ à C₄, un carbamoyl-cycloalkyl-alkyle en C₁ à C₄ ou un N-mono- ou N,N-di-alkyle en C₁ à C₄-carbamoyle-cycloalkyl-alkyle en C₁ à C₄, un cyano-alkyle en C₁ à C₄, un alcanesulfonyle en C₁ à C₇-alkyle en C₁ à C₄, un thiocarbamoyl-alkyle en C₁ à C₄, un alkyle en C₁ à C₄-thiocarbamoyl-alkyle en C₁ à C₄, un di-alkyle en C₁ à C₄-thiocarbamoyl-alkyle en C₁ à C₄, un sulfamoyl-alkyle en C₁ à C₄, un N-alkyle en C₁ à C₇-sulfamoyl-alkyle en C₁ à C₄, un N,N-di-alkyle en C₁ à C₄-sulfamoyl-alkyle en C₁ à C₄, un radical aza-, diaza-, triaza-, oxadiaza- ou tétraaza-aryle lié par l'intermédiaire d'un atome de carbone, éventuellement partiellement hydrogéné, oxo-substitué et/ou benzo-condensé à 5 chaînons, ou un radical aza- ou diaza-aryle à 6 chaînons ou un radical aza-, diaza-, triaza-, oxadiaza- ou tétra-aza-aryl-alkyle en C₁ à C₄ lié par l'intermédiaire d'un atome de carbone, éventuellement partiellement hydrogéné, oxo-substitué et/ou benzocondensé ou un radical aza- ou diaza-aryl-alkyle en C₁ à C₄,
et leurs sels.

2. Composés selon la revendication 1 de formule I, dans laquelle
R₁ représente un hydrogène,
R₂ représente un alkyle en C₁ à C₇, un alcoxy en C₁ à C₄-alkyle en C₁ à C₄, un alcoxy en C₁ à C₄-alcoxy en C₁ à C₄, un alcoxy en C₁ à C₄-alcoxy en C₁ à C₄-alkyle en C₁ à C₄, un phényl-alcoxy en C₁ à C₄ non substitué ou substitué par un alkyle en C₁ à C₄ , un alcoxy en C₁ à C₄, un hydroxy, un halogène, un nitro et/ou un amino, un pyridyl- ou un N-oxydopyridyl-alcoxy en C₁ à C₄, un alkylthio en C₁ à C₄-alcoxy en C₁ à C₄, un alcane en C₁ à C₇-sulfonyl-alcoxy en C₁ à C₄, un alcanoyle en C₁ à C₇-alcoxy en C₂ à C₄, un cyano-alcoxy en C₁ à C₄, un carboxy-alcoxy en C₁ à C₄, un alcoxy en C₁ à C₄-carbonyl-alcoxy en C₁ à C₄, un carbamoyl-alcoxy en C₁ à C₇ ou un N-mono- ou N,N-di-alkyle en C₁ à C₄-carbamoyl-alcoxy en C₁ à C₄,
R₃ représente un hydrogène, un alkyle en C₁ à C₇, un hydroxy, alcoxy en C₁ à C₄ ou polyhalogène-alcoxy en C₁ à C₄ ou représente ensemble avec R₄ un alkylène-dioxy en C₁ à C₄,
R₄ est un hydrogène ou représente ensemble avec R₃ un alkylène-dioxy en C₁ à C₄,
X représente un méthylène ou un hydroxyméthylène,
R₅ représente un alkyle en C₁ à C₇ ou un cycloalkyle à 3 à 8 chaînons,
R₆ représente un amino, un alkylamino en C₁ à C₄, un di-alkylamino en C₁ à C₄ ou un N-alcanoylamino en C₁ à C₇,
R₇ représente un alkyle en C₁ à C₇, et
R₈ représente un alkyle en C₁ à C₇, un hydroxy-alkyle en C₂ à C₇, un alcanoyloxy en C₁ à C₇-alkyle en C₂ à C₄, un alcoxy en C₁ à C₄-alkyle en C₁ à C₄, un alcényloxy en C₂ à C₇-alkyle en C₁ à C₄, un amino-alkyle en C₁ à C₄, un N-alcanoylamino en C₁ à C₄-alkyle en C₁ à C₄, un N-alkylamino en C₁ à C₄-alkyle en C₁ à C₄, un N,N-di-alkylamino en C₁ à C₄-alkyle en C₁ à C₄, un pipéridino-alkyle en C₁ à C₄, un hydroxypipéridino-alkyle en C₁ à C₄, un alcoxy en C₁ à C₄-pipéridino-alkyle en C₁ à C₄, un morpholino-alkyle en C₁ à C₄, un diméthylmorpholino-alkyle en C₁ à C₄, un thiomorpholino-alkyle en C₁ à C₄, un S,S-dioxothiomorpholino-alkyle en C₁ à C₄, un carboxy-alkyle en C₁ à C₄, un alcoxy en C₁ à C₄-carbonyl-alkyle en C₁ à C₄, un carbamoyl-alkyle en C₁ à C₇, un N-mono- ou N,N-di-alkyle en C₁ à C₄-carbamoyl-alkyle en C₁ à C₄, un carboxy-(hydroxy)alkyle en C₁ à C₇, un alcoxy en C₁ à C₄-carbonyl-(hydroxy)alkyle en C₁ à C₇ ou un carbamoyl-(hydroxy)alkyle en C₁ à C₇, un carboxycycloalkyle à 5 ou 6 chaînons-alkyle en C₁ à C₄, un alcoxy en C₁ à C₄-carbonyl-cycloalkyl-alkyle en C₁ à C₄ à 5 ou 6 chaînons, un carbamoyl-cycloalkyl-alkyle en C₁ à C₄ à 5 ou 6 chaînons, un N-mono- ou N,N-di-alkyle en C₁ à C₄-carbamoyl-cycloalkyl-alkyle en C₁ à C₄ à 5 ou 6 chaînons, un cyano-alkyle en C₁ à C₄, un alcane en C₁ à C₇-sulfonyl-alkyle en C₁ à C₄, un sulfamoyl-alkyle en C₁ à C₄, un imidazolyl-alkyle en C₁ à C₄, un oxopyrrolidinyl-alkyle en C₁ à C₄, un benzimidazolyl-alkyle en C₁ à C₄, un oxadiazolyl-alkyle en C₁ à C₄, un pyridyl-alkyle en C₁ à C₄, un oxopipéridinyl-alkyle en C₁ à C₄ ou un quinolinyl-alkyle en C₁ à C₄, un pipéridin-4-yl-alkyle en C₁ à C₄, ou un 1-alcanoyle en C₁ à C₇-pipéridin-4-yl-alkyle en C₁ à C₄,
et leurs sels.

3. Composés selon la revendication 1 de formule I, dans laquelle
R₁ et R₄ représentent un hydrogène, R₂ représente un alcoxy en C₁ à C₄-alcoxy en C₁ à C₄ ou un alcoxy en C₁ à C₄-alkyle en C₁ à C₄,
R₃ représente un alkyle en C₁ à C₄ ou un alcoxy en C₁ à C₄,
R₆ est un amino,
X est un méthylène,
R₅ et R₇ représentent un alkyle en C₁ à C₄ ramifié et
R₈ représente un carbamoyl-alkyle en C₁ à C₄, N-alkyle en C₁ à C₄-carbamoyl-alkyle en C₁ à C₄, un N,N-di-alkyle en C₁ à C₄-carbamoyl-alkyle en C₁ à C₄, un morpholino-alkyle en C₁ à C₄, un thiomorpholino-alkyle en C₁ à C₄, un 4-(1-alcanoyle en C₁ à C₄-pipéridyl)-alkyle en C₁ à C₄ ou un 2-oxo-pyrrolidinyl-alkyle en C₁ à C₄,
et leurs sels.

4. Composés selon l'une des revendications 1 à 3 de formule I, dans laquelle au moins un atome de carbone asymétrique de la chaîne principale présente la stéréotaxie indiquée dans la formule Ia 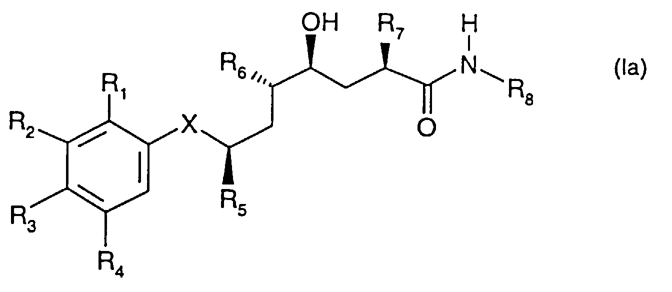 où les variables ont toujours les significations indiquées dans la revendications 1 à 3, et leurs sels, pharmaceutiquement acceptables.

5. Composés selon l'une des revendications 1 à 4, de formules I et Ia, dans lesquelles X représente un méthylène, et leurs sels pharmaceutiquement acceptables.

6. N-(2-carbamoyl-2,2-diméthyl-éthyl)-amide de l'acide 5(S)-amino-4(S)-hydroxy-2(S),7(S)-diisopropyl-8-[4-méthoxy-3-(3-méthoxypropyloxy)-phényl]-octanoïque selon la revendication 1 ou un de ses sels.

7. (N-butyl)-amide de l'acide 5(S)-amino-4(S),8(R,S)-dihydroxy-2(S),7(S)-diisopropyl-8-[4-méthoxy-3-(2-méthoxyméthoxyéthyl)-phényl]-octanoïque;
N-(2-carbamoyl-2,2-diméthyl-éthyl)-amide de l'acide 5(S)-amino-4(S),8(R)-dihydroxy-2(S),7(S)-diisopropyl-8-[4-méthoxy-3-(3-méthoxypropyloxy)-phényl]-octanoïque ;
N-(2-carbamoyl-2,2-diméthyl-éthyl)-amide de l'acide 5(S)-amino-4(S)-hydroxy-2(S),7(S)-diisopropyl-8-[4-méthoxy-3-(3-méthoxypropyloxy)-phényl] octanoïque ou
[N-2-(morpholino-4-yl)-éthyl]-amide de l'acide 5(S)-amino-2(S),7(S)-diisopropyl-4(S)-hydroxy-8-[4-tert.butyl-3-(3-méthoxypropoxy)-phényl]- octanoïque selon la revendication 1 ou chaque fois l'un de leurs sels.

8. (N-butyl)amide de l'acide 2(R,S)-méthyl-4(S)-hydroxy-5(S)-amino-7(S)-isopropyl-8-(p-tert.butyl-phényl)-octanoïque ;
(N-butyl)amide de l'acide 2(R,S)-méthyl-4(S)-hydroxy-5(S)-amino-7(S)-éthyl-8-(p-tert.butyl-phényl)-octanoïque ;
(N-butyl)amide de l'acide 2(R,S)-méthyl-4(S)-hydroxy-5(S)-amino-7(S)-méthyl-8-biphényl-octanoïque;
(N-butyl)amide de l'acide 2(R,S)-méthyl-4(S)-hydroxy-5(S)-amino-7(S)-éthyl-8-(4-propyloxyméthyl-napht-2-yl)-octanoïque;
(N-butyl)amide de l'acide 2(R)-méthyl-4(S)-hydroxy-5(S)-amino-7(S)-isopropyl-8-(3-hydroxy-4-tert.butyl-phényl)-octanoïque ;
(N-butyl)amide de l'acide 2(R,S)-méthyl-4(S)-hydroxy-5(S)-amino-7(S)-isopropyl-8-(2-hydroxy-4-tert.butyl-phényl)-octanoïque;
(N-butyl)amide de l'acide 2(R)-méthyl-4(S)-hydroxy-5(S)-amino-7(S)-isopropyl-8-(3-éthoxycarbonylméthoxy-4-tert.butyl-phényl)-octanoïque;
(N-butyl)amide de l'acide 2(R)-méthyl-4(S)-hydroxy-5(S)-amino-7(S)-isopropyl-8-(3-allyloxy-4-tert.butyl-phényl)-octanoïque ;
(N-butyl)amide de l'acide 2(R,S)-méthyl-4(S)-hydroxy-5(S)-amino-7(S)-isopropyl-8-(3-méthoxycarbonylallyloxy-4-tert.butyl-phényl)-octanoïque;
(N-butyl)amide de l'acide 2(R,S)-méthyl-4(S)-hydroxy-5(S)-amino-7(S)-isopropyl-8-(3-méthoxycarbonylméthoxy-4-tert.butyl-phényl)-octanoïque ;
(N-butyl)amide de l'acide 2(R,S)-méthyl-4(S)-hydroxy-5(S)-amino-7(S)-isopropyl-8-(3-carbamoyl-méthoxy-4-tert-butyl-phényl)-octanoïque ;
(N-butyl)amide de l'acide 2(R,S)-méthyl-4(S)-hydroxy-5(S)-amino-7(S)-isopropyl-8-[3-(pyrid-2-yl-méthoxy)-4-tert.butyl-phényl]-octanoïque ;
(N-butyl)amide de l'acide 2(R,S)-méthyl-4(S)-hydroxy-5(S)-amino-7(S)-isopropyl-8-[3-(pyrid-4-yl-méthoxy)-4-tert.butyl-phényl]-octanoïque ;
(N-butyl)amide de l'acide 2(R,S)-méthyl-4(S)-hydroxy-5(S)-amino-7(S)-isopropyl-8-[3-(N-oxydo-pyrid-2-yl-méthoxy)-4-tert.butyl-phényl]-octanoïque ;
(N-butyl)amide de l'acide 2(R,S)-méthyl-4(S)-hydroxy-5(S)-amino-7(S)-isopropyl-8-[3-(2-éthoxycarbonylallyloxy)-4-tert.butyl-phényl]-octanoïque;
(N-butyl)amide de l'acide 2(R,S)-méthyl-4(S)-hydroxy-5(S)-amino-7(S)-isopropyl-8-[3-(2-éthoxycarbonylpropyloxy)-4-tert.butyl-phényl]- octanoïque ;
(N-butyl)amide de l'acide 2(R,S)-méthyl-4(S)-hydroxy-5(S)-amino-7(S)-isopropyl-8-[3-(méthylthio-méthoxy)-4-tert.butyl-phényl]-octanoïque;
(N-butyl)amide de l'acide 2(R,S)-méthyl-4(S)-hydroxy-5(S)-amino-7(S)-isopropyl-8-[3-(méthylsulfonyl-méthoxy)-4-tert.butyl-phényl]-octanoïque ;
(N-butyl)amide de l'acide 2(R,S)-méthyl-4(S)-hydroxy-5(S)-amino-7(S)-isopropyl-8-[3-(carboxy-méthoxy)-4-tert.butyl-phényl]-octanoïque ;
(N-butyl)amide de l'acide 2(R,S)-méthyl-4(S)-hydroxy-5(S)-amino-7(S)-isopropyl-8-[3-(3,3-diméthyl-2-oxo-butyloxy)-4-tert.butyl-phényl]-octanoïque ;
(N-butyl)amide de l'acide 2(R,S)-méthyl-4(S)-hydroxy-5(S)-amino-7(S)-isopropyl-8-[3-(2-nitrobenzyloxy)-4-tert.butyl-phényl]-octanoïque ;
(N-butyl)amide de l'acide 2(R,S)-méthyl-4(S)-hydroxy-5(S)-amino-7(S)-isopropyl-8-[3-(2-aminobenzyloxy)-4-tert.butyl-phényl]-octanoïque ;
(N-butyl)amide de l'acide 2(R,S)-méthyl-4(S)-hydroxy-5(S)-amino-7(S)-isopropyl-8-[3-(3-chloro-2(R,S)-hydroxypropyloxy)-4-tert.butyl-phényl]-octanoïque ;
(N-butyl)amide de l'acide 2(R,S)-méthyl-4(S)-hydroxy-5(S)-amino-7(S)-isopropyl-8-[3-(3-méthylthio-2(S,R)-hydroxypropyloxy)-4-tert.butyl-phényl]-octanoïque ;
(N-butyl)amide de l'acide 2(R,S)-méthyl-4(S)-hydroxy-5(S)-amino-7(S)-isopropyl-8-[3-(3-méthylsulfonyl-2(S,R)-hydroxypropyloxy)-4-tert.butyl-phényl]-octanoïque ;
(N-3-morpholino-propyl)amide de l'acide 2(R)-méthyl-4(S)-hydroxy-5(S)-amino-7(S)-isopropyl-8-[3-(méthylsulfonyl-méthoxy)-4-tert.butyl-phényl]-octanoïque ;
(N-butyl)amide de l'acide 2(R)-méthyl-4(S)-hydroxy-5(S)-amino-7(S)-isopropyl-8-(3-méthoxycarbonylméthoxy-phényl)-octanoïque ;
(N-butyl)amide de l'acide 2(R)-méthyl-4(S)-hydroxy-5(S)-amino-7(S)-isopropyl-8-[3-(méthoxycarbonylméthoxy)-4-méthoxy-phényl]-octanoïque;
(N-butyl)amide de l'acide 2(R)-méthyl-4(S)-hydroxy-5(S)-amino-7(S)-isopropyl-8-[3-(N-méthyl-carbamoylméthoxy)-4-méthoxy-phényl]-octanoïque;
(N-butyl)amide de l'acide 2(R)-méthyl-4(S)-hydroxy-5(S)-amino-7(S)-isopropyl-8-[3-(3-méthylsulfonyl-propyloxy)-4-méthoxy-phényl]- octanoïque;
(N-butyl)amide de l'acide 2(R)-méthyl-4(S)-hydroxy-5(S)-amino-7(S)-isopropyl-8-[3-(méthylsulfonyl-méthoxy)-4-méthoxy-phényl]-octanoïque;
(N-butyl)amide de l'acide 2(R)-méthyl-4(S)-hydroxy-5(S)-amino-7(S)-isopropyl-8-[3-(3-méthoxy-propyloxy)-4-méthoxy-phényl]-octanoïque;
(N-butyl)amide de l'acide 2(R)-méthyl-4(S)-hydroxy-5(S)-amino-7(S)-isopropyl-8-[3-(2-méthoxy-éthoxy)-4-méthoxy-phényl]-octanoïque;
(N-butyl)amide de l'acide 2(R)-méthyl-4(S)-hydroxy-5(S)-amino-7(S)-isopropyl-8-[3-(3-hydroxy-propyloxy)-4-méthoxy-phényl]-octanoïque;
(N-butyl)amide de l'acide 2(R)-méthyl-4(S)-hydroxy-5(S)-amino-7(S)-isopropyl-8-[3-(carbamoylméthoxy)-4-méthoxy-phényl]-octanoïque;
(N-butyl)amide de l'acide 2(R)-méthyl-4(S)-hydroxy-5(S)-amino-7(S)-isopropyl-8-(3-cyanométhoxy-4-méthoxy-phényl)-octanoïque;
(N-butyl)amide de l'acide 2(R)-méthyl-4(S)-hydroxy-5(S)-amino-7(S)-isopropyl-8-[3-(4-méthoxy-butoxy)-4-méthoxy-phényl]-octanoïque;
(N-butyl)amide de l'acide 2(R)-méthyl-4(S)-hydroxy-5(S)-amino-7(S)-isopropyl-8-[3-(4-éthoxy-éthoxy)-4-méthoxy-phényl]-octanoïque;
(N-butyl)amide de l'acide 2(R)-méthyl-4(S)-hydroxy-5(S)-amino-7(S)-isopropyl-8-{3-[2-(2-méthoxy-éthoxy)éthoxy]-4-méthoxy-phényl}-octanoïque;
(N-butyl)amide de l'acide 2(R)-méthyl-4(S)-hydroxy-5(S)-amino-7(S)-isopropyl-8-(3-pentoxy-4-méthoxy-phényl)-octanoïque;
(N-butyl)amide de l'acide 2(R)-méthyl-4(S)-hydroxy-5(S)-amino-7(S)-isopropyl-8-(3-benzyloxy-4-méthoxy-phényl)-octanoïque;
(N-butyl)amide de l'acide 2(R)-méthyl-4(S)-hydroxy-5(S)-amino-7(S)-isopropyl-8-[3-(3-éthoxy-propyloxy)-4-méthoxy-phényl]-octanoïque;
(N-butyl)amide de l'acide 2(R)-méthyl-4(S)-hydroxy-5(S)-amino-7(S)-isopropyl-8-[3-(pyrid-4-ylméthoxy)-4-méthoxy-phényl]-octanoïque;
(N-butyl)amide de l'acide 2(R,S)-méthyl-4(S)-hydroxy-5(S)-amino-7(S)-isopropyl-8-(2-éthoxycarbonylméthoxy-4-tert.butyl-phényl)-octanoïque;
(N-butyl)amide de l'acide 2(R,S)-méthyl-4(S)-hydroxy-5(S)-amino-7(S)-isopropyl-8-(2-éthoxycarbonyl-4-tert.butyl-phényl)-octanoïque;
(N-butyl)mude de l'acide 2(R)-méthyl-4(S)-hydroxy-5(S)-amino-7(S)-isopropyl-8-[3-(3-méthoxy-propyloxy)-4,5-éthylènedioxy-phényl]-octanoïque;
[N-(2-carbamoyl-2,2-diméthyl-éthyl)]-amide de l'acide 5(S)-amino-4(S)-hydroxy-2(S),7(S)-diisopropyl-8-[4-(3-hydroxypropyloxy)-3-(3-méthoxypropyloxy)-phényl]-octanoïque;
[N-(2-carbamoyl-2,2-diméthyl-éthyl)]-amide de l'acide 5(S)-amino-4(S)-hydroxy-2(S),7(S)-diisopropyl-8-[4-isopropyl-3-(3-méthoxypropyloxy)-phényl]-octanoïque;
[N-(2-carbamoyl-2,2-diméthyl-éthyl)]-amide de l'acide 5(S)-amino-4(S)-hydroxy-2(S),7(S)-diisopropyl-8-[4-tert.butyl-3-(3-méthoxypropyloxy)-phényl]-octanoïque;
(N-2-morpholinoéthyl)amide de l'acide 5(S)-amino-4(S)-hydroxy-2(S),7(S)-diisopropyl-8-[4-(3-méthyl-sulfonyl-propyloxy)-3-(3-méthoxypropyloxy)-phényl]-octanoïque;
[N-(2-carbamoyl-2,2-diméthyl-éthyl)]-amide de l'acide 5(S)-amino-4(S)-hydroxy-2(S),7(S)-diisopropyl-8-[4-(3-méthylsulfonyl-propyloxy)-3-(3-méthoxypropyloxy)-phényl]-octanoïque;
(N-2-morpholinoéthyl)amide de l'acide 5(S)-amino-4(S)-hydroxy-2(S),7(S)-diisopropyl-8-[3,4-di-(3-hydroxypropyloxy)-phényl]-octanoïque;
[N-(2-carbamoyl-2,2-diméthyl-éthyl)]-amide de l'acide 5(S)-amino-4(S)-hydroxy-2(S),7(S)-diisopropyl-8-[3,4-di-(3-hydroxypropyloxy)-phényl]-octanoïque;
(N-2-morpholinoéthyl)amide de l'acide 5(S)-amino-4(S)-hydroxy-2(S),7(S)-diisopropyl-8-[4-(3-N-méthylcarbamoyl-propyl)-3-(3-méthoxy-propyloxy)-phényl]-octanoïque;
[N-(2-carbamoyl-2,2-diméthyl-éthyl)]-amide de l'acide 5(S)-amino-4(S)-hydroxy-2(S),7(S)-diisopropyl-8-[4-(2-morpholinoéthoxy)-3-(3-méthoxypropyloxy)-phényl]-octanoïque;
(N-2-morpholinoéthyl)amide de l'acide 5(S)-amino-4(S)-hydroxy-2(S),7(S)-diisopropyl-8-[3-(3-méthoxypropyloxy)-4,5-éthylène-dioxy-phényl]-octanoïque;
[N-(2-carbamoyl-2,2-diméthyl-éthyl)]-amide de l'acide 5(S)-amino-4(S)-hydroxy-2(S),7(S)-diisopropyl-8-[3-(3-méthoxypropyloxy)-4,5-éthylène-dioxy-phényl]-octanoïque;
(N-2-morpholinoéthyl)amide de l'acide 5(S)-amino-4(S)-hydroxy-2(S),7(S)-diisopropyl-8-[3-(3-méthoxypropyloxy)-4,5-méthylènedioxy-phényl]-octanoïque;
[N-(2-carbamoyl-2,2-diméthyl-éthyl)]-amide de l'acide 5(S)-amino-4(S)-hydroxy-2(S),7(S)-diisopropyl-8-[3-(3-méthoxypropyloxy)-4,5-méthylènedioxy-phényl]-octanoïque;
[N-(2-carbamoyl-2,2-éthylène-éthyl)]-amide de l'acide 5(S)-amino-4(S)-hydroxy-2(S),7(S)-diisopropyl-8-[4-méthoxy-3-(3-méthoxypropyloxy)-phényl]-octanoïque;
[N-(3(S)-2-oxo-pyrrolidin-3-yl-méthyl)]amide de l'acide 5(S)-amino-4(S)-hydroxy-2(S),7(S)-diisopropyl-8-[4-méthoxy-3-(3-méthoxypropyloxy)-phényl]-octanoïque;
(N-butyl)amide de l'acide 5(S)-amino-4(S)-hydroxy-7(S)-isopropyl-2(R)-méthyl-8-[4-méthoxy-3-(4-méthoxy-but-2-énoxy)-phényl]-octanoïque;
(N-butyl)amide de l'acide 5(S)-amino-4(S)-hydroxy-7(S)-isopropyl-2(R)-méthyl-8-[4-hydroxy-3-(3-méthoxypropyloxy)-phényl]-octanoïque;
(N-butyl)amide de l'acide 5(S)-amino-4(S)-hydroxy-7(S)-isopropyl-2(R)-méthyl-8-[4-benzyloxy-3-(3-méthoxypropyloxy)-phényl]-octanoïque;
(N-butyl)amide de l'acide 5(S)-amino-4(S)-hydroxy-7(S)-isopropyl-2(R)-méthyl-8-[3,4-di-(3-méthoxypropyloxy)-phényl]-octanoïque;
(N-butyl)amide de l'acide 5(S)-amino-4(S)-hydroxy-7(S)-isopropyl-2(R)-méthyl-8-[4-(2,2,2-trifluoroéthoxy)-3-(3-méthoxypropyloxy)-phényl]-octanoïque;
(N-butyl)amide de l'acide 5(S)-amino-4(S)-hydroxy-7(S)-isopropyl-2(R)-méthyl-8-[4-(3-hydroxypropyloxy)-3-(3-méthoxypropyloxy)-phényl]-octanoïque;
(N-butyl)amide de l'acide 5(S)-amino-4(S)-hydroxy-7(S)-isopropyl-2(R)-méthyl-8-[4-(2-amino-éthoxy)-3-(3-méthoxypropyloxy)-phényl]-octanoïque;
(N-butyl)amide de l'acide 5(S)-amino-4(S)-hydroxy-7(S)-isopropyl-2(R)-méthyl-8-[4-(5-amino-pentyloxy)-3-(3-méthoxypropyloxy)-phényl]-octanoïque;
(N-butyl)amide de l'acide 5(S)-amino-4(S)-hydroxy-7(S)-isopropyl-2(R)-méthyl-8-[4-(4-amino-butyloxy)-3-(3-méthoxypropyloxy)-phényl]-octanoïque;
(N-butyl)amide de l'acide 5(S)-amino-4(S)-hydroxy-7(S)-isopropyl-2(R)-méthyl-8-[4-(4-N,N-diméthylamino-butyloxy)-3-(3-méthoxypropyloxy)-phényl]-octanoïque;
(N-butyl)amide de l'acide 5(S)-amino-4(S)-hydroxy-7(S)-isopropyl-2(R)-méthyl-8-{4-[4-N-(trifluorométhanesulfonylamino-butyloxy)-3-(3-méthoxypropyloxy)-phényl]}-octanoïque;
(N-butyl)amide de l'acide 5(S)-amino-4(S)-hydroxy-7(S)-isopropyl-2(R)-méthyl-8-[4-carboxyméthoxy-3-(3-méthoxypropyloxy)-phényl]-octanoïque;
(N-butyl)amide de l'acide 5(S)-amino-4(S)-hydroxy-7(S)-isopropyl-2(R)-méthyl-8-[4-(3-éthoxycarbonyl-propyloxy)-3-(3-méthoxypropyloxy)-phényl]-octanoïque;
(N-butyl)amide de l'acide 5(S)-amino-4(S)-hydroxy-7(S)-isopropyl-2(R)-méthyl-8-[4-(3-carboxy-propyloxy)-3-(3-méthoxypropyloxy)-phényl]-octanoïque;
(N-butyl)amide de l'acide 5(S)-amino-4(S)-hydroxy-7(S)-isopropyl-2(R)-méthyl-8-[4-(4-méthoxycarbonylbutyloxy)-3-(3-méthoxypropyloxy)-phényl]-octanoïque;
(N-butyl)amide de l'acide 5(S)-amino-4(S)-hydroxy-7(S)-isopropyl-2(R)-méthyl-8-[4-(4-carboxy-butyloxy)-3-(3-méthoxypropyloxy)-phényl]-octanoïque;
(N-butyl)amide de l'acide 5(S)-amino-4(S)-hydroxy-2(S),7(S)-diisopropyl-8-[4-méthoxy-3-(3-méthoxypropyloxy)-phényl]-octanoïque;
(N-butyl)amide de l'acide 5(S)-amino-4(S)-hydroxy-2(S),7(S)-diisopropyl-8-[4-méthoxy-3-(2-méthoxyméthoxyéthyl)-phényl]-octanoïque;
N-(2-morpholinoéthyl)amide de l'acide 5(S)-amino-4(S)-hydroxy-2(S),7(S)-diisopropyl-8-[4-(3-hydroxypropyloxy)-3-(3-méthoxypropyloxy)-phényl]-octanoïque;
Dichlorhydrate de N-[2-(4-hydroxypipéridin-1-yl)éthyl]amide de l'acide 5(S)-amino-4(S)-hydroxy-7(S)-isopropyl-2(R)-méthyl-8-[4-méthoxy-3-(3-méthoxypropyloxy)-phényl]-octanoïque;
N-[2-(trans-2,6-diméthyl-morpholino)éthyl]amide de l'acide 5(S)-amino-4(S)-hydroxy-7(S)-isopropyl-2(R)-méthyl-8-[4-méthoxy-3-(3-méthoxypropyloxy)-phényl]-octanoïque;
N-[2-(cis-2,6-diméthyl-morpholino)éthyl]amide de l'acide 5(S)-amino-4(S)-hydroxy-7(S)-isopropyl-2(R)-méthyl-8-[4-méthoxy-3-(3-méthoxypropyloxy)-phényl]-octanoïque;
N-(2-pipéridinoéthyl)amide de l'acide 5(S)-amino-4(S)-hydroxy-7(S)-isopropyl-2(R)-méthyl-8-[4-méthoxy-3-(3-méthoxypropyloxy)-phényl]- octanoïque;
N-[2-(4-méthoxypipéridino)-éthyl]amide de l'acide 5(S)-amino-4(S)-hydroxy-7(S)-isopropyl-2(R)-méthyl-8-[4-méthoxy-3-(3-méthoxypropyloxy)-phényl]-octanoïque;
N-(2-thiomorpholinoéthyl)amide de l'acide 5(S)-amino-4(S)-hydroxy-7(S)-isopropyl-2(R)-méthyl-8-[4-méthoxy-3-(3-méthoxypropyloxy)-phényl]-octanoïque;
[N-(3-hydroxypropyl)]amide de l'acide 5(S)-amino-4(S)-hydroxy-7(S)-isopropyl-2(R)-méthyl-8-[4-méthoxy-3-(3-méthoxypropyloxy)-phényl]-octanoïque;
[N-(4-acétoxybutyl)]amide de l'acide 5(S)-amino-4(S)-hydroxy-7(S)-isopropyl-2(R)-méthyl-8-[4-méthoxy-3-(3-méthoxypropyloxy)-phényl]-octanoïque;
[N-(3-cyanopropyl)]amide de l'acide 5(S)-amino-4(S)-hydroxy-7(S)-isopropyl-2(R)-méthyl-8-[4-méthoxy-3-(3-méthoxypropyloxy)-phényl]-octanoïque;
[N-(3-méthoxypropyl)]amide de l'acide 5(S)-amino-4(S)-hydroxy-7(S)-isopropyl-2(R)-méthyl-8-[4-méthoxy-3-(3-méthoxypropyloxy)-phényl]-octanoïque;
[N-(2-acétylamino-éthyl)]amide de l'acide 5(S)-amino-4(S)-hydroxy-7(S)-isopropyl-2(R)-méthyl-8-[4-méthoxy-3-(3-méthoxypropyloxy)-phényl]-octanoïque;
{N-[2-(2-pyridyl)-éthyl]}-amide de l'acide 5(S)-amino-4(S)-hydroxy-7(S)-isopropyl-2(R)-méthyl-8-[4-méthoxy-3-(3-méthoxypropyloxy)-phényl]-octanoïque;
N-[2-(N'-oxomorpholino)-éthyl]-amide de l'acide 5(S)-amino-4(S)-hydroxy-7(S)-isopropyl-2(R)-méthyl-8-[4-méthoxy-3-(3-méthoxypropyloxy)-phényl]-octanoïque;
{N-[3-(tert.butyl-sulfonyl)-propyl]}-amide de l'acide 5(S)-amino-4(S)-hydroxy-7(S)-isopropyl-2(R)-méthyl-8-[4-méthoxy-3-(3-méthoxypropyloxy)-phényl]-octanoïque;
{N-[3-(éthyl-sulfonyl)-propyl]}-amide de l'acide 5(S)-amino-4(S)-hydroxy-7(S)-isopropyl-2(R)-méthyl-8-[4-méthoxy-3-(3-méthoxypropyloxy)-phényl]-octanoïque;
{N-[2-(éthylsulfonyl)-éthyl]}-amide de l'acide 5(S)-amino-4(S)-hydroxy-7(S)-isopropyl-2(R)-méthyl-8-[4-méthoxy-3-(3-méthoxypropyloxy)- phényl]-octanoïque;
{N-[2-(N-butylsulfamoyl)-éthyl]}-amide de l'acide 5(S)-amino-4(S)-hydroxy-7(S)-isopropyl-2(R)-méthyl-8-[4-méthoxy-3-(3-méthoxypropyloxy)-phényl]-octanoïque;
{N-[2-(N,N-diméthylsulfamoyl)-éthyl]}-amide de l'acide 5(S)-amino-4(S)-hydroxy-7(S)-isopropyl-2(R)-méthyl-8-[4-méthoxy-3-(3-méthoxypropyloxy)-phényl]-octanoïque;
{N-[3-(1H-tétrazol-5-yl)-propyl]}-amide de l'acide 5(S)-amino-4(S)-hydroxy-7(S)-isopropyl-2(R)-méthyl-8-[4-méthoxy-3-(3-méthoxypropyloxy)-phényl]-octanoïque;
{N-[3-(1H-imidazol-5-yl)-propyl]}-amide de l'acide 5(S)-amino-4(S)-hydroxy-7(S)-isopropyl-2(R)-méthyl-8-[4-méthoxy-3-(3-méthoxypropyloxy)-phényl]-octanoïque;
{N-[3-(3-méthyl-1,2,4-oxadiazol-5-yl)-propyl]}-amide de l'acide 5(S)-amino-4(S)-hydroxy-7(S)-isopropyl-2(R)-méthyl-8-[4-méthoxy-3-(3-méthoxypropyloxy)-phényl]-octanoïque;
[N-(3-aminopropyl)]-amide de l'acide 5(S)-amino-4(S)-hydroxy-7(S)-isopropyl-2(R)-méthyl-8-[4-méthoxy-3-(3-méthoxypropyloxy)-phényl]-octanoïque;
[N-(2-diméthylamino-éthyl)]-amide de l'acide 5(S)-amino-4(S)-hydroxy-7(S)-isopropyl-2(R)-méthyl-8-[4-méthoxy-3-(3-méthoxypropyloxy)-phényl]-octanoïque;
N-(2-morpholinoéthyl)amide de l'acide 5(S)-amino-4(S)-hydroxy-7(S)-isopropyl-2(R)-méthyl-8-[4-méthoxy-3-(3-méthoxypropyloxy)-phényl]-octanoïque;
N-(3-morpholinopropyl)amide de l'acide 5(S)-amino-4(S)-hydroxy-7(S)-isopropyl-2(R)-méthyl-8-[4-méthoxy-3-(3-méthoxypropyloxy)-phényl]-octanoïque;
N-[2-(1,1-dioxothiomorpholino)éthyl]amide de l'acide 5(S)-amino-4(S)-hydroxy-7(S)-isopropyl-2(R)-méthyl-8-[4-méthoxy-3-(3-méthoxypropyloxy)-phényl]-octanoïque;
N-(2-éthoxycarbonyléthyl)amide de l'acide 5(S)-amino-4(S)-hydroxy-7(S)-isopropyl-2(R)-méthyl-8-[4-méthoxy-3-(3-méthoxypropyloxy)-phényl]-octanoïque;
[N-(2-carboxy-éthyl)]-amide de l'acide 5(S)-amino-4(S)-hydroxy-7(S)-isopropyl-2(R)-méthyl-8-[4-méthoxy-3-(3-méthoxypropyloxy)-phényl]-octanoïque;
[N-(3-méthoxycarbonyl-éthyl)]-amide de l'acide 5(S)-amino-4(S)-hydroxy-7(S)-isopropyl-2(R)-méthyl-8-[4-méthoxy-3-(3-méthoxypropyloxy)-phényl]-octanoïque;
[N-(3-carboxypropyl)]-amide de l'acide 5(S)-amino-4(S)-hydroxy-7(S)-isopropyl-2(R)-méthyl-8-[4-méthoxy-3-(3-méthoxypropyloxy)-phényl]-octanoïque;
[N-(2-carbamoyléthyl)]-amide de l'acide 5(S)-amino-4(S)-hydroxy-7(S)-isopropyl-2(R)-méthyl-8-[4-méthoxy-3-(3-méthoxypropyloxy)-phényl]-octanoïque;
[N-(4-carbamoylbutyl)]-amide de l'acide 5(S)-amino-4(S)-hydroxy-7(S)-isopropyl-2(R)-méthyl-8-[4-méthoxy-3-(3-méthoxypropyloxy)-phényl]-octanoïque;
N-{3-[N-(2-méthoxyéthyl)carbamoyl]propyl}amide de l'acide 5(S)-amino-4(S)-hydroxy-7(S)-isopropyl-2(R)-méthyl-8-[4-méthoxy-3-(3-méthoxypropyloxy)-phényl]-octanoïque;
N-(4-morpholino-4-oxo-butyl)amide de l'acide 5(S)-amino-4(S)-hydroxy-7(S)-isopropyl-2(R)-méthyl-8-[4-méthoxy-3-(3-méthoxypropyloxy)-phényl]-octanoïque;
[N-(2-carbamoyl-2,2-diméthyl-éthyl)]-amide de l'acide 5(S)-amino-4(S)-hydroxy-7(S)-isopropyl-2(R)-méthyl-8-[4-méthoxy-3-(3-méthoxypropyloxy)-phényl]-octanoïque;
N-(1,1-diméthyl-2-morpholino-éthyl)amide de l'acide 5(S)-amino-4(S)-hydroxy-2(S),7(S)-diisopropyl-8-[4-méthoxy-3-(3-méthoxypropyloxy)-phényl]-octanoïque;
N-[1(R,S)-méthyl-2-morpholino-éthyl]amide de l'acide 5(S)-amino-4(S)-hydroxy-2(S),7(S)-diisopropyl-8-[4-méthoxy-3-(3-méthoxypropyloxy)-phényl]-octanoïque;
[N-(1-carbamoyl-1-méthyl-éthyl)]-amide de l'acide 5(S)-amino-4(S)-hydroxy-2(S),7(S)-diisopropyl-8-[4-méthoxy-3-(3-méthoxypropyloxy)-phényl]-octanoïque;
[N-(1-carbamoyl-méthyl)]-amide de l'acide 5(S)-amino-4(S)-hydroxy-2(S),7(S)-diisopropyl-8-[4-méthoxy-3-(3-méthoxypropyloxy)-phényl]- octanoïque;
[N-(2-carbamoyl-éthyl)]-amide de l'acide 5(S)-amino-4(S)-hydroxy-2(S),7(S)-diisopropyl-8-[4-méthoxy-3-(3-méthoxypropyloxy)-phényl]-octanoïque;
N-[2-(N-méthyl-carbamoyl)éthyl]amide de l'acide 5(S)-amino-4(S)-hydroxy-2(S),7(S)-diisopropyl-8-[4-méthoxy-3-(3-méthoxypropyloxy)-phényl]-octanoïque;
N-(3-morpholino-3-oxo-propyl)amide de l'acide 5(S)-amino-4(S)-hydroxy-2(S),7(S)-diisopropyl-8-[4-méthoxy-3-(3-méthoxypropyloxy)-phényl]-octanoïque;
{N-[2-(N,N-diméthyl-carbamoyl)-1(R,S)-méthyl-éthyl]}-amide de l'acide 5(S)-amino-4(S)-hydroxy-2(S),7(S)-diisopropyl-8-[4-méthoxy-3-(3-méthoxypropyloxy)-phényl]-octanoïque;
[N-(2-carbamoyl-1(R)-isopropyl-éthyl)]-amide de l'acide 5(S)-amino-4(S)-hydroxy-2(S),7(S)-diisopropyl-8-[4-méthoxy-3-(3-méthoxypropyloxy)-phényl]-octanoïque;
{N-[2-(N-méthylcarbamoyl-1(R)-isopropyl-éthyl]}-amide de l'acide 5(S)-amino-4(S)-hydroxy-2(S),7(S)-diisopropyl-8-[4-méthoxy-3-(3-méthoxypropyloxy)-phényl]-octanoïque;
{N-[2-(N,N-diméthylcarbamoyl-1(R)-isopropyl-éthyl]}-amide de l'acide 5(S)-amino-4(S)-hydroxy-2(S),7(S)-diisopropyl-8-[4-méthoxy-3-(3-méthoxypropyloxy)-phényl]-octanoïque;
[N-(1(S)-carbamoyl-2-hydroxy-éthyl)]-amide de l'acide 5(S)-amino-4(S)-hydroxy-2(S),7(S)-diisopropyl-8-[4-méthoxy-3-(3-méthoxypropyloxy)-phényl]-octanoïque;
[N-(1(S),2-dicarbamoyl-éthyl)]-amide de l'acide 5(S)-amino-4(S)-hydroxy-2(S),7(S)-diisopropyl-8-[4-méthoxy-3-(3-méthoxypropyloxy)-phényl]-octanoïque;
[N-(1(S),3-dicarbamoyl-propyl)]-amide de l'acide 5(S)-amino-4(S)-hydroxy-2(S),7(S)-diisopropyl-8-[4-méthoxy-3-(3-méthoxypropyloxy)-phényl]-octanoïque;
[N-(1(S)-carbamoyl-propyl)]-amide de l'acide 5(S)-amino-4(S)-hydroxy-2(S),7(S)-diisopropyl-8-[4-méthoxy-3-(3-méthoxypropyloxy)-phényl]-octanoïque;
{N-(1(S)-carbamoyl-2(S)-méthyl-butyl)]-amide de l'acide 5(S)-amino-4(S)-hydroxy-2(S),7(S)-diisopropyl-8-[4-méthoxy-3-(3-méthoxypropyloxy)- phényl]-octanoïque;
N-[2(R,S)-carbamoyl-2(R,S)-méthyl-éthyl]-amide de l'acide 5(S)-amino-4(S)-hydroxy-2(S),7(S)-diisopropyl-8-[4-méthoxy-3-(3-méthoxypropyloxy)-phényl]-octanoïque;
[N-(2-carbamoyl-1(S)-méthyl-éthyl)]-amide de l'acide 5(S)-amino-4(S)-hydroxy-2(S),7(S)-diisopropyl-8-[4-méthoxy-3-(3-méthoxypropyloxy)-phényl]-octanoïque;
[N-(2-carbamoyl-1(R)-méthyl-éthyl)]-amide de l'acide 5(S)-amino-4(S)-hydroxy-2(S),7(S)-diisopropyl-8-[4-méthoxy-3-(3-méthoxypropyloxy)-phényl]-octanoïque;
[N-(2(S)-carbamoyl-2(S)-méthyl-éthyl)]-amide de l'acide 5(S)-amino-4(S)-hydroxy-2(S),7(S)-diisopropyl-8-[4-méthoxy-3-(3-méthoxypropyloxy)-phényl]-octanoïque;
{N-[2(S)-(N-méthyl-carbamoyl)-2(S)-méthyl-éthyl]}-amide de l'acide 5(S)-amino-4(S)-hydroxy-2(S),7(S)-diisopropyl-8-[4-méthoxy-3-(3-méthoxypropyloxy)-phényl]-octanoïque;
[N-(2-carboxy-2,2-diméthyl-éthyl)]-amide de l'acide 5(S)-amino-4(S)-hydroxy-2(S),7(S)-diisopropyl-8-[4-méthoxy-3-(3-méthoxypropyloxy)-phényl]-octanoïque;
[N-(2-carboxy-2,2-diéthyl-éthyl)]-amide de l'acide 5(S)-amino-4(S)-hydroxy-2(S),7(S)-diisopropyl-8-[4-méthoxy-3-(3-méthoxypropyloxy)-phényl]-octanoïque; N-[(1-carboxy-cyclopentyl)]-méthylamide de l'acide 5(S)-amino-4(S)-hydroxy-2(S),7(S)-diisopropyl-8-[4-méthoxy-3-(3-méthoxypropyloxy)-phényl]-octanoïque;
{N-[2-(1H-tétrazol-5-yl)-éthyl]}-amide de l'acide 5(S)-amino-4(S)-hydroxy-2(S),7(S)-diisopropyl-8-[4-méthoxy-3-(3-méthoxypropyloxy)-phényl]-octanoïque;
N-[1(S)-(5-oxopyrrolidin-2-yl)méthyl]-amide de l'acide 5(S)-amino-4(S)-hydroxy-2(S),7(S)-diisopropyl-8-[4-méthoxy-3-(3-méthoxypropyloxy)-phényl]-octanoïque;
N-[1(R)-(5-oxopyrrolidin-2-yl)méthyl]-amide de l'acide 5(S)-amino-4(S)-hydroxy-2(S),7(S)-diisopropyl-8-[4-méthoxy-3-(3-méthoxypropyloxy)-phényl]-octanoïque;
N-[(morpholin-4-yl)carbonyl-méthyl]amide de l'acide 5(S)-amino-4(S)-hydroxy-2(S),7(S)-diisopropyl-8-[4-méthoxy-3-(3-méthoxypropyloxy)-phényl]-octanoïque;
[N-(1(S)-carbamoyl-éthyl)]-amide de l'acide 5(S)-amino-4(S)-hydroxy-2(S),7(S)-diisopropyl-8-[4-méthoxy-3-(3-méthoxypropyloxy)-phényl]-octanoïque;
N-{1(S)-[(N-méthyl)-carbamoyl]-éthyl}-amide de l'acide 5(S)-amino-4(S)-hydroxy-2(S),7(S)-diisopropyl-8-[4-méthoxy-3-(3-méthoxypropyloxy)-phényl]-octanoïque;
N-{1(S)-[(N,N-diméthyl)-carbamoyl]-éthyl}-amide de l'acide 5(S)-amino-4(S)-hydroxy-2(S),7(S)-diisopropyl-8-[4-méthoxy-3-(3-méthoxypropyloxy)-phényl]-octanoïque;
N-{1(S)-N-[(morpholin-4-yl)-carbonyl]-éthyl}-amide de l'acide 5(S)-amino-4(S)-hydroxy-2(S),7(S)-diisopropyl-8-[4-méthoxy-3-(3-méthoxypropyloxy)-phényl]-octanoïque;
N-[1(S)-carbamoyl-butyl]amide de l'acide 5(S)-amino-4(S)-hydroxy-2(S),7(S)-diisopropyl-8-[4-méthoxy-3-(3-méthoxypropyloxy)-phényl]-octanoïque;
N-[1(S)-carbamoyl-2-méthyl-propyl]-amide de l'acide 5(S)-amino-4(S)-hydroxy-2(S),7(S)-diisopropyl-8-[4-méthoxy-3-(3-méthoxypropyloxy)-phényl]-octanoïque;
N-[1(S)-(N-méthylcarbamoyl)-2-méthyl-propyl]-amide de l'acide 5(S)-amino-4(S)-hydroxy-2(S),7(S)-diisopropyl-8-[4-méthoxy-3-(3-méthoxypropyloxy)-phényl]-octanoïque;
N-[1(S)-(N,N-diméthylcarbamoyl)-2-méthyl-propyl]-amide de l'acide 5(S)-amino-4(S)-hydroxy-2(S),7(S)-diisopropyl-8-[4-méthoxy-3-(3-méthoxypropyloxy)-phényl]-octanoïque;
N-{1(S)-[(morpholino-4-yl)carbonyl]-2-méthyl-propyl}amide de l'acide 5(S)-amino-4(S)-hydroxy-2(S),7(S)-diisopropyl-8-[4-méthoxy-3-(3-méthoxypropyloxy)-phényl]-octanoïque;
N-[2-(N-méthylsulfamoyl)éthyl]amide de l'acide 5(S)-amino-4(S)-hydroxy-2(S),7(S)-diisopropyl-8-[4-méthoxy-3-(3-méthoxypropyloxy)-phényl]-octanoïque;
N-{2-[N-(morpholin-4-yl)-sulfonyl]éthyl}amide de l'acide 5(S)-amino-4(S)-hydroxy-2(S),7(S)-diisopropyl-8-[4-méthoxy-3-(3-méthoxypropyloxy)- phényl]-octanoïque;
N-[(N-acétyl-pipéridin-4-yl)méthyl]amide de l'acide 5(S)-amino-4(S)-hydroxy-2(S),7(S)-diisopropyl-8-[4-méthoxy-3-(3-méthoxypropyloxy)-phényl]-octanoïque;
N-(2-carbamoyl-2,2-diméthyl-éthyl)amide de l'acide 5(S)-amino-4(S)-hydroxy-2(S),7(S)-diisopropyl-8-[4-méthoxy-3-(4-méthoxy-butyl)-phényl]-octanoïque;
N-[2-(N,N-diméthylcarbamoyl)éthyl]amide de l'acide 5(S)-amino-4(S)-hydroxy-2(S),7(S)-diisopropyl-8-[4-méthoxy-3-(3-méthoxypropyloxy)-phényl]-octanoïque;
N-(2-morpholinoéthyl)amide de l'acide 5(S)-amino-4(S)-hydroxy-2(S),7(S)-diisopropyl-8-[4-méthory-3-(4-méthoxybutyl)-phényl]-octanoïque;
[N-(3(R)-2-oxo-pyrrolidin-3-yl-méthyl)]-amide de l'acide 5(S)-amino-4(S)-hydroxy-2(S),7(S)-diisopropyl-8-[4-méthoxy-3-(3-méthoxypropyloxy)-phényl]-octanoïque;
[N-(3(S)-2-oxo-pipéridin-3-yl-méthyl)]-amide de l'acide 5(S)-amino-4(S)-hydroxy-2(S),7(S)-diisopropyl-8-[4-méthoxy-3-(3-méthoxypropyloxy)-phényl]-octanoïque;
[N-(3(R)-2-oxo-pipéridin-3-yl-méthyl)]-amide de l'acide 5(S)-amino-4(S)-hydroxy-2(S),7(S)-diisopropyl-8-[4-méthoxy-3-(3-méthoxypropyloxy)-phényl]-octanoïque;
[N-(3-carbamoyl-3,3-diméthyl-propyl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(5(S)-2-pyrrolidinon-5-yl-méthyl)]-amide de l'acide 5(S)-amino-4(S)-hydroxy-2(S),7(5)-diisopropyl-8-[4-méthoxy-3-(4-méthoxy-butyl)-phényl]-octanoïque;
[N-(5(R)-2-pyrrolidinon-5-yl-méthyl)]-amide de l'acide 5(S)-amino-4(S)-hydroxy-2(S),7(S)-diisopropyl-8-[4-méthoxy-3-(4-méthoxy-butyl)-phényl]-octanoïque;
[N-(6(S)-2-oxo-pipéridin-6-yl-méthyl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(6(R)-2-oxo-pipéridin-6-yl-méthyl)]-amide de l'acide 5(S)-amino-4(S)-hydroxy-2(S),7(S)-diisopropyl-8-[4-méthoxy-3-(3-méthoxy-propyloxy)- phényl]-octanoïque;
[N-(2-thiazol-2-yl-éthyl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(4(S)-2-oxazolidinon-4-yl-méthyl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(4(R)-2-oxazolidinon-4-yl-méthyl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(3(S)-2,5-dioxo-pyrrolidin-3-yl-méthyl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(3(R)-2,5-dioxo-pyrrolidin-3-yl-méthyl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(2,6-dioxo-pipéridin-4-yl-méthyl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(4(S)-2-oxothiazolin-4-yl-méthyl)]-amide de l'acide 5(S)-amino-4(S)-hydroxy-2(S),7(S)-diisopropyl-8-[4-méthory-3-(3-méthoxy-propyloxy)-phényl]-octanoïque;
N-(2-carbamoyl-2,2-diméthyl-éthyl)-amide de l'acide 5(S)-amino-4(S)-hydroxy-2(S),7(S)-diisopropyl-8-[3-(3-méthoxy propoxy)-4,5-éthylène-dioxy-phényl]-octanoïque;
[N-(4(R)-2-oxothiazolin-4-yl-méthyl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(tétrahydro-2-pyrimidon-5-yl-méthyl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(N-acétyl-2-amino-2-méthyl-propyl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(N-formyl-2-amino-2-méthyl-propyl)]-amide de l'acide 5(S)-amino-4(S)-hydroxy-2(S),7(S)-diisopropyl-8-[4-méthoxy-3-(3-méthoxy-propyloxy)- phényl]-octanoïque;
[N-(4-acétyl-pipérazinyl-éthyl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(2,4-imidazolinedion-5-yl-méthyl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(2-hydroxy-pyridin-6-yl-méthyl)]-amide de l'acide 5(S)-amino-4(S)-hydroxy-2(S),7(S)-diisopropyl-8-[4-méthoxy-3-(4-méthoxy-butyl)-phényl]-octanoïque;
[N-(2,2-diméthyl-2-sulfamoyl-éthyl)]-amide de l'acide 5(S)-amino-4(S)-hydroxy-2(S),7(S)-diisopropyl-8-[4-méthoxy-3-(3-méthoxy-propyloxy)-phényl]-octanoïque;
Chlorhydrate de [N-(2,2-diméthyl-2-(N,N-diméthyl)-sulfamoyl-éthyl)]-amide de l'acide 5(S)-amino-4(S)-hydroxy-2(S),7(S)-diisopropyl-8-[4-méthoxy-3-(3-méthoxy-propyloxy)-phényl]-octanoïque; [N-(2-oxo-pipéridin-3(R)-yl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(2-oxo-pipéridin-3(S)-yl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(2-oxo-pipéridin-4-yl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(N-acétyl-pipéridin-4-yl)]-amide de l'acide 5(S)-amino-4(S)-hydroxy-2(S),7(S)-diisopropyl-8-[4-méthoxy-3-(3-méthoxy-propyloxy)-phényl]-octanoïque;
[N-(2-carbamoyl-2,2-diméthyl-éthyl)]-amide de l'acide 5(S)-amino-4(S)-hydroxy-2(S),7(S)-diisopropyl-8-[4-méthoxy-3-(4-méthoxy-but-1-én-yl)-phényl]-octanoïque;
N-(3-morpholinopropyl)amide de l'acide 5(S)-amino-4(S)-hydroxy-2(S),7(S)-diisopropyl-8-[4-méthoxy-3-(3-méthoxy-propyloxy)-phényl]-octanoïque;
N-(2-morpholinoéthyl)amide de l'acide 5(S)-amino-4(S)-hydroxy-2(S),7(S)-diisopropyl-8-[4-méthoxy-3-(3-méthoxy-propyloxy)-phényl]-octanoïque; {N-[2-(N-méthyl-carbamoyl)-1-(R,S)-méthyl-éthyl]}-amide de l'acide 5(S)-amino-4(S)-hydroxy-2(S),7(S)-diisopropyl-8-[4-méthoxy-3-(3-méthoxypropyloxy)-phényl]-octanoïque;
N-(3-carbamoylpropyl)amide de l'acide 5(S)-amino-4(S)-hydroxy-2(S),7(S)-diisopropyl-8-[4-méthoxy-3-(3-méthoxy-propyloxy)-phényl]-octanoïque; {N-[2-(R)-(N-méthyl-carbamoyl)-2-(R)-méthyl-éthyl]}-amide de l'acide 5(S)-amino-4(S)-hydroxy-2(S),7(S)-diisopropyl-8-[4-méthoxy-3-(3-méthoxypropyloxy)-phényl]-octanoïque;
N-(2-thiomorpholinoéthyl)amide de l'acide 5(S)-amino-4(S)-hydroxy-2(S),7(S)-diisopropyl-8-[4-méthoxy-3-(3-méthoxypropyloxy)-phényl]-octanoïque;
N-[2-(N,N-diméthyl-carbamoyl)éthyl]amide de l'acide 5(S)-amino-4(S)-hydroxy-2(S),7(S)-diisopropyl-8-[4-méthoxy-3-(3-méthoxypropyloxy)-phényl]-octanoïque;
N-(2-carbamoyl-1(R,S)-méthyl-éthyl)amide de l'acide 5(S)-amino-4(S)-hydroxy-2(S),7(S)-diisopropyl-8-[4-méthoxy-3-(3-méthoxypropyloxy)-phényl]-octanoïque;
N-[2(R)-carbamoyl-2(R)-méthyl-éthyl]-amide de l'acide 5(S)-amino-4(S)-hydroxy-2(S),7(S)-diisopropyl-8-[4-méthoxy-3-(3-méthoxypropyloxy)-phényl]-octanoïque;
N-(2-carbamoyl-2,2-diméthyl-éthyl)amide de l'acide 5(S)-amino-4(S)-hydroxy-2(S),7(S)-diisopropyl-8-[4-méthoxy-3-(3-méthoxypropyloxy)-phényl]-octanoïque;
N-[2-(N-acétyl)-pipéridin-4-yl)éthyl]amide de l'acide 5(S)-amino-4(S)-hydroxy-2(S),7(S)-diisopropyl-8-[4-méthoxy-3-(3-méthoxypropyloxy)-phényl]-octanoïque;
{N-[(N,N-diméthyl)-carbamoyl-méthyl]}-amide de l'acide 5(S)-amino-4(S)-hydroxy-2(S),7(S)-diisopropyl-8-[4-méthoxy-3-(3-méthoxypropyloxy)-phényl]-octanoïque ou
N-[2(R,S)-(N-méthylcarbamoyl)-2(R,S)-méthyl-éthyl]-amide de l'acide 5(S)-amino-4(S)-hydroxy-2(S),7(S)-diisopropyl-8-[4-méthoxy-3-(3-méthoxypropyloxy)-phényl]-octanoïque, selon la revendication 1, ou à chaque fois un de leurs sels.

9. Composés selon l'une quelconque des revendications 1 à 8 aux fins d'utilisation dans un procédé de traitement thérapeutique du corps humain ou animal.

10. Préparations pharmaceutiques contenant, outre des additifs pharmaceutiques habituels, comme substance active pharmaceutique un composé selon l'une des revendications 1 à 8 sous forme libre ou sous forme de sel pharmaceutiquement acceptable.

11. Procédé de préparation d'amides d'acide δ-amino-γ-hydroxy-ω-aryl-alcanoïde de formule (I) selon la revendication 1, et de leurs sels, caractérisé en ce que a) on fait réagir un composé de formule II 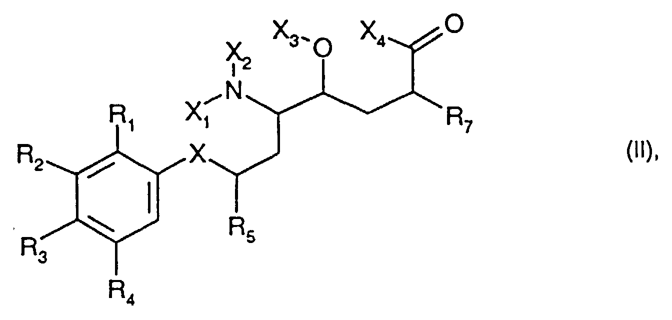 dans laquelle
X₁ représente un alkyle en C₁ à C₄, un alcanoyle en C₁ à C₄ ou un groupe amino protecteur,
X₂ représente un hydrogène ou avec X₃ un groupe protecteur bivalent,
X₃ représente un hydrogène, un groupe hydroxy protecteur ou représente avec X₂ un groupe protecteur bivalent, ou selon les cas représente avec X₄ une liaison directe et
X₄ représente un hydroxy éthérifié ou estérifé éventuellement réactif, ou représente avec X₃ une liaison directe, et R₁, R₂, R₃, R₄, X, R₅, R₆ et R₇ ont les significations indiquées à la formule I, avec une amine de formule III
H₂N-R₈ (III),
dans laquelle R₆ possède l'une des significations données à la formule I, avec formation d'une liaison amide, et en ce qu'on sépare les groupes protecteurs présents, ou en ce que
b) dans un amide d'acide carboxylique de formule IV 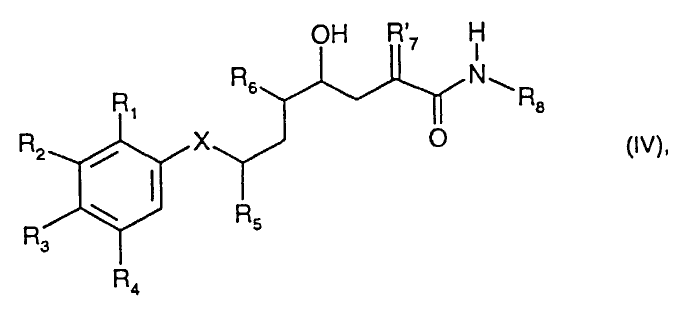 dans laquelle R₁, R₂, R₃, R₄, X, R₅, R₆, R₇, R₈ ont les significations indiquées à la formule I et R'₇ représente l'un des groupes alkylidène en C₁ à C₄ ou aryl-alkylidène en C₁ à C₄ correspondant au groupe alkyle en C₁ à C₄ ou selon les cas au groupe aryl-alkyle en C₁ à C₄ R₇, les groupes fonctionnels libres se présentant le cas échéant sous forme protégée, ou un de leurs sels, le groupe R'₇ étant réduit par traitement avec un agent d'hydrogénation pour donner R₇, ou en ce que c) pour préparer des composés de formule I, dans laquelle R₆ représente un amino, on réduit en amino le groupe azido dans un dérivé d'acide 5-azido carboxylique de formule V 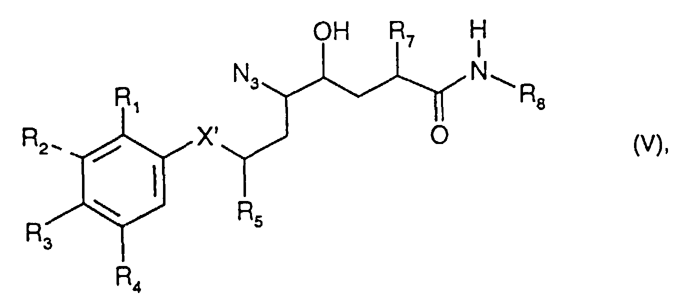 dans laquelle R₁, R₂, R₃, R₄, R₅, R₇, et R₈ ont les significations indiquées à la formule I, X' représente un méthylène ou un hydroxyméthyle éventuellement estérifié ou éthérifié et les groupes fonctionnels libres se présentent éventuellement sous forme protégée, ou dans un de ses sels, et ce si on le désire avec libération d'hydroxyméthyle X ou réduction de X' en méthylène X, et on sépare les groupes protecteurs présents, et si on le désire on transforme un composé de formule I que l'on peut obtenir selon un des procédés a) à c) mentionnés ci-dessus avec au moins un groupe salificateur, en un sel, ou un sel que l'on peut obtenir en un composé libre ou en un autre sel, et/ou si on le désire on sépare les mélanges d'isomère que l'on peut obtenir et/ou on transforme un composé de formule I selon l'invention en un autre composé de formule I selon l'invention.

12. Composés de formule II 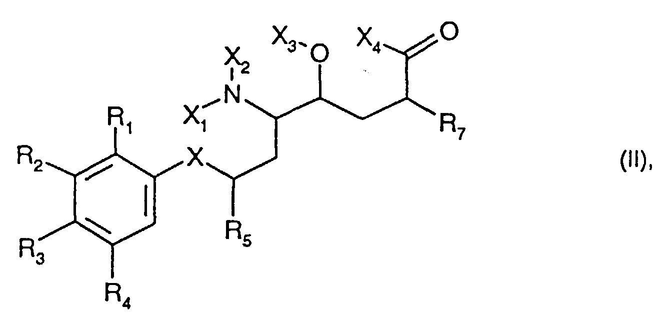 dans laquelle
R₁ représente un hydrogène, un hydroxy, un alcoxy en C₁ à C₇, un cycloalcoxy à 3 à 8 chaînons, un alcoxy en C₁ à C₄-alcoxy en C₂ à C₄ ou un carboxy-alcoxy en C₁ à C₄, un alcoxy en C₁ à C₄-carbonyl-alcoxy en C₁ à C₄, un carbamoyl-alcoxy en C₁ à C₄, un carbamoyl-alcoxy en C₁ à C₄, un N-alkyle en C₁ à C₇-carbamoyl-alcoxy en C₁ à C₄ ou un N-mono- ou N,N-di-alkyle en C₁ à C₄-carbamoyle-alcoxy en C₁ à C₄,
R₂ représente un hydrogène, un alkyle en C₁ à C₇, un cycloalkyle à 3 à 8 chaînons, un alcoxy en C₁ à C₄-alkyle en C₁ à C₄, un alcoxy en C₁ à C₄-alcoxy en C₁ à C₄-alkyle en C₁ à C₄, un cycloalcoxy à 3 à 8 chaînons-alkyle en C₁ à C₄, un hydroxy, un alcanoyloxy en C₁ à C₇-alkyle en C₂ à C₄, un hydroxy-alcoxy en C₂ à C₇, un halogène-(hydroxy)alcoxy en C₂ à C₇, un alcane en C₁ à C₇-sulfonyl-(hydroxy)alcoxy en C₁ à C₄, un amino-alkyle en C₁ à C₄, un alkylamino en C₁ à C₄-alkyle en C₁ à C₄, un N,N-di-alkylamino en C₁ à C₄-alkyle en C₁ à C₄, un N-alcanoylamino en C₁ à C₄-alkyle en C₁ à C₄, un alcoxy en C₁ à C₇-carbonylamino-alkyle en C₂ à C₇, un pyridyl- ou N-oxydopyridyl-alkyle en C₁ à C₄ éventuellement partiellement hydrogéné, un amino-alcoxy en C₁ à C₄, un alkylamino en C₁ à C₄-alcoxy en C₁ à C₄, un N,N-di-alkylamino en C₁ à C₄-alcoxy en C₁ à C₄, un alcanoylamino en C₁ à C₄-alcoxy en C₁ à C₄, un alcoxy en C₁ à C₇-carbonylamino-alcoxy en C₂ à C₇, un alcanoyle en C₁ à C₇-alcoxy en C₂ à C₄ portant le groupe alcanoyle en une position supérieure à la position α, un alcoxy en C₁ à C₇, un cycloalcoxy à 3 à 8 chaînons, un alcényloxy en C₂ à C₇, un cycloalcoxy à 3 à 8 chaînons-alcoxy en C₁ à C₄, un alcoxy en C₁ à C₄-alcoxy en C₂ à C₄, un alcoxy en C₁ à C₄-alcényle en C₂ à C₄, un alcényloxy en C₂ à C₇-alcoxy en C₁ à C₄, un alcoxy en C₁ à C₄-alcényloxy en C₂ à C₄, un alcényloxy en C₂ à C₇-alkyle en C₁ à C₄, un alkylthio en C₁ à C₄-alcoxy en C₁ à C₄, un alcane sulfonyle en C₁ à C₇-alcoxy en C₁ à C₄, un alkylthio en C₁ à C₄-(hydroxy)alcoxy en C₁ à C₄, un phényl- ou naphtyl-alcoxy en C₁ à C₄ non substitué ou mono-, di- ou trisubstitué par un alkyle en C₁ à C₄, un alcoxy en C₁ à C₄, un hydroxy, un alkylamino en C₁ à C₄, un di-alkylamino en C₁ à C₄, un halogène et/ou un trifluorométhyle, un pyridyl- ou N-oxydopyridyl-alcoxy en C₁ à C₄ éventuellement partiellement hydrogéné, un thiazolyl-alcoxy en C₁ à C₄, un morpholino-alcoxy en C₁ à C₄ éventuellement N-oxydé, un cyano-alcoxy en C₁ à C₄, un carboxy-alcoxy en C₁ à C₄, un alcoxy en C₁ à C₄-carbonyl-alcoxy en C₁ à C₄, un carbamoyle-alcoxy en C₁ à C₄, un N-alkyle en C₁ à C₇-carbamoyl-alcoxy en C₁ à C₄ ou un N-mono- ou N,N-di-alkyle en C₁ à C₄-carbamoyl-alcoxy en C₁ à C₄, un carboxy-alkyle en C₁ à C₄, un alcoxy en C₁ à C₄-carbonyl-alkyle en C₁ à C₄, un carbamoyl-alkyle en C₁ à C₇ ou un N-mono- ou N,N-di-alkyle en C₁ à C₄-carbamoyl-alkyle en C₁ à C₄,
R₃ représente un hydrogène, un alkyle en C₁ à C₇, un polyhalogène-alkyle en C₁ à C₄, un alcoxy en C₁ à C₄-alkyle en C₁ à C₄, un cycloalcoxy à 3 à 8 chaînons-alkyle en C₁ à C₄, un hydroxy-alkyle en C₂ à C₇, un alkylthio en C₁ à C₄-alkyle en C₁ à C₄, un alcanesulfonyle en C₁ à C₇-alkyle en C₁ à C₄, un thiazolylthio-alkyle en C₁ à C₄ ou selon les cas un thiazolinylthio-alkyle en C₁ à C₄, un imidazolylthio-alkyle en C₁ à C₄, un piridylthio-alkyle en C₁ à C₄ éventuellement N-oxydé, un pyrimidinylthio-alkyle en C₁ à C₄, un pyridyl- ou N-oxydopyridyl-alkyle en C₁ à C₄ éventuellement partiellement hydrogéné, un amino-alkyle en C₁ à C₄, un alkylamino en C₁ à C₄-alkyle en C₁ à C₄, un N,N-di-alkylamino en C₁ à C₄-alkyle en C₁ à C₄, un N-alcanoylamino en C₁ à C₄-alkyle en C₁ à C₄, un alcane en C₁ à C₄-sulfonylamino-alkyle en C₁ à C₄, un trifluoro-alcane en C₁ à C₇-sulfonylamino-alkyle en C₁ à C₄, un pyrrolidino-alkyle en C₁ à C₄, un pipéridino-alkyle en C₁ à C₄, un pipérazino-alkyle en C₁ à C₄, un N'-alkyle en C₁ à C₄-pipérazino-alkyle en C₁ à C₄, un N'-alcanoyle en C₂ à C₇-pipérazino-alkyle en C₁ à C₄, un morpholino-alkyle en C₁ à C₄, un thiomorpholino-alkyle en C₁ à C₄, un S-oxothiomorpholino-alkyle en C₁ à C₄, un S,S-dioxo-thiomorpholino-alkyle en C₁ à C₄, un cyano-alkyle en C₁ à C₄, un carboxy-alkyle en C₁ à C₄, un alcoxy en C₁ à C₄-carbonyl-alkyle en C₁ à C₄, un carbamoyl-alkyle en C₁ à C₇, un N-mono- ou N,N-di-alkyle en C₁ à C₄-carbamoyl-alkyle en C₁ à C₄, un cycloalkyle à 3 à 8 chaînons, un phényle ou naphtyle non substitué ou mono-, di- ou trisubstitué par un alkyle en C₁ à C₄, un alcoxy en C₁ à C₄, un hydroxy, un alkylamino en C₁ à C₄, un di-alkylamino en C₁ à C₄, un halogène et/ou un trifluorométhyle, un hydroxy, un alcoxy en C₁ à C₄, un cycloalcoxy à 3 à 8 chaînons, un alcoxy en C₁ à C₄-alcoxy en C₂ à C₄, un cycloalcoxy à 3 à 8 chaînons-alcoxy en C₁ à C₄, un hydroxy-alcoxy en C₂ à C₇, un phényl-alcoxy en C₁ à C₄ ou un naphtyl-alcoxy en C₁ à C₄ non substitué ou mono-, di- ou trisubstitué par un alkyle en C₁ à C₄, un alcoxy en C₁ à C₄, un hydroxy, un alkylamino en C₁ à C₄, un di-alkylamino en C₁ à C₄, un halogène et/ou un trifluorométhyle, un alcoxy en C₁ à C₄, un polyhalogène-alcoxy en C₁ à C₄, un alkylthio en C₁ à C₄-alcoxy en C₁ à C₄, un alcane en C₁ à C₇-sulfonyl-alcoxy en C₁ à C₄, un pyridyl- ou un N-oxydo-pyridyl-alcoxy en C₁ à C₄ éventuellement partiellement hydrogéné, un thiazolyl-alcoxy en C₁ à C₄, un morpholino-alcoxy en C₁ à C₄, un thiazolylthio-alcoxy en C₁ à C₄, un thiazolinylthio-alcoxy en C₁ à C₄, un imidazolylthio-alcoxy en C₁ à C₄, un pyridylthio-alcoxy en C₁ à C₄ éventuellement N-oxydé, un pyrimidinylthio-alcoxy en C₁ à C₄, un amino-alcoxy en C₁ à C₄, un alkylamino en C₁ à C₄-alcoxy en C₁ à C₄, un N,N-dialkylamino en C₁ à C₄-alcoxy en C₁ à C₄, un alcanoylamino en C₁ à C₇-alcoxy en C₁ à C₄, un alcane en C₁ à C₇-sulfonylamino-alcoxy en C₁ à C₄, un trifluoro-alcane en C₁ à C₇-sulfonyl-alcoxy en C₁ à C₄, un pyrrolidino-alcoxy en C₂ à C₄, un pypéridino-alcoxy en C₁ à C₄, un pipérazino-alkyle en C₁ à C₄, un N'-alkyle en C₁ à C₄-pipérazino-alkyle en C₁ à C₄, un N'-alcanoyle en C₂ à C₇-pipérazino-alkyle en C₁ à C₄, un morpholino-alkyle en C₁ à C₄, un thiomorpholino-alkyle en C₁ à C₄, un S-oxothiomorpholino-alkyle en C₁ à C₄, un S,S-dioxothiomorpholino-alkyle en C₁ à C₄, un cyano-alcoxy en C₁ à C₄, un carboxy-alcoxy en C₁ à C₄, un alcoxy en C₁ à C₄-carbonyl-alcoxy en C₁ à C₄, un carbamoyl-alcoxy en C₁ à C₇, un N-mono- ou N,N-di-alkyle en C₁ à C₄-carbamoyl-alcoxy en C₁ à C₄ ou représente ensemble avec R₄ un alkylène-dioxy en C₁ à C₄ ou un noyau benzo- ou cyclohexéno condensé,
R₄ représente ensemble avec R₃ un alkylène dioxy en C₁ à C₄ ou un noyau benzo- ou cyclohexéno condensé, ou représente un hydrogène, un alkyle en C₁ à C₇, un hydroxy, un alcoxy en C₁ à C₄ ou un cycloalcoxy à 3 à 8 chaînons,
X représente un méthylène ou un hydroxyméthylène,
R₅ représente un alkyle en C₁ à C₇ ou un cycloalkyle à 3 à 8 chaînons,
R₇ représente un alkyle en C₁ à C₇, un alcényle en C₂ à C₇, un cycloalkyle à 3 à 8 chaînons ou un phényl- ou naphtyl-alkyle en C₁ à C₄ non substitué ou mono-, di- ou tri-substitué par un alkyle en C₁ à C₄, un alcoxy en C₁ à C₄, un hydroxy, un alkylamino en C₁ à C₄, un di-alkylamino en C₁ à C₄, un halogène et/ou un trifluorométhyle,
X₁ représente un groupe amino protecteur, X₂ représente un hydrogène ou ensemble avec X₃ un groupe protecteur bivalent,
X₃ représente un hydrogène, un groupe hydroxy protecteur ou avec X₂ un groupe protecteur bivalent ou selon les cas avec X₄ une liaison directe et
X₄ représente un hydroxy éthérifié ou estérifié éventuellement réactif, ou représente ensemble avec X₃ une liaison directe,
et leurs sels.

13. Composés selon la revendication 12 de formule IId 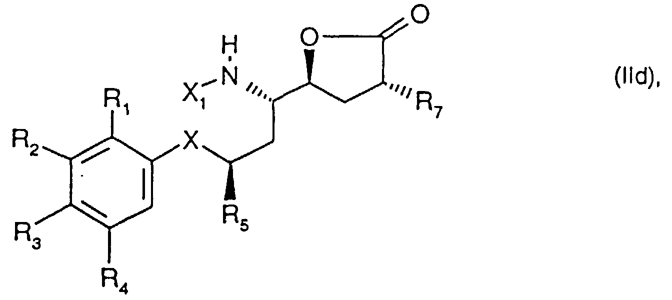 dans laquelle R₁ et R₄ représentent un hydrogène,
R₂ représente un alcoxy en C₁ à C₄-alcoxy en C₁ à C₄ ou un alcoxy en C₁ à C₄-alkyle en C₁ à C₄,
R₃ représente un alkyle en C₁ à C₄ ou un alcoxy en C₁ à C₄, X est un méthylène,
R₅ et R₇ représentent un alkyle en C₁ à C₄ ramifié et
X₁ représente un "alcoxy en C₁ à C₇-carbonyle ou α-phényl-alcoxy en C₁ à C₄-carbonyle éventuellement substitué par un alkyle en C₁ à C₄, alcoxy en C₁ à C₄, nitro et/ou halogène" et leur sels.

14. 3(S)-isopropyl-5(S)-{1(S)-tert.butoxycarbonylamino-3(S)-isopropyl-4-[4-(3-hydroxy-propyloxy)-3-(3-méthoxypropyloxy)-phényl]-butyl}-tétrahydrofuran-2-one ;
3(S)-isopropyl-5(S)-{1(S)-benzyloxycarbonylamino-3(5)-isopropyl-4-[4-méthoxy-3-(3-méthoxypropyloxy)-phényl]-butyl}-tétrahydrofuran-2-one; Acide 5(S)-tert.butoxycarbonylamino-4(S)-tert.butyldiméthylsilyloxy-7(S)-isopropyl-2(R)-méthyl-8-[4-méthoxy-3-(3-méthoxypropyloxy)-phényl]-octanoïque ;
Acide 5(S)-tert.butoxycarbonylamino-4(S)-hydroxy-7(5)-isopropyl-2(R)-méthyl-8-[4-méthoxy-3-(3-méthoxypropyloxy)-phényl]-octanoïque ou 2-{1(S)-tert.butoxycarbonylamino-3(S)-isopropyl-4-[4-méthoxy-3-(3-méthoxy-propyloxy)-phényl)-butyl}-4(R)-méthyl-tétrahydrofuran-5-one selon la revendication 12 ou à chaque fois un de leurs sels.

15. Composés de formule XVI 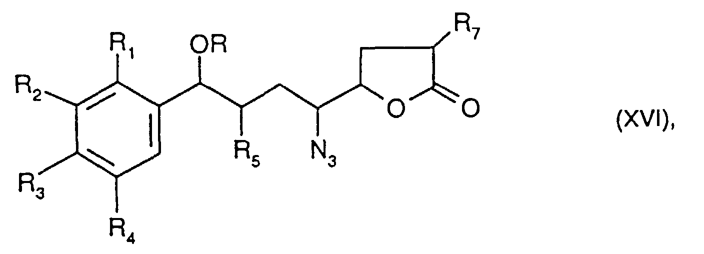 dans laquelle
R₁ représente un hydrogène, un hydroxy, un alcoxy en C₁ à C₇, un cycloalcoxy à 3 à 8 chaînons, un alcoxy en C₁ à C₄-alcoxy en C₂ à C₄ ou un carboxy-alcoxy en C₁ à C₄, un alcoxy en C₁ à C₄-carbonyl-alcoxy en C₁ à C₄, un carbamoyl-alcoxy en C₁ à C₄, un carbamoyl-alcoxy en C₁ à C₄, un N-alkyle en C₁ à C₇-carbamoyl-alcoxy en C₁ à C₄ ou un N-mono- ou N,N-di-alkyle en C₁ à C₄-carbamoyle-alcoxy en C₁ à C₄,
R₂ représente un hydrogène, un alkyle en C₁ à C₇, un cycloalkyle à 3 à 8 chaînons, un alcoxy en C₁ à C₄-alkyle en C₁ à C₄, un alcoxy en C₁ à C₄-alcoxy en C₁ à C₄-alkyle en C₁ à C₄, un cycloalcoxy à 3 à 8 chaînons-alkyle en C₁ à C₄, un hydroxy, un alcanoyloxy en C₁ à C₇-alkyle en C₂ à C₄, un hydroxy-alcoxy en C₂ à C₇, un halogène-(hydroxy)alcoxy en C₂ à C₇, un alcane en C₁ à C₇-sulfonyl-(hydroxy)alcoxy en C₁ à C₄, un amino-alkyle en C₁ à C₄, un alkylamino en C₁ à C₄-alkyle en C₁ à C₄, un N,N-di-alkylamino en C₁ à C₄-alkyle en C₁ à C₄, un N-alcanoylamino en C₁ à C₄-alkyle en C₁ à C₄, un alcoxy en C₁ à C₇-carbonylamino-alkyle en C₂ à C₇, un pyridyl- ou N-oxydopyridyl-alkyle en C₁ à C₄ éventuellement partiellement hydrogéné, un amino-alcoxy en C₁ à C₄, un alkylamino en C₁ à C₄-alcoxy en C₁ à C₄, un N,N-di-alkylamino en C₁ à C₄-alcoxy en C₁ à C₄, un alcanoylamino en C₁ à C₄-alcoxy en C₁ à C₄, un alcoxy en C₁ à C₇-carbonylamino-alcoxy en C₂ à C₇, un alcanoyle en C₁ à C₇-alcoxy en C₂ à C₄ portant le groupe alcanoyle en une position supérieure à la position α, un alcoxy en C₁ à C₇, un cycloalcoxy à 3 à 8 chaînons, un alcényloxy en C₂ à C₇, un cycloalcoxy à 3 à 8 chaînons-alcoxy en C₁ à C₄, un alcoxy en C₁ à C₄-alcoxy en C₂ à C₄, un alcoxy en C₁ à C₄-alcényle en C₂ à C₄, un alcényloxy en C₂ à C₇-alcoxy en C₁ à C₄, un alcoxy en C₁ à C₄-alcényloxy en C₂ à C₄, un alcényloxy en C₂ à C₇-alkyle en C₁ à C₄, un alkylthio en C₁ à C₄-alcoxy en C₁ à C₄, un alcane sulfonyle en C₁ à C₇-alcoxy en C₁ à C₄, un alkylthio en C₁ à C₄-(hydroxy)alcoxy en C₁ à C₄, un phényl- ou naphtyl-alcoxy en C₁ à C₄ non substitué ou mono-, di- ou trisubstitué par un alkyle en C₁ à C₄, un alcoxy en C₁ à C₄, un hydroxy, un alkylamino en C₁ à C₄, un di-alkylamino en C₁ à C₄, un halogène et/ou un trifluorométhyle, un pyridyl- ou N-oxydopyridyl-alcoxy en C₁ à C₄ éventuellement partiellement hydrogéné, un thiazolyl-alcoxy en C₁ à C₄, un morpholino-alcoxy en C₁ à C₄ éventuellement N-oxydé, un cyano-alcoxy en C₁ à C₄, un carboxy-alcoxy en C₁ à C₄, un alcoxy en C₁ à C₄-carbonyl-alcoxy en C₁ à C₄, un carbamoyle-alcoxy en C₁ à C₄, un N-alkyle en C₁ à C₇-carbamoyl-alcoxy en C₁ à C₄ ou un N-mono- ou N,N-di-alkyle en C₁ à C₄-carbamoyl-alcoxy en C₁ à C₄, un carboxy-alkyle en C₁ à C₄, un alcoxy en C₁ à C₄-carbonyl-alkyle en C₁ à C₄, un carbamoyl-alkyle en C₁ à C₇ ou un N-mono- ou N,N-di-alkyle en C₁ à C₄-carbamoyl-alkyle en C₁ à C₄,
R₃ représente un hydrogène, un alkyle en C₁ à C₇, un polyhalogène-alkyle en C₁ à C₄, un alcoxy en C₁ à C₄-alkyle en C₁ à C₄, un cycloalcoxy à 3 à 8 chaînons-alkyle en C₁ à C₄, un hydroxy-alkyle en C₂ à C₇, un alkylthio en C₁ à C₄-alkyle en C₁ à C₄, un alcanesulfonyle en C₁ à C₇-alkyle en C₁ à C₄, un thiazolylthio-alkyle en C₁ à C₄ ou selon les cas un thiazolinylthio-alkyle en C₁ à C₄, un imidazolylthio-alkyle en C₁ à C₄, un piridylthio-alkyle en C₁ à C₄ éventuellement N-oxydé, un pyrimidinylthio-alkyle en C₁ à C₄, un pyridyl- ou N-oxydopyridyl-alkyle en C₁ à C₄ éventuellement partiellement hydrogéné, un amino-alkyle en C₁ à C₄, un alkylamino en C₁ à C₄-alkyle en C₁ à C₄, un N,N-di-alkylamino en C₁ à C₄-alkyle en C₁ à C₄, un N-alcanoylamino en C₁ à C₄-alkyle en C₁ à C₄, un alcane en C₁ à C₄-sulfonylamino-alkyle en C₁ à C₄, un trifluoro-alcane en C₁ à C₇-sulfonylamino-alkyle en C₁ à C₄, un pyrolidino-alkyle en C₁ à C₄, un pipéridino-alkyle en C₁ à C₄, un pipérazino-alkyle en C₁ à C₄, un N'-alkyle en C₁ à C₄-pipérazino-alkyle en C₁ à C₄, un N'-alcanoyle en C₂ à C₇-pipérazino-alkyle en C₁ à C₄, un morpholino-alkyle en C₁ à C₄, un thiomorpholino-alkyle en C₁ à C₄, un S-oxothiomorpholino-alkyle en C₁ à C₄, un S,S-dioxo-thiomorpholino-alkyle en C₁ à C₄, un cyano-alkyle en C₁ à C₄, un carboxy-alkyle en C₁ à C₄, un alcoxy en C₁ à C₄-carbonyl-alkyle en C₁ à C₄, un carbamoyl-alkyle en C₁ à C₇, un N-mono- ou N,N-di-alkyle en C₁ à C₄-carbamoyl-alkyle en C₁ à C₄, un cycloalkyle à 3 à 8 chaînons, un phényle ou naphtyle non substitué ou mono-, di- ou trisubstitué par un alkyle en C₁ à C₄, un alcoxy en C₁ à C₄, un hydroxy, un alkylamino en C₁ à C₄, un di-alkylamino en C₁ à C₄, un halogène et/ou un trifluorométhyle, un hydroxy, un alcoxy en C₁ à C₄, un cycloalcoxy à 3 à 8 chaînons, un alcoxy en C₁ à C₄-alcoxy en C₂ à C₄, un cycloalcoxy à 3 à 8 chaînons-alcoxy en C₁ à C₄, un hydroxy-alcoxy en C₂ à C₇, un phényl-alcoxy en C₁ à C₄ ou un naphtyl-alcoxy en C₁ à C₄ non substitué ou mono-, di- ou trisubstitué par un alkyle en C₁ à C₄, un alcoxy en C₁ à C₄, un hydroxy, un alkylamino en C₁ à C₄, un di-alkylamino en C₁ à C₄, un halogène et/ou un trifluorométhyle, un alcoxy en C₁ à C₄, un polyhalogène-alcoxy en C₁ à C₄, un alkylthio en C₁ à C₄-alcoxy en C₁ à C₄, un alcane en C₁ à C₇-sulfonyl-alcoxy en C₁ à C₄, un pyridyl- ou un N-oxydo-pyridyl-alcoxy en C₁ à C₄ éventuellement partiellement hydrogéné, un thiazolyl-alcoxy en C₁ à C₄, un morpholino-alcoxy en C₁ à C₄, un thiazolylthio-alcoxy en C₁ à C₄, un thiazolinylthio-alcoxy en C₁ à C₄, un imidazolylthio-alcoxy en C₁ à C₄, un pyridylthio-alcoxy en C₁ à C₄ éventuellement N-oxydé, un pyrimidinylthio-alcoxy en C₁ à C₄, un amino-alcoxy en C₁ à C₄, un alkylamino en C₁ à C₄-alcoxy en C₁ à C₄, un N,N-dialkylamino en C₁ à C₄-alcoxy en C₁ à C₄, un alcanoylamino en C₁ à C₇-alcoxy en C₁ à C₄, un alcane en C₁ à C₇-sulfonylamino-alcoxy en C₁ à C₄, un trifluoro-alcane en C₁ à C₇-sulfonyl-alcoxy en C₁ à C₄, un pyrrolidino-alcoxy en C₂ à C₄, un pipéridino-alcoxy en C₁ à C₄, un pipérazino-alkyle en C₁ à C₄, un N'-alkyle en C₁ à C₄-pipérazino-alkyle en C₁ à C₄, un N'-alcanoyle en C₂ à C₇-pipérazino-alkyle en C₁ à C₄, un morpholino-alkyle en C₁ à C₄, un thiomorpholino-alkyle en C₁ à C₄, un S-oxothiomorpholino-alkyle en C₁ à C₄, un S,S-dioxothiomorpholino-alkyle en C₁ à C₄, un cyano-alcoxy en C₁ à C₄, un carboxy-alcoxy en C₁ à C₄, un alcoxy en C₁ à C₄-carbonyl-alcoxy en C₁ à C₄, un carbamoyl-alcoxy en C₁ à C₇, un N-mono- ou N,N-di-alkyle en C₁ à C₄-carbamoyl-alcoxy en C₁ à C₄, ou représente ensemble avec R₄ un alkylène-dioxy en C₁ à C₄ ou un noyau benzo- ou cyclohexène condensé,
R₄ représente ensemble avec R₃ un alkylène dioxy en C₁ à C₄ ou un noyau benzo- ou cyclohexéno condensé, ou représente un hydrogène, un alkyle en C₁ à C₇, un hydroxy, un alcoxy en C₁ à C₄ ou un cycloalcoxy à 3 à 8 chaînons,
R représente un groupe hydroxy protecteur,
R₇ représente un alkyle en C₁ à C₇, un alcényle en C₂ à C₇, un cycloalkyle à 3 à 8 chaînons ou un phényl- ou naphtyl-alkyle en C₁ à C₄ non substitué ou mono-, di- ou trisubstitué par un alkyle en C₁ à C₄, un alcoxy en C₁ à C₄, un hydroxy, un alkylamino en C₁ à C₄, un di-alkylamino en C₁ à C₄, un halogène et/ou un triflurométhyle,
et leurs sels.

16. 3(S)-isopropyl-5(5)-{1(5)-azido-3(S)-isopropyl-4(R,S)-hydroxy-4-[4-méthoxy-3-(2-méthoxyméthoxyéthyl)-phényl]-butyl}-tétrahydrofuran-2-one ;
3(S)-isopropyl-5(5)-{1(S)-azido-3(S)-isopropyl-4(R,S)-isobutyryloxy-4-[4-méthoxy-3-(2-méthoxyméthoxyéthyl)-phényl]-butyl}-tétrahydrofuran-2-one;
3(S)-isopropyl-5(S)-{1(S)-azido-3(S)-isopropyl-4(R,S)-hydroxy-4-[4-(3-benzyloxypropyloxy)-3-(3-méthoxypropyloxy)-phényl]-butyl}-tétrahydrofuran-2-one ;
3(S)-isopropyl-5(S)-{1(S)-azido-3(S)-isopropyl-4(R,S)-acétoxy-4-[4-(3-benzyloxypropyloxy)-3-(3-méthoxypropyloxy)-phényl]-butyl}-tétrahydrofuran-2-one ;
3(S)-isopropyl-5(S)-{4(R,S)-acétoxy-1(S)-azido-3(S)-isopropyl-4(R,S)-[4-tert.butyl-3-(3-méthoxypropyloxy)-phényl]-butyl}-tétrahydrofuran-2-one ;
3(S)-isopropyl-5(S)-{4(R,S)-hydroxy-1(S)-azido-3(S)-isopropyl-4(R,S)-[4-tert.butyl-3-(3-méthoxypropyloxy)-phényl]-butyl}-tétrahydrofuran-2-one ou
3(S)-isopropyl-5(S)-{1(S)-azido-4(S,R)-hydroxy-3(S)-isopropyl-4-[4-méthoxy-3-(3-méthoxypropyloxy)-phényl]-butyl}-tétrahydrofuran-2-one selon la revendication 15.
